# EUROPÄISCHE PATENTSCHRIFT

(11) **EP 2 681 191 B1**
(45) Veröffentlichungstag und Bekanntmachung des Hinweises auf die Patenterteilung: **02.09.2015**
(21) Anmeldenummer: 12705881.6
(22) Anmeldetag: 27.02.2012
(51) Int. Cl.: C07D 207/38, C07D 209/54, C07D 471/10, C07D 495/10, C07D 491/113, C07D 491/107, A01N 43/36, A01N 43/38, A01N 43/90

(54) **2-ACYLOXY-PYRROLIN-4-ONE**
2-ACYLOXY-PYRROLIN-4-ONES
2-ACYLOXY-PYRROLIN-4-ONES

(30) Priorität: 01.03.2011 EP 11156415; 01.03.2011 US 201161447858 P
(43) Veröffentlichungstag der Anmeldung: 08.01.2014
(73) Patentinhaber: Bayer Intellectual Property GmbH, 40789 Monheim am Rhein (DE)
(72) Erfinder: BOJACK, Guido, 65207 Wiesbaden-Naurod (DE); FARIDA, Taraneh, 50259 Pulheim (DE); FISCHER, Reiner, 40789 Monheim (DE); LEHR, Stefan, 69006 Lyon (FR); SCHNATTERER, Albert, 51373 Leverkusen (DE); AULER, Thomas, 51465 Bergisch Gladbach (DE); GÖRGENS, Ulrich, 40882 Ratingen (DE); KEHNE, Heinz, 65719 Hofheim (DE); MALSAM, Olga, 51503 Rösrath (DE)
(74) Vertreter: BIP Patents
(86) Internationale Anmeldenummer: PCT/EP2012/053289
(87) Internationale Veröffentlichungsnummer: WO 2012/116960

(56) Entgegenhaltungen:
- US-B1- 6 451 843
- US-B1- 6 894 005
- DATABASE REGISTRY [Online] CHEMICAL ABSTRACTS SERVICE, COLUMBUS, OHIO, US; 10. Juni 2008 (2008-06-10), XP002646335, gefunden im STN Database accession no. 1027045-70-0

## Beschreibung

Die vorliegende Erfindung betrifft neue 2-Acyloxy-pyrrolin-4-one, mehrere Verfahren zu ihrer Herstellung und ihre Verwendung als Schädlingsbekämpfungsmittel und/oder Herbizide. Gegenstand der Erfindung sind auch selektiv herbizide Mittel, die 2-Acyloxy-pyrrolin-4-one einerseits und eine die Kulturpflanzen-verträglichkeit verbessernde Verbindung andererseits enthalten.

Die vorliegende Erfindung betrifft weiterhin die Steigerung der Wirkung von Pflanzenschutzmitteln enthaltend insbesondere 2-Acyloxy-pyrrolin-4-one, durch die Zugabe von Ammonium- oder Phosphoniumsalzen und gegebenenfalls Penetrationsförderern, die entsprechenden Mittel, Verfahren zu ihrer Herstellung und ihre Anwendung im Pflanzenschutz als Insektizide und/oder Akarizide und/oder Nematizide und/oder zur Verhinderung von unerwünschten Pflanzenwuchs.

Von 3-Acyl-pyrrolidin-2,4-dionen sind pharmazeutische Eigenschaften vorbeschrieben (S. Suzuki et al. Chem. Pharm. Bull. 15 1120 (1967)). Weiterhin wurden N-Phenylpyrrolidin-2,4-dione von R. Schmierer und H. Mildenberger (Liebigs Ann. Chem. 1985, 1095) synthetisiert. Eine biologische Wirksamkeit dieser Verbindungen wurde nicht beschrieben.

In EP-A-0 262 399 und GB-A-2 266 888 werden ähnlich strukturierte Verbindungen (3-Aryl-pyrrolidin-2,4-dione) offenbart, von denen jedoch keine herbizide, insektizide oder akarizide Wirkung bekannt geworden ist. Bekannt mit herbizider, insektizider oder akarizider Wirkung sind unsubstituierte, bicyclische 3-Aryl-pyrrolidin-2,4-dion-Derivate (EP-A-355 599, EP-A-415 211 und JP-A-12-053 670) sowie substituierte monocyclische 3-Aryl-pyrrolidin-2,4-dion-Derivate (EP-A-377 893, EP-A-442 077 und WO 10/066780).

Weiterhin bekannt sind polycyclische 3-Arylpyrrolidin-2,4-dion-Derivate (EP-A-442 073) sowie 1H-Arylpyrrolidin-dion-Derivate (EP-A-456 063, EP-A-521 334, EP-A-596 298, EP-A-613 884, EP-A-613 885, WO 95/01 971, WO 95/26 954, WO 95/20 572, EP-A-0 668 267, WO 96/25 395, WO 96/35 664, WO 97/01535, WO 97/02 243, WO 97/36 868, WO 97/43275, WO 98/05638, WO 98/06721, WO 98/25928, WO 99/24437, WO 99/43649, WO 99/48869, WO 99/55673, WO 01/17972, WO 01/23354, WO 01/74770, WO 03/013249, WO 03/062244, WO 2004/007448, WO 2004/024 688, WO 04/065366, WO 04/080962, WO 04/111042, WO 05/044791, WO 05/044796, WO 05/048710, WO 05/049569, WO 05/066125, WO 05/092897, WO 06/000355, WO 06/029799, WO 06/056281, WO 06/056282, WO 06/089633, WO 07/048545, DEA 102 00505 9892, WO 07/073856, WO 07/096058, WO 07/121868, WO 07/140881, WO 08/067873, WO 08/067910, WO 08/067911, WO 08/138551, WO 09/015801, WO 09/039975, WO 09/049851, WO 09/115262, WO 10/052161, WO 10/102758, WO 10/063378, WO 10/063670, WO 11/098440, WO 11/098443, WO 11/067135, WO 11/067240). Außerdem sind ketalsubstituierte 1-H-Arylpyrrolidin-2,4-dione aus WO 99/16748 und (spiro)-ketalsubstituierte N-Alkoxy-alkoxy-substituierte Aryl-pyrrolidindione aus JP-A-14 205 984 und Ito M. et al.. Bioscience, Biotechnology and Biochemistry 67, 1230-1238, (2003) bekannt. Der Zusatz von Safenern zu Ketoenolen ist ebenfalls prinzipiell aus der WO 03/013249 bekannt. Außerdem sind aus WO 06/024411 herbizide Mittel enthaltend Ketoenole bekannt.

Mit pharmazeutischer Wirkung sind bisher bekannt WO 2011/098433, DE-A-102010008642, DE-A-102010008643 und DE-Anmeldenummer 102010008640.

Außerdem sind biphenylsubstituierte 1H-Pyrrolidin-dion Derivate mit fungizider Wirkung bekannt (WO 03/059065).

Die Wirksamkeit und Wirkungsbreite dieser Verbindungen ist jedoch insbesondere bei niedrigen Aufwandmengen und Konzentrationen nicht immer völlig zufriedenstellend. Weiterhin ist die Pflanzenverträglichkeit dieser Verbindungen gegenüber den Kulturpflanzen nicht immer ausreichend. Außerdem sind die toxikologischen Eigenschaften und/oder Umwelteigenschaften dieser Verbindungen nicht immer völlig zufriedenstellend.

Es wurden nun neue Verbindungen der Formel (I) 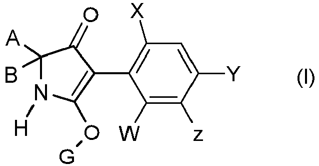 gefunden,
in welcher
- W: für Wasserstoff, Halogen, Alkyl, Alkenyl, Alkinyl, gegebenenfalls substituiertes Cycloalkyl, Alkoxy, Alkenyloxy, Halogenalkyl, Halogenalkoxy oder Cyano steht,
- X: für Halogen, Alkyl, Alkenyl, Alkinyl, gegebenenfalls substituiertes Cycloalkyl, Alkoxy, Alkenyloxy, Alkylthio, Alkylsulfinyl, Alkylsulfonyl, Halogenalkyl, Halogenalkoxy, Halogenalkenyloxy, Nitro oder Cyano steht,
- Y und Z: unabhängig voneinander für Wasserstoff, Alkyl, Alkenyl, Alkinyl, gegebenenfalls substituiertes Cycloalkyl, Alkoxy, Halogen, Halogenalkyl, Halogenalkoxy, Cyano, Nitro oder jeweils gegebenenfalls substituiertes Aryl oder Hetaryl stehen,
- A: für Wasserstoff, für jeweils gegebenenfalls durch Halogen substituiertes Alkyl, Alkenyl, Alkoxyalkyl, Alkylthioalkyl, gesättigtes oder ungesättigtes, gegebenenfalls substituiertes Cycloalkyl, in welchem gegebenenfalls mindestens ein Ringatom durch ein Heteroatom ersetzt ist, oder jeweils gegebenenfalls durch Halogen, Alkyl, Halogenalkyl, Alkoxy, Halogenalkoxy, Cyano oder Nitro substituiertes Aryl, Arylalkyl oder Hetaryl steht,
- B: für Wasserstoff, Alkyl oder Alkoxyalkyl steht, mit der Maßgabe, dass A und B nur dann jeweils für Methyl stehen dürfen, wenn, im Fall dass W, X und Y jeweils für Alkyl und Z für Wasserstoff stehen, W und X jeweils für Methyl stehen oder W und X jeweils für Ethyl stehen,
oder
- A und B: gemeinsam mit dem Kohlenstoffatom an das sie gebunden sind für einen gesättigten oder ungesättigten, gegebenenfalls mindestens ein Heteroatom enthaltenden unsubstituierten oder substituierten Cyclus stehen,
- G: für eine der Gruppen 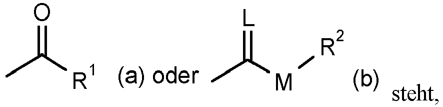
worin
- L: für Sauerstoff oder Schwefel steht,
- M: für Sauerstoff oder Schwefel steht,
- R¹: für jeweils gegebenenfalls durch Halogen oder Cyano substituiertes Alkyl, Alkenyl, Alkoxyalkyl, Alkylthioalkyl oder Polyalkoxyalkyl oder für jeweils gegebenenfalls durch Halogen, Alkyl oder Alkoxy substituiertes Cycloalkyl oder Heterocyclyl oder für jeweils gegebenenfalls substituiertes Phenyl, Phenylalkyl, Hetaryl, Phenoxyalkyl oder Hetaryloxyalkyl steht,
- R²: für jeweils gegebenenfalls durch Halogen oder Cyano substituiertes Alkyl, Alkenyl, Alkoxyalkyl oder Polyalkoxyalkyl oder für jeweils gegebenenfalls substituiertes Cycloalkyl, Phenyl oder Benzyl steht.

Die Verbindungen der Formel (I) können, auch in Abhängigkeit von der Art der Substituenten, als optische Isomere oder Isomerengemische, in unterschiedlicher Zusammensetzung vorliegen, die gegebenenfalls in üblicher Art und Weise getrennt werden können. Sowohl die reinen Isomeren als auch die Isomerengemische, deren Herstellung und Verwendung sowie diese enthaltende Mittel sind Gegenstand der vorliegenden Erfindung. Im folgenden wird der Einfachheit halber jedoch stets von Verbindungen der Formel (I) gesprochen, obwohl sowohl die reinen Verbindungen als gegebenenfalls auch Gemische mit unterschiedlichen Anteilen an isomeren Verbindungen gemeint sind.

Unter Einbeziehung der verschiedenen Bedeutungen (a) und (b) der Gruppe G ergeben sich folgende hauptsächliche Strukturen (I-a) bis (I-b),

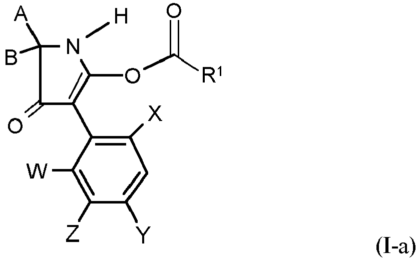

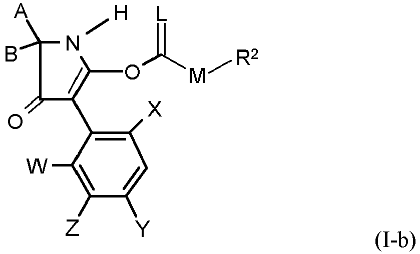

Weiterhin wurde gefunden, dass man die neuen Verbindungen der Formel (I) nach den im Folgenden beschriebenen Verfahren erhält:
(A) Man erhält Verbindungen der oben gezeigten Formel (I-a), in welchen R¹, A, B, W, X, Y und Z die oben angegebenen Bedeutungen haben, wenn man Verbindungen der Formel (II), in welcher A, B, W, X, Y und Z die oben angegebenen Bedeutungen haben, 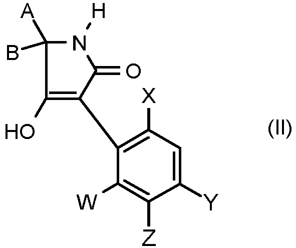 in welcher
   A, B, W, X, Y und Z die oben angegebenen Bedeutungen haben, α) mit Verbindungen der Formel (III) 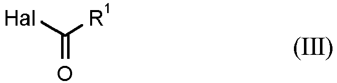 in welcher
      R¹ die oben angegebene Bedeutung hat und
      Hal für Halogen (insbesondere Chlor oder Brom) steht
      oder
   ß) mit Carbonsäureanhydriden der Formel (IV)

      R¹-CO-O-CO-R¹ (IV)

      in welcher
      R¹ die oben angegebene Bedeutung hat,
      gegebenenfalls in Gegenwart eines Verdünnungsmittels und gegebenenfalls in Gegenwart eines Säurebindemittels umsetzt.
(B) Man erhält Verbindungen der oben gezeigten Formel (I-b), in welchen R², A, B, M, W, X, Y und Z die oben angegebenen Bedeutungen haben und L für Sauerstoff steht, erhält, wenn man Verbindungen der oben gezeigten Formel (II), in welcher A, B, W, X, Y und Z die oben angegebenen Bedeutungen haben,
mit Chlorameisensäureestern oder Chlorameisensäurethioestern der Formel (V)

R²-M-CO-Cl (V)

in welcher
R² und M die oben angegebenen Bedeutungen haben,
gegebenenfalls in Gegenwart eines Verdünnungsmittels und gegebenenfalls in Gegenwart eines Säurebindemittels umsetzt.

Weiterhin wurde gefunden, dass die neuen Verbindungen der Formel (I) eine gute Wirksamkeit als Schädlingsbekämpfungsmittel, vorzugsweise als Insektizide und/oder Akarizide und/oder Herbizide aufweisen, darüber hinaus häufig insbesondere gegenüber Kulturpflanzen, sehr gut pflanzenverträglich sind und/oder über günstige toxikologische und/oder umweltrelevante Eigenschaften verfügen.

Überraschenderweise wurde nun auch gefunden, dass bestimmte substituierte, cyclische Ketoenole bei gemeinsamer Anwendung mit den im weiteren beschriebenen, die Kulturpflanzen-Verträglichkeit verbessernden Verbindungen (Safenern/Antidots) ausgesprochen gut die Schädigung der Kulturpflanzen verhindern und besonders vorteilhaft als breit wirksame Kombinationspräparate zur selektiven Bekämpfung von unerwünschten Pflanzen in Nutzpflanzenkulturen, wie z.B. in Getreide aber auch Mais, Raps, Soja und Reis, verwendet werden können.

Gegenstand der Erfindung sind auch selektiv-herbizide Mittel enthaltend einen wirksamen Gehalt an einer Wirkstoffkombination umfassend als Komponenten
a') mindestens eine Verbindung der Formel (I), in welcher A, B, G, W, X, Y und Z die oben angegebene Bedeutung haben
   und
(b') zumindest eine die Kulturpflanzen-Verträglichkeit verbessernde Verbindung (Safener).

Die Safener sind vorzugsweise ausgewählt aus der Gruppe bestehend aus:
S1) Verbindungen der Formel (S1), 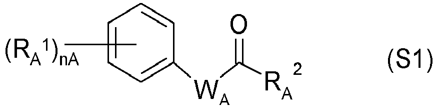 wobei die Symbole und Indizes folgende Bedeutungen haben:
   - n_{A}: ist eine natürliche Zahl von 0 bis 5, vorzugsweise 0 bis 3;
   - R_{A}¹: ist Halogen, (C₁-C₄)Alkyl, (C₁-C₄)Alkoxy, Nitro oder (C₁-C₄)Haloalkyl;
   - W_{A}: ist ein unsubstituierter oder substituierter divalenter heterocyclischer Rest aus der Gruppe der teilungesättigten oder aromatischen Fünfring-Heterocyclen mit 1 bis 3 Heteroringatomen aus der Gruppe N und O, wobei mindestens ein N-Atom und höchstens ein O-Atom im Ring 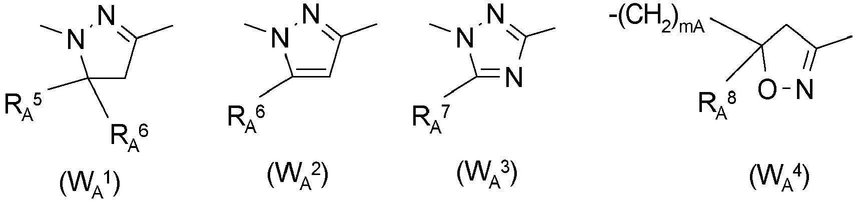 enthalten ist, vorzugsweise ein Rest aus der Gruppe (W_{A}¹) bis (W_{A}⁴),
   - m_{A}: ist 0 oder 1;
   - R_{A}²: ist OR_{A}³, SR_{A}³ oder NR_{A}³R_{A}⁴ oder ein gesättigter oder ungesättigter 3- bis 7-gliedriger Heterocyclus mit mindestens einem N-Atom und bis zu 3 Heteroatomen, vorzugsweise aus der Gruppe O und S, der über das N-Atom mit der Carbonylgruppe in (S1) verbunden ist und unsubstituiert oder durch Reste aus der Gruppe (C₁-C₄)Alkyl, (C₁-C₄)Alkoxy oder gegebenenfalls substituiertes Phenyl substituiert ist, vorzugsweise ein Rest der Formel OR_{A}³, NHR_{A}⁴ oder N(CH₃)₂, insbesondere der Formel OR_{A}³;
   - R_{A}³: ist Wasserstoff oder ein unsubstituierter oder substituierter aliphatischer Kohlenwasserstoffrest, vorzugsweise mit insgesamt 1 bis 18 C-Atomen;
   - R_{A}⁴: ist Wasserstoff, (C₁-C₆)Alkyl, (C₁-C₆)Alkoxy oder substituiertes oder unsubstituiertes Phenyl;
   - R_{A}⁵: ist H, (C₁-C₈)Alkyl, (C₁-C₈)Haloalkyl, (C₁-C₄)Alkoxy(C₁-C₈)Alkyl, Cyano oder COOR_{A}⁹, worin R_{A}⁹ Wasserstoff, (C₁-C₈)Alkyl, (C₁-C₈)Haloalkyl, (C₁-C₄)Alkoxy-(C₁-C₄)alkyl, (C₁-C₆)Hydroxyalkyl, (C₃-C₁₂)Cycloalkyl oder Tri-(C₁-C₄)-alkyl-silyl ist;
   - R_{A}⁶, R_{A}⁷, R_{A}⁸: sind gleich oder verschieden Wasserstoff, (C₁-C₈)Alkyl, (C₁-C₈)Haloalkyl, (C₃-C₁₂)Cycloalkyl oder substituiertes oder unsubstituiertes Phenyl;
   vorzugsweise:
   a) Verbindungen vom Typ der Dichlorphenylpyrazolin-3-carbonsäure (S1^{a}), vorzugsweise Verbindungen wie 1-(2,4-Dichlorphenyl)-5-ethoxycarbonyl)-5-methyl-2-pyrazolin-3-carbonsäure, 1-(2,4-Dichlorphenyl)-5-ethoxycarbonyl)-5-methyl-2-pyrazolin-3-carbonsäureethylester (S1-1) ("Mefenpyr-diethyl"), und verwandte Verbindungen, wie sie in der WO-A-91/07874 beschrieben sind;
   b) Derivate der Dichlorphenylpyrazolcarbonsäure (S1^{b}), vorzugsweise Verbindungen wie 1-(2,4-Dichlorphenyl)-5-methyl-pyrazol-3-carbonsäureethylester (S1-2), 1-(2,4-Dichlorphenyl)-5-isopropyl-pyrazol-3-carbonsäureethylester (S1-3), 1-(2,4-Dichlorphenyl)-5-(1,1-dimethyl-ethyl)pyrazol-3-carbonsäureethyl-ester (S1-4) und verwandte Verbindungen, wie sie in EP-A-333 131 und EP-A-269 806 beschrieben sind;
   c) Derivate der 1,5-Diphenylpyrazol-3-carbonsäure (S1^{c}), vorzugsweise Verbindungen wie 1-(2,4-Dichlorphenyl)-5-phenylpyrazol-3-carbonsäureethylester (S1-5), 1-(2-Chlorphenyl)-5-phenylpyrazol-3-carbonsäuremethylester (S1-6) und verwandte Verbindungen wie sie beispielsweise in der EP-A-268554 beschrieben sind;
   d) Verbindungen vom Typ der Triazolcarbonsäuren (S1^{d}), vorzugsweise Verbindungen wie Fenchlorazol(-ethylester), d.h. 1-(2,4-Dichlorphenyl)-5-richlormethyl-(1H)-1,2,4-triazol-3-carbonsäureethylester (S1-7), und verwandte Verbindungen wie sie in EP-A-174 562 und EP-A-346 620 beschrieben sind;
   e) Verbindungen vom Typ der 5-Benzyl- oder 5-Phenyl-2-isoxazolin-3- carbonsäure oder der 5,5-Diphenyl-2-isoxazolin-3-carbonsäure (S1^{e}), vorzugsweise Verbindungen wie 5-(2,4-Dichlorbenzyl)-2-isoxazolin-3-carbonsäureethylester (S1-8) oder 5-Phenyl-2-isoxazolin-3-carbonsäureethylester (S 1-9) und verwandte Verbindungen, wie sie in WO-A-91/08202 beschrieben sind, bzw. 5,5-Diphenyl-2-isoxazolin-carbonsäure (S1-10) oder 5,5-Diphenyl-2-isoxazolin-carbonsäureethylester (S1-11) ("Isoxadifen-ethyl") oder -n-propylester (S 1-12) oder der 5-(4-Fluorphenyl)-5-phenyl-2-isoxazolin-3-carbonsäureethylester (S1-13), wie sie in der Patentanmeldung WO-A-95/07897 beschrieben sind.
S2) Chinolinderivate der Formel (S2), 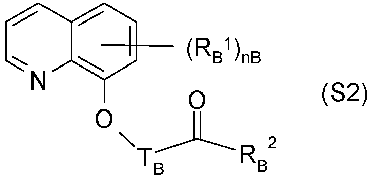 wobei die Symbole und Indizes folgende Bedeutungen haben:
   - R_{B}¹: ist Halogen, (C₁-C₄)Alkyl, (C₁-C₄)Alkoxy, Nitro oder (C₁-C₄)Haloalkyl;
   - n_{B}: ist eine natürliche Zahl von 0 bis 5, vorzugsweise 0 bis 3;
   - R_{B}²: ist OR_{B}³, SR_{B}³ oder NR_{B}³R_{B}⁴ oder ein gesättigter
   oder ungesättigter 3- bis 7-gliedriger Heterocyclus mit mindestens einem N-Atom und bis zu 3 Heteroatomen, vorzugsweise aus der Gruppe O und S, der über das N-Atom mit der Carbonylgruppe in (S2) verbunden ist und unsubstituiert oder durch Reste aus der Gruppe (C₁-C₄)Alkyl, (C₁-C₄)Alkoxy oder gegebenenfalls substituiertes Phenyl substituiert ist, vorzugsweise ein Rest der Formel OR_{B}³, NHR_{B}⁴ oder N(CH₃)₂, insbesondere der Formel OR_{B}³;
   - R_{B}³: ist Wasserstoff oder ein unsubstituierter oder substituierter aliphatischer Kohlenwasserstoffrest, vorzugsweise mit insgesamt 1 bis 18 C-Atomen;
   - R_{B}⁴: ist Wasserstoff, (C₁-C₆)Alkyl, (C₁-C₆)Alkoxy oder substituiertes oder unsubstituiertes Phenyl;
   - T_{B}: ist eine (C₁ oder C₂)-Alkandiylkette, die unsubstituiert oder mit einem oder zwei (C₁-C₄)Alkylresten oder mit [(C₁-C₃)-Alkoxy]-carbonyl substituiert ist;
   vorzugsweise:
   a) Verbindungen vom Typ der 8-Chinolinoxyessigsäure (S2^{a}), vorzugsweise (5-Chlor-8-chinolinoxy)essigsäure-(1-methylhexyl)ester ("Cloquintocet-mexyl") (S2-1), (5-Chlor-8-chinolinoxy)essigsäure-(1,3-dimethyl-but-1-yl)ester (S2-2), (5-Chlor-8-chinolinoxy)essigsäure-4-allyloxy-butylester (S2-3), (5-Chlor-8-chinolinoxy)essigsäure-1-allyloxy-prop-2-ylester (S2-4), (5-Chlor-8-chinolinoxy)essigsäureethylester (S2-5), (5-Chlor-8-chinolinoxy)essigsäuremethylester (S2-6), (5-Chlor-8-chinolinoxy)essigsäureallylester (S2-7), (5-Chlor-8-chinolinoxy)essigsäure-2-(2-propyliden-iminoxy)-1-ethylester (S2-8), (5-Chlor-8-chinolinoxy)essigsäure-2-oxo-prop-1-ylester (S2-9) und verwandte Verbindungen, wie sie in EP-A-86 750, EP-A-94 349 und EP-A-191 736 oder EP-A-0 492 366 beschrieben sind, sowie (5-Chlor-8-chinolinoxy)essigsäure (S2-10), deren Hydrate und Salze, beispielsweise deren Lithium-, Natrium- Kalium-, Kalzium-, Magnesium-, Aluminium-, Eisen-, Ammonium-, quartäre Ammonium-, Sulfonium-, oder Phosphoniumsalze wie sie in der WO-A-2002/34048 beschrieben sind;
   b) Verbindungen vom Typ der (5-Chlor-8-chinolinoxy)malonsäure (S2^{b}), vorzugsweise Verbindungen wie (5-Chlor-8-chinolinoxy)malonsäurediethylester, (5-Chlor-8-chinolinoxy)malonsäurediallylester, (5-Chlor-8-chinolin-oxy)malonsäure-methyl-ethylester und verwandte Verbindungen, wie sie in EP-A-0 582 198 beschrieben sind.
S3) Verbindungen der Formel (S3) 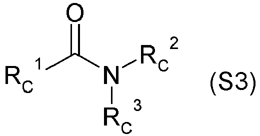 wobei die Symbole und Indizes folgende Bedeutungen haben:
   - R_{C}¹: ist (C₁-C₄)Alkyl, (C₁-C₄)Haloalkyl, (C₂-C₄)Alkenyl, (C₂-C₄)Haloalkenyl, (C₃-C₇)Cycloalkyl, vorzugsweise Dichlormethyl;
   - R_{C}², R_{C}³: sind gleich oder verschieden Wasserstoff, (C₁-C₄)Alkyl, (C₂-C₄)Alkenyl, (C₂-C₄)Alkinyl, (C₁-C₄)Haloalkyl, (C₂-C₄)Haloalkenyl, (C₁-C₄)Alkylcarbamoyl-(C₁-C₄)alkyl, (C₂-C₄)Alkenylcarbamoyl-(C₁-C₄)alkyl, (C₁-C₄)Alkoxy-(C₁-C₄)alkyl, Dioxolanyl-(C₁-C₄)alkyl, Thiazolyl, Furyl, Furylalkyl, Thienyl, Piperidyl, substituiertes oder unsubstituiertes Phenyl, oder R_{C}² und R_{C}³ bilden zusammen einen substituierten oder unsubstituierten heterocyclischen Ring, vorzugsweise einen Oxazolidin-, Thiazolidin-, Piperidin-, Morpholin-, Hexahydropyrimidin- oder Benzoxazinring;
   vorzugsweise:
   Wirkstoffe vom Typ der Dichloracetamide, die häufig als Vorauflaufsafener (bodenwirksame Safener) angewendet werden, wie z. B.
   "Dichlormid" (N,N-Diallyl-2,2-dichloracetamid) (S3-1),
   "R-29148" (3-Dichloracetyl-2,2,5-trimethyl-1,3-oxazolidin) der Firma Stauffer (S3-2),
   "R-28725" (3-Dichloracetyl-2,2,-dimethyl-1,3-oxazolidin) der Firma Stauffer (S3-3),
   "Benoxacor" (4-Dichloracetyl-3,4-dihydro-3-methyl-2H-1,4-benzoxazin) (S3-4),
   "PPG-1292" (N-Allyl-N-[(1,3-dioxolan-2-yl)-methyl]-dichloracetamid) der Firma PPG Industries (S3-5),
   "DKA-24" (N-Allyl-N-[(allylaminocarbonyl)methyl]-dichloracetamid) der Firma Sagro-Chem (S3-6),
   "AD-67" oder "MON 4660" (3-Dichloracetyl-1-oxa-3-aza-spiro[4,5]decan) der Firma Nitrokemia bzw. Monsanto (S3-7),
   "TI-35" (1-Dichloracetyl-azepan) der Firma TRI-Chemical RT (S3-8),
   "Diclonon" (Dicyclonon) oder "BAS145138" oder "LAB145138" (S3-9) (3-Dichloracetyl-2,5,5-trimethyl-1,3-diazabicyclo[4.3.0]nonan) der Firma BASF,
   "Furilazol" oder "MON 13900" ((RS)-3-Dichloracetyl-5-(2-furyl)-2,2-dimethyloxazolidin)
   (S3-10); sowie dessen (R)-Isomer (S3-11).
S4) N-Acylsulfonamide der Formel (S4) und ihre Salze, 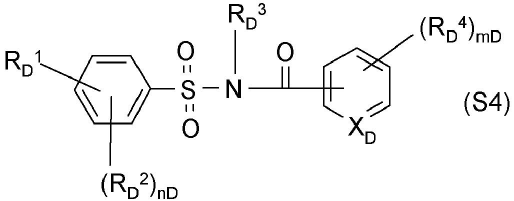 worin die Symbole und Indizes folgende Bedeutungen haben:
   - X_{D}: ist CH oder N;
   - R_{D}¹: ist CO-NR_{D}⁵R_{D}⁶ oder NHCO-R_{D}⁷;
   - R_{D}²: ist Halogen, (C₁-C₄)Haloalkyl, (C₁-C₄)Haloalkoxy, Nitro, (C₁-C₄)Alkyl, (C₁-C₄)Alkoxy, (C₁-C₄)Alkylsulfonyl, (C₁-C₄)Alkoxycarbonyl oder (C₁-C₄)Alkylcarbonyl;
   - R_{D}³: ist Wasserstoff, (C₁-C₄)Alkyl, (C₂-C₄)Alkenyl oder (C₂-C₄)Alkinyl;
   - R_{D}⁴: ist Halogen, Nitro, (C₁-C₄)Alkyl, (C₁-C₄)Haloalkyl, (C₁-C₄)Haloalkoxy, (C₃-C₆)Cycloalkyl, Phenyl, (C₁-C₄)Alkoxy, Cyano, (C₁-C₄)Alkylthio, (C₁-C₄)Alkylsulfinyl, (C₁-C₄)Alkylsulfonyl, (C₁-C₄)Alkoxycarbonyl oder (C₁-C₄)Alkylcarbonyl;
   - R_{D}⁵: ist Wasserstoff, (C₁-C₆)Alkyl, (C₃-C₆)Cycloalkyl, (C₂-C₆)Alkenyl, (C₂-C₆)Alkinyl, (C₅-C₆)Cycloalkenyl, Phenyl oder 3- bis 6-gliedriges Heterocyclyl enthaltend v_{D} Heteroatome aus der Gruppe Stickstoff, Sauerstoff und Schwefel, wobei die sieben letztgenannten Reste durch v_{D} Substituenten aus der Gruppe Halogen, (C₁-C₆)Alkoxy, (C₁-C₆)Haloalkoxy, (C₁-C₂)Alkylsulfinyl, (C₁-C₂)Alkylsulfonyl, (C₃-C₆)Cycloalkyl, (C₁-C₄)Alkoxycarbonyl, (C₁-C₄)Alkylcarbonyl und Phenyl und im Falle cyclischer Reste auch (C₁-C₄) Alkyl und (C₁-C₄)Haloalkyl substituiert sind;
   - R_{D}⁶: ist Wasserstoff, (C₁-C₆)Alkyl, (C₂-C₆)Alkenyl oder (C₂-C₆)Alkinyl, wobei die drei letztgenannten Reste durch v_{D} Reste aus der Gruppe Halogen, Hydroxy, (C₁-C₄)Alkyl, (C₁-C₄)Alkoxy und (C₁-C₄)Alkylthio substituiert sind, oder
   - R_{D}⁵ und R_{D}⁶: gemeinsam mit dem sie tragenden Stickstoffatom einen Pyrrolidinyl- oder Piperidinyl-Rest bilden;
   - R_{D}⁷: ist Wasserstoff, (C₁-C₄)Alkylamino, Di-(C₁-C₄)alkylamino, (C₁-C₆)Alkyl, (C₃-C₆)Cycloalkyl, wobei die 2 letztgenannten Reste durch v_{D} Substituenten aus der Gruppe Halogen, (C₁-C₄)Alkoxy, (C₁-C₆)Haloalkoxy und (C₁-C₄)Alkylthio und im Falle cyclischer Reste auch (C₁-C₄)Alkyl und (C₁-C₄)Haloalkyl substituiert sind;
   - n_{D}: ist 0, 1 oder 2;
   - m_{D}: ist 1 oder 2;
   - v_{D}: ist 0, 1, 2 oder 3;
   davon bevorzugt sind Verbindungen von Typ der N-Acylsulfonamide, z.B. der nachfolgenden Formel (S4^{a}), die z. B. bekannt sind aus WO-A-97/45016 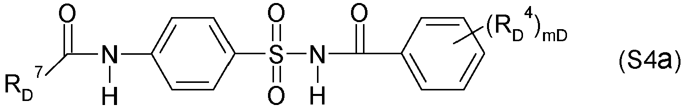 worin
   - R_{D}⁷: (C₁-C₆)Alkyl, (C₃-C₆)Cycloalkyl, wobei die 2 letztgenannten Reste durch v_{D} Substituenten aus der Gruppe Halogen, (C₁-C₄)Alkoxy, (C₁-C₆)Haloalkoxy und (C₁-C₄)Alkylthio und im Falle cyclischer Reste auch (C₁-C₄)Alkyl und (C₁-C₄)Haloalkyl substituiert sind;
   - R_{D}⁴: Halogen, (C₁-C₄)Alkyl, (C₁-C₄)Alkoxy, CF_{3;}
   - m_{D}: 1 oder 2;
   - v_{D}: ist 0, 1, 2 oder 3 bedeutet;
   sowie
   Acylsulfamoylbenzoesäureamide, z.B. der nachfolgenden Formel (S4^{b}), die z.B. bekannt sind aus WO-A-99/16744, 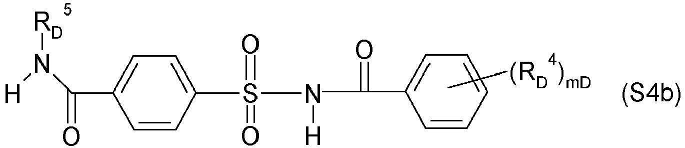
   z.B. solche worin
   R_{D}⁵ = Cyclopropyl und (R_{D}⁴) = 2-OMe ist ("Cyprosulfamide", S4-1),
   R_{D}⁵ = Cyclopropyl und (R_{D}⁴) = 5-Cl-2-OMe ist (S4-2),
   R_{D}⁵ = Ethyl und (R_{D}⁴) = 2-OMe ist (S4-3),
   R_{D}⁵ = Isopropyl und (R_{D}⁴) = 5-Cl-2-OMe ist (S4-4) und
   R_{D}⁵ = Isopropyl und (R_{D}⁴) = 2-OMe ist (S4-5)
      sowie
   Verbindungen vom Typ der N-Acylsulfamoylphenylharnstoffe der Formel (S4^{c}), die z.B. bekannt sind aus der EP-A-365484, 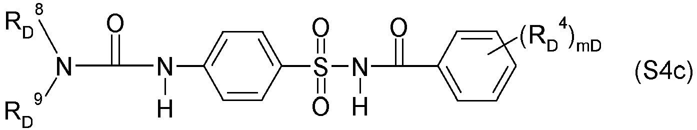 worin
      - R_{D}⁸ und R_{D}⁹: unabhängig voneinander Wasserstoff, (C₁-C₈)Alkyl, (C₃-C₈)Cycloalkyl, (C₃-C₆)Alkenyl, (C₃-C₆)Alkinyl,
      - R_{D}⁴: Halogen, (C₁-C₄)Alkyl, (C₁-C₄)Alkoxy, CF₃
      - m_{D}: 1 oder 2 bedeutet;
      beispielsweise
      1-[4-(N-2-Methoxybenzoylsulfamoyl)phenyl]-3-methylharnstoff,
      1-[4-(N-2-Methoxybenzoylsulfamoyl)phenyl]-3,3-dimethylharnstoff,
      1-[4-(N-4,5-Dimethylbenzoylsulfamoyl)phenyl]-3-methylharnstoff.
S5) Wirkstoffe aus der Klasse der Hydroxyaromaten und der aromatisch-aliphatischen Carbonsäurederivate (S5), z.B. 3,4,5-Triacetoxybenzoesäureethylester, 3,5-Dimethoxy-4-hydroxybenzoesäure, 3,5-Dihydroxybenzoesäure, 4-Hydroxysalicylsäure, 4-Fluorsalicyclsäure, 2-Hydroxyzimtsäure, 1,2-Dihydro-2-oxo-6-trifluoromethylpyridin-3-carboxamid, 2,4-Dichlorzimtsäure, wie sie in der WO-A-2004/084631, WO-A-2005/015994, WO-A-2005/016001 beschrieben sind.
S6) Wirkstoffe aus der Klasse der 1,2-Dihydrochinoxalin-2-one (S6), z.B. 1-Methyl-3-(2-thienyl)-1,2-dihydrochinoxalin-2-on, 1-Methyl-3-(2-thienyl)-1,2-dihydro-chinoxalin-2-thion, 1-(2-Aminoethyl)-3-(2-thienyl)-1,2-dihydro-chinoxalin-2-on-hydrochlorid, 1-[2-(Diethylamino)ethyl]-6,7-dimethyl-3-thiophen-2-ylchinoxalin-2(1H)-on, 1-(2-Methylsulfonylaminoethyl)-3-(2-thienyl)-1,2-dihydro-chinoxalin-2-on, wie sie in der WO-A-2005/112630 beschrieben sind.
S7) Verbindungen der Formel (S7),wie sie in der WO-A-1998/38856 beschrieben sind 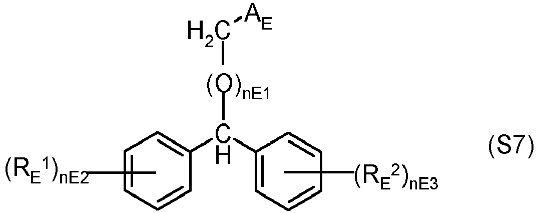 worin die Symbole und Indizes folgende Bedeutungen haben:
   - R_{E}¹, R_{E}²: sind unabhängig voneinander Halogen, (C₁-C₄)Alkyl, (C₁-C₄)Alkoxy, (C₁-C₄)Haloalkyl, (C₁-C₄)Alkylamino, Di-(C₁-C₄)Alkylamino, Nitro;
   - A_{E}: ist COOR_{E}³ oder COSR_{E}⁴
   - R_{E}³, R_{E}⁴: sind unabhängig voneinander Wasserstoff, (C₁-C₄)Alkyl, (C₂-C₆)Alkenyl, (C₂-C₄)Alkinyl, Cyanoalkyl, (C₁-C₄)Haloalkyl, Phenyl, Nitrophenyl, Benzyl, Halobenzyl, Pyridinylalkyl und Alkylammonium,
   - n_{E}¹: ist 0 oder 1
   - n_{E}², n_{E}³: sind unabhängig voneinander 0, 1 oder 2,
   vorzugsweise:
   Diphenylmethoxyessigsäure,
   Diphenylmethoxyessigsäureethylester,
   Diphenylmethoxyessigsäuremethylester (CAS-Reg.Nr. 41858-19-9) (S7-1).
S8) Verbindungen der Formel (S8),wie sie in der WO-A-98/27049 beschrieben sind 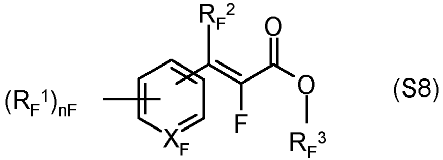 worin
   - X_{F}: CH oder N,
   - n_{F}: für den Fall, dass X_{F}=N ist, eine ganze Zahl von 0 bis 4 und für den Fall, dass X_{F}=CH ist, eine ganze Zahl von 0 bis 5 ,
   - R_{F}¹: Halogen, (C₁-C₄)Alkyl, (C₁-C₄)Haloalkyl, (C₁-C₄)Alkoxy, (C₁-C₄)Haloalkoxy, Nitro, (C₁-C₄)Alkylthio, (C₁-C₄)-Alkylsulfonyl, (C₁-C₄)Alkoxycarbonyl, ggf. substituiertes. Phenyl, ggf. substituiertes Phenoxy,
   - R_{F}²: Wasserstoff oder (C₁-C₄)Alkyl
   - R_{F}³: Wasserstoff, (C₁-C₈)Alkyl, (C₂-C₄)Alkenyl, (C₂-C₄)Alkinyl, oder Aryl, wobei jeder der vorgenannten C-haltigen Reste unsubstituiert oder durch einen oder mehrere, vorzugsweise bis zu drei gleiche oder verschiedene Reste aus der Gruppe, bestehend aus Halogen und Alkoxy substituiert ist;bedeuten, oder deren Salze,
   vorzugsweise Verbindungen worin
   X_{F} CH,
   - n_{F}: eine ganze Zahl von 0 bis 2 ,
   - R_{F}¹: Halogen, (C₁-C₄)Alkyl, (C₁-C₄)Haloalkyl, (C₁-C₄)Alkoxy, (C₁-C₄)Haloalkoxy,
   - R_{F}²: Wasserstoff oder (C₁-C₄)Alkyl,
   - R_{F}³: Wasserstoff, (C₁-C₈)Alkyl, (C₂-C₄)Alkenyl, (C₂-C₄)Alkinyl, oder Aryl, wobei jeder der vorgenannten C-haltigen Reste unsubstituiert oder durch einen oder mehrere, vorzugsweise bis zu drei gleiche oder verschiedene Reste aus der Gruppe, bestehend aus Halogen und Alkoxy substituiert ist, bedeuten,oder deren Salze.
S9) Wirkstoffe aus der Klasse der 3-(5-Tetrazolylcarbonyl)-2-chinolone (S9), z.B. 1,2-Dihydro-4-hydroxy-1-ethyl-3-(5-tetrazolylcarbonyl)-2-chinolon (CAS-Reg.Nr. 219479-18-2), 1,2-Dihydro-4-hydroxy-1-methyl-3-(5-tetrazolyl-carbonyl)-2-chinolon (CAS-Reg.Nr. 95855-00-8), wie sie in der WO-A-1999/000020 beschrieben sind.
S10) Verbindungen der Formeln (S10^{a}) oder (S10^{b})
   wie sie in der WO-A-2007/023719 und WO-A-2007/023764 beschrieben sind 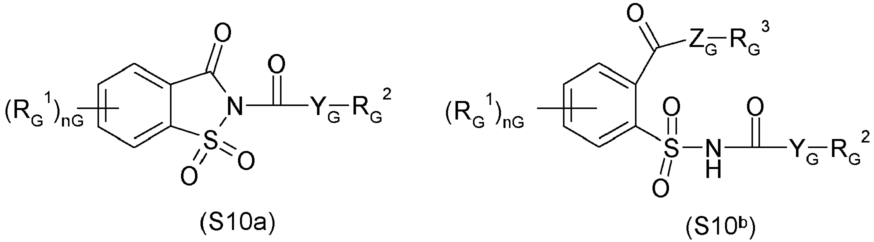 worin
   - R_{G}¹: Halogen, (C₁-C₄)Alkyl, Methoxy, Nitro, Cyano, CF₃, OCF₃
   - Y_{G}, Z_{G}: unabhängig voneinander O oder S,
   - n_{G}: eine ganze Zahl von 0 bis 4,
   - R_{G}²: (C₁-C₁₆)Alkyl, (C₂-C₆)Alkenyl, (C₃-C₆)Cycloalkyl, Aryl; Benzyl, Halogenbenzyl,
   - R_{G}³: Wasserstoff oder (C₁-C₆)Alkyl bedeutet.
S11) Wirkstoffe vom Typ der Oxyimino-Verbindungen (S11), die als Saatbeizmittel bekannt sind, wie z. B. "Oxabetrinil" ((Z)-1,3-Dioxolan--ylmethoxyimino(phenyl)acetonitril) (S11-1), das als Saatbeiz-Safener für Hirse gegen Schäden von Metolachlor bekannt ist,
   "Fluxofenim" (1-(4-Chlorphenyl)-2,2,2-trifluor-1-ethanon-O-(1,3-dioxolan-2-ylmethyl)-oxim) (S 11-2), das als Saatbeiz-Safener für Hirse gegen Schäden von Metolachlor bekannt ist, und
   "Cyometrinil" oder "CGA-43089" ((Z)-Cyanomethoxyimino(phenyl)acetonitril) (S11-3), das als Saatbeiz-Safener für Hirse gegen Schäden von Metolachlor bekannt ist.
S12) Wirkstoffe aus der Klasse der Isothiochromanone (S 12), wie z.B. Methyl-[(3-oxo-1H-2-benzothiopyran-4(3H)-yliden)methoxy]acetate (CAS-Reg.Nr. 205121-04-6) (S12-1) und verwandte Verbindungen aus WO-A-1998/13361.
S13) Eine oder mehrere Verbindungen aus Gruppe (S13):
   "Naphthalic anhydrid" (1,8-Naphthalindicarbonsäureanhydrid) (S13-1), das als Saatbeiz-Safener für Mais gegen Schäden von Thiocarbamatherbiziden bekannt ist,
   "Fenclorim" (4,6-Dichlor-2-phenylpyrimidin) (S 13-2), das als Safener für Pretilachlor in gesätem Reis bekannt ist,
   "Flurazole" (Benzyl-2-chlor-4-trifluormethyl-1,3-thiazol-5-carboxylat) (S13-3), das als Saatbeiz-Safener für Hirse gegen Schäden von Alachlor und Metolachlor bekannt ist,
   "CL 304415" (CAS-Reg.Nr. 31541-57-8) (4-Carboxy-3,4-dihydro-2H-1-benzopyran-4-essigsäure) (S 13-4) der Firma American Cyanamid, das als Safener für Mais gegen Schäden von Imidazolinonen bekannt ist,
   "MG 191" (CAS-Reg.Nr. 96420-72-3) (2-Dichlormethyl-2-methyl-1,3-dioxolan) (S13-5) der Firma Nitrokemia, das als Safener für Mais bekannt ist,
   "MG-838" (CAS-Reg.Nr. 133993-74-5) (2-propenyl 1-oxa-4-azaspiro[4.5]decane-4-carbodithioate) (S 13-6) der Firma Nitrokemia,
   "Disulfoton" (O,O-Diethyl S-2-ethylthioethyl phosphordithioat) (S 13-7),
   "Dietholate" (O,O-Diethyl-O-phenylphosphorotioat) (S 13-8),
   "Mephenate" (4-Chlorphenyl-methylcarbamat) (S 13-9).
S14) Wirkstoffe, die neben einer herbiziden Wirkung gegen Schadpflanzen auch Safenerwirkung an Kulturpflanzen wie Reis aufweisen, wie z. B. "Dimepiperate" oder "MY-93" (S-1-Methyl-1-phenylethyl-piperidin-1-carbothioat), das als Safener für Reis gegen Schäden des Herbizids Molinate bekannt ist,
   "Daimuron" oder "SK 23" (1-(1-Methyl-1-phenylethyl)-3-p-tolyl-harnstoff), das als Safener für Reis gegen Schäden des Herbizids Imazosulfuron bekannt ist,
   "Cumyluron" = "JC-940" (3-(2-Chlorphenylmethyl)-1-(1-methyl-1-phenyl-ethyl)harnstoff, siehe JP-A-60087254), das als Safener für Reis gegen Schäden einiger Herbizide bekannt ist,
   "Methoxyphenon" oder "NK 049" (3,3'-Dimethyl-4-methoxy-benzophenon), das als Safener für Reis gegen Schäden einiger Herbizide bekannt ist,
   "CSB" (1-Brom-4-(chlormethylsulfonyl)benzol) von Kumiai, (CAS-Reg.Nr. 54091-06-4), das als Safener gegen Schäden einiger Herbizide in Reis bekannt ist.
S15) Verbindungen der Formel (S15) oder deren Tautomere 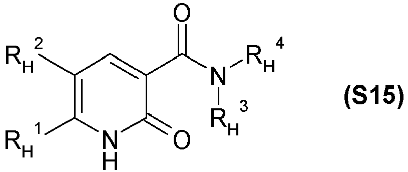 wie sie in der WO-A-2008/131861 und WO-A-2008/131860 beschrieben sind
   worin
   - R_{H}¹: einen (C₁-C₆)Haloalkylrest bedeutet und
   - R_{H}²: Wasserstoff oder Halogen bedeutet und
   - R_{H}³, R_{H}⁴: unabhängig voneinander Wasserstoff, (C₁-C₁₆)Alkyl, (C₂-C₁₆)Alkenyl oder (C₂-C₁₆)Alkinyl,
   wobei jeder der letztgenannten 3 Reste unsubstituiert oder durch einen oder mehrere Reste aus der Gruppe Halogen, Hydroxy, Cyano, (C₁-C₄)Alkoxy, (C₁-C₄)Haloalkoxy, (C₁-C₄)Alkylthio, (C₁-C₄)Alkylamino, Di[(C₁-C₄)alkyl]-amino, [(C₁-C₄)Alkoxy]-carbonyl, [(C₁-C₄)Haloalkoxy]-carbonyl, (C₃-C₆)Cycloalkyl, das unsubstituiert oder substituiert ist, Phenyl, das unsubstituiert oder substituiert ist, und Heterocyclyl, das unsubstituiert oder substituiert ist, substituiert ist,
   oder (C₃-C₆)Cycloalkyl, (C₄-C₆)Cycloalkenyl, (C₃-C₆)Cycloalkyl, das an einer Seite des Rings mit einem 4 bis 6-gliedrigen gesättigten oder ungesättigten carbocyclischen Ring kondensiert ist, oder (C₄-C₆)Cycloalkenyl, das an einer Seite des Rings mit einem 4 bis 6-gliedrigen gesättigten oder ungesättigten carbocyclischen Ring kondensiert ist,
   wobei jeder der letztgenannten 4 Reste unsubstituiert oder durch einen oder mehrere Reste aus der Gruppe Halogen, Hydroxy, Cyano, (C₁-C₄)Alkyl, (C₁-C₄)Haloalkyl, (C₁-C₄)Alkoxy, (C₁-C₄)Haloalkoxy, (C₁-C₄)Alkylthio, (C₁-C₄)Alkylamino, Di[(C₁-C₄)alkyl]-amino, [(C₁-C₄)Alkoxy]-carbonyl, [(C₁-C₄)Haloalkoxy]-carbonyl, (C₃-C₆)Cycloalkyl, das unsubstituiert oder substituiert ist, Phenyl, das unsubstituiert oder substituiert ist, und Heterocyclyl, das unsubstituiert oder substituiert ist, substituiert ist,
   bedeutet oder
   - R_{H}³: (C₁-C₄)-Alkoxy, (C₂-C₄)Alkenyloxy, (C₂-C₆)Alkinyloxy oder (C₂-C₄)Haloalkoxy bedeutet und
   - R_{H}⁴: Wasserstoff oder (C₁-C₄)-Alkyl bedeutet oder
   - R_{H}³ und R_{H}⁴: zusammen mit dem direkt gebundenen N-Atom einen vier- bis achtgliedrigen heterocyclischen Ring, der neben dem N-Atom auch weitere Heteroringatome, vorzugsweise bis zu zwei weitere Heteroringatome aus der Gruppe N, O und S enthalten kann und der unsubstituiert oder durch einen oder mehrere Reste aus der Gruppe Halogen, Cyano, Nitro, (C₁-C₄)Alkyl, (C₁-C₄)Haloalkyl, (C₁-C₄)Alkoxy, (C₁-C₄)Haloalkoxy und (C₁-C₄)Alkylthio substituiert ist, bedeutet.
S16) Wirkstoffe, die vorrangig als Herbizide eingesetzt werden, jedoch auch Safenerwirkung auf Kulturpflanzen aufweisen, z.B.
   (2,4-Dichlorphenoxy)essigsäure (2,4-D),
   (4-Chlorphenoxy)essigsäure,
   (R,S)-2-(4-Chlor-o-tolyloxy)propionsäure (Mecoprop),
   4-(2,4-Dichlorphenoxy)buttersäure (2,4-DB),
   (4-Chlor-o-tolyloxy)essigsäure (MCPA),
   4-(4-Chlor-o-tolyloxy)buttersäure,
   4-(4-Chlorphenoxy)buttersäure,
   3,6-Dichlor-2-methoxybenzoesäure (Dicamba),
   1-(Ethoxycarbonyl)ethyl-3,6-dichlor-2-methoxybenzoat (Lactidichlor-ethyl).

Als die die Kulturpflanzen-Verträglichkeit verbessernde Verbindung [Komponente (b')] sind Cloquintocetmexyl, Fenchlorazol-ethylester, Isoxadifen-ethyl, Mefenpyr-diethyl, Fenclorim, Cumyluron, S4-1 und S4-5 am meisten bevorzugt, wobei Cloquintocet-mexyl und Mefenpyr-diethyl besonders hervorgehoben seien.

Es wurde nun überraschend gefunden, dass die oben definierten Wirkstoffkombinationen aus Verbindungen der allgemeinen Formel (I) und Safenern (Antidots) aus der oben aufgeführten Gruppe (b') bei sehr guter Nutzpflanzen-Verträglichkeit eine besonders hohe herbizide Wirksamkeit aufweisen und in verschiedenen Kulturen, insbesondere in Getreide (vor allem Weizen), aber auch in Soja, Kartoffeln, Mais und Reis zur selektiven Unkrautbekämpfung verwendet werden können.

Dabei ist es als überraschend anzusehen, dass aus einer Vielzahl von bekannten Safenern oder Antidots, die befähigt sind, die schädigende Wirkung eines Herbizids auf die Kulturpflanzen zu antagonisieren, gerade die oben aufgeführten Verbindungen der Gruppe (b') geeignet sind, die schädigende Wirkung von Verbindungen der Formel (I) auf die Kulturpflanzen annähernd vollständig aufzuheben, ohne dabei die herbizide Wirksamkeit gegenüber den Unkräutern maßgeblich zu beeinträchtigen.

Hervorgehoben sei hierbei die besonders vorteilhafte Wirkung der besonders und am meisten bevorzugten Kombinationspartner aus der Gruppe (b'), insbesondere hinsichtlich der Schonung von Getreidepflanzen, wie z.B. Weizen, Gerste und Roggen, aber auch Mais und Reis, als Kulturpflanzen.

Die erfindungsgemäßen Verbindungen sind durch die Formel (I) allgemein definiert. Bevorzugte Substituenten bzw. Bereiche der in der oben und nachstehend erwähnten Formeln aufgeführten Reste werden im Folgenden erläutert:
- W: steht bevorzugt für Wasserstoff, Halogen, C₁-C₆-Alkyl, C₂-C₆-Alkenyl, C₂-C₆-Alkinyl, gegebenenfalls einfach bis zweifach durch C₁-C₂-Alkyl, C₁-C₂-Alkoxy, Fluor, Chlor, Trifluormethyl oder C₃-C₆-Cycloalkyl substituiertes C₃-C₆-Cycloalkyl, C₁-C₆-Alkoxy, C₁-C₄-Halogenalkyl, C₁-C₄-Halogenalkoxy oder Cyano,
- X: steht bevorzugt für Halogen, C₁-C₆-Alkyl, C₂-C₆-Alkenyl, C₂-C₆-Alkinyl, gegebenenfalls einfach bis zweifach durch C₁-C₂-Alkyl, C₁-C₂-Alkoxy, Fluor, Chlor, Trifluormethyl oder C₃-C₆-Cycloalkyl substituiertes C₃-C₆-Cycloalkyl, C₁-C₆-Halogenalkyl, C₁-C₆-Alkoxy, C₃-C₆-Alkenyloxy, C₁-C₆-Alkylthio, C₁-C₆-Alkylsulfinyl, C₁-C₆-Alkylsulfonyl, C₁-C₆-Halogenalkoxy, C₃-C₆-Halogenalkenyloxy, Nitro oder Cyano,
- Y und Z: stehen bevorzugt unabhängig voneinander für Wasserstoff, Halogen, C₁-C₆-Alkyl, C₂-C₆-Alkenyl, C₂-C₆-Alkinyl, gegebenenfalls einfach bis zweifach durch C₁-C₂-Alkyl, C₁-C₂-Alkoxy, Fluor, Chlor, Trifluormethyl oder C₃-C₆-Cycloalkyl substituiertes C₃-C₆-Cycloalkyl, C₁-C₆-Alkoxy, C₁-C₆-Halogenalkyl, C₁-C₆-Halogenalkoxy, Cyano, C₂-C₆-Alkenyl, C₂-C₆-Alkinyl oder für einen der (Het)-arylreste 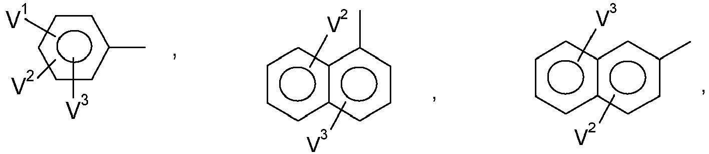 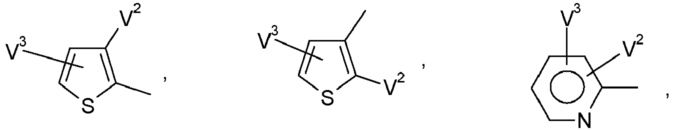 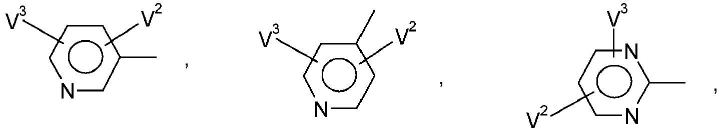 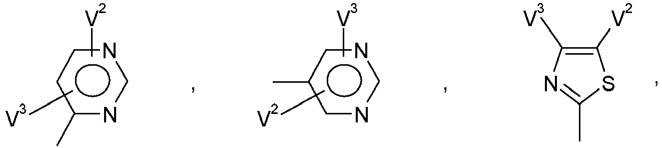 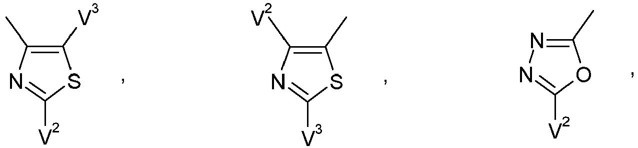 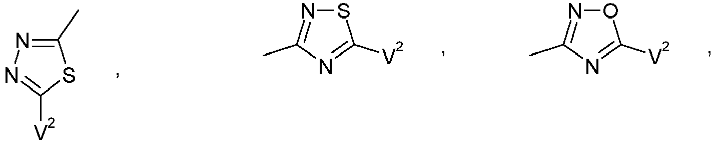 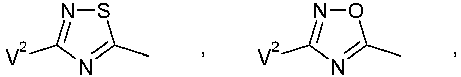
wobei im Falle von (Het)-aryl nur einer der Reste Y oder Z für (Het)-aryl stehen darf,
- V¹: steht bevorzugt für Wasserstoff, Halogen, C₁-C₁₂-Alkyl, C₁-C₆-Alkoxy, C₁-C₆-Alkylthio, C₁-C₆-Alkylsulfinyl, C₁-C₆-Alkylsulfonyl, C₁-C₄-Halogenalkyl, C₁-C₄-Halogenalkoxy, Nitro, Cyano oder jeweils gegebenenfalls einfach oder mehrfach durch Halogen, C₁-C₆-Alkyl, C₁-C₆-Alkoxy, C₁-C₄-Halogenalkyl, C₁-C₄-Halogenalkoxy, Nitro oder Cyano substituiertes Phenyl, Phenoxy, Phenoxy-C₁-C₄-alkyl, Phenyl-C₁-C₄-alkoxy, Phenylthio-C₁-C₄-alkyl oder Phenyl-C₁-C₄-alkylthio,
- V² und V³: stehen bevorzugt unabhängig voneinander für Wasserstoff, Halogen, C₁-C₆-Alkyl, C₁-C₆-Alkoxy, C₁-C₄-Halogenalkyl oder C₁-C₄-Halogenalkoxy,
- A: steht bevorzugt für Wasserstoff oder jeweils gegebenenfalls durch Halogen substituiertes C₁-C₁₂-Alkyl, C₃-C₈-Alkenyl, C₁-C₁₀-Alkoxy-C₁-C₈-alkyl, C₁-C₁₀-Alkylthio-C₁-C₆-alkyl, gegebenenfalls durch Halogen, C₁-C₆-Alkyl oder C₁-C₆-Alkoxy substituiertes C₃-C₈-Cycloalkyl, in welchem gegebenenfalls ein oder zwei nicht direkt benachbarte Ringglieder durch Sauerstoff und/oder Schwefel ersetzt sind oder für jeweils gegebenenfalls durch Halogen, C₁-C₆-Alkyl, C₁-C₆-Halogenalkyl, C₁-C₆-Alkoxy, C₁-C₆-Halogenalkoxy, Cyano oder Nitro substituiertes Phenyl, Naphthyl, Hetaryl mit 5 bis 6 Ringatomen (beispielsweise Furanyl, Pyridyl, Imidazolyl, Triazolyl, Pyrazolyl, Pyrimidyl, Thiazolyl oder Thienyl), Phenyl-C₁-C₆-alkyl oder Naphthyl-C₁-C₆-alkyl,
- B: steht bevorzugt für Wasserstoff, C₁-C₁₂-Alkyl oder C₁-C₈-Alkoxy-C₁-C₆-alkyl, mit der Maßgabe, dass A und B nur dann jeweils für Methyl stehen dürfen, wenn, im Fall dass W, X und Y jeweils für C₁-C₆-Alkyl und Z für Wasserstoff stehen, W und X jeweils für Methyl stehen oder W und X jeweils für Ethyl stehen,
oder
- A, B: und das Kohlenstoffatom an das sie gebunden sind, stehen bevorzugt für gesättigtes C₃-C₁₀-Cycloalkyl oder ungesättigtes C₅-C₁₀-Cycloalkyl, worin gegebenenfalls ein Ringglied durch Stickstoff, Sauerstoff oder Schwefel ersetzt ist und welche gegebenenfalls einfach oder zweifach durch C₁-C₈-Alkyl, C₁-C₈-Alkoxy, C₃-C₈-Alkenyloxy, C₁-C₆-Alkoxy-C₁-C₆-Alkyl, C₃-C₆-Cycloalkyl-C₁-C₂-alkoxy, C₃-C₁₀-Cycloalkyl, C₁-C₈-Halogenalkyl, C₂-C₆-Halogenalkoxy, C₁-C₆-Alkoxy-C₁-C₄-alkoxy substituiert sind, wobei die zuvor genannten Reste (ausgenommen Trifluormethyl) auch als N-Substituenten in Frage kommen, oder
- A, B: und das Kohlenstoffatom, an das sie gebunden sind, stehen bevorzugt für C₃-C₆-Cycloalkyl, welches durch eine gegebenenfalls ein oder zwei nicht direkt benachbarte Sauerstoff- und/oder Schwefelatome enthaltende gegebenenfalls durch C₁-C₄-Alkyl substituierte Alkylendiyl- oder durch eine Alkylendioxyl- oder durch eine Alkylendithioyl-Gruppe substituiert ist, die mit dem Kohlenstoffatom, an das sie gebunden ist, einen weiteren fünf- bis achtgliedrigen Ring bildet oder
- A, B: und das Kohlenstoffatom, an das sie gebunden sind, stehen bevorzugt für C₃-C₈-Cycloalkyl oder C₅-C₈-Cycloalkenyl, in welchen zwei Substituenten gemeinsam mit den Kohlenstoffatomen, an die sie gebunden sind, für jeweils gegebenenfalls durch C₁-C₆-Alkyl, C₁-C₆-Alkoxy oder Halogen substituiertes C₂-C₆-Alkandiyl, C₂-C₆-Alkendiyl oder C₄-C₆-Alkandiendiyl stehen, worin gegebenenfalls eine Methylengruppe durch Sauerstoff oder Schwefel ersetzt ist,
- G: steht bevorzugt für eine der Gruppen 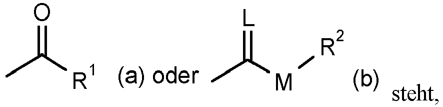 worin
L für Sauerstoff oder Schwefel steht,
M für Sauerstoff oder Schwefel steht,
R¹ steht bevorzugt für jeweils gegebenenfalls durch Halogen oder Cyano substituiertes C₁-C₂₀-Alkyl, C₂-C₂₀-Alkenyl, C₁-C₈-Alkoxy-C₁-C₈-alkyl, C₁-C₈-Alkylthio-C₁-C₈-alkyl oder Poly-C₁-C₈-alkoxy-C₁-C₈-alkyl oder für gegebenenfalls durch Halogen, C₁-C₆-Alkyl oder C₁-C₆-Alkoxy substituiertes C₃-C₈-Cycloalkyl, in welchem gegebenenfalls eine oder zwei nicht direkt benachbarte Methylengruppen durch Sauerstoff und/oder Schwefel ersetzt sind,
für gegebenenfalls durch Halogen, Cyano, Nitro, C₁-C₆-Alkyl, C₁-C₆-Alkoxy, C₁-C₆-Halogenalkyl, C₁-C₆-Halogenalkoxy, C₁-C₆-Alkylthio oder C₁-C₆-Alkylsulfonyl substituiertes Phenyl,
für gegebenenfalls durch Halogen, Nitro, Cyano, C₁-C₆-Alkyl, C₁-C₆-Alkoxy, C₁-C₆-Halogen-alkyl oder C₁-C₆-Halogenalkoxy substituiertes Phenyl-C₁-C₆-alkyl,
für gegebenenfalls durch Halogen oder C₁-C₆-Alkyl substituiertes 5- oder 6-gliedriges Hetaryl mit ein oder zwei Heteroatomen aus der Reihe Sauerstoff, Schwefel und Stickstoff,
für gegebenenfalls durch Halogen oder C₁-C₆-Alkyl substituiertes Phenoxy-C₁-C₆-alkyl oder
für gegebenenfalls durch Halogen, Amino oder C₁-C₆-Alkyl substituiertes 5- oder 6-gliedriges Hetaryloxy-C₁-C₆-alkyl mit ein oder zwei Heteroatomen aus der Reihe Sauerstoff, Schwefel und Stickstoff,
R² steht bevorzugt für jeweils gegebenenfalls durch Halogen oder Cyano substituiertes C₁-C₂₀-Alkyl, C₂-C₂₀-Alkenyl, C₁-C₈-Alkoxy-C₂-C₈-alkyl oder Poly-C₁-C₈-alkoxy-C₂-C₈-alkyl,
für gegebenenfalls durch Halogen, C₁-C₆-Alkyl oder C₁-C₆-Alkoxy substituiertes C₃-C₈-Cycloalkyl oder
für jeweils gegebenenfalls durch Halogen, Cyano, Nitro, C₁-C₆-Alkyl, C₁-C₆-Alkoxy, C₁-C₆-Halogenalkyl oder C₁-C₆-Halogenalkoxy substituiertes Phenyl oder Benzyl.

In den als bevorzugt genannten Restedefinitionen steht Halogen für Fluor, Chlor, Brom und Iod, insbesondere für Fluor, Chlor und Brom.
- W: steht besonders bevorzugt für Wasserstoff, Chlor, Brom, C₁-C₄-Alkyl, C₂-C₄-Alkenyl, C₂-C₄-Alkinyl, gegebenenfalls einfach durch Methyl, Ethyl, Methoxy, Fluor, Chlor, Trifluormethyl oder Cyclopropyl substituiertes C₃-C₆-Cycloalkyl, C₁-C₄-Alkoxy, C₁-C₂-Halogenalkyl oder C₁-C₂-Halogenalkoxy,
- X: steht besonders bevorzugt für Chlor, Brom, Iod, C₁-C₄-Alkyl, C₂-C₄-Alkenyl, C₂-C₄-Alkinyl, gegebenenfalls einfach durch Methyl, Ethyl, Methoxy, Fluor, Chlor, Trifluormethyl oder Cyclopropyl substituiertes C₃-C₆-Cycloalkyl, C₁-C₄-Alkoxy, C₁-C₄-Halogenalkyl, C₁-C₄-Halogenalkoxy oder Cyano,
- Y und Z: stehen besonders bevorzugt unabhängig voneinander für Wasserstoff, Fluor, Chlor, Brom, Iod, C₁-C₄-Alkyl, C₂-C₄-Alkenyl, C₂-C₄-Alkinyl, gegebenenfalls einfach durch Methyl, Ethyl, Methoxy, Fluor, Chlor, Trifluormethyl oder Cyclopropyl substituiertes C₃-C₆-Cycloalkyl, C₁-C₆-Alkoxy, C₁-C₄-Halogenalkyl, C₁-C₄-Halogenalkoxy, Cyano, C₂-C₄-Alkenyl, C₂-C₄-Alkinyl oder für einen der (Het)-arylreste, 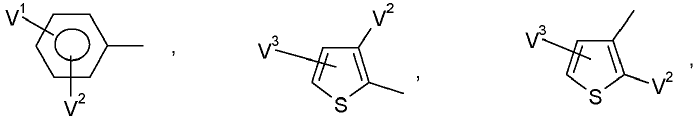 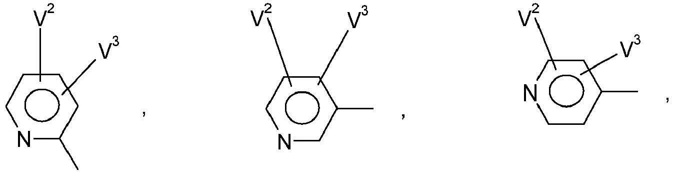 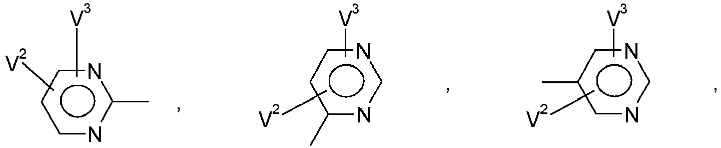 wobei im Falle von (Het)-aryl nur einer der Reste Y oder Z für (Het)-aryl stehen darf,
- V¹: steht besonders bevorzugt für Wasserstoff, Fluor, Chlor, Brom, C₁-C₆-Alkyl, C₁-C₄-Alkoxy, C₁-C₂- Halogenalkyl, C₁-C₂-Halogenalkoxy, Nitro, Cyano oder gegebenenfalls einfach bis zweifach durch Fluor, Chlor, Brom, C₁-C₄-Alkyl, C₁-C₄-Alkoxy, C₁-C₂-Halogenalkyl, C₁-C₂-Halogenalkoxy, Nitro oder Cyano substituiertes Phenyl,
- V² und V³: stehen besonders bevorzugt unabhängig voneinander für Wasserstoff, Fluor, Chlor, Brom, C₁-C₄-Alkyl, C₁-C₄-Alkoxy, C₁-C₂-Halogenalkyl oder C₁-C₂-Halogenalkoxy,
- A: steht besonders bevorzugt für Wasserstoff, jeweils gegebenenfalls einfach bis dreifach durch Fluor oder Chlor substituiertes C₁-C₆-Alkyl, C₁-C₄-Alkoxy-C₁-C₂-alkyl, gegebenenfalls einfach bis zweifach durch C₁-C₂-Alkyl oder C₁-C₂-Alkoxy substituiertes und gegebenenfalls durch ein Sauerstoffatom unterbrochenes C₃-C₆-Cycloalkyl oder jeweils gegebenenfalls einfach bis zweifach durch Fluor, Chlor, Brom, C₁-C₄-Alkyl, C₁-C₂-Halogenalkyl, C₁-C₄-Alkoxy, C₁-C₂-Halogenalkoxy, Cyano oder Nitro substituiertes Phenyl, Pyridyl oder Benzyl,
- B: steht besonders bevorzugt für Wasserstoff, C₁-C₄-Alkyl oder C₁-C₂-Alkoxyl-C₁-C₂-alkyl, mit der Maßgabe, dass A und B nur dann jeweils für Methyl stehen dürfen, wenn, im Fall dass W, X und Y jeweils für C₁-C₄-Alkyl und Z für Wasserstoff stehen, W und X jeweils für Methyl stehen oder W und X jeweils für Ethyl stehen,
oder
- A, B: und das Kohlenstoffatom an das sie gebunden sind, stehen besonders bevorzugt für gesättigtes oder ungesättigtes C₃-C₇-Cycloalkyl, worin gegebenenfalls ein Ringglied durch Stickstoff, Sauerstoff oder Schwefel ersetzt ist und welches gegebenenfalls einfach bis zweifach durch C₁-C₆-Alkyl, C₁-C₄-Alkoxy-C₁-C₂-alkyl, Trifluormethyl, C₁-C₆-Alkoxy, C₃-C₆-Alkenyloxy, Trifluorethoxy, C₁-C₃-Alkoxy-C₁-C₃-alkoxy oder C₃-C₆-Cycloalkylmethoxy substituiert ist, wobei die zuvor genannten Reste (ausgenommen Trifluormethyl) auch als N-Substituenten in Frage kommen, oder
- A, B: und das Kohlenstoffatom, an das sie gebunden sind, stehen besonders bevorzugt für C₅-C₆-Cycloalkyl, welches durch eine gegebenenfalls ein oder zwei nicht direkt benachbarte Sauerstoff- oder Schwefelatome enthaltende gegebenenfalls durch Methyl oder Ethyl substituierte Alkylendiyl- oder durch eine Alkylendioxyl- oder durch eine Alkylendithiol-Gruppe substituiert ist, die mit dem Kohlenstoffatom, an das sie gebunden ist, einen weiteren fünf- oder sechsgliedrigen Ring bildet, oder
- A, B: und das Kohlenstoffatom, an das sie gebunden sind, stehen besonders bevorzugt für C₃-C₆-Cycloalkyl oder C₅-C₆-Cycloalkenyl, in welchen zwei Substituenten gemeinsam mit den Kohlenstoffatomen, an die sie gebunden sind, für jeweils gegebenenfalls durch C₁-C₂-Alkyl oder C₁-C₂-Alkoxy substituiertes C₂-C₄-Alkandiyl, C₂-C₄-Alkendiyl oder Butadiendiyl stehen,
- G: steht besonders bevorzugt für eine der Gruppen 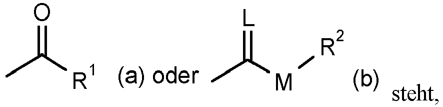
worin
- L: für Sauerstoff oder Schwefel steht,
- M: für Sauerstoff oder Schwefel steht,
- R¹: steht besonders bevorzugt für jeweils gegebenenfalls einfach bis dreifach durch Fluor oder Chlor substituiertes C₁-C₁₆-Alkyl, C₂-C₁₆-Alkenyl, C₁-C₆-Alkoxy-C₁-C₄-alkyl, C₁-C₆-Alkylthio-C₁-C₄-alkyl oder Poly-C₁-C₆-alkoxy-C₁-C₄-alkyl oder für gegebenenfalls einfach bis zweifach durch Fluor, Chlor, C₁-C₅-Alkyl oder C₁-C₅-Alkoxy substituiertes C₃-C₇-Cycloalkyl, in welchem gegebenenfalls eine oder zwei nicht direkt benachbarte Methylengruppen durch Sauerstoff und/oder Schwefel ersetzt sind,
für gegebenenfalls einfach bis dreifach durch Fluor, Chlor, Brom, Cyano, Nitro, C₁-C₄-Alkyl, C₁-C₄-Alkoxy, C₁-C₃-Halogenalkyl, C₁-C₃-Halogenalkoxy, C₁-C₄-Alkylthio oder C₁-C₄-Alkyl-sulfonyl substituiertes Phenyl,
für gegebenenfalls einfach bis zweifach durch Fluor, Chlor, Brom, C₁-C₄-Alkyl, C₁-C₄-Alkoxy, C₁-C₃-Halogenalkyl oder C₁-C₃-Halogenalkoxy substituiertes Phenyl-C₁-C₄-alkyl,
für jeweils gegebenenfalls einfach bis zweifach durch Fluor, Chlor, Brom oder C₁-C₄-Alkyl substituiertes Pyrazolyl, Thiazolyl, Pyridyl, Pyrimidyl, Furanyl oder Thienyl,
für gegebenenfalls einfach bis zweifach durch Fluor, Chlor, Brom oder C₁-C₄-Alkyl substituiertes Phenoxy-C₁-C₅-alkyl oder
für jeweils gegebenenfalls einfach bis zweifach durch Fluor, Chlor, Brom, Amino oder C₁-C₄-Alkyl substituiertes Pyridyloxy-C₁-C₅-alkyl, Pyrimidyloxy-C₁-C₅-alkyl oder Thiazolyloxy-C₁-C₅-alkyl,
- R²: steht besonders bevorzugt für jeweils gegebenenfalls einfach bis dreifach durch Fluor oder Chlor substituiertes C₁-C₁₆-Alkyl, C₂-C₁₆-Alkenyl, C₁-C₆-Alkoxy-C₂-C₆-alkyl oder Poly-C₁-C₆-alkoxy-C₂-C₆-alkyl,
für gegebenenfalls einfach bis zweifach durch Fluor, Chlor, C₁-C₄-Alkyl oder C₁-C₄-Alkoxy substituiertes C₃-C₇-Cycloalkyl oder
für jeweils gegebenenfalls einfach bis dreifach durch Fluor, Chlor, Brom, Cyano, Nitro, C₁-C₄-Alkyl, C₁-C₃-Alkoxy, C₁-C₃-Halogenalkyl oder C₁-C₃-Halogenalkoxy substituiertes Phenyl oder Benzyl.

In den als besonders bevorzugt genannten Restedefinitionen steht Halogen für Fluor, Chlor und Brom, insbesondere für Fluor und Chlor.
- W: steht ganz besonders bevorzugt für Wasserstoff, Chlor, Brom, Methyl, Ethyl, Propyl, Vinyl, Ethinyl, Propinyl, Cyclopropyl, Methoxy, Ethoxy oder Trifluormethyl,
- X: steht ganz besonders bevorzugt für Chlor, Brom, Methyl, Ethyl, Propyl, iso-Propyl, Vinyl, Ethinyl, Propinyl, Cyclopropyl, Methoxy, Ethoxy, Trifluormethyl, Difluormethoxy, Trifluormethoxy oder Cyano,
- Y und Z: stehen ganz besonders bevorzugt unabhängig voneinander für Wasserstoff, Fluor, Chlor, Brom, Iod, Methyl, Ethyl, Vinyl, Ethinyl, Propinyl, Cyclopropyl, Methoxy, Trifluormethyl, Trifluormethoxy, Cyano oder einen Phenylrest, 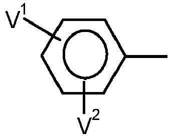 wobei im Falle von Phenyl nur einer der Reste Y oder Z für Phenyl stehen darf,
- V¹: steht ganz besonders bevorzugt für Wasserstoff, Fluor oder Chlor,
- V²: steht ganz besonders bevorzugt für Wasserstoff, Fluor, Chlor, Methyl, Ethyl, n-Propyl, iso-Propyl, Methoxy, Ethoxy oder Trifluormethyl,
- A: steht ganz besonders bevorzugt für Wasserstoff, jeweils gegebenenfalls einfach bis dreifach durch Fluor substituiertes C₁-C₄-Alkyl oder C₁-C₂-Alkoxy-C₁-C₂-alkyl, für Cyclopropyl, Cyclopentyl oder Cyclohexyl,
- B: steht ganz besonders bevorzugt für Wasserstoff, Methyl oder Ethyl, mit der Maßgabe, dass A und B nur dann jeweils für Methyl stehen dürfen, wenn, im Fall dass W, X und Y jeweils für Methyl oder Ethyl und Z für Wasserstoff stehen, W und X jeweils für Methyl stehen oder W und X jeweils für Ethyl stehen,
oder
- A, B: und das Kohlenstoffatom an das sie gebunden sind, stehen ganz besonders bevorzugt für gesättigtes C₅-C₆-Cycloalkyl, in welchem gegebenenfalls ein Ringglied durch Stickstoff, Sauerstoff oder Schwefel ersetzt ist und welches gegebenenfalls einfach oder zweifach durch Methyl, Ethyl, Methoxymethyl, Ethoxymethyl, Methoxyethyl, Ethoxyethyl, Trifluormethyl, Methoxy, Ethoxy, Propoxy, Butoxy, Methoxyethoxy, Ethoxyethoxy, Allyloxy, Trifluorethoxy oder Cyclopropylmethoxy substituiert ist, wobei die zuvor genannten Reste (ausgenommen Trifluormethyl) auch als N-Substituenten in Frage kommen, oder
- A, B: und das Kohlenstoffatom, an das sie gebunden sind, stehen ganz besonders bevorzugt für C₆-Cycloalkyl, welches gegebenenfalls durch eine gegebenenfalls durch ein Sauerstoffatom unterbrochene Alkylidendiyl-Gruppe oder durch eine mit zwei nicht direkt benachbarten Sauerstoffatomen enthaltende Alkylendioxy-Gruppe substituiert ist, wobei ein weiter 5- oder 6-Ring gebildet wird (der gegebenenfalls einfach oder zweifach durch Methyl substituiert sein kann), oder
- A, B: und das Kohlenstoffatom, an das sie gebunden sind, stehen ganz besonders bevorzugt für C₅-C₆-Cycloalkyl oder C₅-C₆-Cycloalkenyl, worin zwei Substituenten gemeinsam mit den Kohlenstoffatomen, an die sie gebunden sind, für C₂-C₄-Alkandiyl oder C₂-C₄-Alkendiyl oder Butadiendiyl stehen,
- G: steht ganz besonders bevorzugt für eine der Gruppen 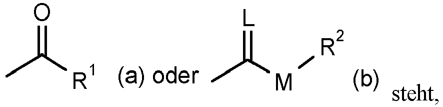
worin
- L: für Sauerstoff oder Schwefel steht,
- M: für Sauerstoff oder Schwefel steht,
- R¹: steht ganz besonders bevorzugt für jeweils gegebenenfalls einfach bis dreifach durch Fluor oder Chlor substituiertes C₁-C₁₀-Alkyl, C₂-C₁₀-Alkenyl, C₁-C₄-Alkoxy-C₁-C₂-alkyl, C₁-C₄-Alkylthio-C₁-C₂-alkyl oder für gegebenenfalls einfach durch Fluor, Chlor, Methyl, Ethyl oder Methoxy substituiertes C₃-C₆-Cycloalkyl,
für gegebenenfalls einfach bis zweifach durch Fluor, Chlor, Brom, Cyano, Nitro, Methyl, Ethyl, n-Propyl, i-Propyl, Methoxy, Ethoxy, Trifluormethyl oder Trifluormethoxy substituiertes Phenyl,
für jeweils gegebenenfalls einfach durch Chlor, Brom oder Methyl substituiertes Furanyl, Thienyl oder Pyridyl,
- R²: steht ganz besonders bevorzugt für jeweils gegebenenfalls einfach bis dreifach durch Fluor oder Chlor substituiertes C₁-C₁₀-Alkyl, C₂-C₁₀-Alkenyl oder C₁-C₄-Alkoxy-C₂-C₄-alkyl.
für Cyclopentyl oder Cyclohexyl
oder für jeweils gegebenenfalls einfach bis zweifach durch Fluor, Chlor, Cyano, Nitro, Methyl, Ethyl, Methoxy, Trifluormethyl oder Trifluormethoxy substituiertes Phenyl oder Benzyl.
- W: steht insbesonders bevorzugt für Methyl, Ethyl oder Propyl,
- X: steht insbesonders bevorzugt für Methyl, Ethyl, Chlor, Brom oder Methoxy,
- Y: steht insbesonders bevorzugt für Methyl, Chlor, Jod oder Brom,
- Z: steht insbesonders bevorzugt für Wasserstoff,
- A: steht insbesonders bevorzugt für Methyl,
- B: steht insbesonders bevorzugt für Methyl,
mit der Maßgabe, dass A und B nur dann jeweils für Methyl stehen dürfen, wenn, im Fall dass W, Xund Y jeweils für Methyl oder Ethyl und Z für Wasserstoff stehen, W und Xjeweils für Methyl stehen oder Wund Xjeweils für Ethyl stehen,
oder
- A, B: und das Kohlenstoff an das sie gebunden sind, stehen insbesonders bevorzugt für C₆-Cycloalkyl, welches gegebenenfalls durch Methyl, Methoxy oder Methoxymethyl substituiert ist, wobei der Substituent in der 4-Stellung des Cyclus stehen kann,
- G: steht insbesonders bevorzugt für eine der Gruppen 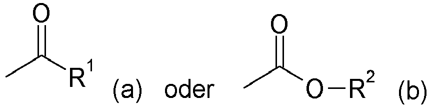
worin
- R¹: insbesonders bevorzugt für Methyl, Ethyl, Propyl, iso-Propyl, Butyl, iso-Butyl, sec-Butyl oder tert. Butyl (hervorgehoben für iso-Propyl oder tert.-Butyl) steht,
- R²: insbesonders bevorzugt für Methyl, Ethyl, Propyl, iso-Propyl, Butyl, iso-Butyl, sec.-Butyl oder tert.-Butyl (hervorgehoben für Ethyl) steht.
- W: steht ebenfalls insbesonders bevorzugt für Wasserstoff,
- X: steht ebenfalls insbesonders bevorzugt für Methyl,
- Y: steht ebenfalls insbesonders bevorzugt für Wasserstoff,
- Z: steht ebenfalls insbesonders bevorzugt für Methyl,
- A, B: und das Kohlenstoff an das sie gebunden sind, stehen ebenfalls insbesonders bevorzugt für C₆-Cycloalkyl, welches gegebenenfalls durch Methoxy oder Methoxymethyl substituiert ist, wobei der Substituent sowohl in der 3- als auch in der 4-Stellung des Cyclus stehen kann,
- G: steht ebenfalls insbesonders bevorzugt für eine der Gruppen 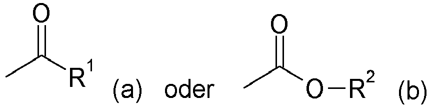
worin
- R¹: ebenfalls insbesonders bevorzugt für Methyl, Ethyl, Propyl, iso-Propyl, Butyl, iso-Butyl, sec-Butyl oder tert. Butyl (besonders hervorgehoben für C(CH₃)₂C₂H₅) steht,
- R²: ebenfalls insbesonders bevorzugt für Methyl, Ethyl, Propyl, iso-Propyl, Butyl, iso-Butyl, sec.-Butyl oder tert.-Butyl (hervorgehoben für Ethyl) steht.

Die oben aufgeführten allgemeinen oder in Vorzugsbereichen aufgeführten Restedefinitionen bzw. Erläuterungen können untereinander, also auch zwischen den jeweiligen Bereichen und Vorzugsbereichen beliebig kombiniert werden. Sie gelten für die Endprodukte sowie für die Vor- und Zwischenprodukte entsprechend.

Erfindungsgemäß bevorzugt werden die Verbindungen der Formel (I), in welchen eine Kombination der vorstehend als bevorzugt (vorzugsweise) aufgeführten Bedeutungen vorliegt.

Erfindungsgemäß besonders bevorzugt werden die Verbindungen der Formel (I), in welchen eine Kombination der vorstehend als besonders bevorzugt aufgeführten Bedeutungen vorliegt.

Erfindungsgemäß ganz besonders bevorzugt werden die Verbindungen der Formel (I), in welchen eine Kombination der vorstehend als ganz besonders bevorzugt aufgeführten Bedeutungen vorliegt.

Erfindungsgemäß insbesonders bevorzugt werden die Verbindungen der Formel (I), in welchen eine Kombination der vorstehend als insbesonders bevorzugt aufgeführten Bedeutungen vorliegt. Hervorgehoben sind die Verbindungen der Formel (I) in welcher A und B gemeinsam mit dem Kohlenstoffatom an das sie gebunden sind für einen gesättigten oder ungesättigten, gegebenenfalls mindestens ein Heteroatom enthaltenden unsubstituierten oder substituierten Cyclus stehen, bzw. die entsprechenden bevorzugten, besonders bevorzugten, ganz besonders bevorzugten und insbesonders bevorzugten Definitionen.

Gesättigte oder ungesättigte Kohlenwasserstoffreste wie Alkyl oder Alkenyl können, auch in Verbindung mit Heteroatomen, wie z.B. in Alkoxy, soweit möglich, jeweils geradkettig oder verzweigt sein.

Gegebenenfalls substituierte Reste können, sofern nichts anderes angegeben ist, einfach oder mehrfach substituiert sein, wobei bei Mehrfachsubstitutionen die Substituenten gleich oder verschieden sein können. Im Einzelnen seien außer den bei den Beispielen genannten Verbindungen die folgenden Verbindungen der Formel (I) mit G = COCH₃ genannt:

**Tabelle 1**

| | | | | | |
|---|---|---|---|---|---|
| 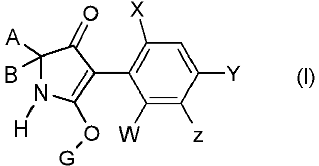 | | | | | |

| **A** | **B** | **X** | **W** | **Y** | **Z** |
|---|---|---|---|---|---|
| CH₃ | CH₃ | CH₃ | H | H | H |
| CH₃ | CH₃ | Br | H | H | H |
| CH₃ | CH₃ | Cl | H | H | H |
| CH₃ | CH₃ | CF₃ | H | H | H |
| CH₃ | CH₃ | OCH₃ | H | H | H |
| CH₃ | CH₃ | Br | H | Cl | H |
| CH₃ | CH₃ | Cl | H | Br | H |
| CH₃ | CH₃ | Cl | H | Cl | H |
| CH₃ | CH₃ | Cl | H | CH₃ | H |
| CH₃ | CH₃ | CH₃ | H | Cl | H |
| CH₃ | CH₃ | Cl | Cl | H | H |
| CH₃ | CH₃ | Cl | OCH₃ | H | H |
| CH₃ | CH₃ | Cl | CH₃ | H | H |
| CH₃ | CH₃ | Cl | OC₂H₅ | H | H |
| CH₃ | CH₃ | OCH₃ | OCH₃ | H | H |
| CH₃ | CH₃ | CH₃ | CH₃ | H | H |
| CH₃ | CH₃ | C₂H₅ | CH₃ | H | H |
| CH₃ | CH₃ | C₂H₅ | C₂H₅ | H | H |
| CH3 | CH₃ | Br | CH₃ | Br | H |
| CH3 | CH₃ | Cl | CH₃ | Cl | H |
| CH3 | CH₃ | CH₃ | Br | CH₃ | H |
| CH3 | CH₃ | CH₃ | Cl | CH₃ | H |
| CH3 | CH₃ | OCH₃ | CH₃ | CH₃ | H |
| CH3 | CH₃ | OC₂H₅ | CH₃ | CH₃ | H |
| CH3 | CH₃ | 0C₃H₇ | CH₃ | CH₃ | H |
| CH3 | CH₃ | CH₃ | CH₃ | CH₃ | H |
| CH3 | CH₃ | Br | Br | CH₃ | H |
| CH3 | CH₃ | Cl | Cl | CH₃ | H |
| CH₃ | CH₃ | CH₃ | CH₃ | Br | H |
| CH₃ | CH₃ | OCH₃ | C₂H₅ | CH₃ | H |
| CH₃ | CH₃ | OC₂H₅ | C₂H₅ | CH₃ | H |
| CH₃ | CH₃ | CH₃ | CH₃ | OCH₃ | H |
| CH₃ | CH₃ | Br | Cl | CH₃ | H |
| CH₃ | CH₃ | Br | CH₃ | Cl | H |
| CH₃ | CH₃ | Cl | CH₃ | Br | H |
| CH₃ | CH₃ | CH₃ | CH₃ | Cl | H |
| CH₃ | CH₃ | C₂H₅ | CH₃ | CH₃ | H (Vergleichsbeispiel) |
| CH₃ | CH₃ | C₂H₅ | C₂H₅ | CH₃ | H |
| CH₃ | CH₃ | C₂H₅ | CH₃ | C₂H₅ | H (Vergleichsbeispiel) |
| CH₃ | CH₃ | C₂H₅ | C₂H₅ | C₂H₅ | H |
| CH₃ | CH₃ | C₂H₅ | CH₃ | Cl | H |
| CH₃ | CH₃ | C₂H₅ | C₂H₅ | Cl | H |
| CH₃ | CH₃ | C₂H₅ | CH₃ | Br | H |
| CH₃ | CH₃ | C₂H₅ | C₂H₅ | Br | H |
| CH₃ | CH₃ | C₂H₅ | Cl | CH₃ | H |
| CH₃ | CH₃ | C₂H₅ | Br | CH₃ | H |
| CH₃ | CH₃ | C₂H₅ | Cl | Cl | H |
| CH₃ | CH₃ | C₂H₅ | Br | Br | H |
| CH₃ | CH₃ | C₂H₅ | Cl | Br | H |
| CH₃ | CH₃ | C₂H₅ | Br | Cl | H |
| CH₃ | CH₃ | OCH₃ | CH₃ | Cl | H |
| CH₃ | CH₃ | OCH₃ | C₂H₅ | Cl | H |
| CH₃ | CH₃ | OC₂H₅ | CH₃ | Cl | H |
| CH₃ | CH₃ | OC₂H₅ | C₂H₅ | Cl | H |
| CH₃ | CH₃ | Cl | OCH₃ | CH₃ | H |
| CH₃ | CH₃ | Cl | OC₂H₅ | CH₃ | H |
| CH₃ | CH₃ | CH₃ | CH₃ | Cl | H |
| CH₃ | CH₃ | Cl | H | Cl | Cl |
| CH₃ | CH₃ | CH₃ | H | CH₃ | CH₃ |
| CH₃ | CH₃ | CH₃ | H | Cl | CH₃ |
| CH₃ | CH₃ | Br | H | Cl | CH₃ |
| CH₃ | CH₃ | Br | H | CH₃ | CH₃ |
| CH₃ | CH₃ | Cl | H | Br | CH₃ |
| CH₃ | CH₃ | Cl | H | Cl | CH₃ |
| CH₃ | CH₃ | CH₃ | H | Br | CH₃ |
| CH₃ | CH₃ | Cl | H | CH₃ | Cl |
| CH₃ | CH₃ | CH₃ | H | H | CH₃ |
| CH₃ | CH₃ | Cl | H | H | CH₃ |
| CH₃ | CH₃ | Br | H | H | CH₃ |
| CH₃ | CH₃ | CH₃ | H | H | Cl |
| CH₃ | CH₃ | CH₃ | H | H | Br |
| CH₃ | CH₃ | CH₃ | CH₃ | CH₃ | CH₃ |
| CH₃ | CH₃ | CH₃ | CH₃ | CH₃ | F |
| CH₃ | CH₃ | CH₃ | CH₃ | CH₃ | Cl |
| CH₃ | CH₃ | CH₃ | CH₃ | CH₃ | Br |
| CH₃ | CH₃ | CH₃ | CH₃ | H | Cl |
| CH₃ | CH₃ | CH₃ | CH₃ | H | Br |
| CH₃ | CH₃ | Cl | Cl | H | Br |
| CH₃ | CH₃ | CH₃ | CH₃ | 4-Cl-C₆H4 | H |
| CH₃ | CH₃ | C₂H₅ | CH₃ | 4-Cl-C₆H₄ | H |
| CH₃ | CH₃ | C₂H₅ | C₂H₅ | 4-Cl-C₆H₄ | H |
| CH₃ | CH₃ | Cl | CH₃ | 4-Cl-C₆H₄ | H |
| CH₃ | CH₃ | Cl | C₂H₅ | 4-Cl-C₆H₄ | H |
| CH₃ | CH₃ | CH₃ | H | H | 4-Cl-C₆H₄ |
| CH₃ | CH₃ | CH₃ | CH₃ | H | 4-Cl-C₆H₄ |
| CH₃ | CH₃ | CH₃ | H | CH₃ | 4-Cl-C₆H₄ |
| CH₃ | CH₃ | CH₃ | CH₃ | CH₃ | 4-Cl-C₆H₄ |
| CH₃ | CH₃ | Cl | H | H | 4-Cl-C₆H₄ |
| CH₃ | CH₃ | J | H | H | H |
| CH₃ | CH₃ | J | H | CH₃ | H |
| CH₃ | CH₃ | J | CH₃ | H | H |
| CH₃ | CH₃ | J | C₂H₅ | H | H |
| CH₃ | CH₃ | CH₃ | H | H | J |
| CH₃ | CH₃ | CH₃ | H | CH₃ | J |
| CH₃ | CH₃ | J | CH₃ | CH₃ | H |
| CH₃ | CH₃ | J | C₂H₅ | CH₃ | H |
| CH₃ | CH₃ | J | CH₃ | Cl | H |
| CH₃ | CH₃ | J | C₂H₅ | Cl | H |
| CH₃ | CH₃ | J | Cl | CH₃ | H |
| CH₃ | CH₃ | J | H | CH₃ | CH₃ |
| CH₃ | CH₃ | CH₃ | H | J | H |
| CH₃ | CH₃ | C₂H₅ | H | J | H |
| CH₃ | CH₃ | CH₃ | CH₃ | J | H |
| CH₃ | CH₃ | C₂H₅ | CH₃ | J | H |
| CH₃ | CH₃ | C₂H₅ | C₂H₅ | J | H |
| CH₃ | CH₃ | Cl | CH₃ | J | H |
| CH₃ | CH₃ | Cl | C₂H₅ | J | H |
| CH₃ | CH₃ | CH₃ | H | J | CH₃ |
| CH₃ | CH₃ | CH₃ | CH₃ | H | J |
| CH₃ | CH₃ | J | H | H | CH₃ |
| CH₃ | CH₃ | C₂H_{5.} | H | H | H |
| CH₃ | CH₃ | 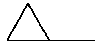 | H | H | H |
| CH₃ | CH₃ | 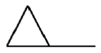 | CH₃ | H | H |
| CH₃ | CH₃ | 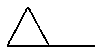 | H | CH₃ | H |
| CH₃ | CH₃ | 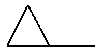 | C₂H₅ | H | H |
| CH₃ | CH₃ | 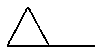 | CH₃ | CH₃ | H |
| CH₃ | CH₃ | 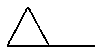 | C₂H₅ | CH₃ | H |
| CH₃ | CH₃ | 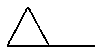 | CH₃ | Cl | H |
| CH₃ | CH₃ | 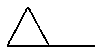 | C₂H₅ | Cl | H |
| CH₃ | CH₃ | 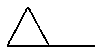 | Cl | CH₃ | H |
| CH₃ | CH₃ | CH₃ | H | 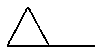 | H |
| CH₃ | CH₃ | C₂H₅ | H | 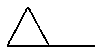 | H |
| CH₃ | CH₃ | CH₃ | CH₃ | 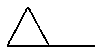 | H |
| CH₃ | CH₃ | C₂H₅ | CH₃ | 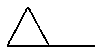 | H |
| CH₃ | CH₃ | C₂H₅ | C₂H₅ | 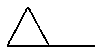 | H |
| CH₃ | CH₃ | Cl | CH₃ | 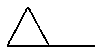 | H |
| CH₃ | CH₃ | Cl | C₂H₅ | 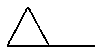 | H |

Weiterhin seien außer den bei den Beispielen genannten Verbindungen die folgenden Verbindungen der Formel (I) mit G = COCH₃ und

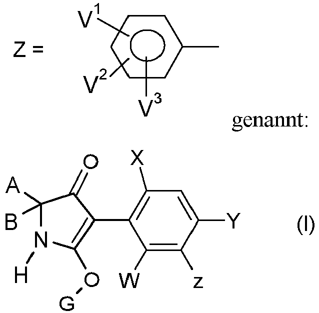

**Tabelle 2**

| **A** | **B** | **W** | **X** | **Y** | **V¹** | **V²** | **V³** |
|---|---|---|---|---|---|---|---|
| CH₃ | CH₃ | H | Cl | H | 2-F | H | H |
| CH₃ | CH₃ | H | Cl | H | 3-F | H | H |
| CH₃ | CH₃ | H | Cl | H | 4-F | H | H |
| CH₃ | CH₃ | H | Cl | H | 2-F | 4-F | H |
| CH₃ | CH₃ | H | Cl | H | 2-F | 4-Cl | H |
| CH₃ | CH₃ | H | Cl | H | 2-F | 4-CH₃ | H |
| CH₃ | CH₃ | H | Cl | H | 2-F | 4-OCH₃ | H |
| CH₃ | CH₃ | H | Cl | H | 3-F | 4-F | H |
| CH₃ | CH₃ | H | Cl | H | 3-F | 4-Cl | H |
| CH₃ | CH₃ | H | Cl | H | 3-F | 4-CH₃ | H |
| CH₃ | CH₃ | H | Cl | H | 3-F | 4-OCH₃ | H |
| CH₃ | CH₃ | H | Cl | H | 4-F | 3-Cl | H |
| CH₃ | CH₃ | H | Cl | H | 4-F | 3-CH₃ | H |
| CH₃ | CH₃ | H | Cl | H | 4-F | 3-OCH₃ | H |
| CH₃ | CH₃ | H | Cl | H | 2-F | 4-F | 5-F |
| CH₃ | CH₃ | H | Cl | H | 2-F | 4-F | 6-F |
| CH₃ | CH₃ | H | Cl | H | 2-F | 4-Cl | 5-F |
| CH₃ | CH₃ | H | Cl | H | 2-F | 5-Cl | 4-F |
| CH₃ | CH₃ | H | Cl | H | 3-F | 4-F | 5-F |
| CH₃ | CH₃ | H | Cl | H | 3-Cl | 4-Cl | H |
| CH₃ | CH₃ | H | Cl | H | 4-CF₃ | 3-F | H |
| CH₃ | CH₃ | H | Cl | H | 4-CN | H | H |
| CH₃ | CH₃ | H | Cl | H | 3-CF₃ | 4-F | H |
| CH₃ | CH₃ | H | CH₃ | H | 2-F | H | H |
| CH₃ | CH₃ | H | CH₃ | H | 3-F | H | H |
| CH₃ | CH₃ | H | CH₃ | H | 4-F | H | H |
| CH₃ | CH₃ | H | CH₃ | H | 2-F | 4-F | H |
| CH₃ | CH₃ | H | CH₃ | H | 2-F | 4-Cl | H |
| CH₃ | CH₃ | H | CH₃ | H | 2-F | 4-CH₃ | H |
| CH₃ | CH₃ | H | CH₃ | H | 2-F | 4-OCH₃ | H |
| CH₃ | CH₃ | H | CH₃ | H | 3-F | 4-F | H |
| CH₃ | CH₃ | H | CH₃ | H | 3-F | 4-Cl | H |
| CH₃ | CH₃ | H | CH₃ | H | 3-F | 4-CH₃ | H |
| CH₃ | CH₃ | H | CH₃ | H | 3-F | 4-OCH₃ | H |
| CH₃ | CH₃ | H | CH₃ | H | 4-F | 3-Cl | H |
| CH₃ | CH₃ | H | CH₃ | H | 4-F | 3-CH₃ | H |
| CH₃ | CH₃ | H | CH₃ | H | 4-F | 3-OCH₃ | H |
| CH₃ | CH₃ | H | CH₃ | H | 2-F | 4-F | 5-F |
| CH₃ | CH₃ | H | CH₃ | H | 2-F | 4-F | 6-F |
| CH₃ | CH₃ | H | CH₃ | H | 2-F | 4-Cl | 5-F |
| CH₃ | CH₃ | H | CH₃ | H | 2-F | 5-Cl | 4-F |
| CH₃ | CH₃ | H | CH₃ | H | 3-F | 4-F | 5-F |
| CH₃ | CH₃ | H | CH₃ | H | 3-Cl | 4-Cl | H |
| CH₃ | CH₃ | H | CH₃ | H | 4-CF₃ | 3-F | H |
| CH₃ | CH₃ | H | CH₃ | H | 4-CN | H | H |
| CH₃ | CH₃ | H | CH₃ | H | 3-CF₃ | 4-F | H |
| CH₃ | CH₃ | CH₃ | CH₃ | H | 2-F | H | H |
| CH₃ | CH₃ | CH₃ | CH₃ | H | 3-F | H | H |
| CH₃ | CH₃ | CH₃ | CH₃ | H | 4-F | H | H |
| CH₃ | CH₃ | CH₃ | CH₃ | H | 2-F | 4-F | H |
| CH₃ | CH₃ | CH₃ | CH₃ | H | 2-F | 4-Cl | H |
| CH₃ | CH₃ | CH₃ | CH₃ | H | 2-F | 4-CH₃ | H |
| CH₃ | CH₃ | CH₃ | CH₃ | H | 2-F | 4-OCH₃ | H |
| CH₃ | CH₃ | CH₃ | CH₃ | H | 3-F | 4-F | H |
| CH₃ | CH₃ | CH₃ | CH₃ | H | 3-F | 4-Cl | H |
| CH₃ | CH₃ | CH₃ | CH₃ | H | 3-F | 4-CH₃ | H |
| CH₃ | CH₃ | CH₃ | CH₃ | H | 3-F | 4-OCH₃ | H |
| CH₃ | CH₃ | CH₃ | CH₃ | H | 4-F | 3-Cl | H |
| CH₃ | CH₃ | CH₃ | CH₃ | H | 4-F | 3-CH₃ | H |
| CH₃ | CH₃ | CH₃ | CH₃ | H | 4-F | 3-OCH₃ | H |
| CH₃ | CH₃ | CH₃ | CH₃ | H | 2-F | 4-F | 5-F |
| CH₃ | CH₃ | CH₃ | CH₃ | H | 2-F | 4-F | 6-F |
| CH₃ | CH₃ | CH₃ | CH₃ | H | 2-F | 4-Cl | 5-F |
| CH₃ | CH₃ | CH₃ | CH₃ | H | 2-F | 5-Cl | 4-F |
| CH₃ | CH₃ | CH₃ | CH₃ | H | 3-F | 4-F | 5-F |
| CH₃ | CH₃ | CH₃ | CH₃ | H | 3-CF₃ | 4-F | H |
| CH₃ | CH₃ | CH₃ | CH₃ | H | 3-Cl | 4-Cl | H |
| CH₃ | CH₃ | CH₃ | CH₃ | H | 4-CF₃ | 3-F | H |
| CH₃ | CH₃ | CH₃ | CH₃ | H | 4-CN | H | H |
| CH₃ | CH₃ | H | CH₃ | CH₃ | 2-F | H | H |
| CH₃ | CH₃ | H | CH₃ | CH₃ | 3-F | H | H |
| CH₃ | CH₃ | H | CH₃ | CH₃ | 4-F | H | H |
| CH₃ | CH₃ | H | CH₃ | CH₃ | 2-F | 4-F | H |
| CH₃ | CH₃ | H | CH₃ | CH₃ | 2-F | 4-Cl | H |
| CH₃ | CH₃ | H | CH₃ | CH₃ | 2-F | 4-CH₃ | H |
| CH₃ | CH₃ | H | CH₃ | CH₃ | 2-F | 4-OCH₃ | H |
| CH₃ | CH₃ | H | CH₃ | CH₃ | 3-F | 4-F | H |
| CH₃ | CH₃ | H | CH₃ | CH₃ | 3-F | 4-Cl | H |
| CH₃ | CH₃ | H | CH₃ | CH₃ | 3-F | 4-CH₃ | H |
| CH₃ | CH₃ | H | CH₃ | CH₃ | 3-F | 4-OCH₃ | H |
| CH₃ | CH₃ | H | CH₃ | CH₃ | 4-F | 3-Cl | H |
| CH₃ | CH₃ | H | CH₃ | CH₃ | 4-F | 3-CH₃ | H |
| CH₃ | CH₃ | H | CH₃ | CH₃ | 4-F | 3-OCH₃ | H |
| CH₃ | CH₃ | H | CH₃ | CH₃ | 2-F | 4-F | 5-F |
| CH₃ | CH₃ | H | CH₃ | CH₃ | 2-F | 4-F | 6-F |
| CH₃ | CH₃ | H | CH₃ | CH₃ | 2-F | 4-Cl | 5-F |
| CH₃ | CH₃ | H | CH₃ | CH₃ | 2-F | 5-Cl | 4-F |
| CH₃ | CH₃ | H | CH₃ | CH₃ | 3-F | 4-F | 5-F |
| CH₃ | CH₃ | CH₃ | CH₃ | H | 3-Cl | 4-Cl | H |
| CH₃ | CH₃ | CH₃ | CH₃ | H | 4-CF₃ | 3-F | H |
| CH₃ | CH₃ | CH₃ | CH₃ | H | 4-CN | H | H |
| CH₃ | CH₃ | CH₃ | CH₃ | H | 3-CF₃ | 4-F | H |
| CH₃ | CH₃ | CH₃ | CH₃ | CH₃ | 2-F | H | H |
| CH₃ | CH₃ | CH₃ | CH₃ | CH₃ | 3-F | H | H |
| CH₃ | CH₃ | CH₃ | CH₃ | CH₃ | 4-F | H | H |
| CH₃ | CH₃ | CH₃ | CH₃ | CH₃ | 2-F | 4-F | H |
| CH₃ | CH₃ | CH₃ | CH₃ | CH₃ | 2-F | 4-Cl | H |
| CH₃ | CH₃ | CH₃ | CH₃ | CH₃ | 2-F | 4-CH₃ | H |
| CH₃ | CH₃ | CH₃ | CH₃ | CH₃ | 2-F | 4-OCH₃ | H |
| CH₃ | CH₃ | CH₃ | CH₃ | CH₃ | 3-F | 4-F | H |
| CH₃ | CH₃ | CH₃ | CH₃ | CH₃ | 3-F | 4-Cl | H |
| CH₃ | CH₃ | CH₃ | CH₃ | CH₃ | 3-F | 4-CH₃ | H |
| CH₃ | CH₃ | CH₃ | CH₃ | CH₃ | 3-F | 4-OCH₃ | H |
| CH₃ | CH₃ | CH₃ | CH₃ | CH₃ | 4-F | 3-Cl | H |
| CH₃ | CH₃ | CH₃ | CH₃ | CH₃ | 4-F | 3-CH₃ | H |
| CH₃ | CH₃ | CH₃ | CH₃ | CH₃ | 4-F | 3-OCH₃ | H |
| CH₃ | CH₃ | CH₃ | CH₃ | CH₃ | 2-F | 4-F | 5-F |
| CH₃ | CH₃ | CH₃ | CH₃ | CH₃ | 2-F | 4-F | 6-F |
| CH₃ | CH₃ | CH₃ | CH₃ | CH₃ | 2-F | 4-Cl | 5-F |
| CH₃ | CH₃ | CH₃ | CH₃ | CH₃ | 2-F | 5-Cl | 4-F |
| CH₃ | CH₃ | CH₃ | CH₃ | CH₃ | 3-F | 4-F | 5-F |
| CH₃ | CH₃ | CH₃ | CH₃ | H | 3-Cl | 4-Cl | H |
| CH₃ | CH₃ | CH₃ | CH₃ | H | 4-CF₃ | 3-F | H |
| CH₃ | CH₃ | CH₃ | CH₃ | H | 4-CN | H | H |
| CH₃ | CH₃ | CH₃ | CH₃ | H | 3-CF₃ | 4-F | H |

Als erfindungsgemäße Wirkstoffe der Formel (I) mit G = COCH₃ kommen außerdem bevorzugt Verbindungen aus mit den in Tabelle 1 genannten Restedefinitionen für W, X, Y, Z und mit den in Tabelle 2 genannten Restedefinitionen für W, X, Y und 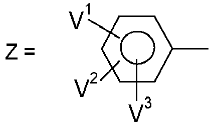 mit den in Tabellen 3 genannten Restedefinitionen für A und B in Frage.

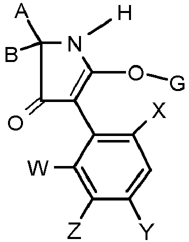

**Tabelle 3**

| **A** | **B** |
|---|---|
| CH₃ | H |
| C₂H₅ | H |
| C₃H₇ | H |
| i-C₃H₇ | H |
| C₄H₉ | H |
| i-C₄H₉ | H |
| s-C₄H₉ | H |
| t-C₄H₉ | H |
| CH₃ | CH₃ |
| C₂H₅ | CH₃ |
| C₃H₇ | CH₃ |
| i-C₃H₇ | CH₃ |
| C₄H₉ | CH₃ |
| i-C₄H₉ | CH₃ |
| s-C₄H₉ | CH₃ |
| t-C₄H₉ | CH₃ |
| C₂H₅ | C₂H₅ |
| C₃H₇ | C₃H₇ |
| 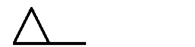 | CH₃ |
| 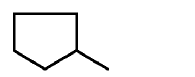 | CH₃ |
| 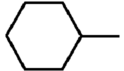 | CH₃ |
| H₃CO-CH₂ | CH₃ |
| H₅C₂O-CH₂- | CH₃ |
| H₃CO-(CH₂)₂- | CH₃ |
| H₅C₂O-(CH₂)₂- | CH₃ |
| 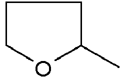 | CH₃ |
| 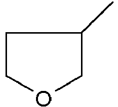 | CH₃ |
| -(CH₂)₂- | |
| -(CH₂)₄- | |
| -(CH₂)₅- | |
| -(CH₂)₆- | |
| -(CH₂)₇- | |
| 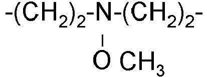 | |
| 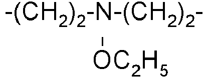 | |
| -(CH₂)₂-O-(CH₂)₂- | |
| -CH₂-O-(CH₂)₃- | |
| -(CH₂)₂-S-(CH₂)₂- | |
| -CH₂-CHCH₃-(CH₂)₃- | |
| -CH₂-CHOCH₃-(CH₂)₂- | |
| -CH₂-CHOC₂H₅-(CH₂)₂- | |
| -CH₂-CHOC₃H₇-(CH₂)₂- | |
| -CH₂-CHOC₄H₉-(CH₂)₂- | |
| -CH₂-CHO(CH₂)₂OCH₃-(CH₂)₂- | |
| 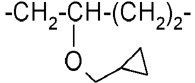 | |
| -CH₂-CHOCH₃-(CH₂)₃- | |
| -CH₂-CHOC₂H₅-(CH₂)₃- | |
| -CH₂-CHOC₃H₇-(CH₂)₃- | |
| -CH₂-CHOC₄H₉-(CH₂)₃- | |
| -CH₂-CHO(CH₂)₂OCH₃-(CH₂)₃- | |
| 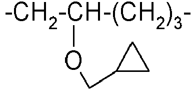 | |
| -(CH₂)₂-CHCH₃-(CH₂)₂- | |
| -(CH₂)₂-CHC₂H₅(CH₂)₂- | |
| -(CH₂)₂-CHC₃H₇-(CH₂)₂- | |
| -(CH₂)₂-CHi-C₃H₇-(CH₂)₂- | |
| -(CH₂)₂-CHOCH₃-(CH₂)₂- | |
| -(CH₂)₂-CHOC₂H₅-(CH₂)₂- | |
| -(CH₂)₂-CHOC₃H₇-(CH₂)₂- | |
| -(CH₂)₂-CHO-CH₂CF₃-(CH₂)₂- | |
| -(CH₂)₂-C(CH₃)₂-(CH₂)₂- | |
| -CH₂-(CHCH₃)₂-(CH₂)₂- | |
| 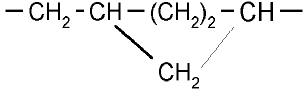 | |
| 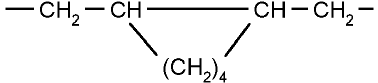 | |
| 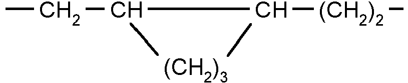 | |
| 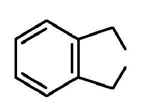 | |
| 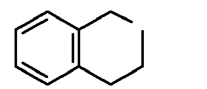 | |
| 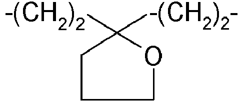 | |
| 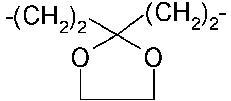 | |
| 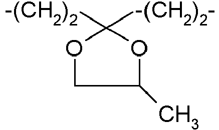 | |
| 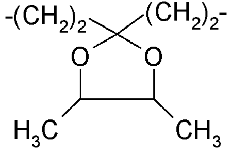 | |
| 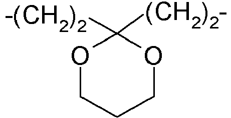 | |
| 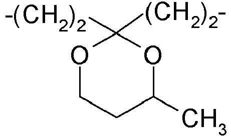 | |
| 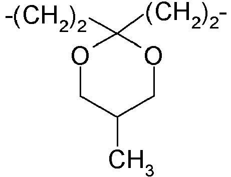 | |
| 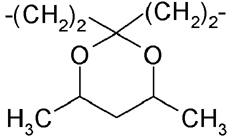 | |
| 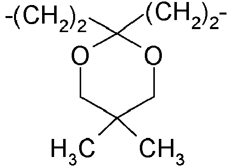 | |
| 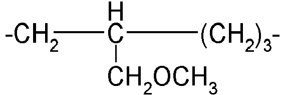 | |
| 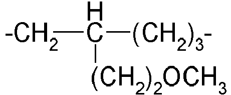 | |
| 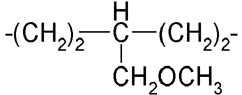 | |
| 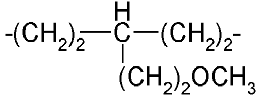 | |
| 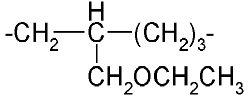 | |
| 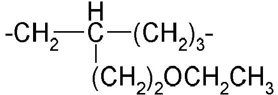 | |
| 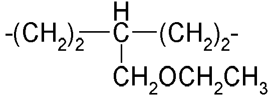 | |
| 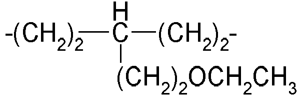 | |

- Tabelle 4: A, B, V¹, V², V³, X, W, Y und Z wie in Tabelle 1, 2 und 3 angegeben G = COC₂H₅
- Tabelle 5: A, B, V¹, V², V³, X, W, Y und Z wie in Tabelle 1, 2 und 3 angegeben G = COC₃H₇
- Tabelle 6: A, B, V¹, V², V³, X, W, Y und Z wie in Tabelle 1, 2 und 3 angegeben G = CO-i-C₃H₇
- Tabelle 7: A, B, V¹, V², V³, X, W, Y und Z wie in Tabelle 1, 2 und 3 angegeben G = CO-c-C₃H₅
- Tabelle 8: A, B, V¹, V², V³, X, W, Y und Z wie in Tabelle 1, 2 und 3 angegeben G = COC₄H₉
- Tabelle 9: A, B, V¹, V², V³, X, W, Y und Z wie in Tabelle 1, 2 und 3 angegeben G = CO-i-C₄H₉
- Tabelle 10: A, B, V¹, V², V³, X, W, Y und Z wie in Tabelle 1, 2 und 3 angegeben G = CO-t-C₄H₉
- Tabelle 11: A, B, V¹, V², V³, X, W, Y und Z wie in Tabelle 1, 2 und 3 angegeben G = CO₂CH₃
- Tabelle 12: A, B, V¹, V², V³, X, W, Y und Z wie in Tabelle 1, 2 und 3 angegeben G = CO₂C₂H₅
- Tabelle 13: A, B, V¹, V², V³, X, W, Y und Z wie in Tabelle 1, 2 und 3 angegeben G = CO₂C₃H₇
- Tabelle 14: A, B, V¹, V², V³, X, W, Y und Z wie in Tabelle 1, 2 und 3 angegeben G = CO₂-i-C₂H₅
- Tabelle 15: A, B, V¹, V², V³, X, W, Y und Z wie in Tabelle 1, 2 und 3 angegeben G = CO₂-t-C₄H₉
- Tabelle 16: A, B, V¹, V², V³, X, W, Y und Z wie in Tabelle 1, 2 und 3 angegeben G = CO₂C₆H₅
- Tabelle 17: A, B, V¹, V², V³, X, W, Y und Z wie in Tabelle 1, 2 und 3 angegeben G = CO₂CH₂C₆H₅
mit der Maßgabe, dass A und B nur dann jeweils für Methyl stehen dürfen, wenn, im Fall dass W, X und Y jeweils für Alkyl und Z für Wasserstoff stehen, W und X jeweils für Methyl stehen oder Wund X jeweils für Ethyl stehen.

In der Literatur wurde bereits beschrieben, dass sich die Wirkung verschiedener Wirkstoffe durch Zugabe von Ammoniumsalzen steigern lässt. Dabei handelt es sich jedoch um als Detergens wirkende Salze (z.B. WO 95/017817) bzw. Salze mit längeren Alkyl- und / oder Arylsubstituenten, die permeabilisierend wirken oder die Löslichkeit des Wirkstoffs erhöhen (z.B. EP-A 0 453 086, EP-A 0 664 081, FR-A 2 600 494, US 4 844 734, US 5 462 912, US 5 538 937, US-A 03/0224939, US-A 05/0009880, US-A 05/0096386). Weiterhin beschreibt der Stand der Technik die Wirkung nur für bestimmte Wirkstoffe und / oder bestimmte Anwendungen der entsprechenden Mittel. In wieder anderen Fällen handelt es sich um Salze von Sulfonsäuren, bei denen die Säuren selber paralysierend auf Insekten wirken (US 2 842 476). Eine Wirkungssteigerung z.B. durch Ammoniumsulfat ist beispielsweise für die Herbizide Glyphosat, Phosphinothricin und für phenylsubstituierte Cyclische Ketoenole beschrieben (US 6 645 914, EP-A2 0 036 106, WO 07/068427). Eine entsprechende Wirkungssteigerung bei Insektiziden wurde bereits durch WO 07/068428 beschrieben.

Auch der Einsatz von Ammoniumsulfat als Formulierhilfsmittel ist für bestimmte Wirkstoffe und Anwendungen beschrieben (WO 92/16108), es dient dort aber zur Stabilisierung der Formulierung, nicht zur Wirkungssteigerung.

Es wurde nun völlig überraschend gefunden, dass sich die Wirkung von Insektiziden und/oder Akariziden und/oder Nematiziden und/oder Herbiziden aus der Klasse der 2-Acyloxy-pyrrolin-4-one der Formel (I) durch den Zusatz von Ammonium- oder Phosphoniumsalzen zur Anwendungslösung oder durch den Einbau dieser Salze in eine Formulierung enthaltend 2-Acyloxy-pyrrolin-4-one der Formel (I) deutlich steigern lässt. Gegenstand der vorliegenden Erfindung ist also die Verwendung von Ammonium- oder Phosphoniumsalzen zur Wirkungssteigerung von Pflanzenschutzmitteln, die insektizid und/oder akarizid und/oder nematizid und/oder herbizid wirksame 2-Acyloxy-pyrrolin-4-one der Formel (I) als Wirkstoff enthalten. Gegenstand der Erfindung sind ebenfalls Mittel, die herbizide und/oder akarizid und/oder insektizid und/oder nematizid wirksame 2-Acyloxy-pyrrolin-4-one der Formel (I) und die Wirkung steigernde Ammonium- oder Phosphoniumsalze enthalten und zwar sowohl formulierte Wirkstoffe als auch anwendungsfertige Mittel (Spritzbrühen). Gegenstand der Erfindung ist schließlich weiterhin die Verwendung dieser Mittel zur Bekämpfung von Schadinsekten und/oder Spinnmilben und/oder unerwünschten Pflanzenwuchs.

Die Verbindungen der Formel (I) besitzen eine breite insektizide und/oder akarizide und/oder nematizide und/oder herbizide Wirkung, die Wirkung und/oder Pflanzenverträglichkeit lässt im Einzelnen aber zu wünschen übrig. Diese Eigenschaften können jedoch durch den Zusatz von Ammonium- oder Phosphoniumsalzen insgesamt oder partiell verbessert werden.

Die Wirkstoffe können in den erfindungsgemäßen Zusammensetzungen in einem breiten Konzentrationsbereich eingesetzt werden. Die Konzentration der Wirkstoffe in der Formulierung beträgt dabei üblicherweise 0,1 - 50 Gew.-%.

Ammonium- und Phosphoniumsalze, die erfindungsgemäß die Wirkung von Pflanzenschutzmitteln enthaltend Wirkstoffe aus der Klasse der 2-Acyloxy-pyrrolin-4-one der Formel (1) steigern, werden durch Formel (III') definiert 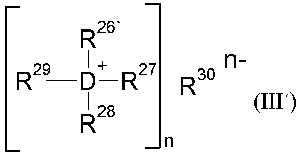 in welcher
- D: für Stickstoff oder Phosphor steht,
- D: bevorzugt für Stickstoff steht,
- R²⁶, R²⁷, R²⁸ und R²⁹: unabhängig voneinander für Wasserstoff oder jeweils gegebenenfalls substituiertes C₁-C₈-Alkyl oder einfach oder mehrfach ungesättigtes, gegebenenfalls substituiertes C₁-C₈-Alkylen stehen, wobei die Substituenten aus Halogen, Nitro und Cyano ausgewählt sein können,
- R²⁶, R²⁷, R²⁸ und R²⁹: bevorzugt unabhängig voneinander für Wasserstoff oder jeweils gegebenenfalls substituiertes C₁-C₄-Alkyl stehen, wobei die Substituenten aus Halogen, Nitro und Cyano ausgewählt sein können,
- R²⁶, R²⁷, R²⁸ und R²⁹: besonders bevorzugt unabhängig voneinander für Wasserstoff, Methyl, Ethyl, n-Propyl, i-Propyl, n-Butyl, i-Butyl, s-Butyl oder t-Butyl stehen,
- R²⁶, R²⁷, R²⁸ und R²⁹: ganz besonders bevorzugt für Wasserstoff stehen,
- n: für 1, 2, 3 oder 4 steht,
- n: bevorzugt für 1 oder 2 steht,
- R³⁰: für ein anorganisches oder organisches Anion steht,
- R³⁰: bevorzugt für Hydrogencarbonat, Tetraborat, Fluorid, Bromid, Jodid, Chlorid, Monohydrogenphosphat, Dihydrogenphosphat, Hydrogensulfat, Tartrat, Sulfat, Nitrat, Thiosulfat, Thiocyanat, Formiat, Laktat, Acetat, Propionat, Butyrat, Pentanoat oder Oxalat steht,
- R³⁰: besonders bevorzugt für Laktat, Sulfat, Nitrat, Thiosulfat, Thiocyanat, Oxalat oder Formiat steht.
- R³⁰: ganz besonders bevorzugt für Sulfat steht.

Die Ammonium- und Phosphoniumsalze der Formel (III') können in einem breiten Konzentrationsbereich zur Steigerung der Wirkung von Pflanzenschutzmitteln enthaltend biphenylsubstituierte cyclische Ketoenole der Formel (I) eingesetzt werden. Im Allgemeinen werden die Ammonium- oder Phosphoniumsalze im anwendungsfertigen Pflanzenschutzmittel in einer Konzentration von 0,5 bis 80 mmol/1, bevorzugt 0,75 bis 37,5 mmol/1, besonders bevorzugt 1,5 bis 25 mmol/l eingesetzt. Im Fall eines formulierten Produktes wird die Ammonium- und/oder Phosphoniumsalzkonzentration in der Formulierung so gewählt, dass sie nach Verdünnung der Formulierung auf die gewünschte Wirkstoffkonzentration in diesen angegebenen allgemeinen, bevorzugten oder besonders bevorzugten Bereichen liegt. Die Konzentration des Salzes in der Formulierung beträgt dabei üblicherweise 1 - 50 Gew.-%.

In einer bevorzugten Ausführungsform der Erfindung wird den Pflanzenschutzmitteln zur Wirkungssteigerung nicht nur ein Ammonium- und/oder Phosphoniumsalz, sondern zusätzlich ein Penetrationsförderer zugegeben. Es ist als völlig überraschend zu bezeichnen, dass selbst in diesen Fällen eine noch weiter gehende Wirkungssteigerung zu beobachten ist. Gegenstand der vorliegenden Erfindung ist also ebenfalls die Verwendung einer Kombination von Penetrationsförderer und Ammonium- und/oder Phosphoniumsalzen zur Wirkungssteigerung von Pflanzenschutzmitteln, die insektizid und/oder akarizid und/oder nematizid und/oder herbizid wirksame, 2-Acyloxy-pyrrolin-4-one der Formel (I) als Wirkstoff enthalten. Gegenstand der Erfindung sind ebenfalls Mittel, die herbizid und/oder akarizid und/oder insektizid und/oder nematizid wirksame 2-Acyloxy-pyrrolin-4-one der Formel (I), Penetrationsförderer und Ammonium- und/oder Phosphoniumsalze enthalten und zwar sowohl formulierte Wirkstoffe als auch anwendungsfertige Mittel (Spritzbrühen). Gegenstand der Erfindung ist schließlich weiterhin die Verwendung dieser Mittel zur Bekämpfung von Schadinsekten und/oder Spinnmilben und/oder unerwünschten Pflanzenwuchs.

Als Penetrationsförderer kommen im vorliegenden Zusammenhang alle diejenigen Substanzen in Betracht, die üblicherweise eingesetzt werden, um das Eindringen von agrochemischen Wirkstoffen in Pflanzen zu verbessern. Penetrationsförderer werden in diesem Zusammenhang dadurch definiert, dass sie aus der wässerigen Spritzbrühe und/oder aus dem Spritzbelag in die Kutikula der Pflanze eindringen und dadurch die Stoffbeweglichkeit (Mobilität) von Wirkstoffen in der Kutikula erhöhen können. Die in der Literatur (Baur et al., 1997, Pesticide Science 51, 131-152) beschriebene Methode kann zur Bestimmung dieser Eigenschaft eingesetzt werden.

Als Penetrationsförderer kommen beispielsweise Alkanol-alkolylate in Betracht. Erfindungsgemäße Penetrationsförderer sind Alkanol-alkoxylate der Formel (IV')

R-O-(-AO)_{V}-R' (IV')

in welcher
- R: für geradkettiges oder verzweigtes Alkyl mit 4 bis 20 Kohlenstoffatomen steht,
- R': für Wasserstoff, Methyl, Ethyl, n-Propyl, i-Propyl, n-Butyl, i-Butyl, t-Butyl, n-Pentyl oder n-Hexyl steht,
- AO: für einen Ethylenoxid-Rest, einen Propylenoxid-Rest, einen Butylenoxid-Rest oder für Gemische aus Ethylenoxid- und Propylenoxid-Resten oder Butylenoxid-Resten steht und
- v: für Zahlen von 2 bis 30 steht.

Eine bevorzugte Gruppe von Penetrationsförderern sind Alkanolalkoxylate der Formel

R-O-(-EO-)ₙ-R' (IV'-a)

in welcher
- R: die oben angegebene Bedeutung hat,
- R': die oben angegebene Bedeutung hat,
- EO: für -CH₂-CH₂-O- steht und
- n: für Zahlen von 2 bis 20 steht.

Eine weitere bevorzugte Gruppe von Penetrationsförderern sind Alkanol-alkoxylate der Formel

R-O-(-EO-)ₚ-(-PO-)_{q}-R' (IV'-b)

in welcher
- R: die oben angegebene Bedeutung hat,
- R': die oben angegebene Bedeutung hat,
- EO: für -CH₂-CH₂-O- steht, PO
für 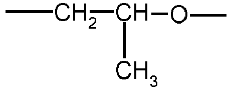 steht,
- p: für Zahlen von 1 bis 10 steht und
- q: für Zahlen von 1 bis 10 steht.

Eine weitere bevorzugte Gruppe von Penetrationsförderern sind Alkanol-Alkoxylate der Formel

R-O-(-PO-)ᵣ-(EO-)ₛ-R' (IV'-c)

in welcher
- R: die oben angegebene Bedeutung hat,
- R': die oben angegebene Bedeutung hat,
- EO: für -CH₂-CH₂-O- steht,
- PO: für 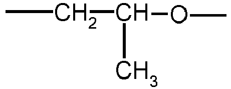 steht,
- r: für Zahlen von 1 bis 10 steht und
- s: für Zahlen von 1 bis 10 steht.

Eine weitere bevorzugte Gruppe von Penetrationsförderern sind Alkanol-alkoxylate der Formel

R-O-(-EO-)ₚ-(-BO-)_{q}-R' (IV'-d)

in welcher
R und R die oben angegebenen Bedeutungen haben,
- EO: für CH₂-CH₂-O- steht,
- BO: für 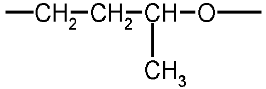 steht,
- p: für Zahlen von 1 bis 10 steht und
- q: für Zahlen von 1 bis 10 steht.

Eine weitere bevorzugte Gruppe von Penetrationsförderern sind Alkanol-alkoxylate der Formel

R-O-(-BO-)ᵣ-(-EO-)ₛ-R' (IV'-e)

in welcher
- R und R: die oben angegebenen Bedeutungen haben,
- BO: 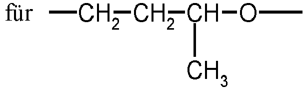 steht,
- EO: für CH₂-CH₂-O- steht,
- r: für Zahlen von 1 bis 10 steht und
- s: für Zahlen von 1 bis 10 steht.

Eine weitere bevorzugte Gruppe von Penetrationsförderern sind Alkanol-Alkoxylate der Formel

CH₃-(CH₂)ₜ-CH₂-O-(-CH₂-CH₂-O-)ᵤ-R' (IV' -f)

in welcher
- R': die oben angegebene Bedeutung hat,
- t: für Zahlen von 8 bis 13 steht
- u: für Zahlen von 6 bis 17 steht.

In den zuvor angegebenen Formeln steht
- R: vorzugsweise für Butyl, i-Butyl, n-Pentyl, i-Pentyl, Neopentyl, n-Hexyl, i-Hexyl, n-Octyl, i-Octyl, 2-Ethyl-hexyl, Nonyl, i-Nonyl, Decyl, n-Dodecyl, i-Dodecyl, Lauryl, Myristyl, i-Tridecyl, Trimethyl-nonyl, Palmityl, Stearyl oder Eicosyl.
- Als: Beispiel für ein Alkanol-Alkoxylat der Formel (IV'-c) sei 2-Ethyl-hexyl-alkoxylat der Formel 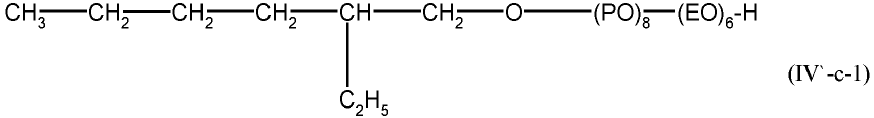
in welcher
- EO: für -CH₂-CH₂-O- steht,
- PO: für 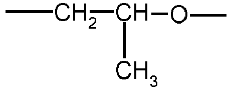 steht und
die Zahlen 8 und 6 Durchschnittswerte darstellen, genannt.

Als Beispiel für ein Alkanol-Alkoxylat der Formel (IV'-d) sei die Formel

CH₃-(CH₂)₁₀-O-(-EO-)₆-(-BO-)₂-CH₃ (IV'-d-1)

in welcher
- EO: für CH₂-CH₂-O- steht,
- BO: 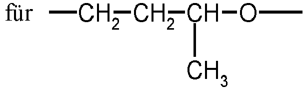 steht und
die Zahlen 10, 6 und 2 Durchschnittswerte darstellen, genannt.

Besonders bevorzugte Alkanol-Alkoxylate der Formel (IV'-f) sind Verbindungen dieser Formel, in denen
- t: für Zahlen von 9 bis 12 und
- u: für Zahlen von 7 bis 9
steht.

Ganz besonders bevorzugt genannt sei Alkanol-Alkoxylat der Formel (IV'-f-1)

CH₃-(CH₂)ₜ-CH₂-O-(-CH₂-CH₂-O-)ᵤ-H (IV'-f-1)

in welcher
- t: für den Durchschnittswert 10,5 steht und
- u: für den Durchschnittswert 8,4 steht.

Die Alkanol-Alkoxylate sind durch die obigen Formeln allgemein definiert. Bei diesen Substanzen handelt es sich um Gemische von Stoffen des angegebenen Typs mit unterschiedlichen Kettenlängen. Für die Indices errechnen sich deshalb Durchschnittswerte, die auch von ganzen Zahlen abweichen können.

Die Alkanol-Alkoxylate der angegebenen Formeln sind bekannt und sind teilweise kommerziell erhältlich oder lassen sich nach bekannten Methoden herstellen (vgl. WO 98/35 553, WO 00/35 278 und EP-A 0 681 865).

Als Penetrationsförderer kommen beispielsweise auch Substanzen in Betracht, die die Verfügbarkeit der Verbindungen der Formel (I) im Spritzbelag fördern. Dazu gehören beispielsweise mineralische oder vegetabile Öle. Als Öle kommen alle üblicherweise in agrochemischen Mitteln einsetzbaren mineralischen oder vegetabilen - gegebenenfalls modifizierte - Öle in Frage. Beispielhaft genannt seien Sonnenblumenöl, Rapsöl, Olivenöl, Rizinusöl, Rüböl, Maiskernöl, Baumwollsaatöl und Sojabohnenöl oder die Ester der genannten Öle. Bevorzugt sind Rapsöl, Sonnenblumenöl und deren Methyl- oder Ethylester.

Die Konzentration an Penetrationsförderer kann in den erfindungsgemäßen Mitteln in einem weiten Bereich variiert werden. Bei einem formulierten Pflanzenschutzmittel liegt sie im allgemeinen bei 1 bis 95 Gew.-%, bevorzugt bei 1 bis 55 Gew.-%, besonders bevorzugt bei 15 - 40 Gew.-%. In den anwendungsfertigen Mitteln (Spritzbrühen) liegen die Konzentration im allgemeinen zwischen 0,1 und 10 g/1, bevorzugt zwischen 0,5 und 5 g/1.

Erfindungsgemäße Pflanzenschutzmittel können auch weitere Komponente, beispielsweise Tenside bzw. Dispergierhilfsmittel oder Emulgatoren enthalten.

Als nicht-ionische Tenside bzw. Dispergierhilfsmittel kommen alle üblicherweise in agrochemischen Mitteln einsetzbaren Stoffe dieses Typs in Betracht. Vorzugsweise genannt seien Polyethylenoxid-polypropylenoxid-Blockcopolymere, Polyethylenglykolether von linearen Alkoholen, Umsetzungsprodukte von Fettsäuren mit Ethylenoxid und/oder Propylenoxid, ferner Polyvinylalkohol, Polyvinylpyrrolidon, Mischpolymerisate aus Polyvinylalkohol und Polyvinylpyrrolidon sowie Copolymerisate aus (Meth)acrylsäure und (Meth)acrylsäureestern, weiterhin Alkylethoxylate und Alkylarylethoxylate, die gegebenenfalls phosphatiert und gegebenenfalls mit Basen neutralisiert sein können, wobei Sorbitolethoxylate beispielhaft genannt seien, sowie Polyoxyalkylenamin-Derivate.

Als anionische Tenside kommen alle üblicherweise in agrochemischen Mitteln einsetzbaren Substanzen dieses Typs in Frage. Bevorzugt sind Alkalimetall- und Erdalkalimetall-Salze von Alkylsulfonsäuren oder Alkylarylsulfonsäuren.

Eine weitere bevorzugte Gruppe von anionischen Tensiden bzw. Dispergierhilfsmitteln sind in Pflanzenöl wenig lösliche Salze von Polystyrolsulfonsäuren, Salze von Polyvinylsulfonsäuren, Salze von Naphthalinsulfonsäure-Formaldehyd-Kondensationsprodukten, Salze von Kondensationsprodukten aus Naphthalinsulfonsäure, Phenolsulfonsäure und Formaldehyd sowie Salze von Ligninsulfonsäure.

Als Zusatzstoffe, die in den erfindungsgemäßen Formulierungen enthalten sein können, kommen Emulgatoren, schaumhemmende Mittel, Konservierungsmittel, Antioxydantien, Farbstoffe und inerte Füllmaterialien in Betracht.

Bevorzugte Emulgatoren sind ethoxylierte Nonylphenole, Umsetzungsprodukte von Alkylphenolen mit Ethylenoxid und/oder Propylenoxid, ethoxylierte Arylalkylphenole, weiterhin ethoxylierte und propoxylierte Arylalkylphenole, sowie sulfatierte oder phosphatierte Arylalkylethoxylate bzw. -ethoxy-propoxylate, wobei Sorbitan-Derivate, wie Polyethylenoxid-Sorbitan-Fettsäureester und SorbitanFettsäureester, beispielhaft genannt seien.

Verwendet man beispielsweise gemäß Verfahren (Aα) 3-[(4-Chlor-2,6-dimethyl)-phenyl]-1-azaspiro[4,5]decan-2,4-dion und Pivaloylchlorid als Ausgangsstoffe, so kann der Verlauf des erfindungsgemäßen Verfahrens durch folgendes Reaktionsschema wiedergegeben werden:

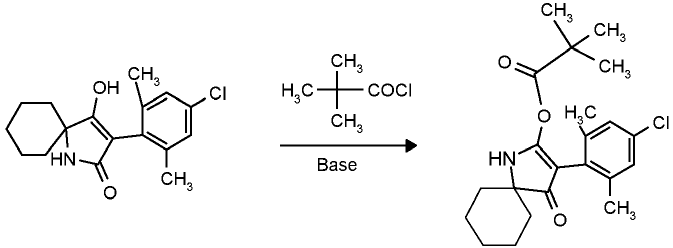

Verwendet man beispielsweise gemäß Verfahren (Aß) 3-[(2,4-Dichlor)-phenyl]-1-azaspiro-[4,5]-decan-2,4-dion und Acetanhydrid als Ausgangsverbindungen, so kann der Verlauf des erfindungsgemäßen Verfahrens durch folgendes Reaktionsschema wiedergegeben werden:

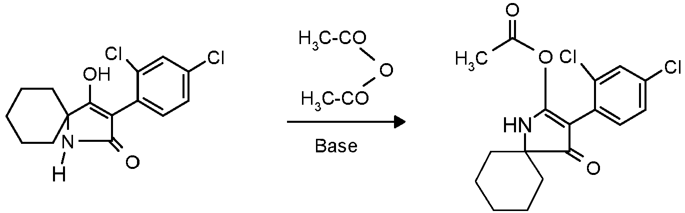

Verwendet man beispielsweise gemäß Verfahren (B) 3-[(2,4-Dichlor-6-methyl)-phenyl]-1-azaspiro[4,5]decan-2,4-dion und Chlorameisensäureethylester als Ausgangsverbindungen, so kann der Verlauf des erfindungsgemäßen Verfahrens durch folgendes Reaktionsschema wiedergegeben werden:

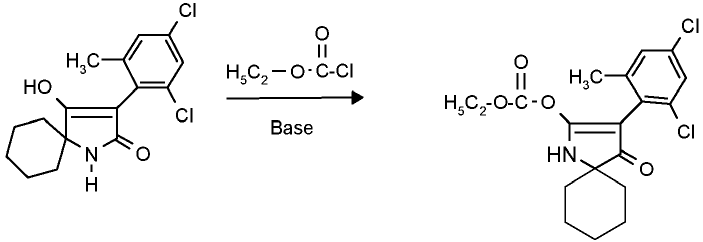

Die bei den erfindungsgemäßen Verfahren (A) und (B) als Ausgangsstoffe benötigten Verbindungen der Formel (II) 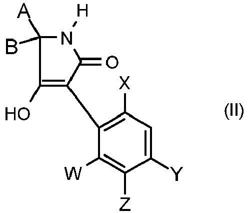 in welcher
A, B, W, X, Y und Z die oben angegebenen Bedeutungen haben,
sind aus der eingangs zitierten Literatur allgemein bekannt oder lassen sich nach den dort beschriebenen Verfahren in Analogie herstellen.

Die zur Durchführung der erfindungsgemäßen Verfahren (A) und (B) als Ausgangsstoffe benötigten Säurehalogenide der Formel (III), Carbonsäureanhydride der Formel (IV) und Chlorameisensäureester oder Chlorameisensäurethioester der Formel (V) sind allgemein bekannte Verbindungen der Organischen Chemie.

Das Verfahren (A_{α}) ist dadurch gekennzeichnet, dass man Verbindungen der Formel (II) jeweils mit Carbonsäurehalogeniden der Formel (III) gegebenenfalls in Gegenwart eines Verdünnungsmittels und gegebenenfalls in Gegenwart eines Säurebindemittels umsetzt.

Als Verdünnungsmittel können bei dem erfindungsgemäßen Verfahren (A_{α}) alle gegenüber den Säurehalogeniden inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenstoff, Chlorbenzol und o-Dichlorbenzol, außerdem Ketone, wie Aceton und Methylisopropylketon, weiterhin Ether, wie Diethylether, Tetrahydrofuran und Dioxan, darüber hinaus Carbonsäureester, wie Ethylacetat, und auch stark polare Solventien, wie Dimethylformamid, Dimethylsulfoxid und Sulfolan. Wenn die Hydrolysestabilität des Säurehalogenids es zulässt, kann die Umsetzung auch in Gegenwart von Wasser durchgeführt werden.

Als Säurebindemittel kommen bei der Umsetzung nach dem erfindungsgemäßen Verfahren (A_{α}) alle üblichen Säureakzeptoren in Betracht. Vorzugsweise verwendbar sind tertiäre Amine, wie Triethylamin, Pyridin, Diazabicyclooctan (DABCO), Diazabicycloundecen (DBU), Diazabicyclononen (DBN), Hünig-Base und N,N-Dimethyl-anilin, ferner Erdalkalimetalloxide, wie Magnesium- und Calciumoxid, außerdem Alkali- und Erdalkali-metall-carbonate, wie Natriumcarbonat, Kaliumcarbonat und Calciumcarbonat sowie Alkalihydroxide wie Natriumhydroxid und Kaliumhydroxid.

Die Reaktionstemperatur kann bei dem erfindungsgemäßen Verfahren (A_{α}) innerhalb eines größeren Bereiches variiert werden. Im allgemeinen arbeitet man bei Temperaturen zwischen -78°C und +100°C, vorzugsweise zwischen -20°C und 100°C.

Bei der Durchführung des erfindungsgemäßen Verfahrens (A_{α}) werden die Ausgangsstoffe der Formel (II) und das Carbonsäurehalogenid der Formel (III) im allgemeinen jeweils in angenähert äquivalenten Mengen verwendet. Es ist jedoch auch möglich, das Carbonsäurehalogenid in einem größeren Überschuss (bis zu 5 Mol) einzusetzen. Die Aufarbeitung erfolgt nach üblichen Methoden.

Das Verfahren (A_{ß}) ist dadurch gekennzeichnet, dass man Verbindungen der Formel (II) jeweils mit Carbonsäureanhydriden der Formel (IV) gegebenenfalls in Gegenwart eines Verdünnungsmittels und gegebenenfalls in Gegenwart eines Säurebindemittels umsetzt.

Als Verdünnungsmittel können bei dem erfindungsgemäßen Verfahren (A_{ß}) vorzugsweise diejenigen Verdünnungsmittel verwendet werden, die auch bei der Verwendung von Säurehalogeniden vorzugsweise in Betracht kommen. Im übrigen kann auch ein im Überschuss eingesetztes Carbonsäureanhydrid gleichzeitig als Verdünnungsmittel fungieren.

Als gegebenenfalls zugesetzte Säurebindemittel kommen beim Verfahren (A_{ß}) vorzugsweise diejenigen Säurebindemittel in Frage, die auch bei der Verwendung von Säurehalogeniden vorzugsweise in Betracht kommen.

Die Reaktionstemperatur kann bei dem erfindungsgemäßen Verfahren (A_{ß}) innerhalb eines größeren Bereiches variiert werden. Im allgemeinen arbeitet man bei Temperaturen zwischen -20°C und +150°C, vorzugsweise zwischen 0°C und 100°C.

Bei der Durchführung des erfindungsgemäßen Verfahrens (A_{β}) werden die Ausgangsstoffe der Formel (II) und das Carbonsäureanhydrid der Formel (IV) im allgemeinen in jeweils angenähert äquivalenten Mengen verwendet. Es ist jedoch auch möglich, das Carbonsäureanhydrid in einem größeren Überschuss (bis zu 5 Mol) einzusetzen. Die Aufarbeitung erfolgt nach üblichen Methoden.

Im allgemeinen geht man so vor, dass man Verdünnungsmittel und im Überschuss vorhandenes Carbonsäureanhydrid sowie die entstehende Carbonsäure durch Destillation oder durch Waschen mit einem organischen Lösungsmittel oder mit Wasser entfernt.

Das Verfahren (B) ist dadurch gekennzeichnet, dass man Verbindungen der Formel (II) jeweils mit Chlorameisensäureestern oder Chlorameisensäurethiolestern der Formel (V) gegebenenfalls in Gegenwart eines Verdünnungsmittels und gegebenenfalls in Gegenwart eines Säurebindemittels umsetzt.

Als Säurebindemittel kommen bei dem erfindungsgemäßen Verfahren (B) alle üblichen Säureakzeptoren in Betracht. Vorzugsweise verwendbar sind tertiäre Amine, wie Triethylamin, Pyridin, DABCO, DBU, DBN, Hünig-Base und N,N-Dimethyl-anilin, ferner Erdalkalimetalloxide, wie Magnesium- und Calciumoxid, außerdem Alkali- und Erdalkalimetallcarbonate, wie Natriumcarbonat, Kaliumcarbonat und Calciumcarbonat sowie Alkalihydroxide wie Natriumhydroxid und Kaliumhydroxid.

Als Verdünnungsmittel können bei dem erfindungsgemäßen Verfahren (B) alle gegenüber den Chlorameisensäureestern bzw. Chlorameisensäurethiolestern inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenwasserstoff, Chlorbenzol und o-Dichlorbenzol, außerdem Ketone, wie Aceton und Methylisopropylketon, weiterhin Ether, wie Diethylether, Tetrahydrofuran und Dioxan, darüber hinaus Carbonsäureester, wie Ethylacetat, außerdem Nitrile wie Acetonitril und auch stark polare Solventien, wie Dimethylformamid, Dimethylsulfoxid und Sulfolan.

Die Reaktionstemperatur kann bei der Durchführung des erfindungsgemäßen Verfahrens (B) innerhalb eines größeren Bereiches variiert werden. Die Reaktionstemperatur liegt im allgemeinen zwischen -78°C und +100°C, vorzugsweise zwischen -20°C und 50°C.

Das erfindungsgemäße Verfahren (B) wird im allgemeinen unter Normaldruck durchgeführt.

Bei der Durchführung des erfindungsgemäßen Verfahrens (B) werden die Ausgangsstoffe der Formeln (II) und der entsprechende Chlorameisensäureester bzw. Chlorameisensäurethiolester der Formel (V) im Allgemeinen jeweils in angenähert äquivalenten Mengen verwendet. Es ist jedoch auch möglich, die eine oder andere Komponente in einem größeren Überschuss (bis zu 2 Mol) einzusetzen. Die Aufarbeitung erfolgt nach üblichen Methoden. Im allgemeinen geht man so vor, dass man ausgefallene Salze entfernt und das verbleibende Reaktionsgemisch durch Abziehen des Verdünnungsmittels einengt.

Die erfindungsgemäßen Wirkstoffe eignen sich bei guter Pflanzenverträglichkeit, günstiger Warmblütertoxizität und guter Umweltverträglichkeit zum Schutz von Pflanzen und Pflanzenorganen, zur Steigerung der Ernteerträge, Verbesserung der Qualität des Erntegutes und zur Bekämpfung von tierischen Schädlingen, insbesondere Insekten, Spinnentieren, Helminthen, Nematoden und Mollusken, die in der Landwirtschaft, im Gartenbau, bei der Tierzucht, in Forsten, in Gärten und Freizeiteinrichtungen, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:

Aus der Ordnung der Anoplura (Phthiraptera) z.B. Damalinia spp., Haematopinus spp., Linognathus spp., Pediculus spp., Trichodectes spp..

Aus der Klasse der Arachnida z.B. Acarus spp., Aceria sheldoni, Aculops spp., Aculus spp., Amblyomma spp., Amphitetranychus viennensis, Argas spp., Boophilus spp., Brevipalpus spp., Bryobia praetiosa, Chorioptes spp., Dermanyssus gallinae, Eotetranychus spp., Epitrimerus pyri, Eutetranychus spp., Eriophyes spp., Halotydeus destructor, Hemitarsonemus spp., Hyalomma spp., Ixodes spp., Latrodectus mactans, Metatetranychus spp., Nuphersa spp., Oligonychus spp., Ornithodoros spp., Panonychus spp., Phyllocoptruta oleivora, Polyphagotarsonemus latus, Psoroptes spp., Rhipicephalus spp., Rhizoglyphus spp., Sarcoptes spp., Scorpio maurus, Stenotarsonemus spp., Tarsonemus spp., Tetranychus spp., Vasates lycopersici.

Aus der Klasse der Bivalva z.B. Dreissena spp..

Aus der Ordnung der Chilopoda z.B. Geophilus spp., Scutigera spp..

Aus der Ordnung der Coleoptera z.B. Acalymma vittatum, Acanthoscelides obtectus, Adoretus spp., Agelastica alni, Agriotes spp., Amphimallon solstitialis, Anobium punctatum, Anoplophora spp., Anthonomus spp., Anthrenus spp., Apion spp., Apogonia spp., Atomaria spp., Attagenus spp., Bruchidius obtectus, Bruchus spp., Cassida spp., Cerotoma trifurcata, Ceutorrhynchus spp., Chaetocnema spp., Cleonus mendicus, Conoderus spp., Cosmopolites spp., Costelytra zealandica, Ctenicera spp., Curculio spp., Cryptorhynchus lapathi, Cylindrocopturus spp., Dermestes spp., Diabrotica spp., Dichocrocis spp., Diloboderus spp., Epilachna spp., Epitrix spp., Faustinus spp., Gibbium psylloides, Hellula undalis, Heteronychus arator, Heteronyx spp., Hylamorpha elegans, Hylotrupes bajulus, Hypera postica, Hypothenemus spp., Lachnosterna consanguinea, Lema spp., Leptinotarsa decemlineata, Leucoptera spp., Lissorhoptrus oryzophilus, Lixus spp., Luperodes spp., Lyctus spp., Megascelis spp., Melanotus spp., Meligethes aeneus, Melolontha spp., Migdolus spp., Monochamus spp., Naupactus xanthographus, Niptus hololeucus, Oryctes rhinoceros, Oryzaephilus surinamensis, Oryzaphagus oryzae, Otiorrhynchus spp., Oxycetonia jucunda, Phaedon cochleariae, Phyllophaga spp., Phyllotreta spp., Popillia japonica, Premnotrypes spp., Psylliodes spp., Ptinus spp., Rhizobius ventralis, Rhizopertha dominica, Sitophilus spp., Sphenophorus spp., Sternechus spp., Symphyletes spp., Tanymecus spp., Tenebrio molitor, Tribolium spp., Trogoderma spp., Tychius spp., Xylotrechus spp., Zabrus spp..

Aus der Ordnung der Collembola z.B. Onychiurus armatus.

Aus der Ordnung der Diplopoda z.B. Blaniulus guttulatus.

Aus der Ordnung der Diptera z.B. Aedes spp., Agromyza spp., Anastrepha spp., Anopheles spp., Asphondylia spp., Bactrocera spp., Bibio hortulanus, Calliphora erythrocephala, Ceratitis capitata, Chironomus spp., Chrysomyia spp., Cochliomyia spp., Contarinia spp., Cordylobia anthropophaga, Culex spp., Cuterebra spp., Dacus oleae, Dasyneura spp., Delia spp., Dermatobia hominis, Drosophila spp., Echinocnemus spp., Fannia spp., Gastrophilus spp., Hydrellia spp., Hylemyia spp., Hyppobosca spp., Hypoderma spp., Liriomyza spp.. Lucilia spp., Musca spp., Nezara spp., Oestrus spp., Oscinella frit, Pegomyia spp., Phorbia spp., Prodiplosis spp., Psila rosae, Rhagoletis spp., Stomoxys spp., Tabanus spp., Tannia spp., Tetanops spp., Tipula spp..

Aus der Klasse der Gastropoda z.B. Arion spp., Biomphalaria spp., Bulinus spp., Deroceras spp., Galba spp., Lymnaea spp., Oncomelania spp., Pomacea spp., Succinea spp..

Aus der Klasse der Helminthen z.B. Ancylostoma duodenale, Ancylostoma ceylanicum, Acylostoma braziliensis, Ancylostoma spp., Ascaris lubricoides, Ascaris spp., Brugia malayi, Brugia timori, Bunostomum spp., Chabertia spp., Clonorchis spp., Cooperia spp., Dicrocoelium spp, Dictyocaulus filaria, Diphyllobothrium latum, Dracunculus medinensis, Echinococcus granulosus, Echinococcus multilocularis, Enterobius vermicularis, Faciola spp., Haemonchus spp., Heterakis spp., Hymenolepis nana, Hyostrongulus spp., Loa Loa, Nematodirus spp., Oesophagostomum spp., Opisthorchis spp., Onchocerca volvulus, Ostertagia spp., Paragonimus spp., Schistosomen spp, Strongyloides fuelleborni, Strongyloides stercoralis, Stronyloides spp., Taenia saginata, Taenia solium, Trichinella spiralis, Trichinella nativa, Trichinella britovi, Trichinella nelsoni, Trichinella pseudopsiralis, Trichostrongulus spp., Trichuris trichuria, Wuchereria bancrofti.

Weiterhin lassen sich Protozoen, wie Eimeria, bekämpfen.

Aus der Ordnung der Heteroptera z.B. Anasa tristis, Antestiopsis spp., Blissus spp., Calocoris spp., Campylomma livida, Cavelerius spp., Cimex spp., Collaria spp., Creontiades dilutus, Dasynus piperis, Dichelops furcatus, Diconocoris hewetti, Dysdercus spp., Euschistus spp., Eurygaster spp., Heliopeltis spp., Horcias nobilellus, Leptocorisa spp., Leptoglossus phyllopus, Lygus spp., Macropes excavatus, Miridae, Monalonion atratum, Nezara spp., Oebalus spp., Pentomidae, Piesma quadrata, Piezodorus spp., Psallus spp., Pseudacysta persea, Rhodnius spp., Sahlbergella singularis, Scaptocoris castanea, Scotinophora spp., Stephanitis nashi, Tibraca spp., Triatoma spp.

Aus der Ordnung der Homoptera z.B. Acyrthosipon spp., Acrogonia spp., Aeneolamia spp., Agonoscena spp., Aleurodes spp., Aleurolobus barodensis, Aleurothrixus spp., Amrasca spp., Anuraphis cardui, Aonidiella spp., Aphanostigma piri, Aphis spp., Arboridia apicalis, Aspidiella spp., Aspidiotus spp., Atanus spp., Aulacorthum solani, Bemisia spp., Brachycaudus helichrysii, Brachycolus spp., Brevicoryne brassicae, Calligypona marginata, Carneocephala fulgida, Ceratovacuna lanigera, Cercopidae, Ceroplastes spp., Chaetosiphon fragaefolii, Chionaspis tegalensis, Chlorita onukii, Chromaphis juglandicola, Chrysomphalus ficus, Cicadulina mbila, Coccomytilus halli, Coccus spp., Cryptomyzus ribis, Dalbulus spp., Dialeurodes spp., Diaphorina spp., Diaspis spp., Drosicha spp., Dysaphis spp., Dysmicoccus spp., Empoasca spp., Eriosoma spp., Erythroneura spp., Euscelis bilobatus, Ferrisia spp., Geococcus coffeae, Hieroglyphus spp., Homalodisca coagulata, Hyalopterus arundinis, Icerya spp., Idiocerus spp., Idioscopus spp., Laodelphax striatellus, Lecanium spp., Lepidosaphes spp., Lipaphis erysimi, Macrosiphum spp., Mahanarva spp., Melanaphis sacchari, Metcalfiella spp., Metopolophium dirhodum, Monellia costalis, Monelliopsis pecanis, Myzus spp., Nasonovia ribisnigri, Nephotettix spp., Nilaparvata lugens, Oncometopia spp., Orthezia praelonga, Parabemisia myricae, Paratrioza spp., Parlatoria spp., Pemphigus spp., Peregrinus maidis, Phenacoccus spp., Phloeomyzus passerinii, Phorodon humuli, Phyllo-xera spp., Pinnaspis aspidistrae, Planococcus spp., Protopulvinaria pyriformis, Pseudaulacaspis pentagona, Pseudococcus spp., Psylla spp., Pteromalus spp., Pyrilla spp., Quadraspidiotus spp., Quesada gigas, Rastrococcus spp., Rhopalosiphum spp., Saissetia spp., Scaphoides titanus, Schizaphis graminum, Selenaspidus articulatus, Sogata spp., Sogatella furcifera, Sogatodes spp., Stictocephala festina, Tenalaphara malayensis, Tinocallis caryaefoliae, Tomaspis spp., Toxoptera spp., Trialeurodes spp., Trioza spp., Typhlocyba spp., Unaspis spp., Viteus vitifolii, Zygina spp..

Aus der Ordnung der Hymenoptera z.B. Athalia spp., Diprion spp., Hoplocampa spp., Lasius spp., Monomorium pharaonis, Vespa spp..

Aus der Ordnung der Isopoda z.B. Armadillidium vulgare, Oniscus asellus, Porcellio scaber.

Aus der Ordnung der Isoptera z.B. Acromyrmex spp., Atta spp., Cornitermes cumulans, Microtermes obesi, Odontotermes spp., Reticulitermes spp,

Aus der Ordnung der Lepidoptera z.B. Acronicta major, Adoxophyes spp., Aedia leucomelas, Agrotis spp., Alabama spp., Amyelois transitella, Anarsia spp., Anticarsia spp., Argyroploce spp., Barathra brassicae, Borbo cinnara, Bucculatrix thurberiella, Bupalus piniarius, Busseola spp., Cacoecia spp., Caloptilia theivora, Capua reticulana, Carpocapsa pomonella, Carposina niponensis, Cheimatobia brumata, Chilo spp., Choristoneura spp., Clysia ambiguella, Cnaphalocerus spp., Cnephasia spp., Conopomorpha spp., Conotrachelus spp., Copitarsia spp., Cydia spp., Dalaca noctuides, Diaphania spp., Diatraea saccharalis, Earias spp., Ecdytolopha aurantium, Elasmopalpus lignosellus, Eldana saccharina, Ephestia kuehniella, Epinotia spp., Epiphyas postvittana, Etiella spp., Eulia spp., Eupoecilia ambiguella, Euproctis spp., Euxoa spp., Feltia spp., Galleria mellonella, Gracillaria spp., Grapholitha spp., Hedylepta spp., Helicoverpa spp., Heliothis spp., Hofmannophila pseudospretella, Homoeosoma spp., Homona spp., Hyponomeuta padella, Kakivoria flavofasciata, Laphygma spp., Laspeyresia molesta, Leucinodes orbonalis, Leucoptera spp., Lithocolletis spp., Lithophane antennata, Lobesia spp., Loxagrotis albicosta, Lymantria spp., Lyonetia spp., Malacosoma neustria, Maruca testulalis, Mamestra brassicae, Mocis spp., Mythimna separata, Nymphula spp., Oiketicus spp., Oria spp., Orthaga spp., Ostrinia spp., Oulema oryzae, Panolis flammea, Parnara spp., Pectinophora spp., Perileucoptera spp., Phthorimaea spp., Phyllocnistis citrella, Phyllonorycter spp., Pieris spp., Platynota stultana, Plusia spp., Plutella xylostella, Prays spp., Prodenia spp., Protoparce spp., Pseudaletia spp., Pseudoplusia includens, Pyrausta nubilalis, Rachiplusia nu, Schoenobius spp., Scirpophaga spp., Scotia segetum, Sesamia spp., Sparganothis spp., Spodoptera spp., Stathmopoda spp., Stomopteryx subsecivella, Synanthedon spp., Tecia solanivora, Thermesia gemmatalis, Tinea pellionella, Tineola bisselliella, Tortrix spp., Trichoplusia spp., Tuta absoluta, Virachola spp..

Aus der Ordnung der Orthoptera z.B. Acheta domesticus, Blatta orientalis, Blattella germanica, Dichroplus spp., Gryllotalpa spp., Leucophaea maderae, Locusta spp., Melanoplus spp., Periplaneta americana, Schistocerca gregaria.

Aus der Ordnung der Siphonaptera z.B. Ceratophyllus spp., Xenopsylla cheopis.

Aus der Ordnung der Symphyla z.B. Scutigerella spp..

Aus der Ordnung der Thysanoptera z.B. Anaphothrips obscurus, Baliothrips biformis, Drepanothris reuteri, Enneothrips flavens, Frankliniella spp., Heliothrips spp., Hercinothrips femoralis, Rhipiphorothrips cruentatus, Scirtothrips spp., Taeniothrips cardamoni, Thrips spp..

Aus der Ordnung der Thysanura z.B. Lepisma saccharina.

Zu den pflanzenparasitären Nematoden gehören z.B. Aphelenchoides spp., Bursaphelenchus spp., Ditylenchus spp., Globodera spp., Heterodera spp., Longidorus spp., Meloidogyne spp., Pratylenchus spp., Radopholus similis, Trichodorus spp., Tylenchulus semipenetrans, Xiphinema spp..

Die Wirkstoffe können in die üblichen Formulierungen überführt werden, wie Lösungen, Emulsionen, Spritzpulver, wasser- und ölbasierte Suspensionen, Pulver, Stäubemittel, Pasten, lösliche Pulver, lösliche Granulate, Streugranulate, Suspensions-Emulsions-Konzentrate, Wirkstoff-imprägnierte Naturstoffe, Wirkstoff-imprägnierte synthetische Stoffe, Düngemittel sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln. Die Herstellung der Formulierungen erfolgt entweder in geeigneten Anlagen oder auch vor oder während der Anwendung.

Als Hilfsstoffe können solche Stoffe Verwendung finden, die geeignet sind, dem Mittel selbst oder und/oder davon abgeleitete Zubereitungen (z.B. Spritzbrühen, Saatgutbeizen) besondere Eigenschaften zu verleihen, wie bestimmte technische Eigenschaften und/oder auch besondere biologische Eigenschaften. Als typische Hilfsmittel kommen in Frage: Streckmittel, Lösemittel und Trägerstoffe.

Als Streckmittel eignen sich z.B. Wasser, polare und unpolare organische chemische Flüssigkeiten z.B. aus den Klassen der aromatischen und nicht-aromatischen Kohlenwasserstoffe (wie Paraffine, Alkylbenzole, Alkylnaphthaline, Chlorbenzole), der Alkohole und Polyole (die ggf. auch substituiert, verethert und/oder verestert sein können), der Ketone (wie Aceton, Cyclohexanon), Ester (auch Fette und Öle) und (poly-)Ether, der einfachen und substituierten Amine, Amide, Lactame (wie N-Alkylpyrrolidone) und Lactone, der Sulfone und Sulfoxide (wie Dimethylsysulfoxid).

Im Falle der Benutzung von Wasser als Streckmittel können z.B. auch organische Lösemittel als Hilfslösungsmittel verwendet werden. Als flüssige Lösemittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylsulfoxid, sowie Wasser.

Erfindungsgemäß bedeutet Trägerstoff eine natürliche oder synthetische, organische oder anorganische Substanz, welcher fest oder flüssig sein kann, mit welchen die Wirkstoffe zur besseren Anwendbarkeit, insbesondere zum Aufbringen auf Pflanzen oder Pflanzenteile, gemischt oder verbunden sind. Der feste oder flüssige Trägerstoff ist im Allgemeinen inert und sollte in der Landwirtschaft verwendbar sein.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Papier, Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage nicht-ionische und/oder ionische Stoffe, z.B. aus den Klassen der Alkohol-POE- und/oder POP-Ether, Säure- und/oder POP- POE-Ester, Alkyl-Aryl- und/oder POP- POE-Ether, Fett- und/oder POP-POE-Addukte, POE- und/oder POP-Polyol Derivate, POE- und/oder POP-Sorbitan- oder-Zucker-Addukte, Alky- oder Aryl-Sulfate, Sulfonate und Phosphate oder die entsprechenden PO-Ether-Addukte. Ferner geeignete Oligo- oder Polymere, z.B. ausgehend von vinylischen Monomeren, von Acrylsäure, aus EO und/oder PO allein oder in Verbindung mit z.B. (poly-) Alkoholen oder (poly-) Aminen. Ferner können Einsatz finden Lignin und seine Sulfonsäure-Derivate, einfache und modifizierte Cellulosen, aromatische und/oder aliphatische Sulfonsäuren sowie deren Addukte mit Formaldehyd.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Weitere Additive können Duftstoffe, mineralische oder vegetabile gegebenenfalls modifizierte Öle, Wachse und Nährstoffe (auch Spurennährstoffe), wie Salze von Eisen, Mangan, Bor, Kupfer, Kobalt, Molybdän und Zink sein.

Weiterhin enthalten sein können Stabilisatoren wie Kältestabilisatoren, Konservierungsmittel, Oxidationsschutzmittel, Lichtschutzmittel oder andere die chemische und / oder physikalische Stabilität verbessernde Mittel.

Die Formulierungen enthalten im allgemeinen zwischen 0,01 und 98 Gew.-% Wirkstoff, vorzugsweise zwischen 0,5 und 90%.

Die erfindungsgemäßen Wirkstoffe können als solche oder in ihren Formulierungen auch in Mischung mit einem oder mehreren geeigneten Fungiziden, Bakteriziden, Akariziden, Nematiziden, Insektiziden, Mikrobiziden, Düngemitteln, Lockstoffen, Sterilantien, Synergisten, Safenern, Semiochemicals und/oder Pflanzenwachstumsregulatoren verwendet werden, um so z.B. das Wirkungsspektrum zu verbreitern, die Wirkdauer zu verlängern, die Wirkgeschwindigkeit zu steigern, Repellenz zu verhindern oder Resistenzentwicklungen vorzubeugen. Des weiteren können solche Wirkstoffkombinationen das Pflanzenwachstum verbessern, die Toleranz gegenüber hohen oder niedrigen Temperaturen, gegen Trockenheit oder gegen Wasser- bzw. Bodensalzgehalt erhöhen, die Blühleistung steigern, die Ernte erleichtern und Ernteerträge steigern, die Reife beschleunigen, die Qualität und/oder den Ernährungswert der Ernteprodukte steigern, die Lagerfähigkeit verlängern und/oder die Bearbeitbarkeit der Ernteprodukte verbessern. Durch Kombination der erfindungsgemäßen Wirkstoffe mit Mischpartnern erhält man synergistische Effekte, d.h. die Wirksamkeit der jeweiligen Mischung ist größer als aufgrund der Wirksamkeiten der Einzelkomponenten zu erwarten war. Generell können die Kombinationen sowohl als Saatgutanwendungen als auch in Vor-, Tank- oder Fertigmischungen verwendet werden.

Jeder zusätzliche Wirkstoff kann in einem weiten Bereich, bevorzugt in einem Verhältnis von 100:1 bis 1.100, besonders bevorzugt von 5:1 bis 1:5 mit den erfindungsgemäßen Wirkstoffen gemischt werden.

### Besonders günstige Mischungspartner sind z.B. die folgenden

### Insektizide / Akarizide / Nematizide:

Die hier mit ihrem "common name" genannten Wirkstoffe sind bekannt und beispielsweise im Pestizidhandbuch ("The Pesticide Manual" 14th Ed., British Crop Protection Council 2006) beschrieben oder im Internet recherchierbar (z.B. http://www.alanwood.net/pesticides).

### (1) Acetylcholinesterase (AChE) Inhibitoren, wie beispielsweise

Carbamate, z.B. Alanycarb, Aldicarb, Bendiocarb, Benfuracarb, Butocarboxim, Butoxycarboxim, Carbaryl, Carbofuran, Carbosulfan, Ethiofencarb, Fenobucarb, Formetanate, Furathiocarb, Isoprocarb, Methiocarb, Methomyl, Metolcarb, Oxamyl, Pirimicarb, Propoxur, Thiodicarb, Thiofanox, Triazamate, Trimethacarb, XMC und Xylylcarb; oder

Organophosphate, z.B. Acephate, Azamethiphos, Azinphos-ethyl, Azinphos-methyl, Cadusafos, Chlorethoxyfos, Chlorfenvinphos, Chlormephos, Chlorpyrifos, Chlorpyrifos-methyl, Coumaphos, Cyanophos, Demeton-S-methyl, Diazinon, Dichlorvos/DDVP, Dicrotophos, Dimethoate, Dimethylvinphos, Disulfoton, EPN, Ethion, Ethoprophos, Famphur, Fenamiphos, Fenitrothion, Fenthion, Fosthiazate, Heptenophos, Imicyafos, Isofenphos, Isopropyl O-(methoxyaminothio-phosphoryl) salicylat, Isoxathion, Malathion, Mecarbam, Methamidophos, Methidathion, Mevinphos, Monocrotophos, Naled, Omethoate, Oxydemeton-methyl, Parathion, Parathion-methyl, Phenthoate, Phorate, Phosalone, Phosmet, Phosphamidon, Phoxim, Pirimiphos-methyl, Profenofos, Propetamphos, Prothiofos, Pyraclofos, Pyridaphenthion, Quinalphos, Sulfotep, Tebupirimfos, Temephos, Terbufos, Tetrachlorvinphos, Thiometon, Triazophos, Triclorfon und Vamidothion.

### (2) GABA-gesteuerte Chlorid-Kanal-Antagonisten, wie beispielsweise

Cyclodien-organochlorine, z.B. Chlordane und Endosulfan; oder
Phenylpyrazole (Fiprole), z.B. Ethiprole und Fipronil.

### (3) Natrium-Kanal-Modulatoren / Spannungsabhängige Natrium-Kanal-Blocker, wie beispielsweise

Pyrethroide, z.B. Acrinathrin, Allethrin, d-cis-trans Allethrin, d-trans Allethrin, Bifenthrin, Bioallethrin, Bioallethrin S-cyclopentenyl Isomer, Bioresmethrin, Cycloprothrin, Cyfluthrin, beta-Cyfluthrin, Cyhalothrin, lambda-Cyhalothrin, gamma-Cyhalothrin, Cypermethrin, alpha-Cypermethrin, beta-Cypermethrin, theta-Cypermethrin, zeta-Cypermethrin, Cyphenothrin [(1R)-trans-Isomere], Deltamethrin, Empenthrin [(EZ)-(1R)-Isomere), Esfenvalerate, Etofenprox, Fenpropathrin, Fenvalerate, Flucythrinate, Flumethrin, tau-Fluvalinate, Halfenprox, Imiprothrin, Kadethrin, Permethrin, Phenothrin [(1R)-trans-Isomer), Prallethrin, Pyrethrine (pyrethrum), Resmethrin, Silafluofen, Tefluthrin, Tetramethrin, Tetramethrin [(1R)- Isomere)], Tralomethrin und Transfluthrin; oder

DDT; oder Methoxychlor.

### (4) Nikotinerge Acetylcholin-Rezeptor (nAChR) Agonisten, wie beispielsweise

Neonikotinoide, z.B. Acetamiprid, Clothianidin, Dinotefuran, Imidacloprid, Nitenpyram, Thiacloprid und Thiamethoxam; oder

Nikotin.

### (5) Nikotinerge Acetylcholin-Rezeptor (nAChR) allosterische Aktivatoren, wie beispielsweise

Spinosine, z.B. Spinetoram und Spinosad.

### (6) Chlorid-Kanal-Aktivatoren, wie beispielsweise

Avermectine/Milbemycine, z.B. Abamectin, Emamectin-benzoat, Lepimectin und Milbemectin.

### (7) Juvenilhormon-Imitatoren, wie beispielsweise

Juvenilhormon-Analoge, z.B. Hydroprene, Kinoprene und Methoprene; oder
Fenoxycarb; oder Pyriproxyfen.

### (8) Wirkstoffe mit unbekannten oder nicht spezifischen Wirkmechanismen, wie beispielsweise Alkylhalide, z.B. Methylbromid und andere Alkylhalide; oder

Chloropicrin; oder Sulfurylfluorid; oder Borax; oder Brechweinstein.

### (9) Selektive Fraßhemmer, z.B. Pymetrozine; oder Flonicamid.

### (10) Milbenwachstumsinhibitoren, z.B. Clofentezine, Hexythiazox und Diflovidazin; oder

Etoxazole.

### (11) Mikrobielle Disruptoren der Insektendarmmembran, z.B. Bacillus thuringiensis Subspezies israelensis, Bacillus sphaericus, Bacillus thuringiensis Subspezies aizawai, Bacillus thuringiensis Subspezies kurstaki, Bacillus thuringiensis Subspezies tenebrionis und BT Pflanzenproteine: Cry1Ab, Cry1Ac, Cry1Fa, Cry2Ab, mCry3A, Cry3Ab, Cry3Bb, Cry34/35Ab1.

### (12) Inhibitoren der oxidativen Phosphorylierung, ATP-Disruptoren, wie beispielsweise Diafenthiuron; oder

Organozinnverbindungen, z.B. Azocyclotin, Cyhexatin und Fenbutatin-oxid; oder

Propargite; oder Tetradifon.

### (13) Entkoppler der oxidativen Phoshorylierung durch Unterbrechung des H-Protongradienten, wie beispielsweise Chlorfenapyr, DNOC und Sulfluramid.

### (14) Nikotinerge Acetylcholin-Rezeptor-Antagonisten, wie beispielsweise Bensultap, Cartap-hydrochlorid, Thiocyclam und Thiosultap-Natrium.

### (15) Inhibitoren der Chitinbiosynthese, Typ 0, wie beispielsweise Bistrifluron, Chlorfluazuron, Diflubenzuron, Flucycloxuron, Flufenoxuron, Hexaflumuron, Lufenuron, Novaluron, Noviflumuron, Teflubenzuron und Triflumuron.

### (16) Inhibitoren der Chitinbiosynthese, Typ 1, wie beispielsweise Buprofezin.

### (17) Häutungsstörende Wirkstoffe, Dipteran, wie beispielsweise Cyromazine.

### (18) Ecdyson-Rezeptor Agonisten, wie beispielsweise Chromafenozide, Halofenozide, Methoxyfenozide und Tebufenozide.

### (19) Oktopaminerge Agonisten, wie beispielsweise Amitraz.

### (20) Komplex-III-Elektronentransportinhibitoren, wie beispielsweise Hydramethylnon; oder Acequinocyl; oder Fluacrypyrim.

### (21) Komplex-I-Elektronentransportinhibitoren, beispielsweise

METI-Akarizide, z.B. Fenazaquin, Fenpyroximate, Pyrimidifen, Pyridaben, Tebufenpyrad und Tolfenpyrad; oder

Rotenone (Derris).

### (22) Spannungsabhängige Natriumkanal-Blocker, z.B. Indoxacarb; oder Metaflumizone.

### (23) Inhibitoren der Acetyl-CoA-Carboxylase, wie beispielsweise

Tetron- und Tetramsäurederivate, z.B. Spirodiclofen, Spiromesifen und Spirotetramat.

### (24) Komplex-IV-Elektronentransportinhibitoren, wie beispielsweise

Phosphine, z.B. Aluminiumphosphid, Calciumphosphid, Phosphin und Zinkphosphid; oder
Cyanid.

### (25) Komplex-II-Elektronentransportinhibitoren, wie beispielsweise Cyenopyrafen.

### (28) Ryanodinrezeptor-Effektoren, wie beispielsweise

Diamide, z.B. Chlorantraniliprole und Flubendiamide.

Weitere Wirkstoffe mit unbekanntem Wirkmechanismus, wie beispielsweise Amidoflumet, Azadirachtin, Benclothiaz, Benzoximate, Bifenazate, Bromopropylate, Chinomethionat, Cryolite, Cyantraniliprole (Cyazypyr), Cyflumetofen, Dicofol, Diflovidazin, Fluensulfone, Flufenerim, Flufiprole, Fluopyram, Fufenozide, Imidaclothiz, Iprodione, Pyridalyl, Pyrifluquinazon und Iodmethan; desweiteren Präparate auf Basis von Bacillus firmus (I-1582, BioNeem, Votivo) sowie folgende bekannte wirksame Verbindungen:
3-Brom-N-{2-brom-4-chlor-6-[(1-cyclopropylethyl)carbamoyl]phenyl}-1-(3-chlorpyridin-2-yl)-1H-pyrazol-5-carboxamid (bekannt aus WO2005/077934), 4-{[(6-Brompyrid-3-yl)methyl](2-fluorethyl)amino}furan-2(5H)-on (bekannt aus WO2007/115644), 4-{[(6-Fluorpyrid-3-yl)methyl](2,2-difluorethyl)amino}furan-2(5H)-on (bekannt aus WO2007/115644), 4-{[(2-Chlor-1,3-thiazol-5-yl)methyl](2-fluorethyl)amino}furan-2(5H)-on (bekannt aus WO2007/115644), 4-{[(6-Chlorpyrid-3-yl)methyl](2-fluorethyl)amino}furan-2(5H)-on (bekannt aus WO2007/115644), 4-{[(6-Chlorpyrid-3-yl)methyl](2,2-difluorethyl)amino}furan-2(5H)-on (bekannt aus WO2007/115644), 4-{[(6-Chlor-5-fluorpyrid-3-yl)methyl](methyl)amino}furan-2(5H)-on (bekannt aus WO2007/115643), 4-{[(5,6-Dichlorpyrid-3-yl)methyl](2-fluorethyl)amino}furan-2(5H)-on (bekannt aus WO2007/115646), 4-{[(6-Chlor-5-fluorpyrid-3-yl)methyl](cyclopropyl)amino}furan-2(5H)-on (bekannt aus WO2007/115643), 4-{[(6-Chlorpyrid-3-yl)methyl](cyclopropyl)amino}furan-2(5H)-on (bekannt aus EP-A-0 539 588), 4-{[(6-Chlorpyrid-3-yl)methyl](methyl)amino}furan-2(5H)-on (bekannt aus EP-A-0 539 588), {[1-(6-Chlorpyridin-3-yl)ethyl](methyl)oxido-λ⁴-sulfanyliden}cyanamid (bekannt aus WO2007/149134) und seine Diastereomere {[(1R)-1-(6-Chlorpyridin-3-yl)ethyl](methyl)oxido-λ⁴-sulfanyliden}cyanamid (A) und {[(1S)-1-(6-Chlorpyridin-3-yl)ethyl](methyl)oxido-λ⁴-sulfanyliden}cyanamid (B) (ebenfalls bekannt aus WO2007/149134) sowie Sulfoxaflor (ebenfalls bekannt aus WO2007/149134) und seine Diastereomere [(R)-Methyl(oxido){(1R)-1-[6-(trifluormethyl)pyridin-3-yl]ethyl}-λ⁴-sulfanyliden]cyanamid (A1) und [(S)-Methyl(oxido){(1S)-1-[6-(trifluormethyl)pyridin-3-yl]ethyl}-λ⁴-sulfanyliden]cyanamid (A2), bezeichnet als Diastereomerengruppe A (bekannt aus WO 2010/074747, WO 2010/074751), [(R)-Methyl(oxido){(1S)-1-[6-(trifluormethyl)pyridin-3-yl]ethyl}-λ⁴-sulfanyliden]cyanamid (B1) und [(S)-Methyl(oxido){(1R)-1-[6-(trifluormethyl)pyridin-3-yl]ethyl}-λ⁴-sulfanyliden]cyanamid (B2), bezeichnet als Diastereomerengruppe B (ebenfalls bekannt aus WO 2010/074747, WO 2010/074751) und 11-(4-Chlor-2,6-dimethylphenyl)-12-hydroxy-1,4-dioxa-9-azadispiro[4.2.4.2]tetradec-11-en-10-on (bekannt aus WO2006/089633), 3-(4'-Fluor-2,4-dimethylbiphenyl-3-yl)-4-hydroxy-8-oxa-1-azaspiro[4.5]dec-3-en-2-on (bekannt aus WO2008/067911), 1-{2-Fluor-4-methyl-5-[(2,2,2-trifluorethyl)sulfinyl]phenyl}-3-(trifluormethyl)-1H-1,2,4-triazol-5-amin (bekannt aus WO2006/043635), [(3S,4aR,12R,12aS,12bS)-3-[(Cyclopropylcarbonyl)oxy]-6,12-dihydroxy-4,12b-dimethyl-11-oxo-9-(pyridin-3-yl)-1,3,4,4a,5,6,6a,12,12a,12b-decahydro-2H,11H-benzo[f]pyrano[4,3-b]chromen-4-yl]methylcyclopropancarboxylat (bekannt aus WO2008/066153), 2-Cyan-3-(difluormethoxy)-N,N-dimethylbenzolsulfonamid (bekannt ausWO2006/056433), 2-Cyan-3-(difluormethoxy)-N-methylbenzolsulfonamid (bekannt aus WO2006/100288), 2-Cyan-3-(difluormethoxy)-N-ethylbenzolsulfonamid (bekannt aus WO2005/035486), 4-(Difluormethoxy)-N-ethyl-N-methyl-1,2-benzothiazol-3-amin-1,1-dioxid (bekannt aus WO2007/057407), N-[1-(2,3-Dimethylphenyl)-2-(3,5-dimethylphenyl)ethyl]-4,5-dihydro-1,3-thiazol-2-a min (bekannt aus WO2008/104503), {1'-[(2E)-3-(4-Chlorphenyl)prop-2-en-1-yl]-5-fluorspiro[indol-3,4'-piperidin]-1(2H)-yl}(2-chlorpyridin-4-yl)methanon (bekannt aus WO2003/106457), 3-(2,5-Dimethylphenyl)-4-hydroxy-8-methoxy-1,8-diazaspiro[4.5]dec-3-en-2-on (bekannt aus WO2009/049851), 3-(2,5-Dimethylphenyl)-8-methoxy-2-oxo-1,8-diazaspiro[4.5]dec-3-en-4-yl-ethylcarbonat (bekannt aus WO2009/049851), 4-(But-2-in-1-yloxy)-6-(3,5-dimethylpiperidin-1-yl)-5-fluorpyrimidin (bekannt aus WO2004/099160), (2,2,3,3,4,4,5,5-Octafluorpentyl)(3,3,3-trifluorpropyl)malononitril (bekannt aus WO2005/063094), (2,2,3,3,4,4,5,5-Octafluorpentyl)(3,3,4,4,4-pentafluorbutyl)malononitril (bekannt aus WO2005/063094), 8-[2-(Cyclopropylmethoxy)-4-(trifluormethyl)phenoxy]-3-[6-(trifluormethyl)pyridazin-3-yl]-3-azabicyclo[3.2.1]octan (bekannt aus WO2007/040280), 2-Ethyl-7-methoxy-3-methyl-6-[(2,2,3,3-tetrafluor-2,3-dihydro-1,4-benzodioxin-6-yl)oxy]chinolin-4-yl-methylcarbonat (bekannt aus JP2008/110953), 2-Ethyl-7-methoxy-3-methyl-6-[(2,2,3,3-tetrafluor-2,3-dihydro-1,4-benzodioxin-6-yl)oxy]chinolin-4-ylacetat (bekannt aus JP2008/110953), PF1364 (CAS-Reg.Nr. 1204776-60-2) (bekannt aus JP2010/018586), 5-[5-(3,5-Dichlorphenyl)-5-(trifluormethyl)-4,5-dihydro-1,2-oxazol-3-yl]-2-(1H-1,2,4-triazol-1-yl)benzonitril (bekannt aus WO2007/075459), 5-[5-(2-Chlorpyridin-4-yl)-5-(trifluormethyl)-4,5-dihydro-1,2-oxazol-3-yl]-2-(1H-1,2,4-triazol-1-yl)benzonitril (bekannt aus WO2007/075459), 4-[5-(3,5-Dichlorphenyl)-5-(trifluormethyl)-4,5-dihydro-1,2-oxazol-3-yl]-2-methyl-N-{2-oxo-2-[(2,2,2-trifluorethyl)amino]ethyl}benzamid (bekannt aus WO2005/085216), 4-{[(6-Chlorpyridin-3-yl)methyl](cyclopropyl)amino}-1,3-oxazol-2(5H)-on, 4-{[(6-Chlorpyridin-3-yl)methyl](2,2-difluorethyl)amino}-1,3-oxazol-2(5H)-on, 4-{[(6-Chlorpyridin-3-yl)methyl](ethyl)amino}-1,3-oxazol-2(5H)-on, 4-{[(6-Chlorpyridin-3-yl)methyl](methyl)amino}-1,3-oxazol-2(5H)-on (alle bekannt aus WO2010/005692), NNI-0711 (bekannt aus WO2002096882), 1-Acetyl-N-[4-(1,1,1,3,3,3-hexafluor-2-methoxypropan-2-yl)-3-isobutylphenyl]-N-isobutyryl-3,5-dimethyl-1H-pyrazol-4-carboxamid (bekannt aus WO2002096882), Methyl-2-[2-({[3-brom-1-(3-chlorpyridin-2-yl)-1H-pyrazol-5-yl]carbonyl}amino)-5-chlor-3-methylbenzoyl]-2-methylhydrazincarboxylat (bekannt aus WO2005/085216), Methyl-2-[2-({[3-brom-1-(3-chlorpyridin-2-yl)-1H-pyrazol-5-yl]carbonyl}amino)-5-cyan-3-methylbenzoyl]-2-ethylhydrazincarboxylat (bekannt aus WO2005/085216), Methyl-2-[2-({[3-brom-1-(3-chlorpyridin-2-yl)-1H-pyrazol-5-yl]carbonyl}amino)-5-cyan-3-methylbenzoyl]-2-methylhydrazincarboxylat (bekannt aus WO2005/085216), Methyl-2-[3,5-dibrom-2-({[3-brom-1-(3-chlorpyridin-2-yl)-1H-pyrazol-5-yl]carbonyl}amino)benzoyl]-1,2-diethylhydrazincarboxylat (bekannt aus WO2005/085216), Methyl-2-[3,5-dibrom-2-({[3-brom-1-(3-chlorpyridin-2-yl)-1H-pyrazol-5-yl]carbonyl}amino)benzoyl]-2-ethylhydrazincarboxylat (bekannt aus WO2005/085216), (5RS,7RS;5RS,7SR)-1-(6-Chlor-3-pyridylmethyl)-1,2,3,5,6,7-hexahydro-7-methyl-8-nitro-5-propoxyimidazo[1,2-a]pyridin (bekannt aus WO2007/101369), 2-{6-[2-(5-Fluorpyridin-3-yl)-1,3-thiazol-5-yl]pyridin-2-yl}pyrimidin (bekannt aus WO2010/006713), 2-{6-[2-(Pyridin-3-yl)-1,3-thiazol-5-yl]pyridin-2-yl}pyrimidin (bekannt aus WO2010/006713), 1-(3-Chlorpyridin-2-yl)-N-[4-cyan-2-methyl-6-(methylcarbamoyl)phenyl]-3-{[5-(trifluormethyl)-1H-tetrazol-1-yl]methyl}-1H-pyrazol-5-carboxamid (bekannt aus WO2010/069502), 1-(3-Chlorpyridin-2-yl)-N-[4-cyan-2-methyl-6-(methylcarbamoyl)phenyl]-3-{[5-(trifluormethyl)-2H-tetrazol-2-yl]methyl}-1H-pyrazol-5-carboxamid (bekannt aus WO2010/069502), N-[2-(tert-Butylcarbamoyl)-4-cyan-6-methylphenyl]-1-(3-chlorpyridin-2-yl)-3-{[5-(trifluormethyl)-1H-tetrazol-1-yl]methyl}-1H-pyrazol-5-carboxamid (bekannt aus WO2010/069502), N-[2-(tert-Butylcarbamoyl)-4-cyan-6-methylphenyl]-1-(3-chlorpyridin-2-yl)-3-{[5-(trifluormethyl)-2H-tetrazol-2-yl]methyl}-1H-pyrazol-5-carboxamid (bekannt aus WO2010/069502) und (1E)-N-[(6-Chlorpyridin-3-yl)methyl]-N'-cyan-N-(2,2-difluorethyl)ethanimidamid (bekannt aus WO2008/009360).

In einer bevorzugten Ausführungsform der Erfindung wird den Pflanzenschutzmitteln zur Wirkungssteigerung zusätzlich ein Penetrationsförderer zugegeben. Als Penetrationsförderer kommen beispielsweise auch Substanzen in Betracht, die die Verfügbarkeit der Verbindungen der Formel (I) im Spritzbelag fördern. Dazu gehören beispielsweise mineralische oder vegetabile Öle. Als Öle kommen alle üblicherweise in agrochemischen Mitteln einsetzbaren mineralischen oder vegetabilen - gegebenenfalls modifizierte - Öle in Frage. Beispielhaft genannt seien Sonnenblumenöl, Rapsöl, Olivenöl, Rizinusöl, Rüböl, Maiskernöl, Baumwollsaatöl und Sojabohnenöl oder die Ester der genannten Öle. Bevorzugt sind Rapsöl, Sonnenblumenöl und deren Methyl- oder Ethylester, insbesondere Rapsölmethylester.

Die Konzentration an Penetrationsförderer kann in den erfindungsgemäßen Mitteln in einem weiten Bereich variiert werden. Bei einem formulierten Pflanzenschutzmittel liegt sie im allgemeinen bei 1 bis 95 Gew.-%, bevorzugt bei 1 bis 55 Gew.-%, besonders bevorzugt bei 15 - 40 Gew.-%. In den anwendungsfertigen Mitteln (Spritzbrühen) liegen die Konzentration im allgemeinen zwischen 0,1 und 10 g/l, bevorzugt zwischen 0,5 und 5 g/l.

Die erfindungsgemäßen Wirkstoffe können ferner beim Einsatz als Insektizide in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Die erfindungsgemäßen Wirkstoffe können ferner beim Einsatz als Insektizide in ihren handelsüblichen Formulierungen sowie in den aus diesen Formulierungen bereiteten Anwendungsformen in Mischungen mit Hemmstoffen vorliegen, die einen Abbau des Wirkstoffes nach Anwendung in der Umgebung der Pflanze, auf der Oberfläche von Pflanzenteilen oder in pflanzlichen Geweben vermindern.

Der Wirkstoffgehalt der aus den handelsüblichen Formulierungen bereiteten Anwendungsformen kann in weiten Bereichen variieren. Die Wirkstoffkonzentration der Anwendungsformen kann von 0,00000001 bis zu 95 Gew.-% Wirkstoff, vorzugsweise zwischen 0,00001 und 1 Gew.-% liegen.

Die Anwendung geschieht in einer den Anwendungsformen angepaßten üblichen Weise.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Beispielhaft seien die wichtigen Kulturpflanzen, wie Getreide (Weizen, Reis), Mais, Soja, Kartoffel, Zuckerrüben, Tomaten, Erbsen und andere Gemüsesorten, Baumwolle, Tabak, Raps, sowie Obstpflanzen (mit den Früchten Äpfel, Birnen, Zitrusfrüchten und Weintrauben) genannt. Unter Pflanzenteilen sollen alle oberirdischen und unterirdischen Teile und Organe der Pflanzen, wie Sproß, Blatt, Blüte und Wurzel verstanden werden, wobei beispielhaft Blätter, Nadeln, Stengel, Stämme, Blüten, Fruchtkörper, Früchte und Saatgut sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Saatgut.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen, Injizieren und bei Vermehrungsmaterial, insbesondere bei Saatgut, weiterhin durch ein- oder mehrschichtiges Umhüllen.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Die Begriffe "Teile" bzw. "Teile von Pflanzen" oder "Pflanzenteile" wurden oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ekto- und Endoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:
Aus der Ordnung der Anoplurida z.B. Haematopinus spp., Linognathus spp., Pediculus spp., Phtirus spp., Solenopotes spp..

Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp..

Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp..

Aus der Ordnung der Siphonapterida z.B. Pulex spp., Ctenocephalides spp. (Ctenocephalides canis, Ctenocephalides felis), Xenopsylla spp., Ceratophyllus spp..

Aus der Ordnung der Heteropterida z.B. Cimex spp., Triatoma spp., Rhodnius spp., Panstrongylus spp..

Aus der Ordnung der Blattarida z.B. Blatta orientalis, Periplaneta americana, Blattela germanica, Supella spp..

Aus der Unterklasse der Acari (Acarina) und den Ordnungen der Meta- sowie Mesostigmata z.B. Argas spp., Ornithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Sternostoma spp., Varroa spp..

Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so daß durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor und bei der Tierhaltung in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spoton), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, daß die erfindungsgemäßen Verbindungen eine hohe insektizide Wirkung gegen Insekten zeigen, die technische Materialien zerstören.

Beispielhaft und vorzugsweise - ohne jedoch zu limitieren - seien die folgenden Insekten genannt:
Käfer wie Hylotrupes bajulus, Chlorophorus pilosis, Anobium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus;
Hautflügler wie Sirex juvencus, Urocerus gigas, Urocerus gigas taignus, Urocerus augur;
Termiten wie Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus;
Borstenschwänze wie Lepisma saccharina.
Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz, Holzverarbeitungsprodukte und Anstrichmittel.

Die anwendungsfertigen Mittel können gegebenenfalls noch weitere Insektizide und gegebenenfalls noch ein oder mehrere Fungizide enthalten.

Hinsichtlich möglicher zusätzlicher Zumischpartner sei auf die oben genannten Insektizide und Fungizide verwiesen.

Zugleich können die erfindungsgemäßen Verbindungen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Weiter können die erfindungsgemäßen Verbindungen allein oder in Kombinationen mit anderen Wirkstoffen als Antifouling-Mittel eingesetzt werden.

Die Wirkstoffe eignen sich auch zur Bekämpfung von tierischen Schädlingen im Haushalts-, Hygiene- und Vorratsschutz, insbesondere von Insekten, Spinnentieren und Milben, die in geschlossenen Räumen, wie beispielsweise Wohnungen, Fabrikhallen, Büros, Fahrzeugkabinen u.ä. vorkommen. Sie können zur Bekämpfung dieser Schädlinge allein oder in Kombination mit anderen Wirk- und Hilfsstoffen in Haushaltsinsektizid-Produkten verwendet werden. Sie sind gegen sensible und resistente Arten sowie gegen alle Entwicklungsstadien wirksam. Zu diesen Schädlingen gehören:
Aus der Ordnung der Scorpionidea z.B. Buthus occitanus.
Aus der Ordnung der Acarina z.B. Argas persicus, Argas reflexus, Bryobia ssp., Dermanyssus gallinae, Glyciphagus domesticus, Ornithodorus moubat, Rhipicephalus sanguineus, Trombicula alfreddugesi, Neutrombicula autumnalis, Dermatophagoides pteronissimus, Dermatophagoides forinae.
Aus der Ordnung der Araneae z.B. Aviculariidae, Araneidae.
Aus der Ordnung der Opiliones z.B. Pseudoscorpiones chelifer, Pseudoscorpiones cheiridium, Opiliones phalangium.
Aus der Ordnung der Isopoda z.B. Oniscus asellus, Porcellio scaber.
Aus der Ordnung der Diplopoda z.B. Blaniulus guttulatus, Polydesmus spp..
Aus der Ordnung der Chilopoda z.B. Geophilus spp..
Aus der Ordnung der Zygentoma z.B. Ctenolepisma spp., Lepisma saccharina, Lepismodes inquilinus.
Aus der Ordnung der Blattaria z.B. Blatta orientalies, Blattella germanica, Blattella asahinai, Leucophaea maderae, Panchlora spp., Parcoblatta spp., Periplaneta australasiae, Periplaneta americana, Periplaneta brunnea, Periplaneta fuliginosa, Supella longipalpa.
Aus der Ordnung der Saltatoria z.B. Acheta domesticus.
Aus der Ordnung der Dermaptera z.B. Forficula auricularia.
Aus der Ordnung der Isoptera z.B. Kalotermes spp., Reticulitermes spp.
Aus der Ordnung der Psocoptera z.B. Lepinatus spp., Liposcelis spp.
Aus der Ordnung der Coleoptera z.B. Anthrenus spp., Attagenus spp., Dermestes spp., Latheticus oryzae, Necrobia spp., Ptinus spp., Rhizopertha dominica, Sitophilus granarius, Sitophilus oryzae, Sitophilus zeamais, Stegobium paniceum.
Aus der Ordnung der Diptera z.B. Aedes aegypti, Aedes albopictus, Aedes taeniorhynchus, Anopheles spp., Calliphora erythrocephala, Chrysozona pluvialis, Culex quinquefasciatus, Culex pipiens, Culex tarsalis, Drosophila spp., Fannia canicularis, Musca domestica, Phlebotomus spp., Sarcophaga carnaria, Simulium spp., Stomoxys calcitrans, Tipula paludosa.
Aus der Ordnung der Lepidoptera z.B. Achroia grisella, Galleria mellonella, Plodia interpunctella, Tinea cloacella, Tinea pellionella, Tineola bisselliella.
Aus der Ordnung der Siphonaptera z.B. Ctenocephalides canis, Ctenocephalides felis, Pulex irritans, Tunga penetrans, Xenopsylla cheopis.
Aus der Ordnung der Hymenoptera z.B. Camponotus herculeanus, Lasius fuliginosus, Lasius niger, Lasius umbratus, Monomorium pharaonis, Paravespula spp., Tetramorium caespitum.
Aus der Ordnung der Anoplura z.B. Pediculus humanus capitis, Pediculus humanus corporis, Pemphigus spp., Phylloera vastatrix, Phthirus pubis.
Aus der Ordnung der Heteroptera z.B. Cimex hemipterus, Cimex lectularius, Rhodinus prolixus, Triatoma infestans.

Die Anwendung im Bereich der Haushaltsinsektizide erfolgt allein oder in Kombination mit anderen geeigneten Wirkstoffen wie Phosphorsäureestern, Carbamaten, Pyrethroiden, Neo-nicotinoiden, Wachstumsregulatoren oder Wirkstoffen aus anderen bekannten Insektizidklassen.

Die Anwendung erfolgt in Aerosolen, drucklosen Sprühmitteln, z.B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propellergetriebenen Verdampfern, energielosen bzw. passiven Verdampfungssystemen, Mottenpapieren, Mottensäckchen und Mottengelen, als Granulate oder Stäube, in Streuködern oder Köderstationen.

Die erfindungsgemäßen Verbindungen der Formel (I) (Wirkstoffe) weisen eine ausgezeichnete herbizide Wirksamkeit gegen ein breites Spektrum wirtschaftlich wichtiger mono- und dikotyler annueller Schadpflanzen auf. Auch schwer bekämpfbare perennierende Schadpflanzen, die aus Rhizomen, Wurzelstöcken oder anderen Dauerorganen austreiben, werden durch die Wirkstoffe gut erfaßt.

Die angewandte Wirkstoffmenge kann in einem größeren Bereich schwanken. Sie hängt im wesentlichen von der Art des gewünschten Effektes ab. Im Allgemeinen liegen die Aufwandmengen zwischen 1 g und 10 kg Wirkstoff pro Hektar Bodenfläche, vorzugsweise zwischen 5 g und 5 kg pro ha.

Der vorteilhafte Effekt der Kulturpflanzen-Verträglichkeit der erfindungsgemäßen Wirkstoffkombinationen ist bei bestimmten Konzentrationsverhältnissen besonders stark ausgeprägt. Jedoch können die Gewichtsverhältnisse der Wirkstoffe in den Wirkstoffkombinationen in relativ großen Bereichen variiert werden. Im allgemeinen entfallen auf 1 Gewichtsteil Wirkstoff der Formel (I) 0,001 bis 1000 Gewichtsteile, vorzugsweise 0,01 bis 100 Gewichtsteile, besonders bevorzugt 0,05 bis 20 Gewichtsteile einer der oben unter (b') genannten, die Kulturpflanzen Verträglichkeit verbessernden Verbindungen (Antidots/Safener).

Die erfindungsgemäßen Wirkstoffkombinationen werden im allgemeinen in Form von Fertigformulierungen zur Anwendung gebracht. Die in den Wirkstoffkombinationen enthaltenen Wirkstoffe können aber auch in Einzelformulierungen bei der Anwendung gemischt, d.h. in Form von Tankmischungen zur Anwendung gebracht werden.

Für bestimmte Anwendungszwecke, insbesondere im Nachauflauf-Verfahren, kann es ferner vorteilhaft sein, in die Formulierungen als weitere Zusatzstoffe pflanzenverträgliche mineralische oder vegetabilische Öle (z.B. das Handelspräparat "Rako Binol") oder Ammoniumsalze wie z.B. Ammoniumsulfat oder Ammoniumrhodanid aufzunehmen.

Die neuen Wirkstoffkombinationen können als solche, in Form ihrer Formulierungen oder der daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Stäuben oder Streuen.

Die Aufwandmengen der erfindungsgemäßen Wirkstoffkombinationen können in einem gewissen Bereich variiert werden; sie hängen u.a. vom Wetter und von den Bodenfaktoren ab. Im allgemeinen liegen die Aufwandmengen zwischen 0,001 und 5 kg pro ha, vorzugsweise zwischen 0,005 und 2 kg pro ha, besonders bevorzugt zwischen 0,01 und 0,5 kg pro ha.

Die erfindungsgemäß einzusetzenden Safener können je nach ihren Eigenschaften zur Vorbehandlung des Saatgutes der Kulturpflanze (Beizung der Samen) verwendet werden oder vor der Saat in die Saatfurchen eingebracht oder vor dem Herbizid separat angewendet werden oder zusammen mit dem Herbizid vor oder nach dem Ablaufen der Pflanzen angewendet werden.

Als Beispiele der Pflanzen werden die wichtigen Kulturpflanzen, wie Getreide (Weizen, Gerste, Reis), Mais, Soja, Kartoffel, Baumwolle, Raps, Rüben, Zuckerrohr sowie Obstpflanzen (mit den Früchten Äpfel, Birnen, Zitrusfrüchten und Weintrauben) erwähnt, wobei Getreide, Mais, Soja, Kartoffel, Baumwolle und Raps besonders hervorgehoben werden.

Mit den erfindungsgemäßen Wirkstoffen können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Spross, Blatt, Blüte und Wurzel verstanden werden, wobei beispielhaft Blätter, Nadeln, Stängel, Stämme, Blüten, Fruchtkörper, Früchte und Saatgut sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Saatgut.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen, Injizieren und bei Vermehrungsmaterial, insbesondere bei Saatgut, weiterhin durch ein- oder mehrschichtiges Umhüllen.

Gegenstand der vorliegenden Erfindung ist daher auch ein Verfahren zur Bekämpfung von unerwünschten Pflanzen oder zur Wachstumsregulierung von Pflanzen, vorzugsweise in Pflanzenkulturen, worin eine oder mehrere erfindungsgemäße Verbindung(en) auf die Pflanzen (z.B. Schadpflanzen wie mono- oder dikotyle Unkräuter oder unerwünschte Kulturpflanzen), das Saatgut (z.B. Körner, Samen oder vegetative Vermehrungsorgane wie Knollen oder Sprossteile mit Knospen) oder die Fläche, auf der die Pflanzen wachsen (z.B. die Anbaufläche), ausgebracht werden. Dabei können die erfindungsgemäßen Verbindungen z.B. im Vorsaat- (ggf. auch durch Einarbeitung in den Boden), Vorauflauf- oder Nachauflaufverfahren ausgebracht werden. Im einzelnen seien beispielhaft einige Vertreter der mono- und dikotylen Unkrautflora genannt, die durch die erfindungsgemäßen Verbindungen kontrolliert werden können, ohne dass durch die Nennung eine Beschränkung auf bestimmte Arten erfolgen soll. Monokotyle Schadpflanzen der Gattungen: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.

Dikotyle Unkräuter der Gattungen: Abutilon, Amaranthus, Ambrosia, Anoda, Anthemis, Aphanes, Artemisia, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.

Die aufgeführten Pflanzen können besonders vorteilhaft erfindungsgemäß mit den Verbindungen der allgemeinen Formel I bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell auf-geführten Verbindungen bzw. Mischungen.

Werden die erfindungsgemäßen Verbindungen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab.

Bei Applikation der Wirkstoffe auf die grünen Pflanzenteile im Nachauflaufverfahren tritt nach der Behandlung Wachstumsstop ein und die Schadpflanzen bleiben in dem zum Applikationszeitpunkt vorhandenen Wachstumsstadium stehen oder sterben nach einer gewissen Zeit ganz ab, so dass auf diese Weise eine für die Kulturpflanzen schädliche Unkrautkonkurrenz sehr früh und nachhaltig beseitigt wird.

Obgleich die erfindungsgemäßen Verbindungen eine ausgezeichnete herbizide Aktivität gegenüber mono-und dikotylen Unkräutern aufweisen, werden Kulturpflanzen wirtschaftlich bedeutender Kulturen z.B. dikotyler Kulturen der Gattungen Arachis, Beta, Brassica, Cucumis, Cucurbita, Helianthus, Daucus, Glycine, Gossypium, Ipomoea, Lactuca, Linum, Lycopersicon, Miscanthus, Nicotiana, Phaseolus, Pisum, Solanum, Vicia, oder monokotyler Kulturen der Gattungen Allium, Ananas, Asparagus, Avena, Hordeum, Oryza, Panicum, Saccharum, Secale, Sorghum, Triticale, Triticum, Zea, abhängig von der Struktur der jeweiligen erfindungsgemäßen Verbindung und deren Aufwandmenge nur unwesentlich oder gar nicht geschädigt. Die vorliegenden Verbindungen eignen sich aus diesen Gründen sehr gut zur selektiven Bekämpfung von unerwünschtem Pflanzenwuchs in Pflanzenkulturen wie landwirtschaftlichen Nutzpflanzungen oder Zierpflanzungen.

Darüberhinaus weisen die erfindungsgemäßen Verbindungen (abhängig von ihrer jeweiligen Struktur und der ausgebrachten Aufwandmenge) hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung wie z.B. durch Auslösen von Desikkation und Wuchsstauchung eingesetzt werden. Desweiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da beispielsweise die Lagerbildung hierdurch verringert oder völlig verhindert werden kann.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Die Begriffe "Teile" bzw. "Teile von Pflanzen" oder "Pflanzenteile" wurden oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Aufgrund ihrer herbiziden und pflanzenwachstumsregulatorischen Eigenschaften können die Wirkstoffe auch zur Bekämpfung von Schadpflanzen in Kulturen von bekannten oder noch zu entwickelnden gentechnisch veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten, Nematoden oder Mikroorganismen wie Pilzen, Bakterien oder Viren. Andere besondere Eigenschaften betreffen z.B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt. Weitere besondere Eigenschaften können in einer Toleranz oder Resistenz gegen abiotische Stressoren z.B. Hitze, Kälte, Trockenheit, Salz und ultraviolette Strahlung liegen. Die Wirkstoffe können auch in transgenen Pflanzen, die sich durch höhere Erträge , z. B. durch eine verbesserte Photosyntheseleistung oder verbesserte Nährstoffaufnahme auszeichnen, eingesetzt werden.

Bevorzugt ist die Anwendung der erfindungsgemäßen Verbindungen der Formel (I) oder deren Salze in wirtschaftlich bedeutenden transgenen Kulturen von Nutz-und Zierpflanzen, z.B. von Getreide wie Weizen, Gerste, Roggen, Hafer, Hirse, Reis, Maniok und Mais oder auch Kulturen von Zuckerrübe, Baumwolle, Soja, Raps, Kartoffel, Tomate, Erbse und anderen Gemüsesorten.

Vorzugsweise können die Verbindungen der Formel (I) als Herbizide in Nutzpflanzenkulturen eingesetzt werden, welche gegenüber den phytotoxischen Wirkungen der Herbizide resistent sind bzw. gentechnisch resistent gemacht worden sind.

Herkömmliche Wege zur Herstellung neuer Pflanzen, die im Vergleich zu bisher vorkommenden Pflanzen modifizierte Eigenschaften aufweisen, bestehen beispielsweise in klassischen Züchtungsverfahren und der Erzeugung von Mutanten. Alternativ können neue Pflanzen mit veränderten Eigenschaften mit Hilfe gentechnischer Verfahren erzeugt werden (siehe z.B. EP 0221044, EP 0131624). Beschrieben wurden beispielsweise in mehreren Fällen
- gentechnische Veränderungen von Kulturpflanzen zwecks Modifikation der in den Pflanzen synthetisierten Stärke (z.B. WO 92/011376 A, WO 92/014827 A, WO 91/019806 A),
- transgene Kulturpflanzen, welche gegen bestimmte Herbizide vom Typ Glufosinate (vgl. z.B. EP 0242236 A, EP 0242246 A) oder Glyphosate (WO 92/000377 A) oder der Sulfonylharnstoffe (EP 0257993 A, US 5,013,659) oder gegen Kombinationen oder Mischungen dieser Herbizide durch "gene stacking" resistent sind, wie transgenen Kulturpflanzen z. B. Mais oder Soja mit dem Handelsnamen oder der Bezeichnung Optimum™ GAT™ (Glyphosate ALS Tolerant). Des weiteren wurden transgene Pflanzen beschrieben, die gegen synthetische Auxine (z. B 2,4 D) HRAC mode of action Class O und Aryloxy-phenoxy Propionate (fops, HRAC, Class A) resistent sind (DHT, Dow Agroscience Herbicide Tolerance Trait)
- transgene Kulturpflanzen, beispielsweise Baumwolle, mit der Fähigkeit Bacillus thuringiensis-Toxine (Bt-Toxine) zu produzieren, welche die Pflanzen gegen bestimmte Schädlinge resistent machen (EP 0142924 A, EP 0193259 A).
- transgene Kulturpflanzen mit modifizierter Fettsäurezusammensetzung (WO 91/013972 A).
- Gentechnisch veränderte Pflanzen, die neue Insektenresistenzen, z. B. basierend auf der Expression von Toxinen aus Photorhabdus, Xenorhabdus Symbionten aus entomopathogenen Nematoden und Toxinen aus Spinnen, Skorpionen, Ameisen, parasitischen Wespen aufweisen.
- gentechnisch veränderte Kulturpflanzen mit neuen Inhalts- oder Sekundärstoffen z. B. neuen Phytoalexinen, die eine erhöhte Krankheitsresistenz verursachen (EP 0309862 A, EP 0464461 A)
- gentechnisch veränderte Pflanzen mit reduzierter Photorespiration, die höhere Erträge und höhere Stresstoleranz aufweisen (EP 0305398 A)
- transgene Kulturpflanzen, die pharmazeutisch oder diagnostisch wichtige Proteine produzieren ("molecular pharming")
- transgene Kulturpflanzen, die sich durch höhere Erträge oder bessere Qualitat auszeichnen
- Transgene Kulturpflanzen, die sich durch erhöhte Toleranzen gegen abiotische und biotische Stressoren auszeichnen
- transgene Kulturpflanzen die sich durch eine Kombinationen z.B. der o. g. neuen Eigenschaften auszeichnen ("gene stacking")

Zahlreiche molekularbiologische Techniken, mit denen neue transgene Pflanzen mit veränderten Eigenschaften hergestellt werden können, sind im Prinzip bekannt; siehe z.B. I. Potrykus und G. Spangenberg (eds.) Gene Transfer to Plants, Springer Lab Manual (1995), Springer Verlag Berlin, Heidelberg. oder Christou, "Trends in Plant Science" 1 (1996) 423-431).

Für derartige gentechnische Manipulationen können Nucleinsäuremoleküle in Plasmide eingebracht werden, die eine Mutagenese oder eine Sequenzveränderung durch Rekombination von DNA-Sequenzen erlauben. Mit Hilfe von Standardverfahren können z.B. Basenaustausche vorgenommen, Teilsequenzen entfernt oder natürliche oder synthetische Sequenzen hinzugefügt werden. Für die Verbindung der DNA-Fragmente untereinander können an die Fragmente Adaptoren oder Linker angesetzt werden, siehe z.B. Sambrook et al., 1989, Molecular Cloning, A Laboratory Manual, 2. Aufl. Cold Spring Harbor Laboratory Press, Cold Spring Harbor, NY; oder Winnacker "Gene und Klone", VCH Weinheim 2. Auflage 1996

Die Herstellung von Pflanzenzellen mit einer verringerten Aktivität eines Genprodukts kann beispielsweise erzielt werden durch die Expression mindestens einer entsprechenden antisense-RNA, einer sense-RNA zur Erzielung eines Cosuppressionseffektes oder die Expression mindestens eines entsprechend konstruierten Ribozyms, das spezifisch Transkripte des obengenannten Genprodukts spaltet.

Hierzu können zum einen DNA-Moleküle verwendet werden, die die gesamte codierende Sequenz eines Genprodukts einschließlich eventuell vorhandener flankierender Sequenzen umfassen, als auch DNA-Moleküle, die nur Teile der codierenden Sequenz umfassen, wobei diese Teile lang genug sein müssen, um in den Zellen einen antisense-Effekt zu bewirken. Möglich ist auch die Verwendung von DNA-Sequenzen, die einen hohen Grad an Homologie zu den codiereden Sequenzen eines Genprodukts aufweisen, aber nicht vollkommen identisch sind.

Bei der Expression von Nucleinsäuremolekülen in Pflanzen kann das synthetisierte Protein in jedem beliebigen Kompartiment der pflanzlichen Zelle lokalisiert sein. Um aber die Lokalisation in einem bestimmten Kompartiment zu erreichen, kann z.B. die codierende Region mit DNA-Sequenzen verknüpft werden, die die Lokalisierung in einem bestimmten Kompartiment gewährleisten. Derartige Sequenzen sind dem Fachmann bekannt (siehe beispielsweise Braun et al., EMBO J. 11 (1992), 3219-3227; Wolter et al., Proc. Natl. Acad. Sci. USA 85 (1988), 846-850; Sonnewald et al., Plant J. 1 (1991), 95-106). Die Expression der Nukleinsäuremoleküle kann auch in den Organellen der Pflanzenzellen stattfinden.

Die transgenen Pflanzenzellen können nach bekannten Techniken zu ganzen Pflanzen regeneriert werden. Bei den transgenen Pflanzen kann es sich prinzipiell um Pflanzen jeder beliebigen Pflanzenspezies handeln, d.h., sowohl monokotyle als auch dikotyle Pflanzen.

So sind transgene Pflanzen erhältlich, die veränderte Eigenschaften durch Überexpression, Suppression oder Inhibierung homologer (= natürlicher) Gene oder Gensequenzen oder Expression heterologer (= fremder) Gene oder Gensequenzen aufweisen.

Vorzugsweise können die erfindungsgemäßen Verbindungen (I) in transgenen Kulturen eingesetzt werden, welche gegen Wuchsstoffe, wie z.B. 2,4 D, Dicamba oder gegen Herbizide, die essentielle Pflanzenenzyme, z.B. Acetyl CoA Carboxylasen, Acetolactatsynthasen (ALS), EPSP Synthasen, Glutaminsynthasen (GS) oder Hydoxyphenylpyruvat Dioxygenasen (HPPD) hemmen, respektive gegen Herbizide aus der Gruppe der FOPs, Sulfonylharnstoffe, der Glyphosate, Glufosinate oder Benzoylisoxazole und analogen Wirkstoffe, oder gegen beliebige Kombinationen dieser Wirkstoffe, resistent sind.
- Besonders bevorzugt können die erfindungsgemäßen Verbindungen in transgenen Kulturpflanzen eingesetzt werden, die gegen eine Kombination von Glyphosaten und Glufosinaten, Glyphosaten und Sulfonylharnstoffen oder Imidazolinonen resistent sind. Ganz besonders bevorzugt können die erfindungsgemäßen Verbindungen in transgenen Kulturpflanzen wie z. B. Mais oder Soja mit dem Handelsnamen oder der Bezeichnung Optimum™ GAT™ (Glyphosate ALS Tolerant) eingesetzt werden. Des weiteren und besonders bevorzugt können die erfindungsgemäßen Verbindungen in transgenen Pflanzen, die gegen synthetische Auxine (z. B 2,4 D) mit "HRAC mode of action Class O" und Aryloxy-phenoxy Propionate (fops) mit "HRAC mode of action Class A" resistent sind (z. B. DHT, Dow Agroscience Herbicide Tolerance Trait) eingesetzt werden.

Bei der Anwendung der erfindungsgemäßen Wirkstoffe in transgenen Kulturen treten neben den in anderen Kulturen zu beobachtenden Wirkungen gegenüber Schadpflanzen oftmals Wirkungen auf, die für die Applikation in der jeweiligen transgenen Kultur spezifisch sind, beispielsweise ein verändertes oder speziell erweitertes Unkrautspektrum, das bekämpft werden kann, veränderte Aufwandmengen, die für die Applikation eingesetzt werden können, vorzugsweise gute Kombinierbarkeit mit den Herbiziden, gegenüber denen die transgene Kultur resistent ist, sowie Beeinflussung von Wuchs und Ertrag der transgenen Kulturpflanzen.

Gegenstand der Erfindung ist deshalb auch die Verwendung der erfindungsgemäßen Verbindungen der Formel (I) als Herbizide zur Bekämpfung von Schadpflanzen in transgenen Kulturpflanzen.

Die erfindungsgemäßen Verbindungen können in Form von Spritzpulvern, emulgierbaren Konzentraten, versprühbaren Lösungen, Stäubemitteln oder Granulaten in den üblichen Zubereitungen angewendet werden. Gegenstand der Erfindung sind deshalb auch herbizide und pflanzenwachstumsregulierende Mittel, welche die erfindungsgemäßen Verbindungen enthalten.

Die erfindungsgemäßen Verbindungen können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser-und Wasser-in-Öl-Emulsionen, versprühbare Lösungen, Suspensionskonzentrate (SC), Dispersionen auf Öl- oder Wasserbasis, ölmischbare Lösungen, Kapselsuspensionen (CS), Stäubemittel (DP), Beizmittel, Granulate für die Streu- und Bodenapplikation, Granulate (GR) in Form von Mikro-, Sprüh-, Aufzugsund Adsorptionsgranulaten, wasserdispergierbare Granulate (WG), wasserlösliche Granulate (SG), ULV-Formulierungen, Mikrokapseln und Wachse.

Diese einzelnen Formulierungstypen sind im Prinzip bekannt und werden beispielsweise beschrieben in: Winnacker-Küchler, "Chemische Technologie", Band 7, C. Hanser Verlag München, 4. Aufl. 1986; Wade van Valkenburg, "Pesticide Formulations", Marcel Dekker, N.Y., 1973; K. Martens, "Spray Drying" Handbook, 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry"; 2nd Ed., J. Wiley & Sons, N.Y.; C. Marsden, "Solvents Guide"; 2nd Ed., Interscience, N.Y. 1963; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1976; Winnacker-Küchler, "Chemische Technologie", Band 7, C. Hanser Verlag München, 4. Aufl. 1986.

Auf der Basis dieser Formulierungen lassen sich auch Kombinationen mit anderen pestizid wirksamen Stoffen, wie z.B. Insektiziden, Akariziden, Herbiziden, Fungiziden, sowie mit Safenern, Düngemitteln und/oder Wachstumsregulatoren herstellen, z.B. in Form einer Fertigformulierung oder als Tankmix.

Als Kombinationspartner für die erfindungsgemäßen Verbindungen in Mischungsformulierungen oder im Tank-Mix sind beispielsweise bekannte Wirkstoffe, die auf einer Inhibition von beispielsweise Acetolactat-Synthase, Acetyl-CoA-Carboxylase, Cellulose-Synthase, Enolpyruvylshikimat-3-phosphat-Synthase, Glutamin-Synthetase, p-Hydroxyphenylpyruvat-Dioxygenase, Phytoendesaturase, Photosystem I, Photosystem II, Protoporphyrinogen-Oxidase beruhen, einsetzbar, wie sie z.B. in Weed Research 26 (1986) 441-445 oder in "The Pesticide Manual", 13th Auflage, The British Crop Protection Council and the Royal Soc. of Chemistry, 2003 und der darin zitierten Literatur beschrieben sind.

Als bekannte Herbizide oder Pflanzenwachstumsregulatoren, die mit den erfindungsgemäßen Verbindungen kombiniert werden können, sind z.B. folgende Wirkstoffe zu nennen:
Acetochlor, Acibenzolar, Acibenzolar-S-methyl, Acifluorfen, Acifluorfen-sodium, Aclonifen, Alachlor, Allidochlor, Alloxydim, Alloxydim-sodium, Ametryn, Amicarbazone, Amidochlor, Amidosulfuron, Aminopyralid, Amitrole, Ammoniumsulfamat, Ancymidol, Anilofos, Asulam, Atrazine, Azafenidin, Azimsulfuron, Aziprotryn, BAH-043, BAS-140H, BAS-693H, BAS-714H, BAS-762H, BAS-776H, BAS-800H, Beflubutamid, Benazolin, Benazolin-ethyl, bencarbazone, Benfluralin, Benfuresate, Bensulide, Bensulfuron-methyl, Bentazone, Benzfendizone, Benzobicyclon, Benzofenap, Benzofluor, Benzoylprop, Bifenox, Bilanafos, Bilanafos-natrium, Bispyribac, Bispyribac-natrium, Bromacil, Bromobutide, Bromofenoxim, Bromoxynil, Bromuron, Buminafos, Busoxinone, Butachlor, Butafenacil, Butamifos, Butenachlor, Butralin, Butroxydim, Butylate, Cafenstrole, Carbetamide, Carfentrazone, Carfentrazone-ethyl, Chlomethoxyfen, Chloramben, Chlorazifop, Chlorazifop-butyl, Chlorbromuron, Chlorbufam, Chlorfenac, Chlorfenac-natrium, Chlorfenprop, Chlorflurenol, Chlorflurenol-methyl, Chloridazon, Chlorimuron, Chlorimuron-ethyl, Chlormequat-chlorid, Chlornitrofen, Chlorophthalim, Chlorthal-dimethyl, Chlorotoluron, Chlorsulfuron, Cinidon, Cinidon-ethyl, Cinmethylin, Cinosulfuron, Clethodim, Clodinafop Clodinafop-propargyl, Clofencet, Clomazone, Clomeprop, Cloprop, Clopyralid, Cloransulam, Cloransulam-methyl, Cumyluron, Cyanamide, Cyanazine, Cyclanilide, Cycloate, Cyclosulfamuron, Cycloxydim, Cycluron, Cyhalofop, Cyhalofop-butyl, Cyperquat, Cyprazine, Cyprazole, 2,4-D, 2,4-DB, Daimuron/Dymron, Dalapon, Daminozide, Dazomet, n-Decanol, Desmedipham, Desmetryn, Detosyl-Pyrazolate (DTP), Diallate, Dicamba, Dichlobenil, Dichlorprop, Dichlorprop-P, Diclofop, Diclofop-methyl, Diclofop-P-methyl, Diclosulam, Diethatyl, Diethatyl-ethyl, Difenoxuron, Difenzoquat, Diflufenican, Diflufenzopyr, Diflufenzopyr-natrium, Dimefuron, Dikegulacsodium, Dimefuron, Dimepiperate, Dimethachlor, Dimethametryn, Dimethenamid, Dimethenamid-P, Dimethipin, Dimetrasulfuron, Dinitramine, Dinoseb, Dinoterb, Diphenamid, Dipropetryn, Diquat, Diquatdibromide, Dithiopyr, Diuron, DNOC, Eglinazine-ethyl, Endothal, EPTC, Esprocarb, Ethalfluralin, Ethametsulfuron-methyl, Ethephon, Ethidimuron, Ethiozin, Ethofumesate, Ethoxyfen, Ethoxyfen-ethyl, Ethoxysulfuron, Etobenzanid, F-5331, d.h. N-[2-Chlor-4-fluor-5-[4-(3fluorpropyl)-4,5-dihydro-5-oxo-1H-tetrazol-1-yl]-phenyl]-ethansulfonamid, Fenoprop, Fenoxaprop, Fenoxaprop-P, Fenoxaprop-ethyl, Fenoxaprop-P-ethyl, Fentrazamide, Fenuron, Flamprop, Flamprop-M-isopropyl, Flamprop-M-methyl, Flazasulfuron, Florasulam, Fluazifop, Fluazifop-P, Fluazifop-butyl, Fluazifop-P-butyl, Fluazolate, Flucarbazone, Flucarbazone-sodium, Flucetosulfuron, Fluchloralin, Flufenacet (Thiafluamide), Flufenpyr, Flufenpyr-ethyl, Flumetralin, Flumetsulam, Flumiclorac, Flumiclorac-pentyl, Flumioxazin, Flumipropyn, Fluometuron, Fluorodifen, Fluoroglycofen, Fluoroglycofen-ethyl, Flupoxam, Flupropacil, Flupropanate, Flupyrsulfuron, Flupyrsulfuron-methyl-sodium, Flurenol, Flurenol-butyl, Fluridone, Flurochloridone, Fluroxypyr, Fluroxypyr-meptyl, Flurprimidol, Flurtamone, Fluthiacet, Fluthiacetmethyl, Fluthiamide, Fomesafen, Foramsulfuron, Forchlorfenuron, Fosamine, Furyloxyfen, Gibberellinsäure, Glufosinate, L-Glufosinate, L-Glufosinate-ammonium, Glufosinate-ammonium, Glyphosate, Glyphosate-isopropylammonium, H-9201, Halosafen, Halosulfuron, Halosulfuron-methyl, Haloxyfop, Haloxyfop-P, Haloxyfop-ethoxyethyl, Haloxyfop-P-ethoxyethyl, Haloxyfop-methyl, Haloxyfop-P-methyl, Hexazinone, HNPC-9908, HOK-201, HW-02, Imazamethabenz, Imazamethabenzmethyl, Imazamox, Imazapic, Imazapyr, Imazaquin, Imazethapyr, Imazosulfuron, Inabenfide, Indanofan, Indolessigsäure (IAA), 4-Indol-3-ylbuttersäure (IBA), Iodosulfuron, Iodosulfuron-methyl-natrium, Ioxynil, Isocarbamid, Isopropalin, Isoproturon, Isouron, Isoxaben, Isoxachlortole, Isoxaflutole, Isoxapyrifop, IDH-100, KUH-043, KUH-071, Karbutilate, Ketospiradox, Lactofen, Lenacil, Linuron, Maleinsäurehydrazid, MCPA, MCPB, MCPB-methyl, -ethyl und -natrium, Mecoprop, Mecopropnatrium, Mecoprop-butotyl, Mecoprop-P-butotyl, Mecoprop-P-dimethylammonium, Mecoprop-P-2-ethylhexyl, Mecoprop-P-kalium, Mefenacet, Mefluidide, Mepiquat-chlorid, Mesosulfuron, Mesosulfuronmethyl, Mesotrione, Methabenzthiazuron, Metam, Metamifop, Metamitron, Metazachlor, Methazole, Methoxyphenone, Methyldymron, 1-Methylcyclopropen, Methylisothiocyanat, Metobenzuron, Metobenzuron, Metobromuron, Metolachlor, S-Metolachlor, Metosulam, Metoxuron, Metribuzin, Metsulfuron, Metsulfuron-methyl, Molinate, Monalide, Monocarbamide, Monocarbamide-dihydrogensulfat, Monolinuron, Monosulfuron, Monuron, MT 128, MT-5950, d. h. N-[3-Chlor-4-(1-methylethyl)-phenyl]-2-methylpentanamid, NGGC-011, Naproanilide, Napropamide, Naptalam, NC-310, d.h. 4-(2,4-dichlorobenzoyl)-1-methyl-5-benzylolypyrazole, Neburon, Nicosulfuron, Nipyraclofen, Nitralin, Nitrofen, Nitrophenolat-natrium (Isomerengemisch), Nitrofluorfen, Nonansäure, Norflurazon, Orbencarb, Orthosulfamuron, Oryzalin, Oxadiargyl, Oxadiazon, Oxasulfuron, Oxaziclomefone, Oxyfluorfen, Paclobutrazol, Paraquat, Paraquat-dichlorid, Pelargonsäure (Nonansäure), Pendimethalin, Pendralin, Penoxsulam, Pentanochlor, Pentoxazone, Perfluidone, Pethoxamid, Phenisopham, Phenmedipham, Phenmedipham-ethyl, Picloram, Picolinafen, Pinoxaden, Piperophos, Pirifenop, Pirifenop-butyl, Pretilachlor, Primisulfuron, Primisulfuron-methyl, Probenazole, Profluazol, Procyazine, Prodiamine, Prifluraline, Profoxydim, Prohexadione, Prohexadione-calcium, Prohydrojasmone, Prometon, Prometryn, Propachlor, Propanil, Propaquizafop, Propazine, Propham, Propisochlor, Propoxycarbazone, Propoxycarbazone-natrium, Propyzamide, Prosulfalin, Prosulfocarb, Prosulfuron, Prynachlor, Pyraclonil, Pyraflufen, Pyraflufen-ethyl, Pyrasulfotole, Pyrazolynate (Pyrazolate), Pyrazosulfuron-ethyl, Pyrazoxyfen, Pyribambenz, Pyribambenz-isopropyl, Pyribenzoxim, Pyributicarb, Pyridafol, Pyridate, Pyriftalid, Pyriminobac, Pyriminobac-methyl, Pyrimisulfan, Pyrithiobac, Pyrithiobac-natrium, Pyroxasulfone, Pyroxsulam, Quinclorac, Quinmerac, Quinoclamine, Quizalofop, Quizalofop-ethyl, Quizalofop-P, Quizalofop-P-ethyl, Quizalofop-P-tefuryl, Rimsulfuron, Secbumeton, Sethoxydim, Siduron, Simazine, Simetryn, SN-106279, Sulcotrione, Sulfallate (CDEC), Sulfentrazone, Sulfometuron, Sulfometuron-methyl, Sulfosate (Glyphosate-trimesium), Sulfosulfuron, SYN-523, SYP-249, SYP-298, SYP-300, Tebutam, Tebuthiuron, Tecnazene, Tefuryltrione, Tembotrione, Tepraloxydim, Terbacil, Terbucarb, Terbuchlor, Terbumeton, Terbuthylazine, Terbutryn, TH-547, Thenylchlor, Thiafluamide, Thiazafluron, Thiazopyr, Thidiazimin, Thidiazuron, Thiencarbazone, Thiencarbazone-methyl, Thifensulfuron, Thifensulfuron-methyl, Thiobencarb, Tiocarbazil, Topramezone, Tralkoxydim, Triallate, Triasulfuron, Triaziflam, Triazofenamide, Tribenuron, Tribenuron-methyl, Trichloressigsäure (TCA), Triclopyr, Tridiphane, Trietazine, Trifloxysulfuron, Trifloxysulfuron-natrium, Trifluralin, Triflusulfuron, Triflusulfuron-methyl, Trimeturon, Trinexapac, Trinexapac-ethyl, Tritosulfuron, Tsitodef, Uniconazole, Uniconazole-P, Vernolate, ZJ-0166, ZJ-0270, ZJ-0543, oder ZJ-0862 sowie die folgenden Verbindungen

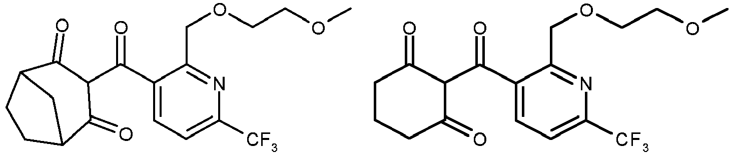

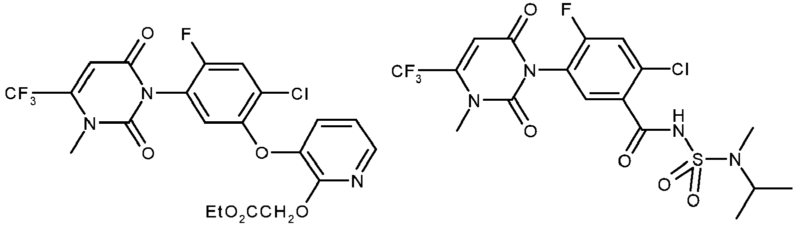

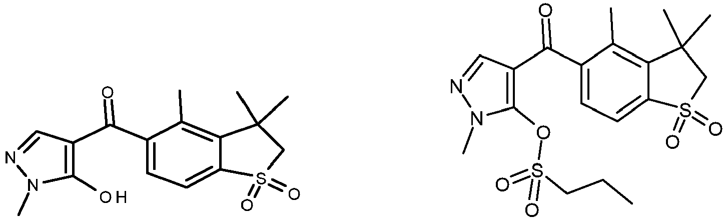

Verbindungen sind entweder mit dem "common name" nach der International Organization for Standardization (ISO) oder mit ihrem chemischen Namen oder Codenummer bezeichnet und umfassen stets sämtliche Anwendungsformen wie Säuren, Salze, Ester oder Modifikationen, wie Isomere, Stereoisomere und optische Isomere. Beispielhaft sind eine oder auch mehrere Anwendungsformen oder Modifikationen genannt.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, ligninsulfonsaures Natrium, 2,2'-dinaphthylmethan-6,6'-disulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen feingemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calzium-Salze wie Ca-Dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanester wie z.B. Sorbitanfettsäureester oder Polyoxethylensorbitanester wie z.B. Polyoxyethylensorbitanfettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z.B. oben bei den anderen Formulierungstypen bereits aufgeführt sind, hergestellt werden.

Emulsionen, z.B. Öl-in-Wasser-Emulsionen (EW), lassen sich beispielsweise mittels Rührern, Kolloidmühlen und/oder statischen Mischern unter Verwendung von wäßrigen organischen Lösungsmitteln und gegebenenfalls Tensiden, wie sie z.B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt.

Zur Herstellung von Teller-, Fließbett-, Extruder- und Sprühgranulate siehe z.B. Verfahren in "Spray-Drying Handbook" 3rd ed. 1979, G. Goodwin Ltd., London; J.E. Browning, "Agglomeration", Chemical and Engineering 1967, Seiten 147 ff; "Perry's Chemical Engineer's Handbook", 5th Ed., McGraw-Hill, New York 1973, S. 8-57.

Für weitere Einzelheiten zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen enthalten in der Regel 0.1 bis 99 Gew.-%, insbesondere 0.1 bis 95 Gew.-%, erfindungsgemäße Verbindungen.

In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0.05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-%.

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel.

Das erfindungsgemäße Behandlungsverfahren wird vorzugsweise auf genetisch modifizierten Organismen, wie beispielsweise Pflanzen oder Pflanzenteile, verwendet.

Genetisch modifizierte Pflanzen, sogenannte transgene Pflanzen, sind Pflanzen, bei denen ein heterologes Gen stabil in das Genom integriert worden ist.

Der Begriff "heterologes Gen" bedeutet im wesentlichen ein Gen, das außerhalb der Pflanze bereitgestellt oder assembliert wird und das bei Einführung in das Zellkerngenom, das Chloroplastengenom oder das Hypochondriengenom der transformierten Pflanze dadurch neue oder verbesserte agronomische oder sonstige Eigenschaften verleiht, daß es ein interessierendes Protein oder Polypeptid exprimiert oder daß es ein anderes Gen, das in der Pflanze vorliegt bzw. andere Gene, die in der Pflanze vorliegen, herunterreguliert oder abschaltet (zum Beispiel mittels Antisense-Technologie, Cosuppressionstechnologie oder RNAi-Technologie [RNA Interference]). Ein heterologes Gen, das im Genom vorliegt, wird ebenfalls als Transgen bezeichnet. Ein Transgen, das durch sein spezifisches Vorliegen im Pflanzengenom definiert ist, wird als Transformations- bzw. transgenes Event bezeichnet.

In Abhängigkeit von den Pflanzenarten oder Pflanzensorten, ihrem Standort und ihren Wachstumsbedingungen (Böden, Klima, Vegetationsperiode, Ernährung) kann die erfindungsgemäße Behandlung auch zu überadditiven ("synergistischen") Effekten führen. So sind zum Beispiel die folgenden Effekte möglich, die über die eigentlich zu erwartenden Effekte hinausgehen: verringerte Aufwandmengen und/oder erweitertes Wirkungsspektrum und/oder erhöhte Wirksamkeit der Wirkstoffe und Zusammensetzungen, die erfindungsgemäß eingesetzt werden können, besseres Pflanzenwachstum, erhöhte Toleranz gegenüber hohen oder niedrigen Temperaturen, erhöhte Toleranz gegenüber Trockenheit oder Wasser- oder Bodensalzgehalt, erhöhte Blühleistung, Ernteerleichterung, Reifebeschleunigung, höhere Erträge, größere Früchte, größere Pflanzenhöhe, intensiver grüne Farbe des Blatts, frühere Blüte, höhere Qualität und/oder höherer Nährwert der Ernteprodukte, höhere Zuckerkonzentration in den Früchten, bessere Lagerfähigkeit und/oder Verarbeitbarkeit der Ernteprodukte.

In gewissen Aufwandmengen können die erfindungsgemäßen Wirkstoffkombinationen auch eine stärkende Wirkung auf Pflanzen ausüben. Sie eignen sich daher für die Mobilisierung des pflanzlichen Abwehrsystems gegen Angriff durch unerwünschte phytopathogene Pilze und/oder Mikroorganismen und/oder Viren. Dies kann gegebenenfalls einer der Gründe für die erhöhte Wirksamkeit der erfindungsgemäßen Kombinationen sein, zum Beispiel gegen Pilze. Pflanzenstärkende (resistenzinduzierende) Substanzen sollen im vorliegenden Zusammenhang auch solche Substanzen oder Substanzkombinationen bedeuten, die fähig sind, das pflanzliche Abwehrsystem so zu stimulieren, daß die behandelten Pflanzen, wenn sie im Anschluß daran mit unerwünschten phytopathogenen Pilzen und/oder Mikroorganismen und/oder Viren inokkuliert werde, einen beträchtlichen Resistenzgrad gegen diese unerwünschten phytopathogenen Pilze und/oder Mikroorganismen und/oder Viren aufweisen. Im vorliegenden Fall versteht man unter unerwünschten phytopathogenen Pilzen und/oder Mikroorganismen und/oder Viren phytopathogene Pilze, Bakterien und Viren. Die erfindungsgemäßen Substanzen lassen sich daher zum Schutz von Pflanzen gegen Angriff durch die erwähnten Pathogene innerhalb eines gewissen Zeitraums nach der Behandlung einsetzen. Der Zeitraum, über den eine Schutzwirkung erzielt wird, erstreckt sich im allgemeinen von 1 bis 10 Tagen, vorzugsweise 1 bis 7 Tagen, nach der Behandlung der Pflanzen mit den Wirkstoffen.

Zu Pflanzen und Pflanzensorten, die vorzugsweise erfindungsgemäß behandelt werden, zählen alle Pflanzen, die über Erbgut verfügen, das diesen Pflanzen besonders vorteilhafte, nützliche Merkmale verleiht (egal, ob dies durch Züchtung und/oder Biotechnologie erzielt wurde).

Pflanzen, die weiterhin vorzugsweise erfindungsgemäß behandelt werden, sind gegen einen oder mehrere biotische Streßfaktoren resistent, d. h. diese Pflanzen weisen eine verbesserte Abwehr gegen tierische und mikrobielle Schädlinge wie Nematoden, Insekten, Milben, phytopathogene Pilze, Bakterien, Viren und/oder Viroide auf.

Neben den vorgenannten Pflanzen und Pflanzensorten, können auch solche erfindungsgemäß behandelt werden, die gegen einen oder mehrere abiotische Streßfaktoren widerstandsfähig sind.

Zu den abiotischen Streßbedingungen können zum Beispiel Dürre, Kälte- und Hitzebedingungen, osmotischer Streß, Staunässe, erhöhter Bodensalzgehalt, erhöhtes Ausgesetztsein an Mineralien, Ozonbedingungen, Starklichtbedingungen, beschränkte Verfügbarkeit von Stickstoffnährstoffen, beschränkte Verfügbarkeit von Phosphornährstoffen oder Vermeidung von Schatten zählen.

Pflanzen und Pflanzensorten, die ebenfalls erfindungsgemäß behandelt werden können, sind solche Pflanzen, die durch erhöhte Ertragseigenschaften gekennzeichnet sind. Ein erhöhter Ertrag kann bei diesen Pflanzen z. B. auf verbesserter Pflanzenphysiologie, verbessertem Pflanzenwuchs und verbesserter Pflanzenentwicklung, wie Wasserverwertungseffizienz, Wasserhalteeffizienz, verbesserter Stickstoffverwertung, erhöhter Kohlenstoffassimilation, verbesserter Photosynthese, verstärkter Keimkraft und beschleunigter Abreife beruhen. Der Ertrag kann weiterhin durch eine verbesserte Pflanzenarchitektur (unter Streß- und nicht-Streß-Bedingungen) beeinflußt werden, darunter frühe Blüte, Kontrolle der Blüte für die Produktion von Hybridsaatgut, Keimpflanzenwüchsigkeit, Pflanzengröße, Internodienzahl und - abstand, Wurzelwachstum, Samengröße, Fruchtgröße, Schotengröße, Schoten- oder Ährenzahl, Anzahl der Samen pro Schote oder Ähre, Samenmasse, verstärkte Samenfüllung, verringerter Samenausfall, verringertes Schotenplatzen sowie Standfestigkeit. Zu weiteren Ertragsmerkmalen zählen Samenzusammensetzung wie Kohlenhydratgehalt, Proteingehalt, Ölgehalt und Ölzusammensetzung, Nährwert, Verringerung der nährwidrigen Verbindungen, verbesserte Verarbeitbarkeit und verbesserte Lagerfähigkeit.

Pflanzen, die erfindungsgemäß behandelt werden können, sind Hybridpflanzen, die bereits die Eigenschaften der Heterosis bzw. des Hybrideffekts exprimieren, was im allgemeinen zu höherem Ertrag, höherer Wüchsigkeit, besserer Gesundheit und besserer Resistenz gegen biotische und abiotische Streßfaktoren führt. Solche Pflanzen werden typischerweise dadurch erzeugt, daß man eine ingezüchtete pollensterile Elternlinie (den weiblichen Kreuzungspartner) mit einer anderen ingezüchteten pollenfertilen Elternlinie (dem männlichen Kreuzungspartner) kreuzt. Das Hybridsaatgut wird typischerweise von den pollensterilen Pflanzen geerntet und an Vermehrer verkauft. Pollensterile Pflanzen können manchmal (z. B. beim Mais) durch Entfahnen (d. h. mechanischem Entfernen der männlichen Geschlechtsorgane bzw. der männlichen Blüten), produziert werden; es ist jedoch üblicher, daß die Pollensterilität auf genetischen Determinanten im Pflanzengenom beruht. In diesem Fall, insbesondere dann, wenn es sich bei dem gewünschten Produkt, da man von den Hybridpflanzen ernten will, um die Samen handelt, ist es üblicherweise günstig, sicherzustellen, daß die Pollenfertilität in Hybridpflanzen, die die für die Pollensterilität verantwortlichen genetischen Determinanten enthalten, völlig restoriert wird. Dies kann erreicht werden, indem sichergestellt wird, daß die männlichen Kreuzungspartner entsprechende Fertilitätsrestorergene besitzen, die in der Lage sind, die Pollenfertilität in Hybridpflanzen, die die genetischen Determinanten, die für die Pollensterilität verantwortlich sind, enthalten, zu restorieren. Genetische Determinanten für Pollensterilität können im Cytoplasma lokalisiert sein. Beispiele für cytoplasmatische Pollensterilität (CMS) wurden zum Beispiel für Brassica-Arten beschrieben. Genetische Determinanten für Pollensterilität können jedoch auch im Zellkerngenom lokalisiert sein. Pollensterile Pflanzen können auch mit Methoden der pflanzlichen Biotechnologie, wie Gentechnik, erhalten werden. Ein besonders günstiges Mittel zur Erzeugung von pollensterilen Pflanzen ist in WO 89/10396 beschrieben, wobei zum Beispiel eine Ribonuklease wie eine Barnase selektiv in den Tapetumzellen in den Staubblättern exprimiert wird. Die Fertilität kann dann durch Expression eines Ribonukleasehemmers wie Barstar in den Tapetumzellen restoriert werden.

Pflanzen oder Pflanzensorten (die mit Methoden der Pflanzenbiotechnologie, wie der Gentechnik, erhalten werden), die erfindungsgemäß behandelt werden können, sind herbizidtolerante Pflanzen, d. h. Pflanzen, die gegenüber einem oder mehreren vorgegebenen Herbiziden tolerant gemacht worden sind. Solche Pflanzen können entweder durch genetische Transformation oder durch Selektion von Pflanzen, die eine Mutation enthalten, die solch eine Herbizidtoleranz verleiht, erhalten werden.

Herbizidtolerante Pflanzen sind zum Beispiel glyphosatetolerante Pflanzen, d. h. Pflanzen, die gegenüber dem Herbizid Glyphosate oder dessen Salzen tolerant gemacht worden sind. So können zum Beispiel glyphosatetolerante Pflanzen durch Transformation der Pflanze mit einem Gen, das für das Enzym 5-Enolpyruvylshikimat-3-phosphatsynthase (EPSPS) kodiert, erhalten werden. Beispiele für solche EPSPS-Gene sind das AroA-Gen (Mutante CT7) des Bakterium *Salmonella typhimurium*, das CP4-Gen des Bakteriums *Agrobacteriiim sp.,* die Gene, die für eine EPSPS aus der Petunie, für eine EPSPS aus der Tomate oder für eine EPSPS aus Eleusine kodieren. Es kann sich auch um eine mutierte EPSPS handeln. Glyphosatetolerante Pflanzen können auch dadurch erhalten werden, daß man ein Gen exprimiert, das für ein Glyphosate-Oxidoreduktase-Enzym kodiert. Glyphosatetolerante Pflanzen können auch dadurch erhalten werden, daß man ein Gen exprimiert, das für ein Glyphosate-acetyltransferase-Enzym kodiert. Glyphosatetolerante Pflanzen können auch dadurch erhalten werden, daß man Pflanzen, die natürlich vorkommende Mutationen der oben erwähnten Gene selektiert.

Sonstige herbizidresistente Pflanzen sind zum Beispiel Pflanzen, die gegenüber Herbiziden, die das Enzym Glutaminsynthase hemmen, wie Bialaphos, Phosphinotricin oder Glufosinate, tolerant gemacht worden sind. Solche Pflanzen können dadurch erhalten werden, daß man ein Enzym exprimiert, das das Herbizid oder eine Mutante des Enzyms Glutaminsynthase, das gegenüber Hemmung resistent ist, entgiftet. Solch ein wirksames entgiftendes Enzym ist zum Beispiel ein Enzym, das für ein Phosphinotricin-acetyltransferase kodiert (wie zum Beispiel das bar- oder pat-Protein aus Streptomyces-Arten). Pflanzen, die eine exogene Phosphinotricin-acetyltransferase exprimieren, sind beschrieben.

Weitere herbizidtolerante Pflanzen sind auch Pflanzen, die gegenüber den Herbiziden, die das Enzym Hydroxyphenylpyruvatdioxygenase (HPPD) hemmen, tolerant gemacht worden sind. Bei den Hydroxyphenylpyruvatdioxygenasen handelt es sich um Enzyme, die die Reaktion, in der paraHydroxyphenylpyruvat (HPP) zu Homogentisat umgesetzt wird, katalysieren. Pflanzen, die gegenüber HPPD-Hemmern tolerant sind, können mit einem Gen, das für ein natürlich vorkommendes resistentes HPPD-Enzym kodiert, oder einem Gen, das für ein mutiertes HPPD-Enzym kodiert, transformiert werden. Eine Toleranz gegenüber HPPD-Hemmern kann auch dadurch erzielt werden, daß man Pflanzen mit Genen transformiert, die für gewisse Enzyme kodieren, die die Bildung von Homogentisat trotz Hemmung des nativen HPPD-Enzyms durch den HPPD-Hemmer ermöglichen. Die Toleranz von Pflanzen gegenüber HPPD-Hemmern kann auch dadurch verbessert werden, daß man Pflanzen zusätzlich zu einem Gen, das für ein HPPD-tolerantes Enzym kodiert, mit einem Gen transformiert, das für ein Prephenatdehydrogenase-Enzym kodiert.

Weitere herbizidresistente Pflanzen sind Pflanzen, die gegenüber Acetolactatsynthase (ALS)-Hemmem tolerant gemacht worden sind. Zu bekannten ALS-Hemmern zählen zum Beispiel Sulfonylharnstoff, Imidazolinon, Triazolopyrimidine, Pyrimidinyloxy(thio)benzoate und/oder Sulfonylaminocarbonyltriazolinon-Herbizide. Es ist bekannt, daß verschiedene Mutationen im Enzym ALS (auch als Acetohydroxysäure-Synthase, AHAS, bekannt) eine Toleranz gegenüber unterschiedlichen Herbiziden bzw. Gruppen von Herbiziden verleihen. Die Herstellung von sulfonylharnstofftoleranten Pflanzen und imidazolinontoleranten Pflanzen ist in der internationalen Veröffentlichung WO 1996/033270 beschrieben. Weitere sulfonylharnstoff und imidazolinontolerante Pflanzen sind auch in z.B. WO 2007/024782 beschrieben.

Weitere herbizidresistente Pflanzen sind Pflanzen, die gegenüber ACCase-Hemmern tolerant gemacht worden sind.

Weitere Pflanzen, die gegenüber Imidazolinon und/oder Sulfonylharnstoff tolerant sind, können durch induzierte Mutagenese, Selektion in Zellkulturen in Gegenwart des Herbizids oder durch Mutationszüchtung erhalten werden.

Pflanzen oder Pflanzensorten (die nach Methoden der pflanzlichen Biotechnologie, wie der Gentechnik, erhalten wurden), die ebenfalls erfindungsgemäß behandelt werden können, sind insektenresistente transgene Pflanzen, d.h. Pflanzen, die gegen Befall mit gewissen Zielinsekten resistent gemacht wurden. Solche Pflanzen können durch genetische Transformation oder durch Selektion von Pflanzen, die eine Mutation enthalten, die solch eine Insektenresistenz verleiht, erhalten werden.

Der Begriff "insektenresistente transgene Pflanze" umfaßt im vorliegenden Zusammenhang jegliche Pflanze, die mindestens ein Transgen enthält, das eine Kodiersequenz umfaßt, die für folgendes kodiert:
1) ein insektizides Kristallprotein aus *Bacillus thuringiensis* oder einen insektiziden Teil davon, wie die insektiziden Kristallproteine, die online bei: http://www.lifesci.sussex.ac.uk/Home/Neil_Crickmore/Bt/ beschrieben sind, zusammengestellt wurden, oder insektizide Teile davon, z.B. Proteine der Cry-Proteinklassen CrylAb, CrylAc, CrylF, Cry2Ab, Cry3Ae oder Cry3Bb oder insektizide Teile davon; oder
2) ein Kristallprotein aus *Bacillus thuringiensis* oder einen Teil davon, der in Gegenwart eines zweiten, anderen Kristallproteins als *Bacillus thuringiensis* oder eines Teils davon insektizid wirkt, wie das binäre Toxin, das aus den Kristallproteinen Cy34 und Cy35 besteht; oder
3) ein insektizides Hybridprotein, das Teile von zwei unterschiedlichen insektiziden Kristallproteinen aus *Bacillus thuringiensis* umfaßt, wie zum Beispiel ein Hybrid aus den Proteinen von 1) oben oder ein Hybrid aus den Proteinen von 2) oben, z. B. das Protein CrylA.105, das von dem Mais-Event MON98034 produziert wird (WO 2007/027777); oder
4) ein Protein gemäß einem der Punkte 1) bis 3) oben, in dem einige, insbesondere 1 bis 10, Aminosäuren durch eine andere Aminosäure ersetzt wurden, um eine höhere insektizide Wirksamkeit gegenüber einer Zielinsektenart zu erzielen und/oder um das Spektrum der entsprechenden Zielinsektenarten zu erweitern und/oder wegen Veränderungen, die in die Kodier-DNA während der Klonierung oder Transformation induziert wurden, wie das Protein Cry3Bb1 in Mais-Events MON863 oder MON88017 oder das Protein Cry3A im Mais-Event MIR 604;
5) ein insektizides sezerniertes Protein aus *Bacillus thuringiensis* oder *Bacillus cereus* oder einen insektiziden Teil davon, wie die vegetativ wirkenden insektentoxischen Proteine (vegetative insekticidal proteins, VIP), die unter http://www.lifesci.sussex.ac.uk/Home/Neil_Crickmore/Bt/vip.html angeführt sind, z. B. Proteine der Proteinklasse VIP3Aa; oder
6) ein sezerniertes Protein aus *Bacillus thuringiensis* oder *Bacillus cereus,* das in Gegenwart eines zweiten sezernierten Proteins aus *Bacillus thuringiensis* oder *B. cereus* insektizid wirkt, wie das binäre Toxin, das aus den Proteinen VIP1A und VIP2A besteht.
7) ein insektizides Hybridprotein, das Teile von verschiedenen sezernierten Proteinen von *Bacillus thuringiensis* oder *Bacillus cereus* umfaßt, wie ein Hybrid der Proteine von 1) oder ein Hybrid der Proteine von 2) oben; oder
8) ein Protein gemäß einem der Punkte 1) bis 3) oben, in dem einige, insbesondere 1 bis 10, Aminosäuren durch eine andere Aminosäure ersetzt wurden, um eine höhere insektizide Wirksamkeit gegenüber einer Zielinsektenart zu erzielen und/oder um das Spektrum der entsprechenden Zielinsektenarten zu erweitern und/oder wegen Veränderungen, die in die Kodier-DNA während der Klonierung oder Transformation induziert wurden (wobei die Kodierung für ein insektizides Protein erhalten bleibt), wie das Protein VIP3Aa im Baumwoll-Event COT 102.

Natürlich zählt zu den insektenresistenten transgenen Pflanzen im vorliegenden Zusammenhang auch jegliche Pflanze, die eine Kombination von Genen umfaßt, die für die Proteine von einer der oben genannten Klassen 1 bis 8 kodieren. In einer Ausführungsform enthält eine insektenresistente Pflanze mehr als ein Transgen, das für ein Protein nach einer der oben genannten 1 bis 8 kodiert, um das Spektrum der entsprechenden Zielinsektenarten zu erweitern oder um die Entwicklung einer Resistenz der Insekten gegen die Pflanzen dadurch hinauszuzögern, daß man verschiedene Proteine einsetzt, die für dieselbe Zielinsektenart insektizid sind, jedoch eine unterschiedliche Wirkungsweise, wie Bindung an unterschiedliche Rezeptorbindungsstellen im Insekt, aufweisen.

Pflanzen oder Pflanzensorten (die nach Methoden der pflanzlichen Biotechnologie, wie der Gentechnik, erhalten wurden), die ebenfalls erfindungsgemäß behandelt werden können, sind gegenüber abiotischen Streßfaktoren tolerant. Solche Pflanzen können durch genetische Transformation oder durch Selektion von Pflanzen, die eine Mutation enthalten, die solch eine Streßresistenz verleiht, erhalten werden. Zu besonders nützlichen Pflanzen mit Streßtoleranz zählen folgende:
a. Pflanzen, die ein Transgen enthalten, das die Expression und/oder Aktivität des Gens für die Poly(ADP-ribose)polymerase (PARP) in den Pflanzenzellen oder Pflanzen zu reduzieren vermag.
b. Pflanzen, die ein streßtoleranzförderndes Transgen enthalten, das die Expression und/oder Aktivität der für PARG kodierenden Gene der Pflanzen oder Pflanzenzellen zu reduzieren vermag;
c. Pflanzen, die ein streßtoleranzförderndes Transgen enthalten, das für ein in Pflanzen funktionelles Enzym des Nicotinamidadenindinukleotid-Salvage-Biosynthesewegs kodiert, darunter Nicotinamidase, Nicotinatphosphoribosyltransferase, Nicotinsäuremononukleotidadenyltransferase, Nicotinamidadenindinukleotidsynthetase oder Nicotinamidphosphoribosyltransferase.

Pflanzen oder Pflanzensorten (die nach Methoden der pflanzlichen Biotechnologie, wie der Gentechnik, erhalten wurden), die ebenfalls erfindungsgemäß behandelt werden können, weisen eine veränderte Menge, Qualität und/oder Lagerfähigkeit des Ernteprodukts und/oder veränderte Eigenschaften von bestimmten Bestandteilen des Ernteprodukts auf, wie zum Beispiel:
1) Transgene Pflanzen, die eine modifizierte Stärke synthetisieren, die bezüglich ihrer chemisch-physikalischen Eigenschaften, insbesondere des Amylosegehalts oder des Amylose/Amylopektin-Verhältnisses, des Verzweigungsgrads, der durchschnittlichen Kettenlänge, der Verteilung der Seitenketten, des Viskositätsverhaltens, der Gelfestigkeit, der Stärkekorngröße und/oder Stärkekornmorphologie im Vergleich mit der synthetisierten Stärke in Wildtyppflanzenzellen oder - pflanzen verändert ist, so daß sich diese modifizierte Stärke besser für bestimmte Anwendungen eignet.
2) Transgene Pflanzen, die Nichtstärkekohlenhydratpolymere synthetisieren, oder Nichtstärkekohlenhydratpolymere, deren Eigenschaften im Vergleich zu Wildtyppflanzen ohne genetische Modifikation verändert sind. Beispiele sind Pflanzen, die Polyfructose, insbesondere des Inulin- und Levantyps, produzieren, Pflanzen, die alpha-1,4-Glucane produzieren, Pflanzen, die alpha-1,6-verzweigte alpha-1,4-Glucane produzieren und Pflanzen, die Alternan produzieren.
3) Transgene Pflanzen, die Hyaluronan produzieren.

Pflanzen oder Pflanzensorten (die nach Methoden der pflanzlichen Biotechnologie, wie der Gentechnik, erhalten wurden), die ebenfalls erfindungsgemäß behandelt werden können, sind Pflanzen wie Baumwollpflanzen mit veränderten Fasereigenschaften. Solche Pflanzen können durch genetische Transformation oder durch Selektion von Pflanzen, die eine Mutation enthalten, die solche veränderten Fasereigenschaften verleiht, erhalten werden; dazu zählen:
a) Pflanzen wie Baumwollpflanzen, die eine veränderte Form von Cellulosesynthasegenen enthalten,
b) Pflanzen wie Baumwollpflanzen, die eine veränderte Form von rsw2- oder rsw3-homologen Nukleinsäuren enthalten;
c) Pflanzen wie Baumwollpflanzen mit einer erhöhten Expression der Saccharosephosphatsynthase;
d) Pflanzen wie Baumwollpflanzen mit einer erhöhten Expression der Saccharosesynthase;
e) Pflanzen wie Baumwollpflanzen bei denen der Zeitpunkt der Durchlaßsteuerung der Plasmodesmen an der Basis der Faserzelle verändert ist, z. B. durch Herunterregulieren der faserselektiven β-1,3-Glucanase;
f) Pflanzen wie Baumwollpflanzen mit Fasern mit veränderter Reaktivität, z. B. durch Expression des N-Acetylglucosamintransferasegens, darunter auch nodC, und von Chitinsynthasegenen.

Pflanzen oder Pflanzensorten (die nach Methoden der pflanzlichen Biotechnologie, wie der Gentechnik, erhalten wurden), die ebenfalls erfindungsgemäß behandelt werden können, sind Pflanzen wie Raps oder verwandte Brassica-Pflanzen mit veränderten Eigenschaften der Ölzusammensetzung. Solche Pflanzen können durch genetische Transformation oder durch Selektion von Pflanzen, die eine Mutation enthalten, die solche veränderten Öleigenschaften verleiht, erhalten werden; dazu zählen:
a) Pflanzen wie Rapspflanzen, die Öl mit einem hohen Ölsäuregehalt produzieren;
b) Pflanzen wie Rapspflanzen, die Öl mit einem niedrigen Linolensäuregehalt produzieren.
c) Pflanzen wie Rapspflanzen, die Öl mit einem niedrigen gesättigten Fettsäuregehalt produzieren.

Besonders nützliche transgene Pflanzen, die erfindungsgemäß behandelt werden können, sind Pflanzen mit einem oder mehreren Genen, die für ein oder mehrere Toxine kodieren, sind die transgenen Pflanzen, die unter den folgenden Handelsbezeichnungen angeboten werden: YIELD GARD® (zum Beispiel Mais, Baumwolle, Sojabohnen), KnockOut® (zum Beispiel Mais), BiteGard® (zum Beispiel Mais), BT-Xtra® (zum Beispiel Mais), StarLink® (zum Beispiel Mais), Bollgard® (Baumwolle), Nucotn® (Baumwolle), Nucotn 33B® (Baumwolle), NatureGard® (zum Beispiel Mais), Protecta® und NewLeaf® (Kartoffel). Herbizidtolerante Pflanzen, die zu erwähnen sind, sind zum Beispiel Maissorten, Baumwollsorten und Sojabohnensorten, die unter den folgenden Handelsbezeichnungen angeboten werden: Roundup Ready® (Glyphosatetoleranz, zum Beispiel Mais, Baumwolle, Sojabohne), Liberty Link® (Phosphinotricintoleranz, zum Beispiel Raps), IMI® (Imidazolinontoleranz) und SCS® (Sylfonylharnstofftoleranz), zum Beispiel Mais. Zu den herbizidresistenten Pflanzen (traditionell auf Herbizidtoleranz gezüchtete Pflanzen), die zu erwähnen sind, zählen die unter der Bezeichnung Clearfield® angebotenen Sorten (zum Beispiel Mais).

Besonders nützliche transgene Pflanzen, die erfindungsgemäß behandelt werden können, sind Pflanzen, die Transformations-Events, oder eine Kombination von Transformations-Events, enthalten und die zum Beispiel in den Dateien von verschiedenen nationalen oder regionalen Behörden angeführt sind (siehe zum Beispiel http://gmoinfo.jrc.it/gmp_browse.aspx und http://www.agbios.com/dbase.php).

Die Bezeichnung "Wirkstoffe" bzw. "Verbindungen" schließt immer auch die hier genannten Wirkstoffkombinationen mit ein.

### Herstellungsbeispiele

### Beispiel I-a-1

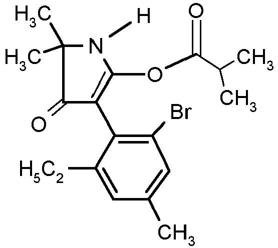

2 g (2.7 mmol) der Verbindung gemäß Beispiel I-1-a-6 aus WO 07/068427 werden zusammen mit 0.29 (2.7 mmol) 2-Methylpropionylchlorid in 53 ml Toluol bei Raumtemperatur vorgelegt. Bei dieser Temperatur werden sehr langsam 0.36 g (3.5 mmol) Triethylamin zugetropft. Nach Einengen und Aufnehmen mit Heptan/Essigsäureethylester fällt das gewünschte Produkt als Feststoff aus, während das isomere Acylierungsprodukt in Lösung bleibt.
Ausbeute: 0.74 g (70% der Theorie), Fp.130°C

1H-NMR (CDCl3, 400 MHz): 7,25 (d, 1H), 7,00 (d, 1H), 6,55 (sbr, 1H), 2,65 (sept, 1H), 2,50 (q, 2H), 2,30 (s, 3H), 1,25 (s, 3H), 1,20 (s, 3H), 1,17 (d, 3H), 1,15 (d, 3H), 1,10 (tr, 3H) ppm.

13C-NMR (CDCl3, 400 MHz): 198,8 ppm (Keto-Gruppe).

In Analogie zu den Beispielen (I-a-1) und gemäß den allgemeinen Angaben zur Herstellung erhält man folgende Verbindungen der Formel (I-a):

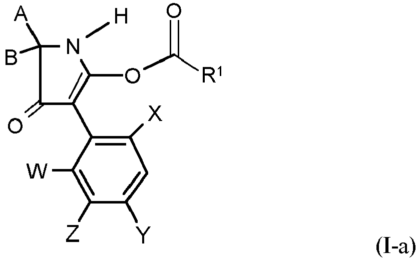

| **Bsp**.-**Nr.** | **W** | **X** | **Y** | **Z** | **A** | **B** | **R¹** | **Fp°C** | **Isomer** |
|---|---|---|---|---|---|---|---|---|---|
| I-a-2 | CH₃ | OCH₃ | CH₃ | H | -(CH₂)₂-CHOCH₃-(CH₂)₂- | | t-C₄H₉ | 124 * | β |
| I-a-3 | H | C₂H₅ | Br | H | -(CH₂)₂-CHCH₃-(CH₂)₂- | | t-C₄H₉ | * | β |
| I-a-4 | C₂H₅ | Cl | Cl | H | -(CH₂)₂-CHCH₃-(CH₂)₂- | | t-C₄H₉ | * | β |
| I-a-5 | CH₃ | C₂H₅ | Br | H | -(CH₂)₂-CHOCH₃-(CH₂)₂- | | t-C₄H₉ | * | β |
| I-a-6 | H | C₂H₅ | CH₃ | H | -(CH₂)₂-CHCH₃-(CH₂)₂- | | t-C₄H₉ | * | β |
| I-a-7 | CH₃ | C₂H₅ | Br | H | -(CH₂)₂-CHCH₃-(CH₂)₂- | | t-C₄H₉ | * | β |
| I-a-8 | C₂H₅ | Br | Br | H | -(CH₂)₅- | | t-C₄H₉ | 136 | - |
| I-a-9 | C₂H₅ | OCH₃ | Cl | H | -(CH₂)₂-CHOCH₃-(CH₂)₂- | | t-C₄H₉ | 146 | β |
| I-a-10 | C₂H₅ | OCH₃ | Cl | H | -(CH₂)₅- | | i-C₃H₇ | 204 | |
| I-a-11 | C₃H₇ | Br | Br | H | CH₃ | CH₃ | t-C₄H₉ | * | |
| I-a-12 | C₂H₅ | OCH₃ | Cl | H | -(CH₂)₂-CHCH₃-(CH₂)₂- | | i-C₃H₇ | Zers. | β |
| I-a-13 | C₂H₅ | Br | CH₃ | H | CH₃ | CH₃ | t-C₄H₉ | 150 | |
| I-a-14 | C₂H₅ | Cl | Br | H | -(CH₂)₅- | | t-C₄H₉ | 234 | |
| I-a-15 | C₂H₅ | OCH₃ | Cl | H | -(CH₂)₅- | | t-C₄H₉ | 166 | |
| I-a-16 | H | CH₃ | H | CH₃ | -(CH₂)₂-CHOCH₃-(CH₂)₂- | | -C(CH₃)₂ C₂H₅ | * | β |

### (I-a-2)

* 1H-NMR (400 MHz, CDCl₃): 6,70 (d, 1H), 6,50 (d, 1H), 3,70 (s, 3H), 3,35 (s, 3H), 3,30 (m, 1H), 2,30 (s, 3H), 2,25 (s, 3H), 1,20 (s, 9H) ppm.

### (I-a-3)

* 1H-NMR (300 MHz, CDCl₃): 7.39 (d, 1H), 7.26 (dd, 1H), 6.88 (d, 1H), 6,72 (s breit, 1H), 2.47 (m, 2H), 2.15 (s, 3H), 1.82 (m, 4H), 1.65 (Signalhaufen breit, 3H), 1.19 (s, 9H), 1,11 (t, 3H), 1.05 (m, 1H), 0,96 (d, 3H), 0,95 (m, 1H) ppm.

### (I-a-4)

* 1H-NMR (300 MHz, CDCl₃): 7.29 (s, 1H), 7.13 (s, 1H), 6,91 (s breit, 1H), 2.56 (q, 2H), 1.92 (m, 4H), 1.60 (Signalhaufen breit, 3H), 1.19 (s, 9H), 1,09 (t, 3H), 1.07 (m, 1H), 0,97 (d, 3H), 0,87 (m, 1H) ppm.

### (I-a-5)

* 1H-NMR (300 MHz, CDCl₃): 7.18 (s, 2H), 6,82 (s breit, 1H), 3.21 (s, 3H), 3.15 8m ,1H), 2.42 (m, 2H), 2.24 (m, 2H), 2.11 (s, 3H), 1.95 (m, 2H), 1.67 (m, 2H), 1.40 (m, 2H), 1.12 (s, 9H), 1,07 (t, 3H) ppm.

### (I-a-6)

* 1H-NMR (300 MHz, CDCl₃): 7.01 (s, 1H), 6.98 (s, 2H), 6,72 (s breit, 1H), 2.46 (m, 2H), 2.15 (s, 3H), 1.82 (m, 4H), 1.65 (Signalhaufen breit, 3H), 1.18 (s, 9H), 1,09 (t, 3H), 1.05 (m, 1H), 0,96 (d, 3H), 0,87 (m, 1H) ppm.

### (I-a-7)

* 1H-NMR (300 MHz, CDCl₃): 7.18 (s, 2H), 6,78 (s breit, 1H), 2.43 (m, 2H), 2.11 (s, 3H), 1.92 (m, 4H), 1.60 (Signalhaufen breit, 3H), 1.13 (s, 9H), 1,07 (t, 3H), 1.05 (m, 2H), 0,97 (d, 3H) ppm.

### (I-a-8)

* 1H-NMR (300 MHz, CDCl₃): 7,60 (d, 1H), 7,30 (d, 1H), 7,00 (sbr, 1H), 2,50 (q, 2H), 2,00-1,50 (m, 8H), 1,50-1,30 (m, 2H), 1,20 (s, 9H), 1,10 (tr, 3H) ppm.

### (I-a-9)

* 1H-NMR (400 MHz, CDCl₃): 6,90 (d, 1H), 6,70 (d, 1H), 3,70 (s, 3H), 3,35 (s, 3H), 3,30 (m, 1H), 2,50 (m, 2H), 1,20 (s, 9H) ppm.

### (I-a-10)

* 1H-NMR (400 MHz, CDCl₃): 6,90 (d, 1H), 6,70 (d, 1H), 3,70 (s, 3H), 2,65 (q, 2H), 2,00-1,30 (m, 10H), 1,30-1,20 (m, 6H) ppm.

### (I-a-11)

* 1H-NMR (400 MHz, CDCl₃): 7,60 (d, 1H), 7,35 (d, 1H), 2,50 (m, 2H), 1,25 (s, 6H), 1,15 (m, 9H) ppm.

### (I-a-12)

* 1H-NMR (300 MHz, CDCl₃): 6,85 (d, 1H), 6,70 (d, 1H), 3,70 (s, 3H), 2,65 (sept, 1H), 2,50 (dq, 2H), 2,00-1,80 (m, 4H), 1,70-1,40 (m, 5H), 1,175 (d, 3H), 1,150 (d, 3H), 1,10 (tr, 3H), 0,95 (d, 3H) ppm.

### (I-a-13)

* 1H-NMR (400 MHz, CDCl₃): 7,30 (d, 1H), 7,05 (d, 1H), 6,55 (sbr, 1H), 2,55 (q, 2H), 2,30 (s, 3H), 1,50 (s, 3H), 1,45 (s, 3H), 1,20 (tr, 3H), 1,10 (s, 9H) ppm.

### (I-a-14)

* 1H-NMR (300 MHz, CDCl₃): 7,40 (d, 1H), 7,30 (d, 1H), 6,95 (sbr, 1H), 2,50 (q, 2H), 2,00-1,60 (m, 8H), 1,50-1,30 (m, 2H), 1,20 (s, 9H), 1,10 (tr, 3H) ppm.

### (I-a-16)

* 1H-NMR (400 MHz, d₆-DMSO): 0.54 (t, 3H), 1.04 (s, 6H), 1.40-1.57 (m, 6H), 1.65-1.72 (m, 2H), 2.05 (s, 3H), 2.19 (s, 3H), 3.21-3.29 (m + s, 1H + 3H), 6.74 (s, 1H), 6.92-6.94 (d, 1H), 7.01-7.03 (d, 1H), 9.05 (sbr, 1H), ppm.

### Beispiel (I-b-1)

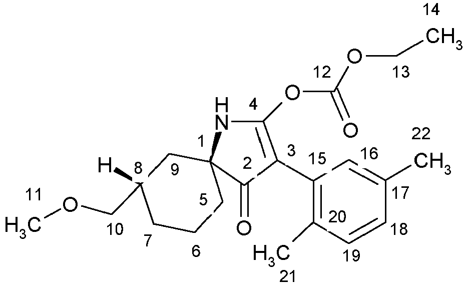

631 mg (2 mmol) der Verbindung gemäß Beispiel (I-1-a-6) bekannt aus WO 07/048545 werden in 20 ml Dichlormethan vorgelegt und mit 0,28 ml (2 mmol) Triethylamin versetzt. Bei 20 ° C tropft man 0.22 ml (2 mmol) Chlorameisensäure-ethylester in 5 ml Dichlormethan zu und rührt 1 h nach. Das Lösungsmittel wird abgedampft und der Rückstand an Kieselgel mit Methylenchlorid/Essigsäureethylester chromatographiert.
Ausbeute 0.3 g (31 % d. Theorie) vom Schmelzpunkt 238 ° C.

¹³C-NMR (600 MHz, CDCl₃): δ= 14.2 (C-14), 19.7 (C-21), 21.0 (C-22), 22.9 (C-6), 28.9 (C-7), 35.4 (C-5, C-8), 36.9 (C-9), 59.1 (C-11), 67.5 (C-13), 69.1 (C-1), 78.7 (C-10), 100.6 (C-3), 129.2 (C-18), 130.4 (C-15), 130.9 (C-19), 132.0 (C-16), 135.9 (C-17), 136.0 (C-20), 150.5 (C-12), 170.2 (C-4), 200.0 (C-2) ppm.

Außerdem werden 0.2 g (20 % d. Theorie) des Produkts Beispiel I-1-c-2 bekannt aus WO 07/048545 isoliert.

In Analogie zu Beispiel (I-b-1) und gemäß den allgemeinen Angaben zur Herstellung erhält man folgende Verbindungen der Formel (I-b-1)

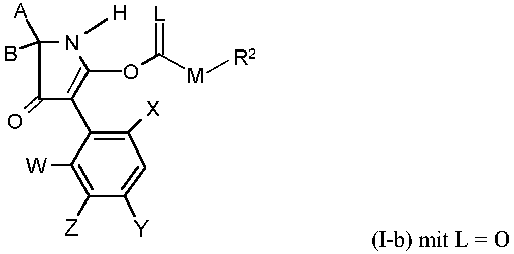

| Bsp. Nr. | W | X | Y | Z | A | B | M | R² | Fp°C | Isomer |
|---|---|---|---|---|---|---|---|---|---|---|
| I-b-2 | CH₃ | C₂H₅ | J | H | -(CH₂)₂-CHOCH₃-(CH₂)₂- | | O | C₂H₅ | * | β |
| I-b-3 | CH₃ | CH₃ | CH₃ | H | 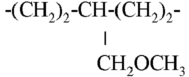 | | O | C₂H₅ | 174, * | β |
| I-b-4 | H | CH₃ | Cl | CH₃ | 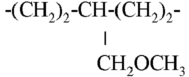 | | O | C₂H₅ | 66 | β |
| I-b-5 | C₂H₅ | c-C₃H₅ | CH₃ | H | CH₃ | CH₃ | O | C₂H₅ | * | - |

### I-b-2

* ¹H-NMR (400 MHz, CD₃CN): δ = 1.02 (t, 3H, Ar-CH₂CH3), 1.20 (t, 3H, OCH₂CH₃), 1.29-1.44 (m, 2H), 1.66-1.89 (m, 4H), 2.05 (s, 3H, Ar-CH₃), 2.36-2.45 (m, 2H), 3.25-3.30 (m, 1H, CHOCH₃), 3.32 (s, 3H, OCH₃), 4.16-4.21 (q, 2H, OCH₂CH₃), 7.05 (s, br, 1H, NH), 7.46 (s, 2H, ArH) ppm.

### I-b-3

* ¹H-NMR (400 MHz, CD₃CN): δ = 1.19 (t, 3H, OCH₂CH₃), 1.21-1.27 ("dt", 2H), 1.61-1.96 (m, 6H), 2.05 (s, 6H, Ar-CH₃), 2.24 (s, 3H, Ar-CH₃), 2.87-2.91 (d, 2H), 3.23-3.25 (d, 2H, CHCH₂O), 3.29 (s, 3H, OCH₃), 6.77 (s, br, 1H, NH), 6.86 (s, 2H, ArH) ppm.

### (I-b-5)

* 1H-NMR (400 MHz, CDCl₃): 6.90 (d, 1H), 6.70 (d, 1H), 6.36 (s, breit, 1H), 4.29 (q, 2H), 2.48 (m, 2H), 2.29 (s, 3H), 1.78 (m, 1H), 1.45 (dd, 6H), 1.32 (t, 3H), 1.11 (dt, 3H), 0,8 - 0,4 (Signalhaufen, 4H) ppm.

### Beispiel 1

### Phaedon -Test (PHAECO Spritzbehandlung)

| | | |
|---|---|---|
| Lösungsmittel: | 78,0 | Gewichtsteile Aceton |
| | 1,5 | Gewichtsteile Dimethylformamid |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration. Chinakohlblattscheiben *(Brassica pekinensis*) werden mit einer Wirkstoffzubereitung der gewünschten Konzentration gespritzt und nach dem Abtrocknen mit Larven des Meerrettichblattkäfers *(Phaedon cochleariae)* besetzt.

Nach 7 Tagen wird die Wirkung in % bestimmt. Dabei bedeutet 100 %, dass alle Käferlarven abgetötet wurden; 0 % bedeutet, dass keine Käferlarven abgetötet wurden.

Bei diesem Test zeigen z. B. die folgenden Verbindungen der Herstellungsbeispiele Wirkung von 83 % bei einer Aufwandmenge von 500g/ha: Bsp. I-a-1, I-a-11, I-a-13, I-b-2

Bei diesem Test zeigen z. B. die folgenden Verbindungen der Herstellungsbeispiele Wirkung von 100 % bei einer Aufwandmenge von 500g/ha: Bsp. I-a-2, I-a-14, I-b-1, I-b-3.

### Beispiel 2

### Spodoptera frugiperda -Test (SPODFR Spritzbehandlung)

| | | |
|---|---|---|
| Lösungsmittel: | 78,0 | Gewichtsteile Aceton |
| | 1,5 | Gewichtsteile Dimethylformamid |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration. Maisblattscheiben (*Zea mays*) werden mit einer Wirkstoffzubereitung der gewünschten Konzentration gespritzt und nach dem Abtrocknen mit Raupen des Heerwurms (*Spodoptera frugiperda*) besetzt.

Nach 7 Tagen wird die Wirkung in % bestimmt. Dabei bedeutet 100 %, dass alle Raupen abgetötet wurden; 0 % bedeutet, dass keine Raupe abgetötet wurde.

Bei diesem Test zeigen z. B. die folgenden Verbindungen der Herstellungsbeispiele Wirkung von 100 % bei einer Aufwandmenge von 500g/ha: Bsp. I-a-2, I-b-3.

### Beispiel 3

### Myzus-Test (MYZUPE Spritzbehandlung)

| | | |
|---|---|---|
| Lösungsmittel: | 78 | Gewichtsteile Aceton |
| | 1,5 | Gewichtsteile Dimethylformamid |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration. Chinakohlblattscheiben *(Brassica pekinensis*), die von allen Stadien der Grünen Pfirsichblattlaus (*Myzus persicae*) befallen sind, werden mit einer Wirkstoffzubereitung der gewünschten Konzentration gespritzt.

Nach 5 Tagen wird die Wirkung in % bestimmt. Dabei bedeutet 100 %, dass alle Blattläuse abgetötet wurden; 0 % bedeutet, dass keine Blattläuse abgetötet wurden.

Bei diesem Test zeigen z. B. die folgenden Verbindungen der Herstellungsbeispiele Wirkung von 80 % bei einer Aufwandmenge von 500g/ha: Bsp. I-a-9, I-a-13

Bei diesem Test zeigen z. B. die folgenden Verbindungen der Herstellungsbeispiele Wirkung von 90 % bei einer Aufwandmenge von 500g/ha: Bsp. I-a-1, I-a-14, I-b-3, I-b-4

Bei diesem Test zeigen z. B. die folgenden Verbindungen der Herstellungsbeispiele Wirkung von 100 % bei einer Aufwandmenge von 500g/ha: Bsp. I-a-2, I-a-16, I-b-1, I-b-2, I-b-5.

### Beispiel 4

### Tetranychus - test, (TETRUR Spritzbehandlung)

| | | |
|---|---|---|
| Lösungsmittel: | 78,0 | Gewichtsteile Aceton |
| | 1,5 | Gewichtsteile Dimethylformamid |
| Emulgator : | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration. Bohnenblattscheiben *(Phaseolus vulgaris),* die von allen Stadien der Gemeinen Spinnmilbe *(Tetranychus urticae)* befallen sind, werden mit einer Wirkstoffzubereitung der gewünschten Konzentration gespritzt.

Nach 5 Tagen wird die Wirkung in % bestimmt. Dabei bedeutet 100 %, dass alle Spinnmilben abgetötet wurden; 0 % bedeutet, dass keine Spinnmilben abgetötet wurden.

Bei diesem Test zeigen z. B. die folgenden Verbindungen der Herstellungsbeispiele Wirkung von 80 % bei einer Aufwandmenge von 100g/ha: Bsp. I-b-2

Bei diesem Test zeigen z. B. die folgenden Verbindungen der Herstellungsbeispiele Wirkung von 90 % bei einer Aufwandmenge von 100g/ha: Bsp. I-b-3

Bei diesem Test zeigen z. B. die folgenden Verbindungen der Herstellungsbeispiele Wirkung von 100 % bei einer Aufwandmenge von 100g/ha: Bsp. I-b-1

Bei diesem Test zeigen z. B. die folgenden Verbindungen der Herstellungsbeispiele Wirkung von 90 % bei einer Aufwandmenge von 500g/ha: Bsp. I-a-14, I-a-16.

### Beispiel 5

### Nilaparvata lugens -Test (NILALU hydroponische Behandlung)

| | | |
|---|---|---|
| Lösungsmittel: | 78,0 | Gewichtsteile Aceton |
| | 1,5 | Gewichtsteile Dimethylformamid |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Die Wirkstoffzubereitung wird in Wasser pipettiert. Die angegebene Konzentration bezieht sich auf die Wirkstoffmenge pro Volumeneinheit Wasser (mg/l = ppm), anschließend wird mit der Braunrückigen Reiszikade (*Nilaparvata lugens*) infiziert.

Nach 7 Tagen wird die Wirkung in % bestimmt. Dabei bedeutet 100 %, dass alle Reiszikaden abgetötet wurden; 0 % bedeutet, dass keine Reiszikaden abgetötet wurden.

Bei diesem Test zeigen z. B. die folgenden Verbindungen der Herstellungsbeispiele Wirkung von 100 % bei einer Aufwandmenge von 20 ppm: Bsp. I-a-13.

### Beispiel 6

### Meloidogyne incognita - test (MELGIN)

| | | |
|---|---|---|
| Lösungsmittel: | 80,0 | Gewichtsteile Aceton |

Zur Herstellung einer zweckmäßigen Wirkstoffzubereitung vermischt man 1 Gewichtsteil Wirkstoff mit den angegebenen Mengen Lösungsmittel und verdünnt das Konzentrat mit Wasser auf die gewünschte Konzentration.

Gefäße werden mit Sand, Wirkstofflösung, Meloidogyne incognita Ei- Larven- Suspension und Salatsamen gefüllt. Die Salatsamen keimen und die Pflänzchen entwickeln sich. An den Wurzeln entwickeln sich die Gallen.

Nach 14 Tagen wird die nematizide Wirkung anhand der Gallenbildung in % bestimmt. Dabei bedeutet 100 %, dass keine Gallen gefunden wurden; 0 % bedeutet, dass die Zahl der Gallen an den behandelten Pflanzen der unbehandelten Kontrolle entspricht.

Bei diesem Test zeigen z. B. die folgenden Verbindungen der Herstellungsbeispiele Wirkung von 100 % bei einer Aufwandmenge von 20 ppm: Bsp. I-a-2.

### Beispiel 7

### Boophilus microplus -Test (BOOPMI Injektion)

| | |
|---|---|
| Lösungsmittel: | Dimethylsulfoxid |

Zur Herstellung einer zweckmäßigen Wirkstoffzubereitung vermischt man 10 mg Wirkstoff mit 0,5 ml Lösungsmittel und verdünnt das Konzentrat mit Lösungsmittel auf die gewünschte Konzentration. Die Wirkstofflösung wird in das Abdomen *(Boophilus microplus)* injiziert, die Tiere werden in Schalen überführt und in einem klimatisierten Raum aufbewahrt. Die Wirkungskontrolle erfolgt auf Ablage fertiler Eier.

Nach 7 Tagen wird die Wirkung in % bestimmt. Dabei bedeutet 100%, dass keine Zecke fertile Eier gelegt hat.

Bei diesem Test zeigen z.B. die folgenden Verbindungen der Herstellungsbeispiele eine Wirkung von 80 % bei einer Aufwandmenge von 20µg/Tier: Bsp. I-a-14

Bei diesem Test zeigen z.B. die folgenden Verbindungen der Herstellungsbeispiele eine Wirkung von 95 % bei einer Aufwandmenge von 20µg /Tier: Bsp. I-b-1

Bei diesem Test zeigen z.B. die folgenden Verbindungen der Herstellungsbeispiele eine Wirkung von 100 % bei einer Aufwandmenge von 20µg /Tier: Bsp. I-b-3.

### Beispiel 8

### Lucilia cuprina-Test (LUCICU)

| | |
|---|---|
| Lösungsmittel: | Dimethylsulfoxid |

Zur Herstellung einer zweckmäßigen Wirkstoffzubereitung vermischt man 10 mg Wirkstoff mit 0,5 ml Dimethylsulfoxid und verdünnt das Konzentrat mit Wasser auf die gewünschte Konzentration. Gefäße, die Pferdefleisch enthalten, das mit der Wirkstoffzubereitung der gewünschten Konzentration behandelt wurde, werden mit ca 20 *Lucilia cuprina* Larven besetzt.

Nach 2 Tagen wird die Abtötung in % bestimmt. Dabei bedeutet 100 %, dass alle Larven abgetötet wurden; 0 % bedeutet, dass keine Larven abgetötet wurden.

Bei diesem Test zeigen z.B. die folgenden Verbindungen der Herstellungsbeispiele eine Wirkung von 90% bei einer Aufwandmenge von 100 ppm: Bsp. I-b-3.

### Beispiel 9a

### 1. Herbizide Wirkung im Vorauflauf

Samen von mono- bzw. dikotylen Unkraut- bzw. Kulturpflanzen werden in Holzfasertöpfen in sandiger Lehmerde ausgelegt und mit Erde abgedeckt. Die in Form von benetzbaren Pulvern (WP) formulierten Testverbindungen werden dann als wässrige Suspension mit einer Wasseraufwandmenge von umgerechnet 600 1/ha unter Zusatz von 0,2% Netzmittel in unterschiedlichen Dosierungen auf die Oberfläche der Abdeckerde appliziert.

Nach der Behandlung werden die Töpfe im Gewächshaus aufgestellt und unter guten Wachstumsbedingungen für die Testpflanzen gehalten. Die visuelle Bonitur der Auflaufschäden an den Versuchspflanzen erfolgt nach einer Versuchszeit von ca. 3 Wochen im Vergleich zu unbehandelten Kontrollen (herbizide Wirkung in Prozent: 100% Wirkung = Pflanzen sind abgestorben, 0 % Wirkung = wie Kontrollpflanzen).

Folgende Verbindungen zeigen neben den zuvor genannten Verbindungen im Vorauflauf mit 320 g/ha a.i. gegen Alopecurus myosuroides, Digitaria sanguinalis, Echinocloa crus-galli, Lolium multiflorum, Setaria viridis und Sorghum halapense eine Wirkung von 90 -100 %: I-a-2, I-a-10, I-a-11, I-a-12.

Folgende Verbindungen zeigen neben den zuvor genannten Verbindungen im Vorauflauf mit 320 g/ha a.i. gegen Alopecurus myosuroides, Echinocloa crus-galli, Lolium multiflorum und Setaria viridis eine Wirkung von 90 - 100 %: I-a-15, I-b-2, I-b-5.

### 2. Herbizide Wirkung im Nachauflauf

Samen von mono- bzw. dikotylen Unkraut- bzw. Kulturpflanzen werden in Holzfasertöpfen in sandigem Lehmboden ausgelegt, mit Erde abgedeckt und im Gewächshaus unter guten Wachstumsbedingungen angezogen. 2-3 Wochen nach der Aussaat werden die Versuchspflanzen im Einblattstadium behandelt. Die als Spritzpulver (WP) formulierten Testverbindungen werden in verschiedenen Dosierungen mit einer Wasseraufwandmenge von umgerechnet 600 1/ha unter Zusatz von 0,2% Netzmittel auf die grünen Pflanzenteile gesprüht. Nach ca. 3 Wochen Standzeit der Versuchspflanzen im Gewächshaus unter optimalen Wachstumsbedingungen wird die Wirkung der Präparate visuell im Vergleich zu unbehandelten Kontrollen bonitiert (herbizide Wirkung in Prozent: 100% Wirkung = Pflanzen sind abgestorben, 0 % Wirkung = wie Kontrollpflanzen).

Neben den zuvor genannten Verbindungen zeigen folgende Verbindungen im Nachauflauf mit 320 g/ha gegen Alopecurus myosuroides, Avena fatua, Digitaria sanguinalis, Echinocloa crus-galli, Lolium multiflorum, Setaria viridis und Sorghum halapense eine Wirkung von 90 -100 %: I-a-2, I-a-4, I-a-5, I-a-7, I-a-9, I-a-10, I-a-11, I-a-12, I-a-13, I-a-15.

Neben den zuvor genannten Verbindungen zeigen folgende Verbindungen im Nachauflauf mit 80 g/ha gegen Alopecurus myosuroides, Avena fatua, Echinocloa crus-galli, Lolium multiflorum und Setaria viridis eine Wirkung von 90 -100 %: I-a-1, I-b-2.

### Beispiel 9b: Vergleichende Daten

### 1. Herbizide Wirkung im Vorauflauf (Pre)

Samen von mono- bzw. dikotylen Unkraut- bzw. Kulturpflanzen werden in Holzfasertöpfen in sandiger Lehmerde ausgelegt und mit Erde abgedeckt. Die in Form von benetzbaren Pulvern (WP) oder Emulsionskonzentrate (EC) formulierten Testverbindungen werden dann als wässrige Suspension mit einer Wasseraufwandmenge von umgerechnet 600 -800 1/ha unter Zusatz von 0,1 - 0,2% Netzmittel in unterschiedlichen Dosierungen auf die Oberfläche der Abdeckerde appliziert.

Nach der Behandlung werden die Töpfe im Gewächshaus aufgestellt und unter guten Wachstumsbedingungen für die Testpflanzen gehalten. Die visuelle Bonitur der Auflaufschäden an den Versuchspflanzen erfolgt nach einer Versuchszeit von ca. 3 Wochen im Vergleich zu unbehandelten Kontrollen (herbizide Wirkung in Prozent: 100% Wirkung = Pflanzen sind abgestorben, 0 % Wirkung = wie Kontrollpflanzen).

Bei diesem Test zeigen z. B. die folgenden Verbindungen der Herstellungsbeispiele überlegene Wirksamkeit gegenüber dem Stand der Technik: siehe Tabelle

### 2. Herbizide Wirkung im Nachauflauf (Post)

Samen von mono- bzw. dikotylen Unkraut- bzw. Kulturpflanzen werden in Holzfasertöpfen in sandigem Lehmboden ausgelegt, mit Erde abgedeckt und im Gewächshaus unter guten Wachstumsbedingungen angezogen. 2-3 Wochen (Stadium 10-13 nach der BBCH Skala) nach der Aussaat werden die Versuchspflanzen bei einer Wuchshöhe von 5 - 10 cm behandelt. Die als Spritzpulver (WP) oder Emulsionskonzentrate (EC) formulierten Testverbindungen werden in verschiedenen Dosierungen mit einer Wasseraufwandmenge von umgerechnet 600 - 800 1/ha unter Zusatz von 0,1 - 0,2% Netzmittel auf die grünen Pflanzenteile gesprüht. Nach ca. 3 Wochen Standzeit der Versuchspflanzen im Gewächshaus unter optimalen Wachstumsbedingungen wird die Wirkung der Präparate visuell im Vergleich zu unbehandelten Kontrollen bonitiert (herbizide Wirkung in Prozent: 100% Wirkung = Pflanzen sind abgestorben, 0 % Wirkung = wie Kontrollpflanzen).

Bei diesem Test zeigen z. B. die folgenden Verbindungen der Herstellungsbeispiele überlegene Wirksamkeit gegenüber dem Stand der Technik: siehe Tabellen

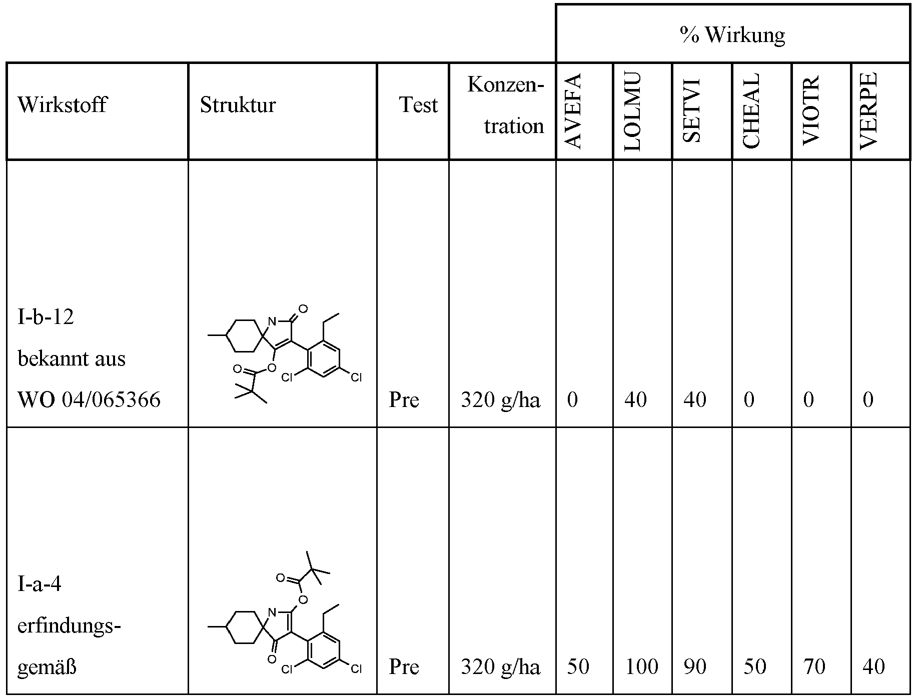

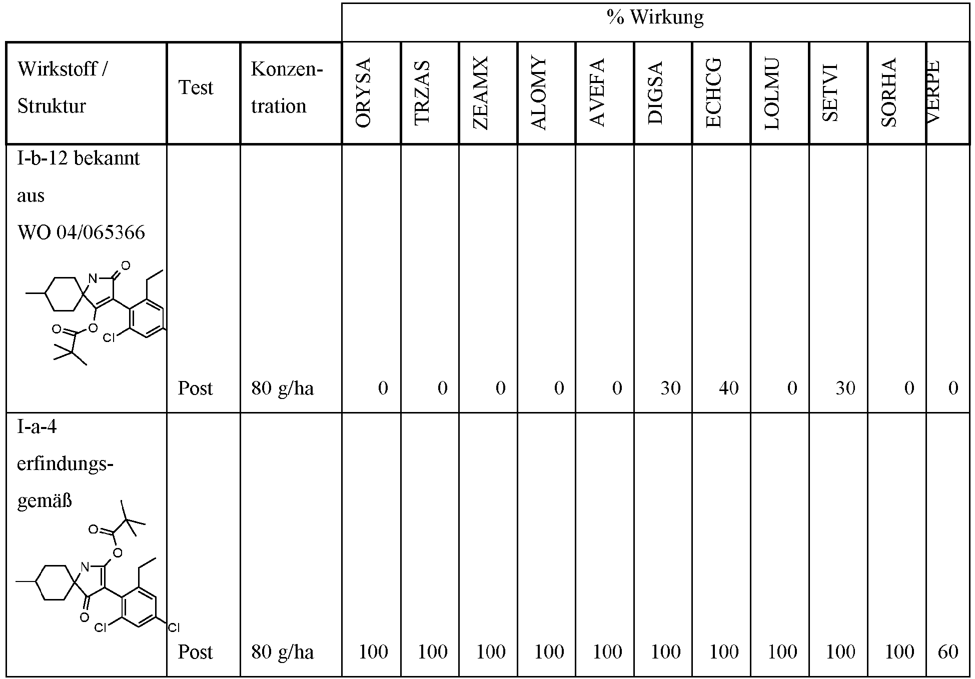

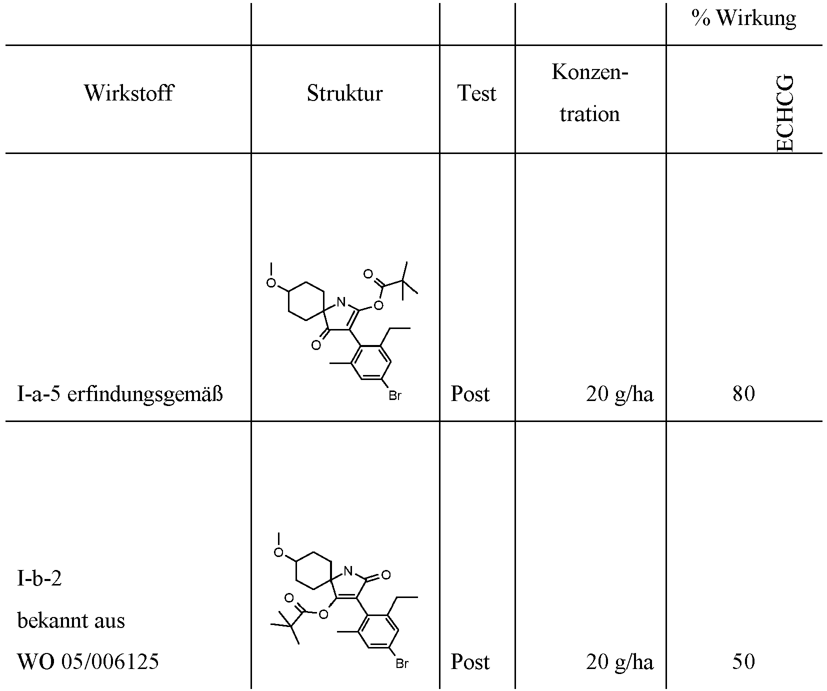

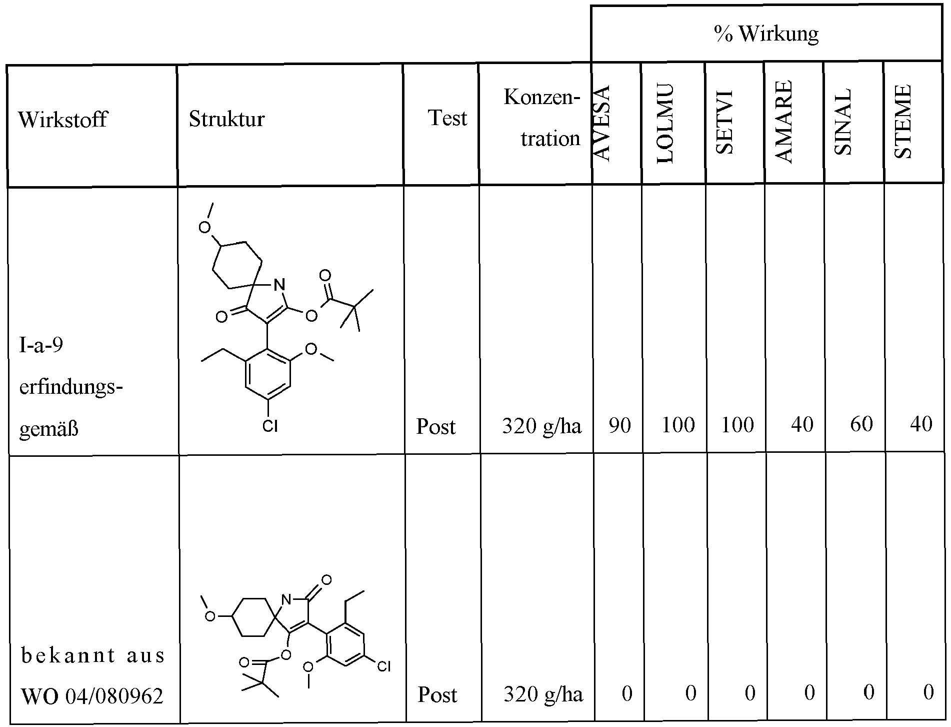

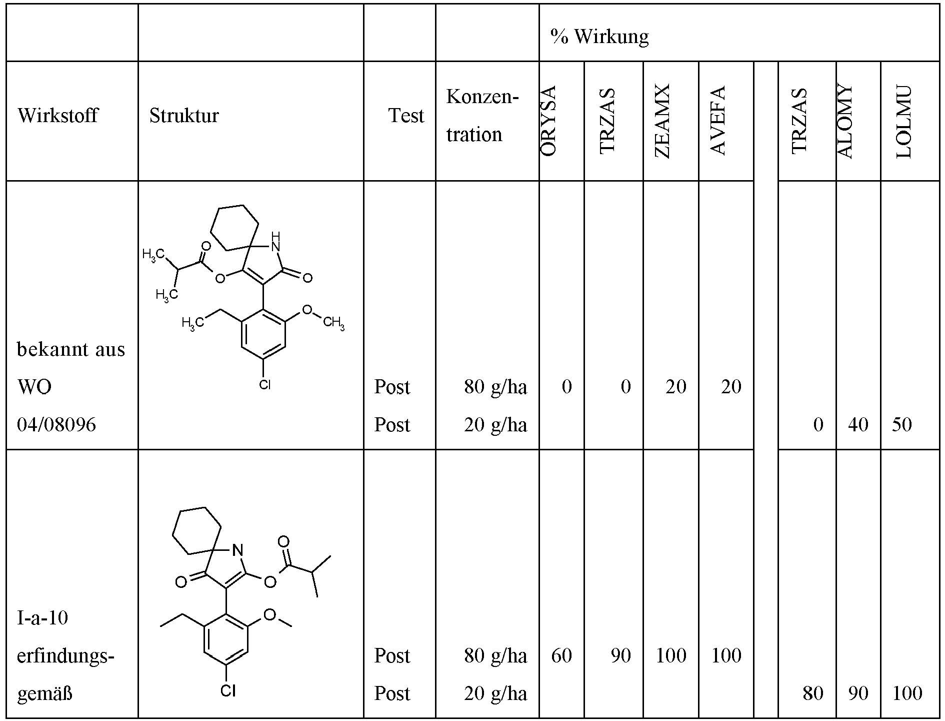

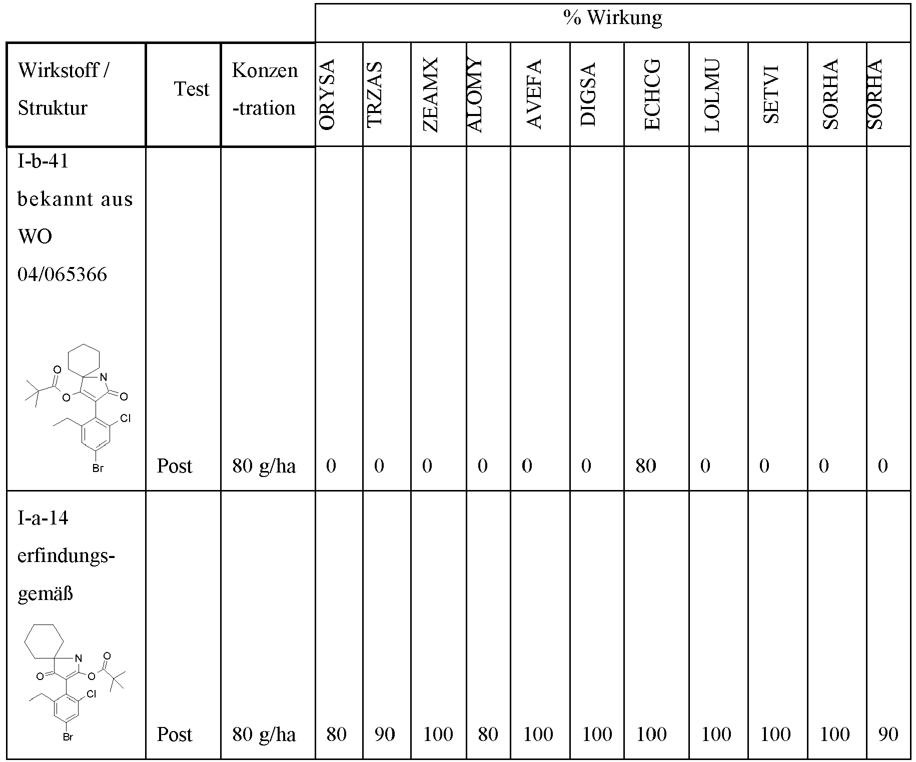

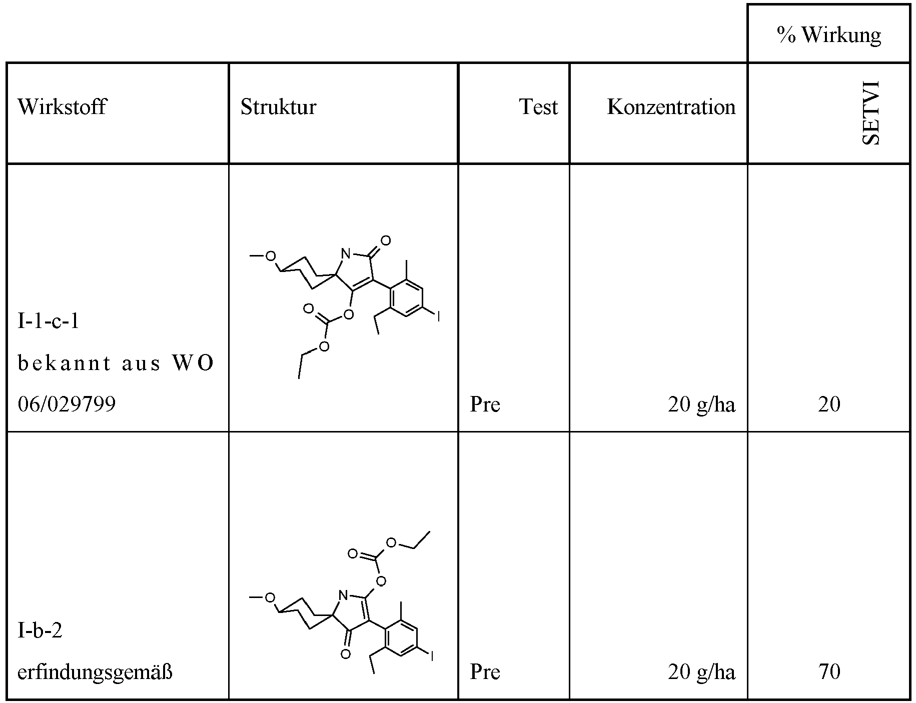

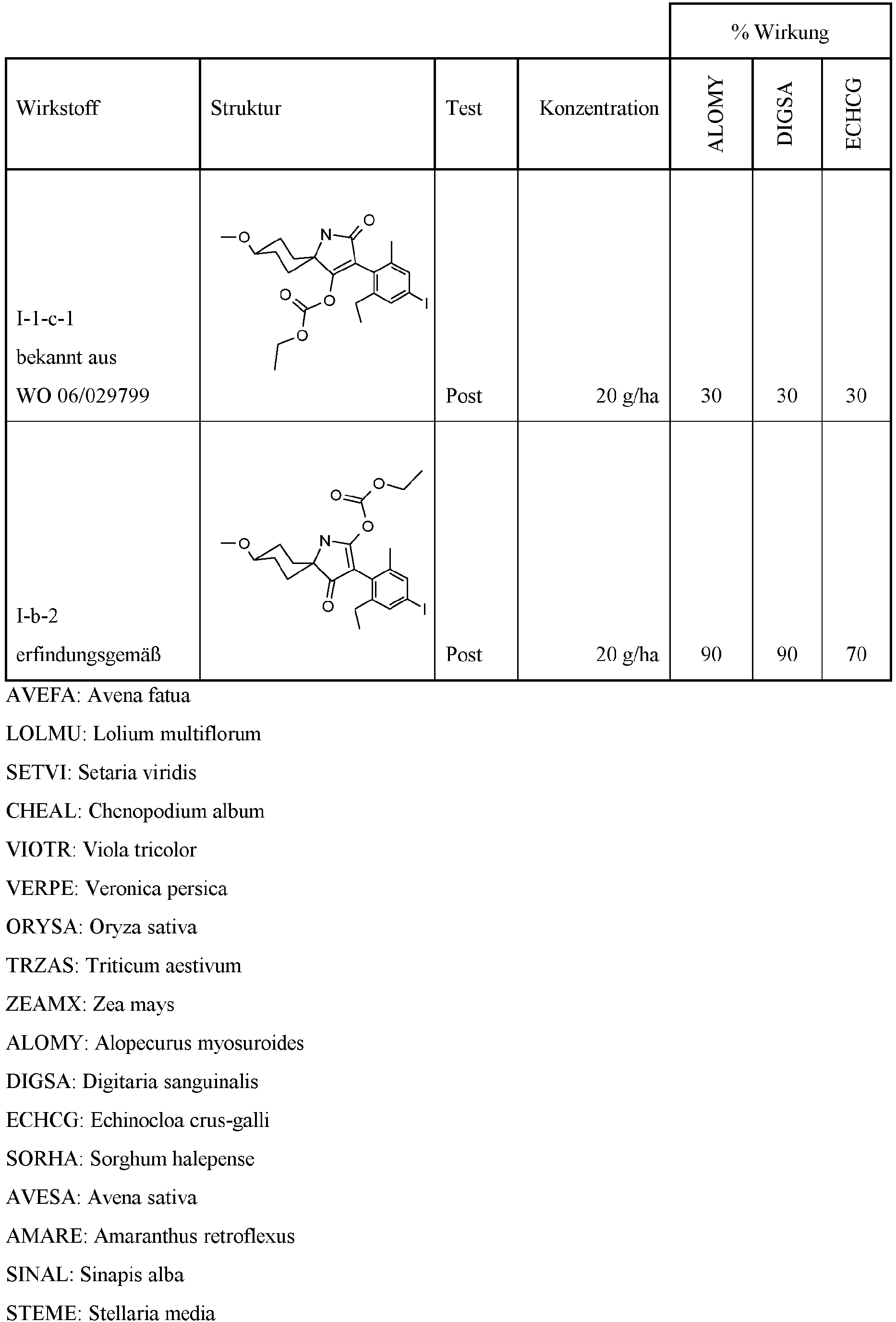

### Beispiel 10: Vergleichende Daten

### Phaedon-Test (PHAECO Spritzbehandlung)

| | |
|---|---|
| Lösungsmittel: | 78,0 Gewichtsteile Aceton |
| | 1,5 Gewichtsteile Dimethylformamid |
| Emulgator: | 0,5 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Chinakohlblattscheiben *(Brassica pekinensis)* werden mit einer Wirkstoffzubereitung der gewünschten Konzentration gespritzt und nach dem Abtrocknen mit Larven des Meerrettichblattkäfers *(Phaedon cochleariae)* besetzt.

Nach der gewünschten Zeit wird die Wirkung in % bestimmt. Dabei bedeutet 100 %, dass alle Käferlarven abgetötet wurden; 0 % bedeutet, dass keine Käferlarven abgetötet wurden. Bei diesem Test zeigen z. B. die folgenden Verbindungen der Herstellungsbeispiele überlegene Wirksamkeit gegenüber dem Stand der Technik: siehe Tabelle

### Myzus-Test (MYZUPE Spritzbehandlung)

| | |
|---|---|
| Lösungsmittel: | 78,0 Gewichtsteile Aceton |
| | 1,5 Gewichtsteile Dimethylformamid |
| Emulgator: | 0,5 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Chinakohlblattscheiben *(Brassica pekinensis),* die von allen Stadien der Grünen Pfirsichblattlaus (*Myzus persicae)* befallen sind, werden mit einer Wirkstoffzubereitung der gewünschten Konzentration gespritzt.
Nach der gewünschten Zeit wird die Wirkung in % bestimmt. Dabei bedeutet 100 %, dass alle Blattläuse abgetötet wurden; 0 % bedeutet, dass keine Blattläuse abgetötet wurden.
Bei diesem Test zeigen z. B. die folgenden Verbindungen der Herstellungsbeispiele überlegene Wirksamkeit gegenüber dem Stand der Technik: siehe Tabelle

### Tetranychus-Test ; OP-resistent (TETRUR Spritzbehandtung)

| | |
|---|---|
| Lösungsmittel: | 78,0 Gewichtsteile Aceton |
| | 1,5 Gewichtsteile Dimethylformamid |
| Emulgator: | 0,5 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Bohnenblattscheiben *(Phaseolus vulgaris),* die von allen Stadien der Gemeinen Spinnmilbe (*Tetranychus urticae)* befallen sind, werden mit einer Wirkstoffzubereitung der gewünschten Konzentration gespritzt.

Nach der gewünschten Zeit wird die Wirkung in % bestimmt. Dabei bedeutet 100 %, dass alle Spinnmilben abgetötet wurden; 0 % bedeutet, dass keine Spinnmilben abgetötet wurden.
Bei diesem Test zeigen z. B. die folgenden Verbindungen der Herstellungsbeispiele überlegene Wirksamkeit gegenüber dem Stand der Technik: siehe Tabelle

| Substanz | Struktur | Objekt | Konzentration | % Wirkung dat |
|---|---|---|---|---|
| | 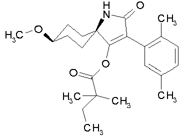 | MYZUPE | 20 g/ha | 0 5dat |
| bekannt aus WO 98/05638 und WO 04/007448 | | TETRUR | 500 g/ha | 0 5dat |
| I-a-16 erfindungsgemäß | 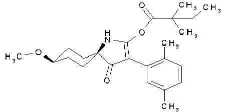 | MYZUPE | 20 g/ha | 100 5dat |
| | | TETRUR | 500 g/ha | 90 5dat |
| I-1-c-2 bekannt aus WO 07/048545 | 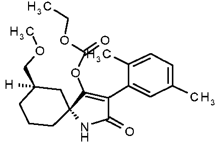 | PHAECO | 100 g/ha | 50 7dat |
| | | MYZUPE | 4 g/ha | 0 5dat |
| I-b-1 erfindungsgemäß | 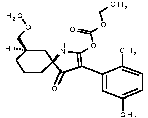 | PHAECO | 100 g/ha | 100 7dat |
| | | MYZUPE | 4 g/ha | 90 5dat |

### Spodoptera frugiperda-Test (SPODFR Spritzbehandlung)

| | |
|---|---|
| Lösungsmittel: | 78,0 Gewichtsteile Aceton |
| | 1,5 Gewichtsteile Dimethylformamid |
| Emulgator: | 0,5 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Maisblattscheiben (*Zea mays*) werden mit einer Wirkstoffzubereitung der gewünschten Konzentration gespritzt und nach dem Abtrocknen mit Raupen des Heerwurms (*Spodoptera frugiperda*) besetzt.

Nach der gewünschten Zeit wird die Wirkung in % bestimmt. Dabei bedeutet 100 %, dass alle Raupen abgetötet wurden; 0 % bedeutet, dass keine Raupe abgetötet wurde.
Bei diesem Test zeigen z. B. die folgenden Verbindungen der Herstellungsbeispiele überlegene Wirksamkeit gegenüber dem Stand der Technik: siehe Tabelle

### Tetranychus-Test ; OP-resistent (TETRUR Spritzbehandtung)

| | |
|---|---|
| Lösungsmittel: | 78,0 Gewichtsteile Aceton |
| | 1,5 Gewichtsteile Dimethylformamid |
| Emulgator: | 0,5 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Bohnenblattscheiben *(Phaseolus vulgaris),* die von allen Stadien der Gemeinen Spinnmilbe *(tetranychus urticae)* befallen sind, werden mit einer Wirkstoffzubereitung der gewünschten Konzentration gespritzt.

Nach der gewünschten Zeit wird die Wirkung in % bestimmt. Dabei bedeutet 100 %, dass alle Spinnmilben abgetötet wurden; 0 % bedeutet, dass keine Spinnmilben abgetötet wurden.
Bei diesem Test zeigen z. B. die folgenden Verbindungen der Herstellungsbeispiele überlegene Wirksamkeit gegenüber dem Stand der Technik: siehe Tabelle

### Liriomyza trifolii - Sprühtest (LIRITR)

| | |
|---|---|
| Lösungsmittel: | 52,5 Gewichtsteile Aceton |
| | 7 Gewichtsteile Dimethylformamid |
| Emulgator: | 0,5 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration. Bei erforderlicher Zugabe von Ammoniumsalzen oder/und Penetrationsförderern werden diese jeweils in einer Konzentration von 1000 ppm der Präparatelösung zugefügt.
Bohnenblattscheiben *(Phaseolus vulgaris),* die von Larven der Minierfliege *(Liriomyza trifolii)* befallen sind, werden mit einer Wirkstoffzubereitung der gewünschten Konzentration gespritzt.

Nach der gewünschten Zeit wird die Wirkung in % bestimmt. Dabei bedeutet 100 %, dass alle Minierfliegen abgetötet wurden; 0 % bedeutet, dass keine Minierfliege abgetötet wurde.

Bei diesem Test zeigt z. B. die folgende Verbindungen der Herstellungsbeispiele überlegene Wirksamkeit gegenüber dem Stand der Technik: siehe Tabelle

### Bemisia tabaci - Sprühtest (BEMITA)

| | |
|---|---|
| Lösungsmittel: | 52,5 GewichtsteileAceton |
| | 7 Gewichtsteile Dimethylformamid |
| Emulgator: | 0,5 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Baumwollblattscheiben *(Gossypium hirsutum),* die von Larven der Weißen Fliege *(Bemisia tabaci)* befallen sind, werden mit einer Wirkstoffzubereitung der gewünschten Konzentration gespritzt. Nach der gewünschten Zeit wird die Wirkung in % bestimmt. Dabei bedeutet 100 %, dass alle Weiße Fliegen abgetötet wurden; 0 % bedeutet, dass keine Weiße Fliege abgetötet wurde.

Bei diesem Test zeigt z. B. die folgende Verbindungen der Herstellungsbeispiele überlegene Wirksamkeit gegenüber dem Stand der Technik: siehe Tabelle

### Myzus persicae - Test (MYZUPE tau)

| | |
|---|---|
| Lösungsmittel: | 7 Gewichtsteile Dimethylformamid |
| Emulgator: | 2 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.
Kohlblätter *(Brassica oleracea),* die stark von der Grünen Pfirsichblattlaus *(Myzus persicae)* befallen sind, werden durch Tauchen in die Wirkstoffzubereitung der gewünschten Konzentration behandelt.
Nach der gewünschten Zeit wird die Abtötung in % bestimmt. Dabei bedeutet 100 %, dass alle Blattläuse abgetötet wurden; 0 % bedeutet, dass keine Blattläuse abgetötet wurden.
Bei diesem Test zeigt z. B. die folgende Verbindungen der Herstellungsbeispiele überlegene Wirksamkeit gegenüber dem Stand der Technik: siehe Tabelle

### Myzus persicae - Test; hydroponische Behandlung (MYZUPE sys)

| | |
|---|---|
| Lösungsmittel: | 7 Gewichtsteile Dimethylformamid |
| Emulgator: | 2 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.
Die Wirkstoffzubereitung wird mit Wasser gemischt. Die angegebene Konzentration bezieht sich auf die Wirkstoffmenge pro Volumeneinheit Wasser (mg/l= ppm). Man füllt das behandelte Wasser in Gefäße mit einer Erbsenpflanze *(Pisum sativum),* anschließend wird mit der Grünen Pfirsichblattlaus *(Myzus persicae)* infiziert.
Nach der gewünschten Zeit wird die Abtötung in % bestimmt. Dabei bedeutet 100 %, dass alle Blattläuse abgetötet wurden; 0 % bedeutet, dass keine Blattläuse abgetötet wurden.

Bei diesem Test zeigt z. B. die folgende Verbindungen der Herstellungsbeispiele überlegene Wirksamkeit gegenüber dem Stand der Technik: siehe Tabelle

| Substanz | Struktur | Objekt | Konzentration | % Wirkung dat |
|---|---|---|---|---|
| I-1-c-16 bekannt aus WO 07/048545 | 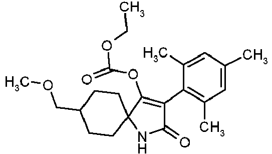 | TETRUR | 20 g/ha | 0 5dat |
| | | SPODFR | 500 g/ha | 67 7dat |
| | | BEMITA | 20 g/ha | 27 7dat |
| | | LIRITRI | 20 g/ha | 0 7dat |
| I-b-3 erfindungsgemäß | 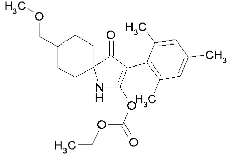 | TETRUR | 20 g/ha | 80 5dat |
| | | SPODFR | 500 g/ha | 100 7dat |
| | | BEMITA | 20 g/ha | 72 7dat |
| | | LIRITRI | 20 g/ha | 80 7dat |
| I-1-c-1 bekannt aus WO 06/029799 | 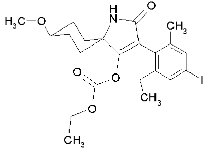 | MYZUPE tau | 20 ppm | 50 6dat |
| | | MYZUPE sys | 20 ppm | 60 6dat |
| I-b-2 erfindungsgemäß | 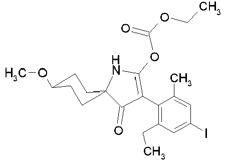 | MYZUPE tau | 20 ppm | 90 6dat |
| | | MYZUPE sys | 20 ppm | 99 6dat |

## Patentansprüche

1. Verbindungen der Formel (I) 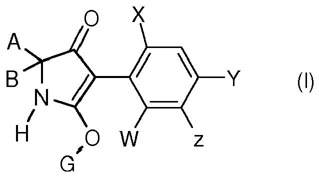 in welcher
W für Wasserstoff, Halogen, Alkyl, Alkenyl, Alkinyl, gegebenenfalls substituiertes Cycloalkyl, Alkoxy, Alkenyloxy, Halogenalkyl, Halogenalkoxy oder Cyano steht,
X für Halogen, Alkyl, Alkenyl, Alkinyl, gegebenenfalls substituiertes Cycloalkyl, Alkoxy, Alkenyloxy, Alkylthio, Alkylsulfinyl, Alkylsulfonyl, Halogenalkyl, Halogenalkoxy, Halogenalkenyloxy, Nitro oder Cyano steht,
Y und Z unabhängig voneinander für Wasserstoff, Alkyl, Alkenyl, Alkinyl, gegebenenfalls substituiertes Cycloalkyl, Alkoxy, Halogen, Halogenalkyl, Halogenalkoxy, Cyano, Nitro oder jeweils gegebenenfalls substituiertes Aryl oder Hetaryl stehen,
A für Wasserstoff, für jeweils gegebenenfalls durch Halogen substituiertes Alkyl, Alkenyl, Alkoxyalkyl, Alkylthioalkyl, gesättigtes oder ungesättigtes, gegebenenfalls substituiertes Cycloalkyl, in welchem gegebenenfalls mindestens ein Ringatom durch ein Heteroatom ersetzt ist, oder jeweils gegebenenfalls durch Halogen, Alkyl, Halogenalkyl, Alkoxy, Halogenalkoxy, Cyano oder Nitro substituiertes Aryl, Arylalkyl oder Hetaryl steht,
B für Wasserstoff, Alkyl oder Alkoxyalkyl steht, mit der Maßgabe, dass A und B nur dann jeweils für Methyl stehen dürfen, wenn, im Fall dass W, X und Y jeweils für Alkyl und Z für Wasserstoff stehen, W und X jeweils für Methyl stehen oder W und X jeweils für Ethyl stehen,
oder
A und B gemeinsam mit dem Kohlenstoffatom an das sie gebunden sind für einen gesättigten oder ungesättigten, gegebenenfalls mindestens ein Heteroatom enthaltenden unsubstituierten oder substituierten Cyclus stehen,
G für eine der Gruppen 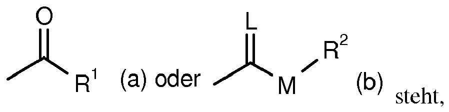 worin
L für Sauerstoff oder Schwefel steht,
M für Sauerstoff oder Schwefel steht,
R¹ für jeweils gegebenenfalls durch Halogen oder Cyano substituiertes Alkyl, Alkenyl, Alkoxyalkyl, Alkylthioalkyl oder Polyalkoxyalkyl oder für jeweils gegebenenfalls durch Halogen, Alkyl oder Alkoxy substituiertes Cycloalkyl oder Heterocyclyl oder für jeweils gegebenenfalls substituiertes Phenyl, Phenylalkyl, Hetaryl, Phenoxyalkyl oder Hetaryloxyalkyl steht,
R² für jeweils gegebenenfalls durch Halogen oder Cyano substituiertes Alkyl, Alkenyl, Alkoxyalkyl oder Polyalkoxyalkyl oder für jeweils gegebenenfalls substituiertes Cycloalkyl, Phenyl oder Benzyl steht.

2. Verbindungen der Formel (I) gemäß Anspruch 1, in welcher
W für Wasserstoff, Halogen, C₁-C₆-Alkyl, C₂-C₆-Alkenyl, C₂-C₆-Alkinyl, gegebenenfalls einfach bis zweifach durch C₁-C₂-Alkyl, C₁-C₂-Alkoxy, Fluor, Chlor, Trifluormethyl oder C₃-C₆-Cycloalkyl substituiertes C₃-C₆-Cycloalkyl, C₁-C₆-Alkoxy, C₁-C₄-Halogenalkyl, C₁-C₄-Halogenalkoxy oder Cyano steht,
X für Halogen, C₁-C₆-Alkyl, C₂-C₆-Alkenyl, C₂-C₆-Alkinyl, gegebenenfalls einfach bis zweifach durch C₁-C₂-Alkyl, C₁-C₂-Alkoxy, Fluor, Chlor, Trifluormethyl oder C₃-C₆-Cycloalkyl substituiertes C₃-C₆-Cycloalkyl, C₁-C₆-Halogenalkyl, C₁-C₆-Alkoxy, C₃-C₆-Alkenyloxy, C₁-C₆-Alkylthio, C₁-C₆-Alkylsulfinyl, C₁-C₆-Alkylsulfonyl, C₁-C₆-Halogenalkoxy, C₃-C₆-Halogenalkenyloxy, Nitro oder Cyano steht,
Y und Z unabhängig voneinander für Wasserstoff, Halogen, C₁-C₆-Alkyl, C₂-C₆-Alkenyl, C₂-C₆-Alkinyl, gegebenenfalls einfach bis zweifach durch C₁-C₂-Alkyl, C₁-C₂-Alkoxy, Fluor, Chlor, Trifluormethyl oder C₃-C₆-Cycloalkyl substituiertes C₃-C₆-Cycloalkyl, C₁-C₆-Alkoxy, C₁-C₆-Halogenalkyl, C₁-C₆-Halogenalkoxy, Cyano, C₂-C₆-Alkenyl, C₂-C₆-Alkinyl oder für einen der (Het)-arylreste stehen 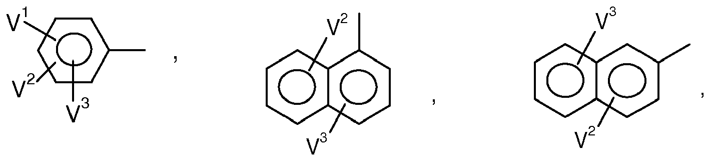 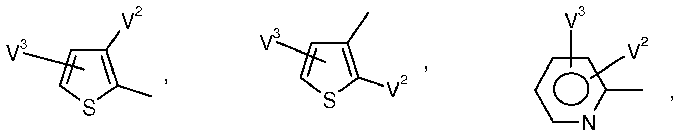 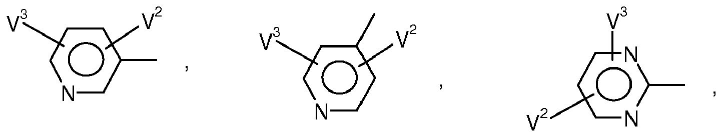 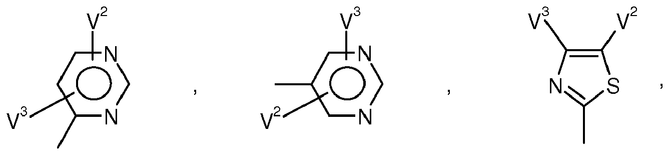 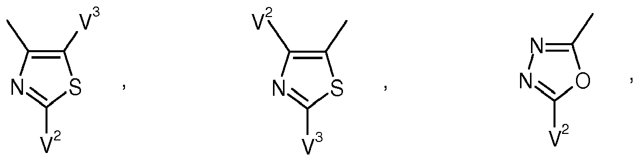 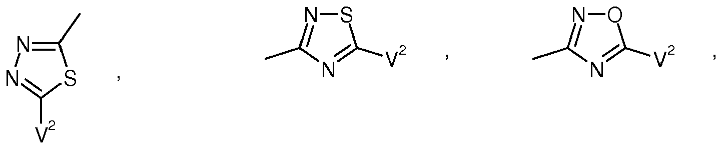 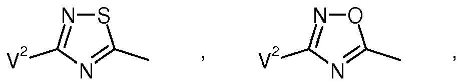 wobei im Falle von (Het)-aryl nur einer der Reste Y oder Z für (Het)-aryl stehen darf,
V¹ für Wasserstoff, Halogen, C₁-C₁₂-Alkyl, C₁-C₆-Alkoxy, C₁-C₆-Alkylthio, C₁-C₆-Alkylsulfinyl, C₁-C₆-Alkylsulfonyl, C₁-C₄-Halogenalkyl, C₁-C₄-Halogen-alkoxy, Nitro, Cyano oder jeweils gegebenenfalls einfach oder mehrfach durch Halogen, C₁-C₆-Alkyl, C₁-C₆-Alkoxy, C₁-C₄-Halogenalkyl, C₁-C₄-Halogenalkoxy, Nitro oder Cyano substituiertes Phenyl, Phenoxy, Phenoxy-C₁-C₄-alkyl, Phenyl-C₁-C₄-alkoxy, Phenylthio-C₁-C₄-alkyl oder Phenyl-C₁-C₄-alkylthio steht,
V² und V³ unabhängig voneinander für Wasserstoff, Halogen, C₁-C₆-Alkyl, C₁-C₆-Alkoxy, C₁₋C₄-Halogenalkyl oder C₁-C₄-Halogenalkoxy stehen,
A für Wasserstoff oder jeweils gegebenenfalls durch Halogen substituiertes C₁-C₁₂-Alkyl, C₃-C₈-Alkenyl, C₁-C₁₀-Alkoxy-C₁-C₈-alkyl, C₁-C₁₀-Alkylthio-C₁-C₆-alkyl, gegebenenfalls durch Halogen, C₁-C₆-Alkyl oder C₁-C₆-Alkoxy substituiertes C₃-C₈-Cycloalkyl, in welchem gegebenenfalls ein oder zwei nicht direkt benachbarte Ringglieder durch Sauerstoff und/oder Schwefel ersetzt sind oder für jeweils gegebenenfalls durch Halogen, C₁-C₆-Alkyl, C₁-C₆-Halogenalkyl, C₁-C₆-Alkoxy, C₁-C₆-Halogenalkoxy, Cyano oder Nitro substituiertes Phenyl, Naphthyl, Hetaryl mit 5 bis 6 Ringatomen, Phenyl-C₁-C₆-alkyl oder Naphthyl-C₁-C₆-alkyl steht,
B für Wasserstoff, C₁-C₁₂-Alkyl oder C₁-C₈-Alkoxy-C₁-C₆-alkyl steht, mit der Maßgabe, dass A und B nur dann jeweils für Methyl stehen dürfen, wenn, im Fall dass W, X und Y jeweils für C₁-C₆-Alkyl und Z für Wasserstoff stehen, W und X jeweils für Methyl stehen oder W und X jeweils für Ethyl stehen,
oder
A, B und das Kohlenstoffatom an das sie gebunden sind, für gesättigtes C₃-C₁₀-Cycloalkyl oder ungesättigtes C₅-C₁₀-Cycloalkyl, worin gegebenenfalls ein Ringglied durch Stickstoff, Sauerstoff oder Schwefel ersetzt ist und welche gegebenenfalls einfach oder zweifach durch C₁-C₈-Alkyl, C₁-C₈-Alkoxy, C₃-C₈-Alkenyloxy, C₁-C₆-Alkoxy-C₁-C₆-Alkyl, C₃-C₆-Cycloalkyl-C₁-C₂-alkoxy, C₃-C₁₀-Cycloalkyl, C₁-C₈-Halogenalkyl, C₂-C₆-Halogenalkoxy, C₁-C₆-Alkoxy-C₁-C₄-alkoxy substituiert sind, wobei die zuvor genannten Reste (ausgenommen Trifluormethyl) auch als N-Substituenten in Frage kommen, oder
A, B und das Kohlenstoffatom, an das sie gebunden sind, für C₃-C₆-Cycloalkyl, welches durch eine gegebenenfalls ein oder zwei nicht direkt benachbarte Sauerstoff- und/oder Schwefelatome enthaltende gegebenenfalls durch C₁-C₄-Alkyl substituierte Alkylendiyl- oder durch eine Alkylendioxyl- oder durch eine Alkylendithioyl-Gruppe substituiert ist, die mit dem Kohlenstoffatom, an das sie gebunden ist, einen weiteren fünf- bis achtgliedrigen Ring bildet oder
A, B und das Kohlenstoffatom, an das sie gebunden sind, für C₃-C₈-Cycloalkyl oder C₅-C₈-Cycloalkenyl stehen, in welchen zwei Substituenten gemeinsam mit den Kohlenstoffatomen, an die sie gebunden sind, für jeweils gegebenenfalls durch C₁-C₆-Alkyl, C₁-C₆-Alkoxy oder Halogen substituiertes C₂-C₆-Alkandiyl, C₂-C₆-Alkendiyl oder C₄-C₆-Alkandiendiyl stehen, worin gegebenenfalls eine Methylengruppe durch Sauerstoff oder Schwefel ersetzt ist,
G für eine der Gruppen 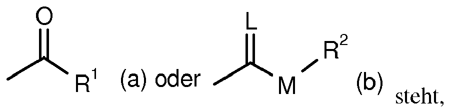
worin
Lfür Sauerstoff oder Schwefel steht,
M für Sauerstoff oder Schwefel steht,
R¹ für jeweils gegebenenfalls durch Halogen oder Cyano substituiertes C₁-C₂₀-Alkyl, C₂-C₂₀-Alkenyl, C₁-C₈-Alkoxy-C₁-C₈-alkyl, C₁-C₈-Alkylthio-C₁-C₈-alkyl oder Poly-C₁-C₈-alkoxy-C₁-C₈-alkyl oder für gegebenenfalls durch Halogen, C₁-C₆-Alkyl oder C₁-C₆-Alkoxy substituiertes C₃-C₈-Cycloalkyl, in welchem gegebenenfalls eine oder zwei nicht direkt benachbarte Methylengruppen durch Sauerstoff und/oder Schwefel ersetzt sind,
für gegebenenfalls durch Halogen, Cyano, Nitro, C₁-C₆-Alkyl, C₁-C₆-Alkoxy, C₁-C₆-Halogenalkyl, C₁-C₆-Halogenalkoxy, C₁-C₆-Alkylthio oder C₁-C₆-Alkylsulfonyl substituiertes Phenyl,
für gegebenenfalls durch Halogen, Nitro, Cyano, C₁-C₆-Alkyl, C₁-C₆-Alkoxy, C₁-C₆-Halogenalkyl oder C₁-C₆-Halogenalkoxy substituiertes Phenyl-C₁-C₆-alkyl,
für gegebenenfalls durch Halogen oder C₁-C₆-Alkyl substituiertes 5- oder 6-gliedriges Hetaryl mit ein oder zwei Heteroatomen aus der Reihe Sauerstoff, Schwefel und Stickstoff,
für gegebenenfalls durch Halogen oder C₁-C₆-Alkyl substituiertes Phenoxy-C₁-C₆-alkyl oder
für gegebenenfalls durch Halogen, Amino oder C₁-C₆-Alkyl substituiertes 5- oder 6-gliedriges Hetaryloxy-C₁-C₆-alkyl mit ein oder zwei Heteroatomen aus der Reihe Sauerstoff, Schwefel und Stickstoff steht,
R² für jeweils gegebenenfalls durch Halogen oder Cyano substituiertes C₁-C₂₀-Alkyl, C₂-C₂₀-Alkenyl, C₁-C₈-Alkoxy-C₂-C₈-alkyl oder Poly-C₁-C₈-alkoxy-C₂-C₈-alkyl, für gegebenenfalls durch Halogen, C₁-C₆-Alkyl oder C₁-C₆-Alkoxy substituiertes C₃-C₈-Cycloalkyl oder
für jeweils gegebenenfalls durch Halogen, Cyano, Nitro, C₁-C₆-Alkyl, C₁-C₆-Alkoxy, C₁-C₆-Halogenalkyl oder C₁-C₆-Halogenalkoxy substituiertes Phenyl oder Benzyl steht.

3. Verbindungen der Formel (I) gemäß Anspruch 1, in welcher
W für Wasserstoff, Chlor, Brom, C₁-C₄-Alkyl, C₂-C₄-Alkenyl, C₂-C₄-Alkinyl, gegebenenfalls einfach durch Methyl, Ethyl, Methoxy, Fluor, Chlor, Trifluormethyl oder Cyclopropyl substituiertes C₃-C₆-Cycloalkyl, C₁-C₄-Alkoxy, C₁-C₂-Halogenalkyl oder C₁-C₂-Halogenalkoxy steht,
X für Chlor, Brom, Iod, C₁-C₄-Alkyl, C₂-C₄-Alkenyl, C₂-C₄-Alkinyl, gegebenenfalls einfach durch Methyl, Ethyl, Methoxy, Fluor, Chlor, Trifluormethyl oder Cyclopropyl substituiertes C₃-C₆-Cycloalkyl, C₁-C₄-Alkoxy, C₁-C₄-Halogenalkyl, C₁-C₄-Halogenalkoxy oder Cyano steht,
Y und Z unabhängig voneinander für Wasserstoff, Fluor, Chlor, Brom, Iod, C₁-C₄-Alkyl, C₂-C₄-Alkenyl, C₂-C₄-Alkinyl, gegebenenfalls einfach durch Methyl, Ethyl, Methoxy, Fluor, Chlor, Trifluormethyl oder Cyclopropyl substituiertes C₃-C₆-Cycloalkyl, C₁-C₆-Alkoxy, C₁-C₄-Halogenalkyl, C₁-C₄-Halogenalkoxy, Cyano, C₂-C₄-Alkenyl, C₂-C₄-Alkinyl oder für einen der (Het)-arylreste steht, 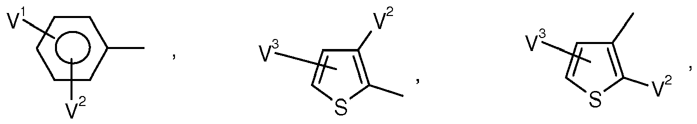 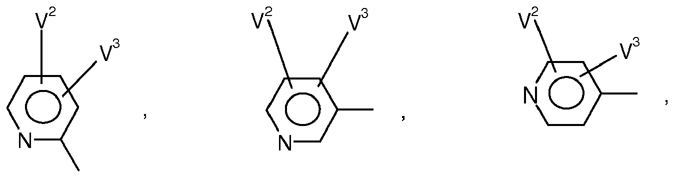 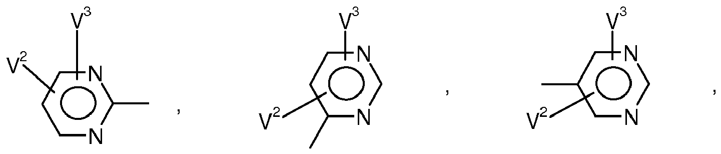 wobei im Falle von (Het)-aryl nur einer der Reste Y oder Z für (Het)-aryl stehen darf,
V¹ für Wasserstoff, Fluor, Chlor, Brom, C₁-C₆-Alkyl, C₁-C₄-Alkoxy, C₁-C₂-Halogenalkyl, C₁-C₂-Halogenalkoxy, Nitro, Cyano oder gegebenenfalls einfach bis zweifach durch Fluor, Chlor, Brom, C₁-C₄-Alkyl, C₁-C₄-Alkoxy, C₁-C₂-Halogenalkyl, C₁-C₂-Halogenalkoxy, Nitro oder Cyano substituiertes Phenyl steht,
V² und V³ unabhängig voneinander für Wasserstoff, Fluor, Chlor, Brom, C₁-C₄-Alkyl, C₁-C₄-Alkoxy, C₁-C₂-Halogenalkyl oder C₁-C₂-Halogenalkoxy stehen,
A für Wasserstoff, jeweils gegebenenfalls einfach bis dreifach durch Fluor oder Chlor substituiertes C₁-C₆-Alkyl, C₁-C₄-Alkoxy-C₁-C₂-alkyl, gegebenenfalls einfach bis zweifach durch C₁-C₂-Alkyl oder C₁-C₂-Alkoxy substituiertes und gegebenenfalls durch ein Sauerstoffatom unterbrochenes C₃-C₆-Cycloalkyl, jeweils gegebenenfalls einfach bis zweifach durch Fluor, Chlor, Brom, C₁-C₄-Alkyl, C₁-C₂-Halogenalkyl, C₁-C₄-Alkoxy, C₁-C₂-Halogenalkoxy, Cyano oder Nitro substituiertes Phenyl, Pyridyl oder Benzyl steht,
B für Wasserstoff, C₁-C₄-Alkyl oder C₁-C₂-Alkoxyl-C₁-C₂-alkyl steht, mit der Maßgabe, dass A und B nur dann jeweils für Methyl stehen dürfen, wenn, im Fall dass W, X und Y jeweils für C₁-C₄ -Alkyl und Z für Wasserstoff stehen, W und X jeweils für Methyl stehen oder W und X jeweils für Ethyl stehen,
oder
A, B und das Kohlenstoffatom an das sie gebunden sind, für gesättigtes oder ungesättigtes C₃-C₇-Cycloalkyl, worin gegebenenfalls ein Ringglied durch Stickstoff, Sauerstoff oder Schwefel ersetzt ist und welches gegebenenfalls einfach bis zweifach durch C₁-C₆-Alkyl, C₁-C₄-Alkoxy-C₁-C₂-alkyl, Trifluormethyl, C₁-C₆-Alkoxy, C₃-C₆-Alkenyloxy, Trifluorethoxy, C₁-C₃-Alkoxy-C₁-C₃-alkoxy oder C₃-C₆-Cycloalkylmethoxy substituiert ist, wobei die zuvor genannten Reste (ausgenommen Trifluormethyl) auch als N-Substituenten in Frage kommen, oder
A, B und das Kohlenstoffatom, an das sie gebunden sind, für C₅-C₆-Cycloalkyl, welches durch eine gegebenenfalls ein oder zwei nicht direkt benachbarte Sauerstoff- oder Schwefelatome enthaltende gegebenenfalls durch Methyl oder Ethyl substituierte Alkylendiyl- oder durch eine Alkylendioxyl- oder durch eine Alkylendithiol-Gruppe substituiert ist, die mit dem Kohlenstoffatom, an das sie gebunden ist, einen weiteren fünf- oder sechsgliedrigen Ring bildet, oder
A, B und das Kohlenstoffatom, an das sie gebunden sind, für C₃-C₆-Cycloalkyl oder C₅-C₆-Cycloalkenyl stehen, in welchen zwei Substituenten gemeinsam mit den Kohlenstoffatomen, an die sie gebunden sind, für jeweils gegebenenfalls durch C₁-C₂-Alkyl
oder C₁-C₂-Alkoxy substituiertes C₂-C₄-Alkandiyl, C₂-C₄-Alkendiyl oder Butadiendiyl stehen,
G für eine der Gruppen 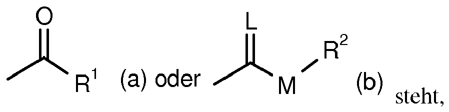
worin
Lfür Sauerstoff oder Schwefel steht,
M für Sauerstoff oder Schwefel steht,
R¹ für jeweils gegebenenfalls einfach bis dreifach durch Fluor oder Chlor substituiertes C₁-C₁₆-Alkyl, C₂-C₁₆-Alkenyl, C₁-C₆-Alkoxy-C₁-C₄-alkyl, C₁-C₆-Alkylthio-C₁-C₄-alkyl oder Poly-C₁-C₆-alkoxy-C₁-C₄-alkyl oder für gegebenenfalls einfach bis zweifach durch Fluor, Chlor, C₁-C₅-Alkyl oder C₁-C₅-Alkoxy substituiertes C₃-C₇-Cycloalkyl, in welchem gegebenenfalls eine oder zwei nicht direkt benachbarte Methylengruppen durch Sauerstoff und/oder Schwefel ersetzt sind,
für gegebenenfalls einfach bis dreifach durch Fluor, Chlor, Brom, Cyano, Nitro, C₁-C₄-Alkyl, C₁-C₄-Alkoxy, C₁-C₃-Halogenalkyl, C₁-C₃-Halogenalkoxy, C₁-C₄-Alkylthio oder C₁-C₄-Alkylsulfonyl substituiertes Phenyl,
für gegebenenfalls einfach bis zweifach durch Fluor, Chlor, Brom, C₁-C₄-Alkyl, C₁-C₄-Alkoxy, C₁-C₃-Halogenalkyl oder C₁-C₃-Halogenalkoxy substituiertes Phenyl-C₁-C₄-alkyl,
für jeweils gegebenenfalls einfach bis zweifach durch Fluor, Chlor, Brom oder C₁-C₄-Alkyl substituiertes Pyrazolyl, Thiazolyl, Pyridyl, Pyrimidyl, Furanyl oder Thienyl,
für gegebenenfalls einfach bis zweifach durch Fluor, Chlor, Brom oder C₁-C₄-Alkyl substituiertes Phenoxy-C₁-C₅-alkyl oder
für jeweils gegebenenfalls einfach bis zweifach durch Fluor, Chlor, Brom, Amino oder C₁-C₄-Alkyl substituiertes Pyridyloxy-C₁-C₅-alkyl, Pyrimidyloxy-C₁-C₅-alkyl oder Thiazolyloxy-C₁-C₅-alkyl steht,
R² für jeweils gegebenenfalls einfach bis dreifach durch Fluor oder Chlor substituiertes C₁-C₁₆-Alkyl, C₂-C₁₆-Alkenyl, C₁-C₆-Alkoxy-C₂-C₆-alkyl oder Poly-C₁-C₆-alkoxy-C₂-C₆-alkyl,
für gegebenenfalls einfach bis zweifach durch Fluor, Chlor, C₁-C₄-Alkyl oder C₁-C₄-Alkoxy substituiertes C₃-C₇-Cycloalkyl oder
für jeweils gegebenenfalls einfach bis dreifach durch Fluor, Chlor, Brom, Cyano, Nitro, C₁-C₄-Alkyl, C₁-C₃-Alkoxy, C₁-C₃-Halogenalkyl oder C₁-C₃-Halogenalkoxy substituiertes Phenyl oder Benzyl steht.

4. Verbindungen der Formel (I) gemäß Anspruch 1, in welcher
W für Wasserstoff, Chlor, Brom, Methyl, Ethyl, Propyl, Vinyl, Ethinyl, Propinyl, Cyclopropyl, Methoxy, Ethoxy oder Trifluormethyl steht,
X für Chlor, Brom, Methyl, Ethyl, Propyl, iso-Propyl, Vinyl, Ethinyl, Propinyl, Cyclopropyl, Methoxy, Ethoxy, Trifluormethyl, Difluormethoxy, Trifluormethoxy oder Cyano steht,
Y und Z unabhängig voneinander für Wasserstoff, Fluor, Chlor, Brom, Iod, Methyl, Ethyl, Vinyl, Ethinyl, Propinyl, Cyclopropyl, Methoxy, Trifluormethyl, Trifluormethoxy, Cyano oder einen Phenylrest stehen 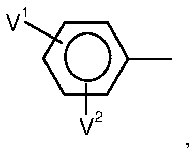 wobei im Falle von Phenyl nur einer der Reste Y oder Z für Phenyl stehen darf,
V¹ für Wasserstoff, Fluor oder Chlor steht,
V² für Wasserstoff, Fluor, Chlor, Methyl, Ethyl, n-Propyl, iso-Propyl, Methoxy, Ethoxy oder Trifluormethyl steht,
A für Wasserstoff, jeweils gegebenenfalls einfach bis dreifach durch Fluor substituiertes C₁-C₄-Alkyl oder C₁-C₂-Alkoxy-C₁-C₂-alkyl, für Cyclopropyl, Cyclopentyl oder Cyclohexyl,
B für Wasserstoff, Methyl oder Ethyl steht,
mit der Maßgabe, dass A und B nur dann jeweils für Methyl stehen dürfen, wenn, im Fall dass W, X und Y jeweils für Methyl oder Ethyl und Z für Wasserstoff stehen, W und X jeweils für Methyl stehen oder W und X jeweils für Ethyl stehen,
oder
A, B und das Kohlenstoffatom an das sie gebunden sind, stehen für gesättigtes C₅-C₆-Cycloalkyl, in welchem gegebenenfalls ein Ringglied durch Stickstoff, Sauerstoff oder Schwefel ersetzt ist und welches gegebenenfalls einfach oder zweifach durch Methyl, Ethyl, Methoxymethyl, Ethoxymethyl, Methoxyethyl, Ethoxyethyl, Trifluormethyl, Methoxy, Ethoxy, Propoxy, Butoxy, Methoxyethoxy, Ethoxyethoxy, Allyloxy, Trifluorethoxy oder Cyclopropylmethoxy substituiert ist, wobei die zuvor genannten Reste (ausgenommen Trifluormethyl) auch als N-Substituenten in Frage kommen, oder
A, B und das Kohlenstoffatom, an das sie gebunden sind, für C₆-Cycloalkyl, welches gegebenenfalls durch eine gegebenenfalls durch ein Sauerstoffatom unterbrochene Alkylidendiyl-Gruppe oder durch eine mit zwei nicht direkt benachbarten Sauerstoffatomen enthaltende Alkylendioxy-Gruppe substituiert ist, wobei ein weiter 5- oder 6-Ring gebildet wird (der gegebenenfalls einfach oder zweifach durch Methyl substituiert sein kann), oder A, B und das Kohlenstoffatom, an das sie gebunden sind, für C₅-C₆-Cycloalkyl oder C₅-C₆-Cycloalkenyl stehen, worin zwei Substituenten gemeinsam mit den Kohlenstoffatomen, an die sie gebunden sind, für C₂-C₄-Alkandiyl oder C₂-C₄-Alkendiyl oder Butadiendiyl stehen,
G für eine der Gruppen 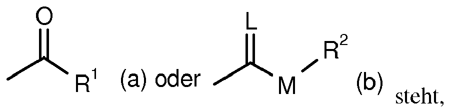
worin
L für Sauerstoff oder Schwefel steht,
M für Sauerstoff oder Schwefel steht,
R¹ für jeweils gegebenenfalls einfach bis dreifach durch Fluor oder Chlor substituiertes C₁-C₁₀-Alkyl, C₂-C₁₀-Alkenyl, C₁-C₄-Alkoxy-C₁-C₂-alkyl, C₁-C₄-Alkylthio-C₁-C₂-alkyl oder für gegebenenfalls einfach durch Fluor, Chlor, Methyl, Ethyl oder Methoxy substituiertes C₃-C₆-Cycloalkyl, für gegebenenfalls einfach bis zweifach durch Fluor, Chlor, Brom, Cyano, Nitro, Methyl, Ethyl, n-Propyl, i-Propyl, Methoxy, Ethoxy, Trifluormethyl oder Trifluormethoxy substituiertes Phenyl,
für jeweils gegebenenfalls einfach durch Chlor, Brom oder Methyl substituiertes Furanyl, Thienyl oder Pyridyl steht,
R² für jeweils gegebenenfalls einfach bis dreifach durch Fluor oder Chlor substituiertes C₁-C₁₀-Alkyl, C₂-C₁₀-Alkenyl oder C₁-C₄-Alkoxy-C₂-C₄-alkyl,
für Cyclopentyl oder Cyclohexyl
oder für jeweils gegebenenfalls einfach bis zweifach durch Fluor, Chlor, Cyano, Nitro, Methyl, Ethyl, Methoxy, Trifluormethyl oder Trifluormethoxy substituiertes Phenyl oder Benzyl steht.

5. Verbindungen der Formel (I) gemäß Anspruch 1, in welcher
W für Methyl, Ethyl oder Propyl steht,
X für Methyl, Ethyl, Chlor, Brom oder Methoxy steht,
Y für Methyl, Chlor, Jod oder Brom steht,
Z für Wasserstoff steht,
A für Methyl steht,
B für Methyl steht,
mit der Maßgabe, dass A und B nur dann jeweils für Methyl stehen dürfen, wenn, im Fall dass W, X und Y jeweils für Methyl oder Ethyl und Z für Wasserstoff stehen, Wund X jeweils für Methyl stehen oder Wund X jeweils für Ethyl stehen,
oder
A, B und das Kohlenstoff an das sie gebunden sind, für C₆-Cycloalkyl stehen, welches gegebenenfalls durch Methyl, Methoxy oder Methoxymethyl substituiert ist, wobei der Substituent in der 4-Stellung des Cyclus stehen kann,
G für eine der Gruppen 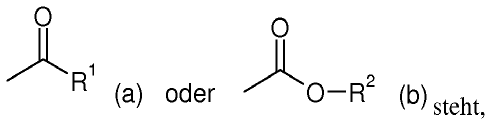
worin
R¹ für Methyl, Ethyl, Propyl, iso-Propyl, Butyl, iso-Butyl, sec-Butyl oder tert. Butyl steht,
R² für Methyl, Ethyl, Propyl, iso-Propyl, Butyl, iso-Butyl, sec.-Butyl oder tert.-Butyl steht.

6. Verbindungen der Formel (I) gemäß Anspruch 1, in welcher
W für Wasserstoff steht,
X für Methyl steht,
Y für Wasserstoff steht,
Z für Methyl steht,
A, B und das Kohlenstoff an das sie gebunden sind, für C₆-Cycloalkyl stehen, welches gegebenenfalls durch Methoxy oder Methoxymethyl substituiert ist, wobei der Substituent sowohl in der 3- als auch in der 4-Stellung des Cyclus stehen kann,
G für eine der Gruppen 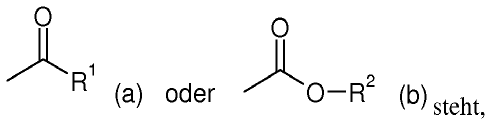
worin
R¹ für Methyl, Ethyl, Propyl, iso-Propyl, Butyl, iso-Butyl, sec-Butyl oder tert. Butyl steht,
R² für Methyl, Ethyl, Propyl, iso-Propyl, Butyl, iso-Butyl, sec.-Butyl oder tert.-Butyl steht.

7. Verfahren zur Herstellung von Verbindungen der Formel (I) gemäß Anspruch 1, **dadurch gekennzeichnet dass** zum Erhalt von
(A) Verbindungen der oben gezeigten Formel (I-a), in welchen R¹, A, B, W, X, Y und Z die oben angegebenen Bedeutungen haben, Verbindungen der Formel (II), in welcher A, B, W, X, Y und Z die oben angegebenen Bedeutungen haben, 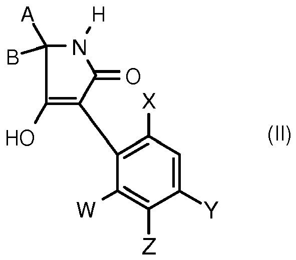 in welcher
A, B, W, X, Y und Z die oben angegebenen Bedeutungen haben,
α) mit Verbindungen der Formel (III) 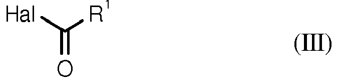 in welcher
R¹ die oben angegebene Bedeutung hat und
Hal für Halogen steht
oder
ß) mit Carbonsäureanhydriden der Formel (IV)
R¹-CO-O-CO-R¹ (IV)
in welcher
R¹ die oben angegebene Bedeutung hat,
gegebenenfalls in Gegenwart eines Verdünnungsmittels und gegebenenfalls in Gegenwart eines Säurebindemittels umsetzt;
(B) Verbindungen der oben gezeigten Formel (I-b), in welchen R², A, B, M, W, X, Y und Z die oben angegebenen Bedeutungen haben und L für Sauerstoff steht, Verbindungen der oben gezeigten Formel (II), in welcher A, B, W, X, Y und Z die oben angegebenen Bedeutungen haben,
mit Chlorameisensäureestern oder Chlorameisensäurethioestern der Formel (V)
R²-M-CO-Cl (V)
in welcher
R² und M die oben angegebenen Bedeutungen haben,
gegebenenfalls in Gegenwart eines Verdünnungsmittels und gegebenenfalls in Gegenwart eines Säurebindemittels umsetzt.

8. Schädlingsbekämpfungsmittel und/oder Herbizide, **gekennzeichnet durch** einen Gehalt an mindestens einer Verbindung der Formel (I) gemäß Anspruch 1.

9. Verfahren zur Bekämpfung von tierischen Schädlingen und/oder unerwünschtem Pflanzenbewuchs, **dadurch gekennzeichnet, dass** man Verbindungen der Formel (I) gemäß Anspruch 1 auf Schädlinge und/oder ihren Lebensraum einwirken lässt, ausgenommen sind Verfahren zur chirurgischen und therapeutischen Behandlung des menschlichen oder tierischen Körpers.

10. Verwendung von Verbindungen der Formel (I) gemäß Anspruch 1 zur Bekämpfung von tierischen Schädlingen und/oder unerwünschtem Pflanzenbewuchs, ausgenommen sind Verfahren zur chirurgischen und therapeutischen Behandlung des menschlichen oder tierischen Körpers.

11. Verfahren zur Herstellung von Schädlingsbekämpfungsmitteln und/oder Herbiziden, **dadurch gekennzeichnet, dass** man Verbindungen der Formel (I) gemäß Anspruch 1 mit Streckmitteln und/oder oberflächenaktiven Stoffen vermischt.

12. Verwendung von Verbindungen der Formel (I) gemäß Anspruch 1 zur Herstellung von Schädlingsbekämpfungsmitteln und/oder Herbiziden.

13. Mittel enthaltend einen wirksamen Gehalt an einer Wirkstoffkombination umfassend als Komponenten.
a') mindestens eine Verbindung der Formel (I) gemäß Anspruch 1, in welcher A, B, W, X, Y und Z die oben angegebene Bedeutung haben und
(b') zumindest eine die Kulturpflanzen-Verträglichkeit verbessernde Verbindung aus der folgenden Gruppe von Verbindungen: S1, S2, S3, S4, S5, S6, S7, S8, S9, S10, S11, S12, S13, S14, S15, S16:
S1) Verbindungen der Formel (S1), 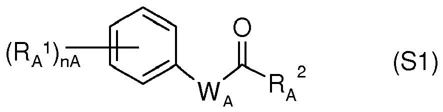 wobei die Symbole und Indizes folgende Bedeutungen haben:
n_{A} ist eine natürliche Zahl von 0 bis 5;
R_{A}¹ ist Halogen, (C₁-C₄)Alkyl, (C₁-C₄)Alkoxy, Nitro oder (C₁-C₄)Haloalkyl;
W_{A} ist ein unsubstituierter oder substituierter divalenter heterocyclischer Rest aus der Gruppe der teilungesättigten oder aromatischen Fünfring-Heterocyclen mit 1 bis 3 Heteroringatomen aus der Gruppe N und O, wobei mindestens ein N-Atom und höchstens ein O-Atom im Ring enthalten ist,
R_{A}² ist OR_{A}³, SR_{A}³ oder NR_{A}³R_{A}⁴ oder ein gesättigter oder ungesättigter 3- bis 7-gliedriger Heterocyclus mit mindestens einem N-Atom und bis zu 3 Heteroatomen, vorzugsweise aus der Gruppe O und S, der über das N-Atom mit der Carbonylgruppe in (S1) verbunden ist und unsubstituiert oder durch Reste aus der Gruppe (C₁-C₄)Alkyl, (C₁-C₄)Alkoxy oder gegebenenfalls substituiertes Phenyl substituiert ist, vorzugsweise ein Rest der Formel OR_{A}³, NHR_{A}⁴ oder N(CH₃)₂, insbesondere der Formel OR_{A}³;
R_{A}³ ist Wasserstoff oder ein unsubstituierter oder substituierter aliphatischer Kohlenwasserstoffrest, vorzugsweise mit insgesamt 1 bis 18 C-Atomen;
R_{A}⁴ ist Wasserstoff, (C₁-C₆)Alkyl, (C₁-C₆)Alkoxy oder substituiertes oder unsubstituiertes Phenyl;
S2) Chinolinderivate der Formel (S2), 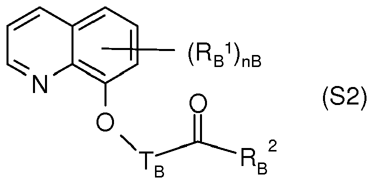 wobei die Symbole und Indizes folgende Bedeutungen haben:
R_{B}¹ ist Halogen, (C₁-C₄)Alkyl, (C₁-C₄)Alkoxy, Nitro oder (C₁-C₄)Haloalkyl;
n_{B} ist eine natürliche Zahl von 0 bis 5;
R_{B}² ist OR_{B}³, SR_{B}³ oder NR_{B}³R_{B}⁴ oder ein gesättigter
oder ungesättigter 3- bis 7-gliedriger Heterocyclus mit mindestens einem N-Atom und bis zu 3 Heteroatomen, der über das N-Atom mit der Carbonylgruppe in (S2) verbunden ist und unsubstituiert oder durch Reste aus der Gruppe (C₁-C₄)Alkyl, (C₁-C₄)Alkoxy oder gegebenenfalls substituiertes Phenyl substituiert ist;
R_{B}³ ist Wasserstoff oder ein unsubstituierter oder substituierter aliphatischer Kohlenwasserstoffrest;
R_{B}⁴ ist Wasserstoff, (C₁-C₆)Alkyl, (C₁-C₆)Alkoxy oder substituiertes oder unsubstituiertes Phenyl;
T_{B} ist eine (C₁, oder C₂)-Alkandiylkette, die unsubstituiert oder mit einem oder zwei (C₁-C₄)Alkylresten oder mit [(C₁-C₃)-Alkoxy]-carbonyl substituiert ist.
S3) Verbindungen der Formel (S3) 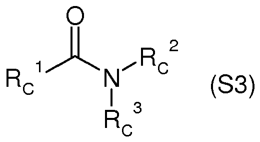 wobei die Symbole und Indizes folgende Bedeutungen haben:
R_{C}¹ ist (C₁-C₄)Alkyl, (C₁-C₄)Haloalkyl, (C₂-C₄)Alkenyl, (C₂-C₄)Haloalkenyl, (C₃-C₇)Cycloalkyl;
R_{C}², R_{C}³ sind gleich oder verschieden Wasserstoff, (C₁-C₄)Alkyl, (C₂-C₄)Alkenyl, (C₂-C₄)Alkinyl, (C₁-C₄)Haloalkyl, (C₂-C₄)Haloalkenyl, (C₁-C₄)Alkylcarbamoyl-(C₁-C₄)alkyl, (C₂-C₄)Alkenylcarbamoyl-(C₁-C₄)alkyl, (C₁-C₄)Alkoxy-(C₁-C₄)alkyl, Dioxolanyl-(C₁-C₄)alkyl, Thiazolyl, Furyl, Furylalkyl, Thienyl, Piperidyl, substituiertes oder unsubstituiertes Phenyl, oder R_{C}² und R_{C}³ bilden zusammen einen substituierten oder unsubstituierten heterocyclischen Ring.
S4) N-Acylsulfonamide der Formel (S4) und ihre Salze, 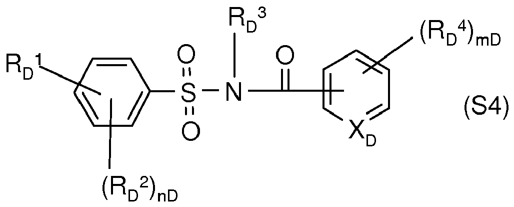 worin die Symbole und Indizes folgende Bedeutungen haben:
X_{D} ist CH oder N;
R_{D}¹ ist CO-NR_{D}⁵R_{D}⁶ oder NHCO-R_{D}⁷;
R_{D}² ist Halogen, (C₁-C₄)Haloalkyl, (C₁-C₄)Haloalkoxy, Nitro, (C₁-C₄)Alkyl, (C₁-C₄)Alkoxy, (C₁-C₄)Alkylsulfonyl, (C₁-C₄)Alkoxycarbonyl oder (C₁-C₄)Alkylcarbonyl;
R_{D}³ ist Wasserstoff, (C₁-C₄)Alkyl, (C₂-C₄)Alkenyl oder (C₂-C₄)Alkinyl;
R_{D}⁴ ist Halogen, Nitro, (C₁-C₄)Alkyl, (C₁-C₄)Haloalkyl, (C₁-C₄)Haloalkoxy, (C₃-C₆)Cycloalkyl, Phenyl, (C₁-C₄)Alkoxy, Cyano, (C₁-C₄)Alkylthio, (C₁-C₄)Alkylsulfinyl, (C₁-C₄)Alkylsulfonyl, (C₁-C₄)Alkoxycarbonyl oder (C₁-C₄)Alkylcarbonyl;
R_{D}⁵ ist Wasserstoff, (C₁-C₆)Alkyl, (C₃-C₆)Cycloalkyl, (C₂-C₆)Alkenyl, (C₂-C₆)Alkinyl, (C₅-C₆)Cycloalkenyl, Phenyl oder 3- bis 6-gliedriges Heterocyclyl enthaltend V_{D} Heteroatome aus der Gruppe Stickstoff, Sauerstoff und Schwefel, wobei die sieben letztgenannten Reste durch V_{D} Substituenten aus der Gruppe Halogen, (C₁-C₆)Alkoxy, (C₁-C₆)Haloalkoxy, (C₁-C₂)Alkylsulfinyl**,** (C₁-C₂)Alkylsulfonyl, (C₃-C₆)Cycloalkyl, (C₁-C₄)Alkoxycarbonyl, (C₁-C₄)Alkylcarbonyl und Phenyl und im Falle cyclischer Reste auch (C₁-C₄) Alkyl und (C₁-C₄)Haloalkyl substituiert sind;
R_{D}⁶ ist Wasserstoff, (C₁-C₆)Alkyl, (C₂-C₆)Alkenyl oder (C₂-C₆)Alkinyl, wobei die drei letztgenannten Reste durch V_{D} Reste aus der Gruppe Halogen, Hydroxy, (C₁-C₄)Alkyl, (C₁-C₄)Alkoxy und (C₁-C₄)Alkylthio substituiert sind, oder
R_{D}⁵ und R_{D}⁶ gemeinsam mit dem sie tragenden Stickstoffatom einen Pyrrolidinyl- oder Piperidinyl-Rest bilden;
R_{D}⁷ ist Wasserstoff, (C₁-C₄)Alkylamino, Di-(C₁-C₄)alkylamino, (C₁-C₆)Alkyl, (C₃-C₆)Cycloalkyl, wobei die 2 letztgenannten Reste durch V_{D} Substituenten aus der Gruppe Halogen, (C₁-C₄)Alkoxy, (C₁-C₆)Haloalkoxy und (C₁-C₄)Alkylthio und im Falle cyclischer Reste auch (C₁-C₄)Alkyl und (C₁-C₄)Haloalkyl substituiert sind;
n_{D} ist 0, 1 oder 2;
m_{D} ist 1 oder 2;
V_{D} ist 0,1,2 oder 3.
S5) Wirkstoffe aus der Klasse der Hydroxyaromaten und der aromatisch-aliphatischen Carbonsäurederivate (S5), z.B.
3,4,5-Triacetoxybenzoesäureethylester, 3,5-Dimethoxy-4-hydroxybenzoesäure, 3,5-Dihydroxybenzoesäure, 4-Hydroxysalicylsäure, 4-Fluorsalicyclsäure, 2-Hydroxy-zimtsäure, 1,2-Dihydro-2-oxo-6-trifluoromethylpyridin-3-carboxamid, 2,4-Dichlorzimtsäure, wie sie in der WO-A-2004/084631, WO-A-2005/015994, WO-A-2005/016001 beschrieben sind.
S6) Wirkstoffe aus der Klasse der 1,2-Dihydrochinoxalin-2-one (S6), z.B. 1-Methyl-3-(2-thienyl)-1,2-dihydrochinoxalin-2-on, 1-Methyl-3-(2-thienyl)-1,2-dihydrochinoxalin-2-thion, 1-(2-Aminoethyl)-3-(2-thienyl)-1,2-dihydro-chinoxalin-2-on-hydrochlorid, 1-[2-(Diethylamino)ethy1]-6,7-dimethyl-3-thiophen-2-ylchinoxalin-2(1H)-on, 1-(2-Methylsulfonylaminoethyl)-3-(2-thienyl)-1,2-dihydro-chinoxalin-2-on, wie sie in der WO-A-2005/112630 beschrieben sind.
S7) Verbindungen der Formel (S7),wie sie in der WO-A-1998/38856 beschrieben sind 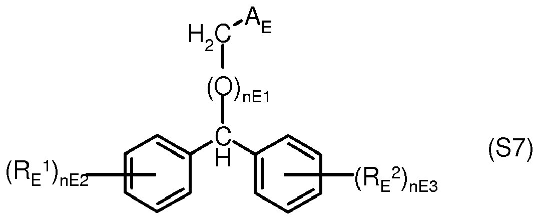 worin die Symbole und Indizes folgende Bedeutungen haben:
R_{E}¹, R_{E}² sind unabhängig voneinander Halogen, (C₁-C₄)Alkyl, (C₁-C₄)Alkoxy, (C₁-C₄)Haloalkyl, (C₁-C₄)Alkylamino, Di-(C₁-C₄)Alkylamino, Nitro;
A_{E} ist COOR_{E}³ oder COSR_{E}⁴
R_{E}³, R_{E}⁴ sind unabhängig voneinander Wasserstoff, (C₁-C₄)Alkyl, (C₂-C₆)Alkenyl, (C₂-C₄)Alkinyl, Cyanoalkyl, (C₁-C₄)Haloalkyl, Phenyl, Nitrophenyl, Benzyl, Halobenzyl, Pyridinylalkyl und Alkylammonium,
n_{E}¹ ist 0 oder 1
n_{E}², n_{E}³ sind unabhängig voneinander 0, 1 oder 2.
S8) Verbindungen der Formel (S8),wie sie in der WO-A-98/27049 beschrieben sind 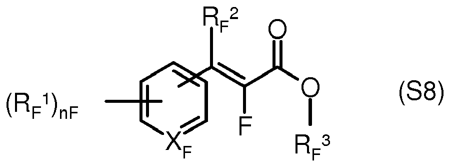 worin
X_{F} CH oder N,
n_{F} für den Fall, dass X_{F}=N ist, eine ganze Zahl von 0 bis 4 und für den Fall, dass X_{F}=CH ist, eine ganze Zahl von 0 bis 5 ,
R_{F}¹ Halogen, (C₁-C₄)Alkyl, (C₁-C₄)Haloalkyl, (C₁-C₄)Alkoxy, (C₁-C₄)Haloalkoxy, Nitro, (C₁-C₄)Alkylthio, (C₁-C₄)-Alkylsulfonyl, (C₁-C₄)Alkoxycarbonyl, ggf. substituiertes. Phenyl, ggf. substituiertes Phenoxy,
R_{F}² Wasserstoff oder (C₁-C₄)Alkyl
R_{F}³ Wasserstoff, (C₁-C₈)Alkyl, (C₂-C₄)Alkenyl, (C₂-C₄)Alkinyl, oder Aryl, wobei jeder der vorgenannten C-haltigen Reste unsubstituiert oder durch einen oder mehrere, gleiche oder verschiedene Reste aus der Gruppe, bestehend aus Halogen und Alkoxy substituiert ist;bedeuten, oder deren Salze.
S9) Wirkstoffe aus der Klasse der 3-(5-Tetrazolylcarbonyl)-2-chinolone (S9), z.B. 1,2-Dihydro-4-hydroxy-1-ethyl-3-(5-tetrazolylcarbonyl)-2-chinolon (CAS-Reg.Nr. 219479-18-2), 1,2-Dihydro-4-hydroxy-1-methyl-3-(5-tetrazolyl-carbonyl)-2-chinolon (CAS-Reg.Nr. 95855-00-8), wie sie in der WO-A-1999/000020 beschrieben sind.
S10) Verbindungen der Formeln (S10^{a}) oder (S10^{b})
wie sie in der WO-A-2007/023719 und WO-A-2007/023764 beschrieben sind 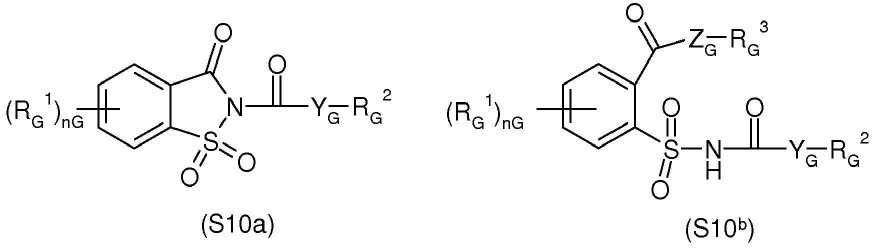 worin
R_{G}¹ Halogen, (C₁-C₄)Alkyl, Methoxy, Nitro, Cyano, CF₃, OCF₃
Y_{G,} Z_{G} unabhängig voneinander O oder S,
n_{G} eine ganze Zahl von 0 bis 4,
R_{G}² (C₁-C₁₆)Alkyl, (C₂-C₆)Alkenyl, (C₃-C₆)Cycloalkyl, Aryl; Benzyl, Halogenbenzyl,
R_{G}³ Wasserstoff oder (C₁-C₆)Alkyl bedeutet.
S11) Wirkstoffe vom Typ der Oxyimino-Verbindungen (S11), die als Saatbeizmittel bekannt sind, wie z. B. "Oxabetrinil" ((Z)-1,3-Dioxolan--ylmethoxyimino-(phenyl)acetonitril) (S11-1), das als Saatbeiz-Safener für Hirse gegen Schäden von Metolachlor bekannt ist,
"Fluxofenim" (1-(4-Chlorphenyl)-2,2,2-trifluor-1-ethanon-O-(1,3-dioxolan-2-ylmethyl)-oxim) (S11-2), das als Saatbeiz-Safener für Hirse gegen Schäden von Metolachlor bekannt ist, und
"Cyometrinil" oder "CGA-43089" ((Z)-Cyanomethoxyimino(phenyl)acetonitril) (S11-3), das als Saatbeiz-Safener für Hirse gegen Schäden von Metolachlor bekannt ist.
S12) Wirkstoffe aus der Klasse der Isothiochromanone (S12), wie z.B. Methyl-[(3-oxo-1H-2-benzothiopyran-4(3H)-yliden)methoxy]acetate (CAS-Reg.Nr. 205121-04-6) (S12-1) und verwandte Verbindungen aus WO-A-1998/13361.
S13) Eine oder mehrere Verbindungen aus Gruppe (S13):
"Naphthalic anhydrid" (1,8-Naphthalindicarbonsäureanhydrid) (S13-1), das als Saatbeiz-Safener für Mais gegen Schäden von Thiocarbamatherbiziden bekannt ist,
"Fenclorim" (4,6-Dichlor-2-phenylpyrimidin) (S13-2), das als Safener für Pretilachlor in gesätem Reis bekannt ist,
"Flurazole" (Benzyl-2-chlor-4-trifluormethyl-1,3-thiazol-5-carboxylat) (S13-3), das als Saatbeiz-Safener für Hirse gegen Schäden von Alachlor und Metolachlor bekannt ist,
"CL 304415" (CAS-Reg.Nr. 31541-57-8) (4-Carboxy-3,4-dihydro-2H-1-benzopyran-4-essigsäure) (S13-4) der Firma American Cyanamid, das als Safener für Mais gegen Schäden von Imidazolinonen bekannt ist,
"MG 191" (CAS-Reg.Nr. 96420-72-3) (2-Dichlormethyl-2-methyl-1,3-dioxolan) (S13-5) der Firma Nitrokemia, das als Safener für Mais bekannt ist,
"MG-838" (CAS-Reg.Nr. 133993-74-5) (2-propenyl 1-oxa-4-azaspiro[4.5]decane-4-carbodithioate) (S13-6) der Firma Nitrokemia,
"Disulfoton" (O,O-Diethyl S-2-ethylthioethyl phosphordithioat) (S13-7),
"Dietholate" (O,O-Diethyl-O-phenylphosphorotioat) (S13-8),
"Mephenate" (4-Chlorphenyl-methylcarbamat) (S13-9).
S14) Wirkstoffe, die neben einer herbiziden Wirkung gegen Schadpflanzen auch Safenerwirkung an Kulturpflanzen wie Reis aufweisen, wie z. B. "Dimepiperate" oder "MY-93" (S-1-Methyl-1-phenylethyl-piperidin-1-carbothioat), das als Safener für Reis gegen Schäden des Herbizids Molinate bekannt ist,
"Daimuron" oder "SK 23" (1-(1-Methyl-1-phenylethyl)-3-p-tolyl-harnstoff), das als Safener für Reis gegen Schäden des Herbizids Imazosulfuron bekannt ist,
"Cumyluron" = "JC-940" (3-(2-Chlorphenylmethyl)-1-(1-methyl-1-phenyl-ethyl)harnstoff, siehe JP-A-60087254), das als Safener für Reis gegen Schäden einiger Herbizide bekannt ist,
"Methoxyphenon" oder "NK 049" (3,3'-Dimethyl-4-methoxy-benzophenon), das als Safener für Reis gegen Schäden einiger Herbizide bekannt ist,
"CSB" (1-Brom-4-(chlormethylsulfonyl)benzol) von Kumiai, (CAS-Reg.Nr. 54091-06-4), das als Safener gegen Schäden einiger Herbizide in Reis bekannt ist.
S15) Verbindungen der Formel (S15) oder deren Tautomere 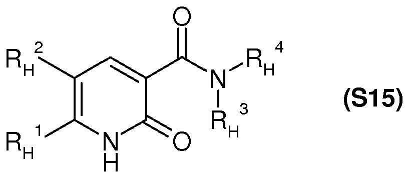 wie sie in der WO-A-2008/131861 und WO-A-2008/131860 beschrieben sind
worin
R_{H}¹ einen (C₁-C₆)Haloalkylrest bedeutet und
Rₕ² Wasserstoff oder Halogen bedeutet und
R_{H}³, R_{H}⁴ unabhängig voneinander Wasserstoff, (C₁-C₁₆)Alkyl, (C₂-C₁₆)Alkenyl oder (C₂-C₁₆)Alkinyl,
wobei jeder der letztgenannten 3 Reste unsubstituiert oder durch einen oder mehrere Reste aus der Gruppe Halogen, Hydroxy, Cyano, (C₁-C₄)Alkoxy, (C₁-C₄)Haloalkoxy, (C₁-C₄)Alkylthio, (C₁-C₄)Alkylamino, Di[(C₁-C₄)alkyl]-amino, [(C₁-C₄)Alkoxy]-carbonyl, [(C₁-C₄)Haloalkoxy]-carbonyl, (C₃-C₆)Cycloalkyl, das unsubstituiert oder substituiert ist, Phenyl, das unsubstituiert oder substituiert ist, und Heterocyclyl, das unsubstituiert oder substituiert ist, substituiert ist,
oder (C₃-C₆)Cycloalkyl, (C₄-C₆)Cycloalkenyl, (C₃-C₆)Cycloalkyl, das an einer Seite des Rings mit einem 4 bis 6-gliedrigen gesättigten oder ungesättigten carbocyclischen Ring kondensiert ist, oder (C₄-C₆)Cycloalkenyl, das an einer Seite des Rings mit einem 4 bis 6-gliedrigen gesättigten oder ungesättigten carbocyclischen Ring kondensiert ist,
wobei jeder der letztgenannten 4 Reste unsubstituiert oder durch einen oder mehrere Reste aus der Gruppe Halogen, Hydroxy, Cyano, (C₁-C₄)Alkyl, (C₁-C₄)Haloalkyl, (C₁-C₄)Alkoxy, (C₁-C₄)Haloalkoxy, (C₁-C₄)Alkylthio, (C₁-C₄)Alkylamino, Di[(C₁-C₄)alkyl]-amino, [(C₁-C₄)Alkoxy]-carbonyl, [(C₁-C₄)Haloalkoxy]-carbonyl, (C₃-C₆)Cycloalkyl, das unsubstituiert oder substituiert ist, Phenyl, das unsubstituiert oder substituiert ist, und Heterocyclyl, das unsubstituiert oder substituiert ist, substituiert ist,
bedeutet oder
R_{H}³ (C₁-C₄)-Alkoxy, (C₂-C₄)Alkenyloxy, (C₂-C₆)Alkinyloxy oder (C₂-C₄)Haloalkoxy bedeutet und
R_{H}⁴ Wasserstoff oder (C₁-C₄)-Alkyl bedeutet oder
R_{H}³ und R_{H}⁴ zusammen mit dem direkt gebundenen N-Atom einen vier- bis achtgliedrigen heterocyclischen Ring, der neben dem N-Atom auch weitere Heteroringatome, enthalten kann und der unsubstituiert oder durch einen oder mehrere Reste aus der Gruppe Halogen, Cyano, Nitro, (C₁-C₄)Alkyl, (C₁-C₄)Haloalkyl, (C₁-C₄)Alkoxy, (C₁-C₄)Haloalkoxy und (C₁-C₄)Alkylthio substituiert ist, bedeutet.
S16) Wirkstoffe, die vorrangig als Herbizide eingesetzt werden, jedoch auch Safenerwirkung auf Kulturpflanzen aufweisen, z.B.
(2,4-Dichlorphenoxy)essigsäure (2,4-D),
(4-Chlorphenoxy)essigsäure,
(R,S)-2-(4-Chlor-o-tolyloxy)propionsäure (Mecoprop),
4-(2,4-Dichlorphenoxy)buttersäure (2,4-DB),
(4-Chlor-o-tolyloxy)essigsäure (MCPA),
4-(4-Chlor-o-tolyloxy)buttersäure,
4-(4-Chlorphenoxy)buttersäure,
3,6-Dichlor-2-methoxybenzoesäure (Dicamba),
1-(Ethoxycarbonyl)ethyl-3,6-dichlor-2-methoxybenzoat (Lactidichlor-ethyl).

14. Verfahren zum Bekämpfen von unerwünschtem Pflanzenwuchs, **dadurch gekennzeichnet, dass** man ein Mittel gemäß Anspruch 13 auf die Pflanzen oder ihre Umgebung einwirken lässt.

15. Verwendung eines Mittels gemäß Anspruch 13 zum Bekämpfen von unerwünschten Pflanzenwuchs.

16. Verfahren zum Bekämpfen von unerwünschtem Pflanzenwuchs, **dadurch gekennzeichnet, dass** man eine Verbindung der Formel (I) gemäß Anspruch 1 und die die Kulturpflanzenverträglichkeit verbessernde Verbindung gemäß Anspruch 13 in zeitlich naher Abfolge getrennt auf die Pflanzen oder ihre Umgebung einwirken lässt.

## Claims

1. Compound of the formula (I) 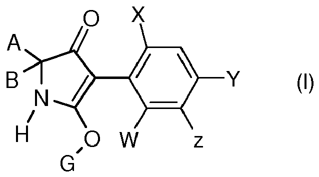 in which
W represents hydrogen, halogen, alkyl, alkenyl, alkynyl, optionally substituted cycloalkyl, alkoxy, alkenyloxy, haloalkyl, haloalkoxy or cyano,
X represents halogen, alkyl, alkenyl, alkynyl, optionally substituted cycloalkyl, alkoxy, alkenyloxy, alkylthio, alkylsulfinyl, alkylsulfonyl, haloalkyl, haloalkoxy, haloalkenyloxy, nitro or cyano,
Y and Z independently of one another represent hydrogen, alkyl, alkenyl, alkynyl, optionally substituted cycloalkyl, alkoxy, halogen, haloalkyl, haloalkoxy, cyano, nitro or in each case optionally substituted aryl or hetaryl,
A represents hydrogen, represents in each case optionally halogen-substituted alkyl, alkenyl, alkoxyalkyl, alkylthioalkyl, saturated or unsaturated, optionally substituted cycloalkyl, in which optionally at least one ring atom is replaced by a heteroatom, or in each case optionally halogen-, alkyl-, haloalkyl-, alkoxy-, haloalkoxy-, cyano- or nitro-substituted aryl, arylalkyl or hetaryl,
B represents hydrogen, alkyl or alkoxyalkyl,
with the proviso that A and B may each only represent methyl if, in the case where W, X and Y each represent alkyl and Z represents hydrogen, W and X each represent methyl or W and X each represent ethyl,
or
A and B together with the carbon atom to which they are attached represent a saturated or unsaturated substituted or unsubstituted cycle which optionally contains at least one heteroatom,
G represents one of the groups 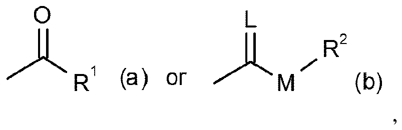 in which
L represents oxygen or sulfur,
M represents oxygen or sulfur,
R¹ represents in each case optionally halogen- or cyano-substituted alkyl, alkenyl, alkoxyalkyl, alkylthioalkyl or polyalkoxyalkyl or represents in each case optionally halogen-, alkyl- or alkoxy-substituted cycloalkyl or heterocyclyl or represents in each case optionally substituted phenyl, phenylalkyl, hetaryl, phenoxyalkyl or hetaryloxyalkyl,
R² represents in each case optionally halogen- or cyano-substituted alkyl, alkenyl, alkoxyalkyl or polyalkoxyalkyl or represents in each case optionally substituted cycloalkyl, phenyl or benzyl,

2. Compound of the formula (I) according to Claim 1 in which
W represents hydrogen, halogen, C₁-C₆-alkyl, C₂-C₆-alkenyl, C₂-C₆-alkynyl, C₃-C₆-cycloalkyl which is optionally mono- to disubstituted by C₁-C₂-alkyl, C₁-C₂-alkoxy, fluorine, chlorine, trifluoromethyl or C₃-C₆-cycloalkyl, C₁-C₆-alkoxy, C₁-C₄ -haloalkyl, C₁-C₄-haloalkoxy or cyano,
X represents halogen, C₁-C₆-alkyl, C₂-C₆-alkenyl, C₂-C₆-alkynyl, C₃-C₆-cycloalkyl which is optionally mono- to disubstituted by C₁-C₂-alkyl, C₁-C₂-alkoxy, fluorine, chlorine, trifluoromethyl or C₃-C₆-cycloalkyl, C₁-C₆-haloalkyl, C₁-C₆-alkoxy, C₃-C₆-alkenyloxy, C₁-C₆-alkylthio, C₁-C₆-alkylsulfinyl, C₁-C₆-alkylsulfonyl, C₁-C₆-haloalkoxy, C₃-C₆-haloalkenyloxy, nitro or cyano,
Y and Z independently of one another represent hydrogen, halogen, C₁-C₆-alkyl, C₂-C₆-alkenyl, C₂-C₆-alkynyl, C₃-C₆-cycloalkyl which is optionally mono- to disubstituted by C₁-C₂-alkyl, C₁-C₂-alkoxy, fluorine, chlorine, trifluoromethyl or C₃-C₆-cycloalkyl, C₁-C₆-alkoxy, C₁-C₆-haloalkyl, C₁-C₆-haloalkoxy, cyano, C₂-C₆-alkenyl, C₂-C₆-alkynyl or represent one of the (het)aryl radicals 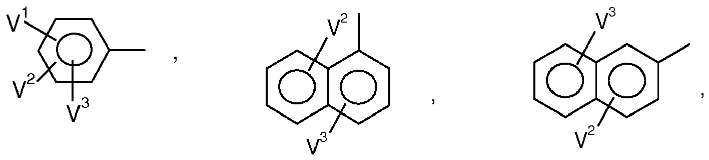 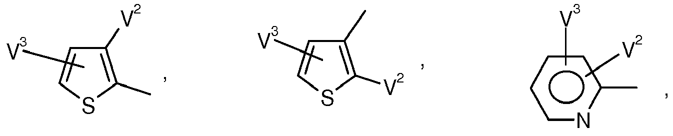 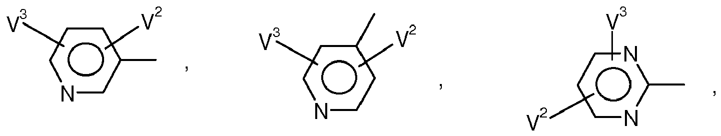 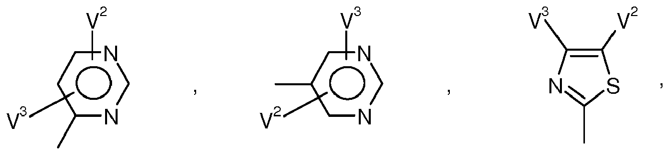 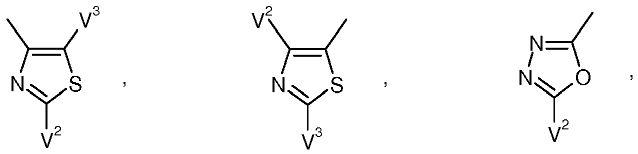 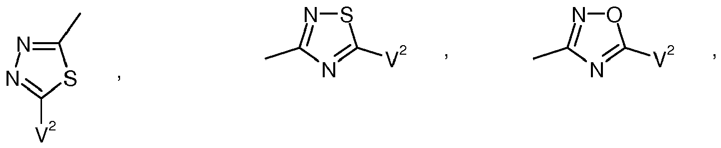 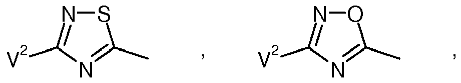 where in the case of (het)aryl only one of the radicals Y or Z may represent (het)aryl,
V¹ represents hydrogen, halogen, C₁-C₁₂-alkyl, C₁-C₆-alkoxy, C₁-C₆-alkylthio, C₁-C₆-alkylsulfinyl, C₁-C₆-alkylsulfonyl, C₁-C₄-haloalkyl, C₁-C₄-haloalkoxy, nitro, cyano or represents phenyl, phenoxy, phenoxy-C₁-C₄-alkyl, phenyl-C₁-C₄-alkoxy, phenylthio-C₁-C₄-alkyl or phenyl-C₁-C₄-alkylthio, each of which is optionally mono- or polysubstituted by halogen, C₁-C₆-alkyl, C₁-C₆-alkoxy, C₁-C₄-haloalkyl, C₁-C₄-haloalkoxy, nitro or cyano,
V² and V³ independently of one another represent hydrogen, halogen, C₁-C₆-alkyl, C₁-C₆-alkoxy, C₁-C₄-haloalkyl or C₁-C₄-haloalkoxy,
A represents hydrogen or in each each case optionally halogen-substituted C₁-C₁₂-alkyl, C₃-C₈-alkenyl, C₁-C₁₀-alkoxy-C₁-C₈-alkyl, C₁-C₁₀-alkylthio-C₁-C₆-alkyl, optionally halogen-, C₁-C₆-alkyl- or C₁-C₆-alkoxy-substituted C₃-C₈-cycloalkyl in which optionally one or two not directly adjacent ring members may be replaced by oxygen and/or sulfur or represents phenyl, naphthyl, hetaryl having 5 or 6 ring atoms, phenyl-C₁-C₆-alkyl or naphthyl-C₁-C₆-alkyl, each of which is optionally substituted by halogen, C₁-C₆-alkyl, C₁-C₆-haloalkyl, C₁-C₆-alkoxy, C₁-C₆-haloalkoxy, cyano or nitro,
B represents hydrogen, C₁-C₁₂-alkyl or C₁-C₈-alkoxy-C₁-C₆-alkyl,
with the proviso that A and B may each only represent methyl if, in the case where W, X and Y each represent C₁-C₆-alkyl and Z represents hydrogen, W and X each represent methyl or W and X each represent ethyl,
or
A, B and the carbon atom to which they are attached represent saturated C₃-C₁₀-cycloalkyl or unsaturated C₅-C₁₀-cycloalkyl in which optionally one ring member is replaced by nitrogen, oxygen or sulfur and which are optionally mono-or disubstituted by C₁-C₈-alkyl, C₁-C₈-alkoxy, C₃-C₈-alkenyloxy, C₁-C₆-alkoxy-C₁-C₆-alkyl, C₃-C₆-cycloalkyl-C₁-C₂-alkoxy, C₃-C₁₀-cycloalkyl, C₁-C₈-haloalkyl, C₂-C₆-haloalkoxy, C₁-C₆-alkoxy-C₁-C₄-alkoxy, where the radicals mentioned above (except for trifluoromethyl) are also suitable as N-substituents, or
A, B and the carbon atom to which they are attached represent C₃-C₆-cycloalkyl which is substituted by an alkylenediyl group, which optionally contains one or two not directly adjacent oxygen and/or sulfur atoms and is optionally substituted by C₁-C₄-alkyl, or by an alkylenedioxyl group or by an alkylenedithioyl group which, together with the carbon atom to which it is attached, forms a further five- to eight-membered ring or
A, B and the carbon atom to which they are attached represent C₃-C₈-cycloalkyl or C₅-C₈-cycloalkenyl in which two substituents together with the carbon atoms to which they are attached form C₂-C₆-alkanediyl, C₂-C₆-alkenediyl or C₄-C₆-alkanedienediyl, each of which is optionally substituted by C₁-C₆-alkyl, C₁-C₆-alkoxy or halogen and in which optionally one methylene group is replaced by oxygen or sulfur,
G represents one of the groups 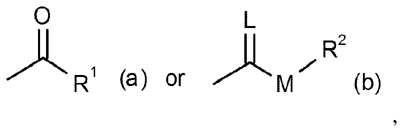 in which
L represents oxygen or sulfur,
M represents oxygen or sulfur,
R¹ represents in each case optionally halogen- or cyano-substituted C₁-C₂₀-alkyl, C₂-C₂₀-alkenyl, C₁-C₈-alkoxy-C₁-C₈-alkyl, C₁-C₈-alkylthio-C₁-C₈-alkyl or poly-C₁-C₈-alkoxy-C₁-C₈-alkyl or represents optionally halogen-, C₁-C₆-alkyl- or C₁-C₆-alkoxy-substituted C₃-C₈-cycloalkyl in which optionally one or two not directly adjacent methylene groups are replaced by oxygen and/or sulfur,
represents optionally halogen-, cyano-, nitro-, C₁-C₆-alkyl-, C₁-C₆-alkoxy-, C₁-C₆-haloalkyl-, C₁-C₆-haloalkoxy-, C₁-C₆-alkylthio- or C₁-C₆-alkylsulfonyl-substituted phenyl,
represents optionally halogen-, nitro-, cyano-, C₁-C₆-alkyl-, C₁-C₆-alkoxy-, C₁-C₆-haloalkyl- or C₁-C₆-haloalkoxy-substituted phenyl-C₁-C₆-alkyl,
represents optionally halogen- or C1-C6-alkyl-substituted 5- or 6-membered hetaryl having one or two heteroatoms from the group consisting of oxygen, sulfur and nitrogen,
represents optionally halogen- or C₁-C₆-alkyl-substituted phenoxy-C₁-C₆-alkyl or
represents optionally halogen- or C1-C6-alkyl-substituted 5- or 6-membered hetaryloxy-C1-C6-alkyl having one or two heteroatoms from the group consisting of oxygen, sulfur and nitrogen,
R² represents in each case optionally halogen- or cyano-substituted C₁-C₂₀-alkyl, C₂-C₂₀-alkenyl, C₁-C₈-alkoxy-C₂-C₈-alkyl or poly-C₁-C₈-alkoxy-C₂-C₈-alkyl,
represents optionally halogen-, C₁-C₆-alkyl- or C₁-C₆-alkoxy-substituted C₃-C₈-cycloalkyl or
represents phenyl or benzyl, each of which is optionally substituted by halogen, cyano, nitro, C₁-C₆-alkyl, C₁-C₆-alkoxy, C₁-C₆-haloalkyl or C₁-C₆-haloalkoxy.

3. Compound of the formula (I) according to Claim 1 in which
W represents hydrogen, chlorine, bromine, C₁-C₄-alkyl, C₂-C₄-alkenyl, C₂-C₄-alkynyl, C₃-C₆-cycloalkyl which is optionally monosubstituted by methyl, ethyl, methoxy, fluorine, chlorine, trifluoromethyl or cyclopropyl, C₁-C₄-alkoxy, C₁-C₂-haloalkyl or C₁-C₂-haloalkoxy,
X represents chlorine, bromine, iodine, C₁-C₄-alkyl, C₂-C₄-alkenyl, C₂-C₄-alkynyl, C₃-C₆-cycloalkyl which is optionally monosubstituted by methyl, ethyl, methoxy, fluorine, chlorine, trifluoromethyl or cyclopropyl, C₁-C₄-alkoxy, C₁₋C₄-haloalkyl, C₁-C₄-haloalkoxy or cyano,
Y and Z independently of one another represent hydrogen, fluorine, chlorine, bromine, iodine, C₁-C₄-alkyl, C₂-C₄-alkenyl, C₂-C₄-alkynyl, C₃-C₆-cycloalkyl which is optionally monosubstituted by methyl, ethyl, methoxy, fluorine, chlorine, trifluoromethyl or cyclopropyl, C₁-C₆-alhoxy, C₁-C₄-haloalkyl, C₁-C₄-haloalkoxy, cyano, C₂-C₄-alkenyl, C₂-C₄-alkynyl or represents one of the (het)aryl radicals, 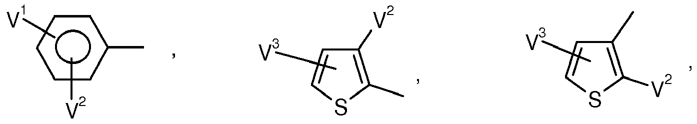 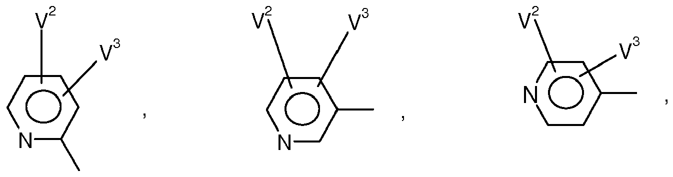 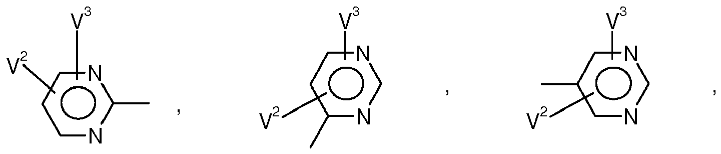 where in the case of (het)aryl only one of the radicals Y or Z may represent (het)aryl,
V¹ represents hydrogen, fluorine, chlorine, bromine, C₁-C₆-alkyl, C₁-C₄-alkoxy, C₁-C₂-haloalkyl, C₁-C₂-haloalkoxy, nitro, cyano or phenyl which is optionally mono- to disubstituted by fluorine, chlorine, bromine, C₁-C₄-alkyl, C₁-C₄-alhoxy, C₁-C₂-haloalkyl, C₁-C₂-haloalkoxy, nitro or cyano
V² and V³ independently of one another represent hydrogen, fluorine, chlorine, bromine, C₁-C₄-alkyl, C₁-C₄-alkoxy, C₁-C₂-haloalkyl or C₁-C₂-haloalkoxy,
A represents hydrogen, represents C₁-C₆-alkyl, C₁-C₄-alkoxy-C₁-C₂-alkyl, each of which is optionally mono- to trisubstituted by fluorine or chlorine, represents C₃-C₆-cycloalkyl which is optionally mono- to disubstituted by C₁-C₂-alkyl or C₁-C₂-alkoxy and optionally interrupted by an oxygen atom or represents phenyl, pyridyl or benzyl, each of which is optionally mono- to disubstituted by fluorine, chlorine, bromine, C₁-C₄-alkyl, C₁-C₂-haloalkyl, C₁-C₄-alkoxy, C₁-C₂-haloalkoxy, cyano or nitro,
B represents hydrogen, C₁-C₄-alkyl or C₁-C₂-alkoxy-C₁-C₂-alkyl,
with the proviso that A and B may each only represent methyl if, in the case where W, X and Y each represent C₁-C₄-alkyl and Z represents hydrogen, W and X each represent methyl or W and X each represent ethyl,
or
A, B and the carbon atom to which they are attached represent saturated or unsaturated C₃-C₇-cycloalkyl in which optionally one ring member is replaced by nitrogen, oxygen or sulfur and which is optionally mono- to disubstituted by C₁-C₆-alkyl, C₁-C₄-alkoxy-C₁-C₂-alkyl, trifluoromethyl, C₁-C₆-alkoxy, C₃-C₆-alkenyloxy, trifluoroethoxy, C₁-C₃-alkoxy-C₁-C₃-alkoxy or C₃-C₆-cycloalkylmethoxy, where the radicals mentioned above (except for trifluoromethyl) are also suitable as N-substituents, or
A, B and the carbon atom to which they are attached represent C₅-C₆-cycloalkyl which is substituted by an alkylendiyl group, which optionally contains one or two not directly adjacent oxygen or sulfur atoms and is optionally substituted by methyl or ethyl, or by an alkylenedioxyl group or by an alkylenedithioyl group which, together with the carbon atom to which it is attached, forms a further five- or six-membered ring, or
A, B and the carbon atom to which they are attached represent C₃-C₆-cycloalkyl or C₅-C₆-cycloalkenyl in which two substituents together with the carbon atoms to which they are attached form C₂-C₄-alkanediyl, C₂-C₄-alkenediyl or butadienediyl, each of which is optionally substituted by C₁-C₂-alkyl or C₁-C₂-alkoxy,
G represents one of the groups 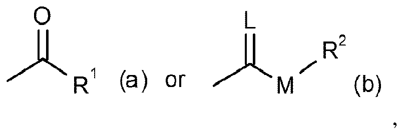 in which
L represents oxygen or sulfur,
M represents oxygen or sulfur,
R¹ represents C₁-C₁₆-alkyl, C₂-C₁₆₋alkenyl, C₁-C₆-alkoxy-C₁-C₄-alkyl, C₁-C₆-alkylthio-C₁-C₄-alkyl or poly-C₁-C₆-alkoxy-C₁-C₄-alkyl, each of which is optionally mono- to trisubstituted by fluorine or chlorine, or represents C₃-C₇-cycloalkyl which is optionally mono- to disubstituted by fluorine, chlorine, C₁-C₅-alkyl or C₁-C₅-alkoxy and in which optionally one or two not directly adjacent methylene groups are replaced by oxygen and/or sulfur,
represents phenyl which is optionally mono- to trisubstituted by fluorine, chlorine, bromine, cyano, nitro, C₁-C₄-alkyl, C₁-C₄-alkoxy, C₁-C₃-haloalkyl, C₁-C₃-haloalkoxy, C₁-C₄-alkylthio or C₁-C₄-alkylsulfonyl,
represents phenyl-C₁-C₄-alkyl, which is optionally mono- to disubstituted by fluorine, chlorine, bromine, C₁-C₄-alkyl, C₁-C₄-alkoxy, C₁-C₃-haloalkyl or C₁-C₃-haloalkoxy,
represents pyrazolyl, thiazolyl, pyridyl, pyrimidyl, furanyl or thienyl, each of which is optionally mono- or disubstituted by fluorine, chlorine, bromine or C₁-C₄-alkyl,
represents phenoxy-C₁-C₅-alkyl which is optionally mono- or disubstituted by fluorine, chlorine, bromine or C₁-C₄-alkyl,
represents pyridyloxy-C₁-C₅-alkyl, pyrimidyloxy-C₁-C₅-alkyl or thiazolyloxy-C₁-C₅-alkyl, each of which is optionally mono- or disubstituted by fluorine, chlorine, bromine, amino or C₁-C₄-alkyl,
R² represents C₁-C₁₆-alkyl, C₂-C₁₆-alkenyl, C₁-C₆-alkoxy-C₂-C₆-alkyl or poly-C₁-C₆-alkoxy-C₂-C₆-alkyl, each of which is optionally mono- to trisubstituted by fluorine or chlorine,
represents C₃-C₇-cycloalkyl which is optionally mono- to disubstituted by fluorine, chlorine, C₁-C₄-alkyl or C₁-C₄-alkoxy or
represents phenyl or benzyl, each of which is optionally mono- to trisubstituted by fluorine, chlorine, bromine, cyano, nitro, C₁-C₄-alkyl, C₁-C₃-alkoxy, C₁-C₃-haloalkyl or C₁-C₃-haloalkoxy.

4. Compound of the formula (I) according to Claim 1 in which
W represents hydrogen, chlorine, bromine, methyl, ethyl, propyl, vinyl, ethynyl, propynyl, cyclopropyl, methoxy, ethoxy or trifluoromethyl,
X represents chlorine, bromine, methyl, ethyl, propyl, isopropyl, vinyl, ethynyl, propynyl, cyclopropyl, methoxy, ethoxy, trifluoromethyl, difluoromethoxy, trifluoromethoxy or cyano,
Y and Z independently of one another represent hydrogen, fluorine, chlorine, bromine, iodine, methyl, ethyl, vinyl, ethynyl, propynyl, cyclopropyl, methoxy, trifluoromethyl, trifluoromethoxy, cyano or a phenyl radical, 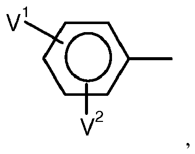 where in the case of phenyl only one of the radicals Y or Z may represent phenyl,
V¹ represents hydrogen, fluorine or chlorine,
V² represents hydrogen, fluorine, chlorine, methyl, ethyl, n-propyl, isopropyl, methoxy, ethoxy or trifluoromethyl,
A represents hydrogen, represents C₁-C₄-alkyl or C₁-C₂-alkoxy-C₁-C₂-alkyl, each of which is optionally mono- to trisubstituted by fluorine, represents cyclopropyl, cyclopentyl or cyclohexyl,
B represents hydrogen, methyl or ethyl,
with the proviso that A and B may each only represent methyl if, in the case where W, X and Y each represent methyl or ethyl and Z represents hydrogen, W and X each represent methyl or W and X each represent ethyl,
or
A, B and the carbon atom to which they are attached represent saturated C₅-C₆-cycloalkyl in which optionally one ring member is replaced by nitrogen, oxygen or sulfur and which is optionally mono- or disubstituted by methyl, ethyl, methoxymethyl, ethoxymethyl, methoxyethyl, ethoxyethyl, trifluoromethyl, methoxy, ethoxy, propoxy, butoxy, methoxyethoxy, ethoxyethoxy, allyloxy, trifluoroethoxy or cyclopropylmethoxy, where the radicals mentioned above (except for trifluoromethyl) are also suitable as N-substituents, or
A, B and the carbon atom to which they are attached represent C₆-cycloalkyl which is optionally substituted by an alkylidenediyl group, which is optionally interrupted by an oxygen atom, or by an alkylenedioxy group, which contains two not directly adjacent oxygen atoms, thus forming a further 5- or 6-membered ring (which may optionally be mono- or disubstituted by methyl), or
A, B and the carbon atom to which they are attached represent C₅-C₆-cycloalkyl or C₅-C₆-cycloalkenyl in which two substituents together with the carbon atoms to which they are attached represent C₂-C₄-alkanediyl or C₂-C₄-alkenediyl or butadienediyl,
G represents one of the groups 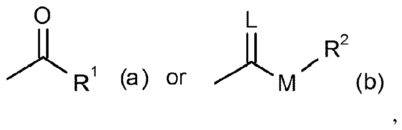 in which
L represents oxygen or sulfur,
M represents oxygen or sulfur,
R1 represents C₁-C₁₀-alkyl, C₂-C₁₀-alkenyl, C₁-C₄-alkoxy-C₁-C₂-alkyl, C₁-C₄-alkylthio-C₁-C₂-alkyl, each of which is optionally mono- to trisubstituted by fluorine or chlorine, or represents C₃-C₆-cycloalkyl which is optionally substituted by fluorine, chlorine, methyl, ethyl or methoxy,
represents phenyl which is optionally mono- to disubstituted by fluorine, chlorine, bromine, cyano, nitro, methyl, ethyl, n-propyl, isopropyl, methoxy, ethoxy, trifluoromethyl or trifluoromethoxy,
represents furanyl, thienyl or pyridyl, each of which is optionally monosubstituted by chlorine, bromine or methyl,
R² represents C₁-C₁₀-alkyl, C₂-C₁₀-alkenyl or C₁-C₄-alkoxy-C₂-C₄-alkyl, each of which is optionally mono- to trisubstituted by fluorine or chlorine,
represents cyclopentyl or cyclohexyl
or represents phenyl or benzyl, each of which is optionally mono- to disubstituted by fluorine, chlorine, cyano, nitro, methyl, ethyl, methoxy, trifluoromethyl or trifluoromethoxy.

5. Compound of the formula (I) according to Claim 1 in which
W represents methyl, ethyl or propyl,
X represents methyl, ethyl, chlorine, bromine or methoxy,
Y represents methyl, chlorine, iodine or bromine,
Z represents hydrogen,
A represents methyl,
B represents methyl,
with the proviso that A and B may each only represent methyl if, in the case where W, X and Y each represent methyl or ethyl and Z represents hydrogen, W and X each represent methyl or W and X each represent ethyl,
or
A, B and the carbon to which they are attached represent C₆-cycloalkyl which is optionally substituted by methyl, methoxy or methoxymethyl, where the substituent may be located in the 4-position of the cycle,
G represents one of the groups 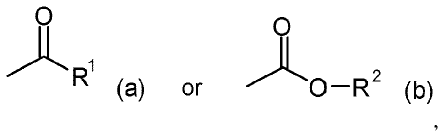 in which
R¹ represents methyl, ethyl, propyl, isopropyl, butyl, isobutyl, sec-butyl or tert-butyl,
R² represents methyl, ethyl, propyl, isopropyl, butyl, isobutyl, sec-butyl or tert-butyl.

6. Compound of the formula (I) according to Claim 1 in which
W represents hydrogen,
X represents methyl,
Y represents hydrogen,
Z represents methyl,
A, B and the carbon to which they are attached represent C₆-cycloalkyl which is optionally substituted by methoxy or methoxymethyl, where the substituent may be located either in the 3- or in the 4-position of the cycle,
G represents one of the groups 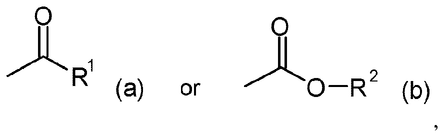 in which
R¹ represents methyl, ethyl, propyl, isopropyl, butyl, isobutyl, sec-butyl or tert-butyl,
R² represents methyl, ethyl, propyl, isopropyl, butyl, isobutyl, sec-butyl or tert-butyl.

7. Process for preparing compounds of the formula (I) according to Claim 1, **characterized in that**, to obtain
(A) compounds of the formula (I-a) shown above in which R¹, A, B, W, X, Y and Z have the meanings given above, compounds of the formula (II) in which A, B, W, X, Y and Z have the meanings given above 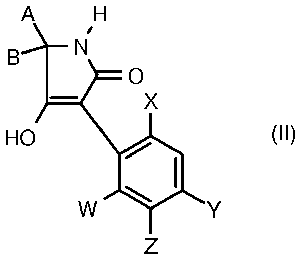 in which
A, B, W, X, Y and Z have the meanings given above,
α) are reacted with compounds of the formula (III) 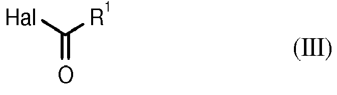 in which
R¹ has the meaning given above and
Hal represents halogen,
or
β) with carboxylic anhydrides of the formula (IV)
R¹-CO-O-CO-R¹ (IV)
in which
R¹ has the meaning given above and
if appropriate in the presence of a diluent and if appropriate in the presence of an acid binder;
(B) compounds of the formula (I-b) shown above in which R², A, B, M, W, X, Y and Z have the meanings given above and L represents oxygen, compounds of the formula (II) shown above in which A, B, W, X, Y and Z have the meanings given above
are reacted with chloroformic esters or chloroformic thioesters of the formula (V)
R²-M-CO-Cl (V)
in which
R² and M have the meanings given above,
if appropriate in the presence of a diluent and if appropriate in the presence of an acid binder.

8. Pesticide and/or herbicide, **characterized in that** it comprises at least one compound of the formula (I) according to Claim 1.

9. Method of controlling animal pests and/or unwanted vegetation, **characterized in that** compounds of the formula (I) according to Claim 1 are allowed to act on pests and/or their habitat, with the exception of processes for the surgical and therapeutic treatment of the human or animal body.

10. Use of compounds of the formula (I) according to Claim 1 for controlling animal pests and/or unwanted vegetation, with the exception of processes for the surgical and therapeutic treatment of the human or animal body.

11. Process for the preparation of pesticides and/or herbicides, **characterized in that** compounds of the formula (I) according to Claim 1 are mixed with extenders and/or surfactants.

12. Use of compounds of the formula (I) according to Claim 1 for the preparation of pesticides and/or herbicides.

13. Composition, comprising an effective amount of an active compound combination comprising, as components,
a') at least one compound of the formula (I) according to claim 1 in which A, B, W, X, Y and Z have the meaning given above, and
(b') at least one crop plant compatibility-improving compound from the following group of compounds: S1, S2, S3, S4, S5, S6, S7, S8, S9, S10, S11, S12, S13, S14, S15, S16,
S1) compounds of the formula (S1) 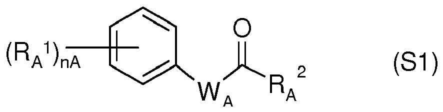 where the symbols and indices have the following meanings:
n_{A} is a natural number from 0 to 5;
R_{A}¹ is halogen, (C₁-C₄)-alkyl, (C₁-C₄)-alkoxy, nitro or (C₁-C₄)-haloalkyl;
W_{A} is an unsubstituted or substituted divalent heterocyclic radical from the group of the partially unsaturated or aromatic five-membered heterocycles having 1 to 3 ring heteroatoms of the N and O group, where at least one nitrogen atom and at most one oxygen atom is present in the ring,
R_{A}² is OR_{A}³, SR_{A}³ or NR_{A}³R_{A}⁴ or a saturated or unsaturated 3- to 7-membered heterocycle having at least one nitrogen atom and up to 3 heteroatoms, preferably from the group of O and S, which is joined to the carbonyl group in (S1) via the nitrogen atom and is unsubstituted or substituted by radicals from the group of (C₁-C₄)alkyl, (C₁-C₄)alkoxy or optionally substituted phenyl, preferably a radical of the formula OR_{A}³, NHR_{A}⁴ or N(CH₃)₂, especially of the formula OR_{A}³;
R_{A}³ is hydrogen or an unsubstituted or substituted aliphatic hydrocarbon radical preferably having a total of 1 to 18 carbon atoms;
R_{A}⁴ is hydrogen, (C₁-C₆)-alkyl, (C₁-C₆)-alkoxy or substituted or unsubstituted phenyl;
S2) Quinoline derivatives of the formula (S2) 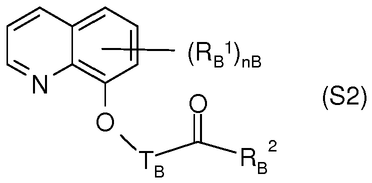 where the symbols and indices have the following meanings:
R_{B}¹ is halogen, (C₁-C₄)-alkyl, (C₁-C₄)-alkoxy, nitro or (C₁-C₄)-haloalkyl;
n_{B} is a natural number from 0 to 5;
R_{B}² is OR_{B}³, SR_{B}³ or NR_{B}³R_{B}⁴ or a saturated
or unsaturated 3- to 7-membered heterocycle having at least one nitrogen atom and up to 3 heteroatoms, which is joined to the carbonyl group in (S2) via the nitrogen atom and is unsubstituted or substituted by radicals from the group of (C₁-C₄)-alkyl, (C₁-C₄)-alkoxy or optionally substituted phenyl;
R_{B}³ is hydrogen or an unsubstituted or substituted aliphatic hydrocarbyl radical;
R_{B}⁴ is hydrogen, (C₁-C₆)-alkyl, (C₁-C₆)-alkoxy or substituted or unsubstituted phenyl;
T_{B} is a (C₁ or C₂)-alkanediyl chain which is unsubstituted or substituted by one or two (C₁-C₄)-alkyl radicals or by [(C₁-C₃)-alkoxy]-carbonyl.
S3) Compounds of the formula (S3) 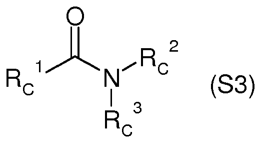 where the symbols and indices have the following meanings:
R_{C}¹ is (C₁-C₄)-alkyl, (C₁-C₄)-haloalkyl, (C₂-C₄)-alkenyl, (C₂-C₄)-haloalkenyl, (C₃-C₇)-cycloalkyl;
R_{C}², R_{C}³ are identical or different and are hydrogen, (C₁-C₄)-alkyl, (C₂-C₄)-alkenyl, (C₂-C₄)-alkynyl, (C₁-C₄)-haloalkyl, (C₂-C₄)-haloalkenyl, (C₁-C₄)-alkylcarbamoyl-(C₁-C₄)-alkyl, (C₂-C₄)-alkenylcarbamoyl-(C₁-C₄)alkyl, (C₁-C₄)-alkoxy-(C₁-C₄)-alkyl, dioxolanyl-(C₁-C₄)-alkyl, thiazolyl, furyl, furylalkyl, thienyl, piperidyl, substituted or unsubstituted phenyl, or R_{C}2 and R_{C}³ together form a substituted or unsubstituted heterocyclic ring, preferably an oxazolidine, thiazolidine, piperidine, morpholine, hexahydropyrimidine or benzoxazine ring.
S4) N-Acylsulfonamides of the formula (S4) and salts thereof 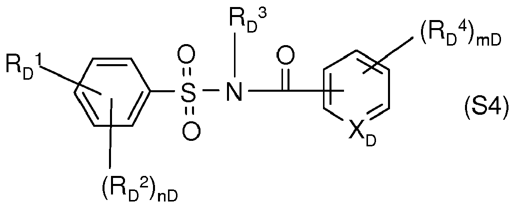 where the symbols and indices have the following meanings:
X_{D} is CH or N;
R_{D}¹ is CO-NR_{D}⁵R_{D}⁶ or NHCO-R_{D}⁷;
R_{D}² is halogen, (C₁-C₄)-haloalkyl, (C₁-C₄)-haloalkoxy, nitro, (C₁-C₄)-alkyl, (C₁-C₄)-alkoxy, (C₁-C₄)-alkylsulfonyl, (C₁-C₄)-alkoxycarbonyl or (C₁-C₄)-alkylcarbonyl;
R_{D}³ is hydrogen, (C₁-C₄)-alkyl, (C₂-C₄)-alkenyl or (C₂-C₄)-alkynyl;
R_{D}⁴ is halogen, nitro, (C₁-C₄)-alkyl, (C₁-C₄)-haloalkyl, (C₁-C₄)-haloalkoxy, (C₃-C₆)-cycloalkyl, phenyl, (C₁-C₄)-alkoxy, cyano, (C₁-C₄)-alkylthio, (C₁-C₄)-alkylsulfinyl, (C₁-C₄)-alkylsulfonyl, (C₁-C₄)-alkoxycarbonyl or (C₁-C₄)-alkylcarbonyl;
R_{D}⁵ is hydrogen, (C₁-C₆)-alkyl, (C₃-C₆)-cycloalkyl, (C₂-C₆)-alkenyl, (C₂-C₆)-alkynyl, (C₅-C₆)-cycloalkenyl, phenyl or 3- to 6-membered heterocyclyl containing V_{D} heteroatoms from the group of nitrogen, oxygen and sulfur, where the seven latter radicals are each substituted by V_{D} substituents from the group of halogen, (C₁-C₆)-alkoxy, (C₁-C₆)-haloalkoxy, (C₁-C₂)-alkylsulfinyl, (C₁-C₂)-alkylsulfonyl, (C₃-C₆)-cycloalkyl, (C₁-C₄)-alkoxycarbonyl, (C₁-C₄)-alkylcarbonyl and phenyl, and in the case of cyclic radicals also (C₁-C₄)-alkyl and (C₁-C₄)-haloalkyl;
R_{D}⁶ is hydrogen, (C₁-C₆)-alkyl, (C₂-C₆)-alkenyl or (C₂-C₆)-alkynyl, where the three latter radicals are substituted by V_{D} radicals from the group of halogen, hydroxyl, (C₁-C₄)-alkyl, (C₁-C₄)-alkoxy and (C₁-C₄)-alkylthio, or
R_{D}⁵ and R_{D}⁶ together with the nitrogen atom which bears them form a pyrrolidinyl or piperidinyl radical;
R_{D}⁷ is hydrogen, (C₁-C₄)-alkylamino, di-(C₁-C₄)-alkylamino, (C₁-C₆)-alkyl, (C₃-C₆)-cycloalkyl, where the 2 latter radicals are substituted by V_{D} substituents from the group of halogen, (C₁-C₄)-alkoxy, (C₁-C₆)-haloalkoxy and (C₁-C₄)-alkylthio, and in the case of cyclic radicals also (C₁-C₄)-alkyl and (C₁-C₄)-haloalkyl;
n_{D} is 0, 1 or 2;
m_{D} is 1 or 2;
V_{D} is 0, 1, 2 or 3.
S5) Active compounds from the class of the hydroxyaromatics and aromatic-aliphatic carboxylic acid derivatives (S5), for example ethyl 3,4,5-triacetoxybenzoate, 3,5-dimethoxy-4-hydroxybenzoic acid, 3,5-dihydroxybenzoic acid, 4-hydroxysalicylic acid, 4-fluorosalicylic acid, 2-hydroxycinnamic acid, 1,2-dihydro-2-oxo-6-trifluoromethylpyridine-3-carboxamide, 2,4-dichlorocinnamic acid, as described in WO-A-2004/084631, WO-A-2005/015994, WO-A-2005/016001.
S6) Active compounds from the class of the 1,2-dihydroquinoxalin-2-ones (S6), for example 1-methyl-3-(2-thienyl)-1,2-dihydroquinoxalin-2-one, 1-methyl-3-(2-thienyl)-1,2-dihydroquinoxaline-2-thione, 1-(2-aminoethyl)-3-(2-thienyl)-1,2-dihydroquinoxalin-2-one hydrochloride, 1-[2-(diethylamino)ethyl]-6,7-dimethyl-3-thiophen-2-ylquinoxalin-2(1*H*)-one, 1-(2-methylsulfonylaminoethyl)-3-(2-thienyl)-1,2-dihydroquinoxalin-2-one, as described in WO-A-2005/112630.
S7) Compounds of the formula (S7), as described in WO-A-1998/38856, 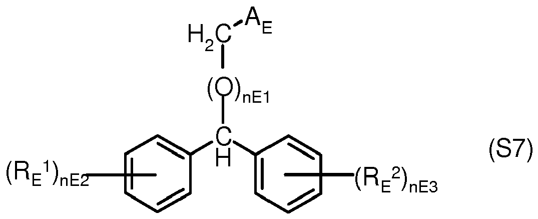 where the symbols and indices have the following meanings:
R_{E}¹, R_{E}² are each independently of one another halogen, (C₁-C₄)-alkyl, (C₁-C₄)-alkoxy, (C₁-C₄)-haloalk-yl, (C₁-C₄)-alkylamino, di-(C₁-C₄)-alkylamino, nitro;
A_{E} is COOR_{E}³ or COSR_{E}⁴
R_{E}³, R_{E}⁴ are each independently of one another hydrogen, (C₁-C₄)-alkyl, (C₂-C₆)-alkenyl, (C₂-C₄)-alkynyl, cyanoalkyl, (C₁-C₄)-haloalkyl, phenyl, nitrophenyl, benzyl, halobenzyl, pyridinylalkyl and alkylammonium,
n_{E}¹ is 0 or 1
n_{E}², n_{E}³ are each independently of one another 0, 1 or 2.
S8) Compounds of the formula (S8), as described in WO-A-98/27049, 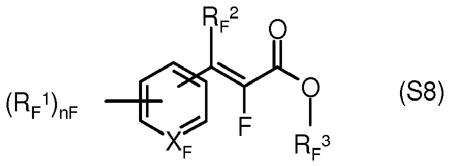 in which
X_{F} is CH or N,
n_{F} in the case that X_{F}=N is an integer from 0 to 4 and
in the case that X_{F}=CH is an integer from 0 to 5,
R_{F}¹ is halogen, (C₁-C₄)-alkyl, (C₁-C₄)-haloalkyl, (C₁-C₄)-alkoxy, (C₁-C₄)-haloalkoxy, nitro, (C₁-C₄)-alkylthio, (C₁-C₄)-alkylsulfonyl, (C₁-C₄)-alkoxycarbonyl, optionally substituted phenyl, optionally substituted phenoxy,
R_{F}² is hydrogen or (C₁-C₄)-alkyl
R_{F}³ is hydrogen, (C₁-C₈)-alkyl, (C₂-C₄)-alkenyl, (C₂-C₄)-alkynyl, or aryl, where each of the aforementioned carbon-containing radicals is unsubstituted or substituted by one or more identical or different radicals from the group consisting of halogen and alkoxy, or salts thereof.
S9) Active compounds from the class of the 3-(5-tetrazolylcarbonyl)-2-quinolones (S9), e.g. 1,2-dihydro-4-hydroxy-1-ethyl-3-(5-tetrazolylcarbonyl)-2-quinolone (CAS reg. no. 219479-18-2), 1,2-dihydro-4-hydroxy-1-methyl-3-(5-tetrazolylcarbonyl)-2-quinolone (CAS reg. no. 95855-00-8), as described in WO-A-1999/000020.
S10) Compounds of the formulae (S10^{a}) or (S10^{b})
as described in WO-A-2007/023719 and WO-A-2007/023764, 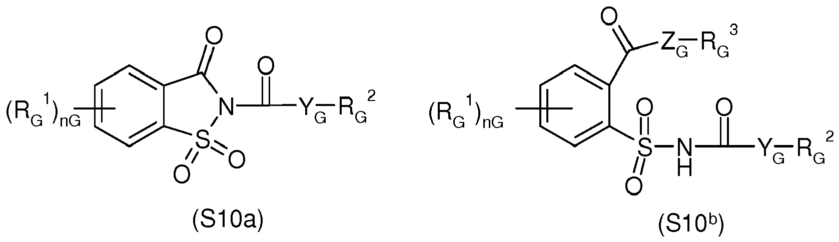 in which
R_{G}¹ is halogen, (C₁-C₄)-alkyl, methoxy, nitro, cyano, CF₃, OCF₃,
Y_{G,} Z_{G} are each independently of one another O or S,
n_{G} is an integer from 0 to 4,
R_{G}² is (C₁-C₁₆)-alkyl, (C₂-C₆)-alkenyl, (C₃-C₆)-cycloalkyl, aryl; benzyl, halobenzyl,
R_{G}³ is hydrogen or (C₁-C₆)-alkyl.
S11) Active compounds of the type of the oxyimino compounds (S11), which are known as seed dressings, such as, for example, "oxabetrinil" ((Z)-1,3-dioxolan--ylmethoxyimino(phenyl)acetonitrile) (S11-1), which is known as seed dressing safener for millet against metolachlor damage,
"fluxofenim" (1-(4-chlorophenyl)-2,2,2-trifluoro-1-ethanone-O-(1,3-dioxolan-2-ylmethyl)oxime) (S11-2), which is known as seed dressing safener for millet against metolachlor damage, and
"cyometrinil" or "CGA-43089" ((Z)-cyanomethoxyimino(phenyl)acetonitrile) (S11-3), which is known as seed dressing safener for millet against metolachlor damage.
S12) Active compounds from the class of the isothiochromanones (S12), for example methyl [(3-oxo-1H-2-benzothiopyran-4(3H)-ylidene)-methoxy] acetate (CAS reg. no. 205121-04-6) (S12-1) and related compounds from WO-A-1998/13361.
S13) One or more compounds from group (S13):
"naphthalic anhydrid" (1,8-naphthalenedicarboxylic anhydride) (S13-1), which is known as seed dressing safener for corn against thiocarbamate herbicide damage,
"fenclorim" (4,6-dichloro-2-phenylpyrimidine) (S13-2), which is known as safener for pretilachlor in sown rice,
"flurazole" (benzyl 2-chloro-4-trifluoromethyl-1,3-thiazole-5-carboxylate) (S13-3), which is known as seed dressing safener for millet against alachlor and metolachlor damage,
"CL 304415" (CAS reg. no. 31541-57-8) (4-carboxy-3,4-dihydro-2H-1-benzopyran-4-acetic acid) (S 13-4) from American Cyanamid, which is known as a safener for corn against damage by imidazolinones,
"MG 191" (CAS reg. no. 96420-72-3) (2-dichloromethyl-2-methyl-1,3-dioxolane) (S13-5) from Nitrokemia, which is known as a safener for corn,
"MG-838" (CAS reg. no. 133993-74-5) (2-propenyl 1-oxa-4-azaspiro[4.5]decane-4-carbodithioate) (S13-6) from Nitrokemia,
"disulfoton" (O,O-diethyl S-2-ethylthioethyl phosphorodithioate) (S13-7),
"dietholate" (O,O-diethyl O-phenyl phosphorothioate) (S13-8),
"mephenate" (4-chlorophenyl methylcarbamate) (S13-9).
S14) Active compounds which, in addition to herbicidal action against harmful plants, also have safener action on crop plants such as rice, for example "dimepiperate" or "MY-93" (S-1-methyl-1-phenylethylpiperidine-1-carbothioate), which is known as a safener for rice against damage by the herbicide molinate,
"daimuron" or "SK 23" (1-(1-methyl-1-phenylethyl)-3-p-tolylurea), which is known as safener for rice against imazosulfuron herbicide damage,
"cumyluron" = "JC-940" (3-(2-chlorophenylmethyl)-1-(1-methyl-1-phenylethyl)urea, see JP-A-60087254), which is known as safener for rice against damage by some herbicides,
"methoxyphenone" or "NK 049" (3,3'-dimethyl-4-methoxybenzophenone), which is known as safener for rice against damage by some herbicides,
"CSB" (1-bromo-4-(chloromethylsulfonyl)benzene) from Kumiai, (CAS reg. no. 54091-06-4), which is known as a safener against damage by some herbicides in rice.
S15) Compounds of the formula (S15) or tautomers thereof 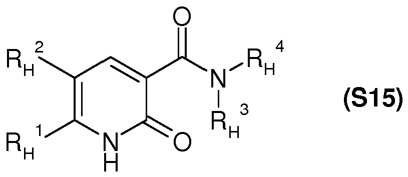 as described in WO-A-2008/131861 and WO-A-2008/131860
in which
R_{H}¹ is a (C₁-C₆)-haloalkyl radical and
R_{H}² is hydrogen or halogen and
R_{H}³, R_{H}⁴ are each independently of one another hydrogen, (C₁-C₁₆)-alkyl, (C₂-C₁₆)-alkenyl or (C₂-C₁₆)-alkynyl,
where each of the latter 3 radicals is unsubstituted or substituted by one or more radicals from the group of halogen, hydroxyl, cyano, (C₁-C₄)-alkoxy, (C₁-C₄)-haloalkoxy, (C₁-C₄)-alkylthio, (C₁-C₄)-alkylamino, di[(C₁-C₄)-alkyl]amino, [(C₁-C₄)-alkoxy]carbonyl, [(C₁-C₄)-haloalkoxy]carbonyl, (C₃-C₆)-cycloalkyl which is unsubstituted or substituted, phenyl which is unsubstituted or substituted, and heterocyclyl which is unsubstituted or substituted,
or (C₃-C₆)-cycloalkyl, (C₄-C₆)-cycloalkenyl, (C₃-C₆)-cycloalkyl which is fused on one side of the ring to a 4- to 6-membered saturated or unsaturated carbocyclic ring, or (C₄-C₆)-cycloalkenyl which is fused on one side of the ring to a 4- to 6-membered saturated or unsaturated carbocyclic ring,
where each of the latter 4 radicals is unsubstituted or substituted by one or more radicals from the group of halogen, hydroxyl, cyano, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylamino, di[(C₁-C₄)-alkyl]amino, [(C₁-C₄)-alkoxy]carbonyl, [(C₁-C₄)-haloalkoxy]carbonyl, (C₃-C₆)-cycloalkyl which is unsubstituted or substituted, phenyl which is unsubstituted or substituted, and heterocyclyl which is unsubstituted or substituted,
or
R_{H}³ is (C₁-C₄)-alkoxy, (C₂-C₄)-alkenyloxy, (C₂-C₆)-alkynyloxy or (C₂-C₄)-haloalkoxy and
R_{H}⁴ is hydrogen or (C₁-C₄)-alkyl or
R_{H}³ and R_{H}⁴ together with the directly bonded nitrogen atom are a four- to eight-membered heterocyclic ring which, as well as the nitrogen atom, may also contain further ring heteroatoms, and which is unsubstituted or substituted by one or more radicals from the group of halogen, cyano, nitro, (C₁-C₄)-alkyl, (C₁-C₄)-haloalkyl, (C₁-C₄)-alkoxy, (C₁-C₄)-haloalkoxy and (C₁-C₄)-alkylthio.
S16) Active compounds which are used primarily as herbicides but also have safener action on crop plants, for example
(2,4-dichlorophenoxy)acetic acid (2,4-D),
(4-chlorophenoxy)acetic acid,
(R,S)-2-(4-chloro-o-tolyloxy)propionic acid (mecoprop),
4-(2,4-dichlorophenoxy)butyric acid (2,4-DB),
(4-chloro-o-tolyloxy)acetic acid (MCPA),
4-(4-chloro-o-tolyloxy)butyric acid,
4-(4-chlorophenoxy)butyric acid,
3,6-dichloro-2-methoxybenzoic acid (dicamba),
1-(ethoxycarbonyl)ethyl 3,6-dichloro-2-methoxybenzoate (lactidichlor-ethyl).

14. Method for controlling unwanted vegetation, **characterized in that** a composition according to Claim 13 is allowed to act on the plants or their surroundings.

15. Use of a composition according to Claim 13 for controlling unwanted vegetation.

16. Method for controlling unwanted vegetation, **characterized in that** a compound of the formula (I) according to Claim 1 and the crop plant compatibility-improving compound according to Claim 13 are allowed to act separately, in close temporal succession on the plants or their surroundings.

## Revendications

1. Composés de formule (I) 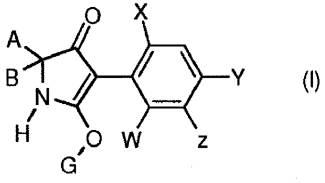 dans laquelle
W représente un atome d'hydrogène, d'halogène, un groupe alkyle, alcényle, alcynyle, un groupe cycloalkyle éventuellement substitué, un groupe alcoxy, alcényloxy, halogénoalkyle, halogénoalcoxy ou cyano,
X représente un atome d'halogène, un groupe alkyle, alcényle, alcynyle, un groupe cycloalkyle éventuellement substitué, un groupe alcoxy, alcényloxy, alkylthio, alkylsulfinyle, alkylsulfonyle, halogénoalkyle, halogénoalcoxy, halogénoalcényloxy, nitro ou cyano,
Y et Z représentent indépendamment l'un de l'autre un atome d'hydrogène, un groupe alkyle, alcényle, alcynyle, un groupe cycloalkyle éventuellement substitué, un groupe alcoxy, un atome d'halogène, un groupe halogénoalkyle, halogénoalcoxy, cyano, nitro ou un groupe aryle ou hétéroaryle chacun éventuellement substitué,
A représente un atome d'hydrogène, un groupe alkyle, alcényle, alcoxyalkyle, alkylthioalkyle chacun éventuellement substitué par halogéno, un groupe cycloalkyle saturé ou insaturé, éventuellement substitué, dans lequel éventuellement au moins un atome formant le cycle est remplacé par un hétéroatome, ou un groupe aryle, arylalkyle ou hétéroaryle chacun éventuellement substitué par halogéno, alkyle, halogénoalkyle, alcoxy, halogénoalcoxy, cyano ou nitro,
B représente un atome d'hydrogène, un groupe alkyle ou alcoxyalkyle
étant entendu que A et B représentent chacun seulement le groupe méthyle lorsque, dans le cas où W, X et Y représentent chacun un groupe alkyle et Z représente un atome d'hydrogène, W et X représentent chacun le groupe méthyle ou W et X représentent chacun le groupe éthyle,
ou
A et B représentent ensemble, avec l'atome de carbone auquel ils sont liés, un cycle saturé ou insaturé, substitué ou non substitué, contenant éventuellement au moins un hétéroatome,
G représente l'un des groupes 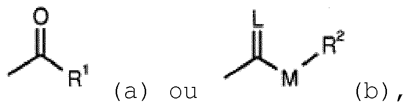 où
L représente un atome d'oxygène ou de soufre,
M représente un atome d'oxygène ou de soufre,
R¹ représente un groupe alkyle, alcényle, alcoxyalkyle, alkylthioalkyle ou polyalcoxy-alkyle chacun éventuellement substitué par halogéno ou cyano ou représente un groupe cycloalkyle ou hétérocyclyle chacun éventuellement substitué par halogéno, alkyle ou alcoxy ou représente un groupe phényle, phénylalkyle, hétéroaryle, phénoxyalkyle ou hétéroaryloxyalkyle chacun éventuellement substitué,
R² représente un groupe alkyle, alcényle, alcoxyalkyle ou polyalcoxyalkyle chacun éventuellement substitué par halogéno ou cyano ou représente un groupe cycloalkyle, phényle ou benzyle chacun éventuellement substitué.

2. Composés de formule (I) selon la revendication 1 dans lesquels
W représente un atome d'hydrogène, d'halogène, un groupe alkyle en C₁-C₆, alcényle en C₂-C₆, alcynyle en C₂-C₆, un groupe cycloalkyle en C₃-C₆ éventuellement une ou deux fois substitué par alkyle en C₁-C₂, alcoxy en C₁-C₂, fluoro, chloro, trifluorométhyle ou cycloalkyle en C₃-C₆, un groupe alcoxy en C₁-C₆, halogénoalkyle(C₁-C₄), halogénoalcoxy(C₁-C₄₎ ou cyano,
X représente un atome d'halogène, un groupe alkyle en C₁-C₆, alcényle en C₂-C₆, alcynyle en C₂-C₆, un groupe cycloalkyle en C₃-C₆ éventuellement une ou deux fois substitué par alkyle en C₁-C₂, alcoxy en C₁-C₂, fluoro, chloro, trifluorométhyle ou cycloalkyle en C₃-C₆, un groupe halogénoalkyle(C₁-C₆) alcoxy en C₁-C₆, alcényloxy(C₃-C₆), alkyl(C₁-C₆) thio, alkyl(C₁-C₆)-sulfinyle, alkyl(C₁-C₆)sulfonyle, halogéno-alcoxy(C₁-C₆), halogénoalcényloxy(C₃-C₆₎, nitro ou cyano,
Y et Z représentent indépendamment l'un de l'autre un atome d'hydrogène, un atome d'halogène, un groupe alkyle en C₁-C₆, alcényle en C₂-C₆, alcynyle en C₂-C₆, un groupe cycloalkyle en C₃-C₆ éventuellement une ou deux fois substitué par alkyle en C₁-C₂, alcoxy en C₁-C₂, fluoro, chloro, trifluorométhyle ou cycloalkyle en C₃-C₆, un groupe alcoxy en C₁-C₆, halogénoalkyle(C₁-C₆), halogénoalcoxy(C₁-C₆), cyano, alcényle en C₂-C₆, alcynyle en C₂-C₆ ou représente l'un des radicaux (hétéro)-aryle, 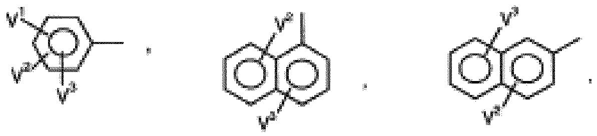 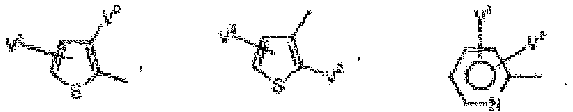 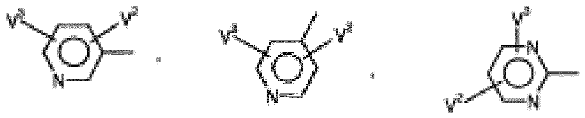 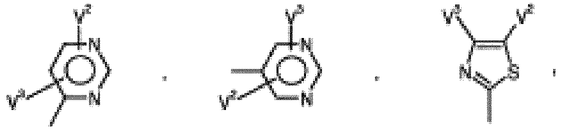 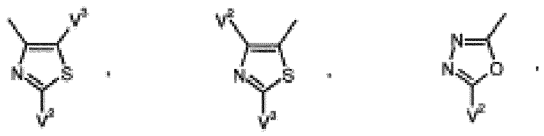 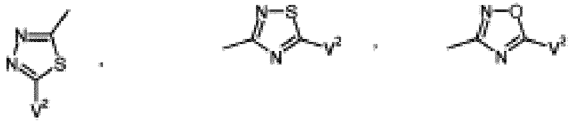 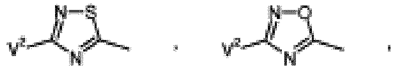 dans le cas d'un radical (hétéro)-aryle un seul des radicaux Y ou Z pouvant représenter un radical (hétéro)-aryle,
V¹ représente un atome d'hydrogène, d'halogène, un groupe alkyle en C₁-C₁₂, alcoxy en C₁-C₆, alkyl(C₁-C₆)thio, alkyl(C₁-C₆)sulfinyle, alkyl(C₁-C₆)sulfonyle, halogénoalkyle(C₁-C₄), halogénoalcoxy(C₁-C₄), nitro, cyano ou un groupe phényle, phénoxy, phénoxyalkyle(C₁-C₄), phényl-alcoxy(C₁-C₄), phénylthio-alkyle(C₁-C₄) ou phényl-alkyl(C₁-C₄)thio chacun éventuellement une ou plusieurs fois substitué par halogéno, alkyle en C₁-C₆ alcoxy en C₁-C_{6,} halogénoalkyle(C₁-C₄), halogénoalcoxy(C₁-C₄), nitro ou cyano,
V² et V³ représentent indépendamment l'un de l'autre un atome d'hydrogène, un atome d'halogène, un groupe alkyle en C₁-C₆, alcoxy en C₁-C₆, halogénoalkyle(C₁-C₄₎ ou halogénoalcoxy(C₁-C₄₎,
A représente un atome d'hydrogène ou un groupe alkyle en C₁-C₁₂, alcényle en C₃-C₈, alcoxy(C₁-C₁₀)alkyle(C₁-C₈), alkyl(C₁-C₁₀)thio-alkyle(C₁-C₆) chacun éventuellement substitué par halogéno, un groupe cycloalkyle en C₃-C₈ éventuellement substitué par halogéno, alkyle en C₁-C₆ ou alcoxy en C₁-C₆, dans lequel éventuellement un ou deux chaînons non contigus du cycle est/sont remplacé(s) par un atome d'oxygène et/ou un atome de soufre, ou un groupe phényle, naphtyle, hétéroaryle à 5 ou 6 chaînons formant le cycle, phénylalkyle(C₁-C₆) ou naphtylalkyle(C₁-C₆) chacun éventuellement substitué par halogéno, alkyle en C₁-C₆, halogénoalkyle(C₁-C₆), alcoxy en C₁-C₆, halogénoalcoxy(C₁-C₆), cyano ou nitro,
B représente un atome d'hydrogène, un groupe alkyle en C₁-C₁₂ ou alcoxy(C₁-C₈)alkyle(C₁-C₆),
étant entendu que A et B ne peuvent représenter chacun que le groupe méthyle lorsque, dans le cas où W, X et Y représentent chacun un groupe alkyle en C₁-C₆ et Z représente un atome d'hydrogène, W et X représentent chacun le groupe méthyle ou W et X représentent chacun le groupe éthyle,
ou A et B représentent ensemble, avec l'atome de carbone auquel ils sont liés, un groupe cycloalkyle en C₃-C₁₀ saturé ou cycloalkyle en C₅-C₁₀ insaturé, dans lesquels éventuellement un chaînon du cycle est remplacé par un atome d'azote, d'oxygène ou de soufre et qui sont éventuellement une ou deux fois substitués par alkyle en C₁-C₈, alcoxy en C₁-C₈, alcényloxy en C₃-C₈, alcoxy(C₁-C₆)-alkyle(C₁-C₆), cycloalkyl(C₃-C₆)-alcoxy(C₁-C₂), cycloalkyle en C₃-C₁₀, halogéno-alkyle(C₁-C₈), halogénoalcoxy(C₂-C₆), alcoxy(C₁-C₆)-alcoxy(C₁-C₄), les radicaux nommés précédemment (hormis trifluorométhyle) étant également pris en considération en tant que substituants sur N, ou
A et B représentent ensemble, avec l'atome de carbone auquel ils sont liés, un groupe cycloalkyle en C₃-C₆ qui est substitué par un groupe alkylènediyle éventuellement substitué par alkyle en C₁-C₄, contenant éventuellement un ou deux atomes d'oxygène et/ou atomes de soufre non directement voisins, ou par un groupe alkylènedioxy ou par un groupe alkylènedithioyle, qui forme, avec l'atome de carbone auquel il est lié, un autre cycle à cinq à huit chaînons ou
A et B représentent ensemble, avec l'atome de carbone auquel ils sont liés, un groupe cycloalkyle en C₃-C₈ ou cycloalcényle en C₅-C₈, dans lequel deux substituants représentent ensemble, avec les atomes de carbone auxquels ils sont liés, un groupe alcane (C₂-C₆)diyle, alcène (C₂-C₆)diyle ou alcanediène(C₄-C₆)diyle chacun éventuellement substitué par alkyle en C₁-C₆, alcoxy en C₁-C₆ ou halogéno, dans lequel éventuellement un groupe méthylène est remplacé par un atome d'oxygène ou de soufre,
G représente l'un des groupes 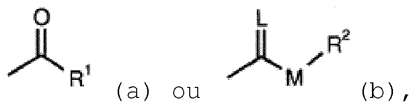 où
L représente un atome d'oxygène ou de soufre,
M représente un atome d'oxygène ou de soufre,
R¹ représente un groupe alkyle en C₁-C_{20,} alcényle en C₂-C₂₀, alcoxy(C₁-C₈)-alkyle(C₁-C₈), alkyl(C₁-C₈)thio-alkyle(C₁-C₈) ou poly-alcoxy(C₁-C₈)-alkyle(C₁-C₈), chacun éventuellement substitué par halogéno ou cyano, ou représente un groupe cycloalkyle en C₃-C₈ éventuellement substitué par halogéno, alkyle en C₁-C₆ ou alcoxy en C₁-C₆, dans lequel éventuellement un ou deux groupes méthylène non directement voisins sont remplacés par un atome d'oxygène et/ou un atome de soufre,
représente un groupe phényle éventuellement substitué par halogéno, cyano, nitro, alkyle en C₁-C₆, alcoxy en C₁-C₆, halogénoalkyle(C₁-C₆), halogénoalcoxy(C₁-C₆), alkyl(C₁-C₆)thio ou alkyl(C₁-C₆)sulfonyle,
représente un groupe phényl-alkyle(C₁-C₆) éventuellement substitué par halogéno, nitro, cyano, alkyle en C₁-C₆, alcoxy en C₁-C₆, halogénoalkyle(C₁-C₆) ou halogéno-alcoxy(C₁-C₆),
représente un groupe hétéroaryle à 5 ou 6 chaînons, comportant un ou deux hétéroatomes choisis dans la série oxygène, soufre et azote, éventuellement substitué par halogéno ou alkyle en C₁-C₆,
représente un groupe phénoxy-alkyle(C₁-C₆) éventuellement substitué par halogéno ou alkyle en C₁-C₆ ou
représente un groupe hétéroaryloxy-alkyle(C₁-C₆) à 5 ou 6 chaînons, comportant un ou deux hétéroatomes choisis dans la série oxygène, soufre et azote, éventuellement substitué par halogéno, amino ou alkyle en C₁-C₆,
R² représente un groupe alkyle en C₁-C₂₀, alcényle en C₂₋C₂₀, alcoxy (C₁-C₈)-alkyle(C₂-C₈) ou poly-alcoxy(C₁-C₈)-alkyle(C₂-C₈) chacun éventuellement substitué par halogéno ou cyano,
représente un groupe cycloalkyle en C₃-C₈ éventuellement substitué par halogéno, alkyle en C₁-C₆ ou alcoxy en C₁-C₆ ou
représente un groupe phényle ou benzyle chacun éventuellement substitué par halogéno, cyano, nitro, alkyle en C₁-C₆, alcoxy en C₁-C₆, halogénoalkyle(C₁-C₆) ou halogéno-alcoxy(C₁-C₆).

3. Composés de formule (I) selon la revendication 1, dans lesquels
W représente un atome d'hydrogène, de chlore, brome, un groupe alkyle en C₁-C₄, alcényle en C₂-C₄, alcynyle en C₂-C₄, un groupe cycloalkyle en C₃-C₆ éventuellement une fois substitué par méthyle, éthyle, méthoxy, fluoro, chloro, trifluorométhyle ou cyclopropyle, un groupe alcoxy en C₁-C₄, halogénoalkyle(C₁-C₂) ou halogénoalcoxy(C₁-C₂),
X représente un atome de chlore, de brome, d'iode, un groupe alkyle en C₁-C₄, alcényle en C₂-C₄, alcynyle en C₂-C₄, un groupe cycloalkyle en C₃-C₆ éventuellement une fois substitué par méthyle, éthyle, méthoxy, fluoro, chloro, trifluorométhyle ou cyclopropyle, un groupe alcoxy en C₁-C₄, halogénoalkyle(C₁-C₄₎, halogénoalcoxy(C₁-C₄₎ ou cyano,
Y et Z représentent indépendamment l'un de l'autre un atome d'hydrogène, de fluor, chlore, brome, d'iode, un groupe alkyle en C₁-C₄, alcényle en C₂-C₄, alcynyle en C₂-C₄, un groupe cycloalkyle en C₃-C₆ éventuellement une fois substitué par méthyle, éthyle, méthoxy, fluoro, chloro, trifluorométhyle ou cyclopropyle, un groupe alcoxy en C₁-C₆, halogénoalkyle(C₁-C₄₎, halogénoalcoxy(C₁-C₄₎, cyano, alcényle en C₂-C₄, alcynyle en C₂-C₄ ou représente l'un des radicaux (hétéro)-aryle, 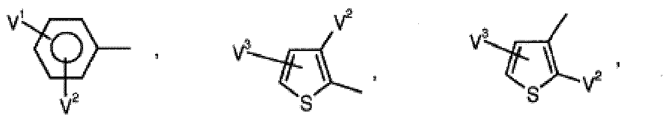 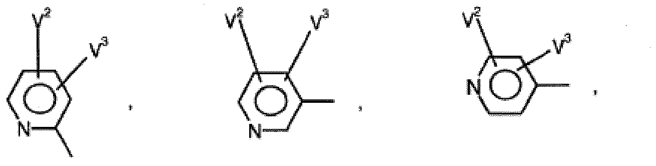 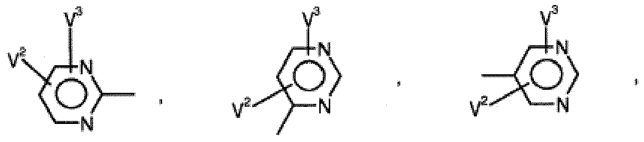 dans le cas d'un radical (hétéro)-aryle un seul des radicaux Y ou Z pouvant représenter un radical (hétéro)-aryle,
V¹ représente un atome d'hydrogène, de fluor, chlore, brome, un groupe alkyle en C₁-C₆, alcoxy en C₁-C₄, halogénoalkyle(C₁-C₂), halogénoalcoxy(C₁-C₂), nitro, cyano ou un groupe phényle éventuellement une ou deux fois substitué par fluoro, chloro, bromo, alkyle en C₁-C₄, alcoxy en C₁-C₄, halogénoalkyle(C₁-C₂), halogénoalcoxy(C₁-C₂), nitro ou cyano,
V² et V³ représentent indépendamment l'un de l'autre un atome d'hydrogène, de fluor, chlore, brome, un groupe alkyle en C₁-C₄, alcoxy en C₁-C₄, halogénoalkyle(C₁-C₂) ou halogénoalcoxy(C₁-C₂),
A représente un atome d'hydrogène, un groupe alkyle en C₁-C₆, alcoxy(C₁-C₄)alkyle(C₁-C₂), chacun éventuellement une à trois fois substitué par fluoro ou chloro, un groupe cycloalkyle en C₃-C₆ éventuellement une ou deux fois substitué par alkyle en C₁-C₂ ou alcoxy en C₁-C₂ et éventuellement interrompu par un atome d'oxygène, un groupe phényle, pyridyle ou benzyle, chacun éventuellement une ou deux fois substitué par fluoro, chloro, bromo, alkyle en C₁-C₄, halogéno-alkyle(C₁-C₂), alcoxy en C₁-C₄, halogénoalcoxy(C₁-C₂), cyano ou nitro,
B représente un atome d'hydrogène, un groupe alkyle en C₁-C₄ ou alcoxy(C₁-C₂)alkyle(C₁-C₂), étant entendu que A et B ne peuvent représenter chacun que le groupe méthyle lorsque, dans le cas où W, X et Y représentent chacun un groupe alkyle en C₁-C₄ et Z représente un atome d'hydrogène, W et X représentent chacun le groupe méthyle ou W et X représentent chacun le groupe éthyle,
ou
A et B représentent ensemble, avec l'atome de carbone auquel ils sont liés, un groupe cycloalkyle en C₃-C₇ saturé ou insaturé, dans lequel éventuellement un chaînon du cycle est remplacé par un atome d'azote, d'oxygène ou de soufre et qui est éventuellement une ou deux fois substitué par alkyle en C₁-C₆, alcoxy (C₁-C₄)-alkyle(C₁-C₂), trifluorométhyle, alcoxy en C₁-C₆, alcényloxy en C₃-C₆, trifluoroéthoxy, alcoxy(C₁-C₃)-alcoxy(C₁-C₃) ou cycloalkyl(C₃-C₆₎-méthoxy, les radicaux nommés précédemment (hormis trifluorométhyle) étant également pris en considération en tant que substituants sur N, ou
A et B représentent ensemble, avec l'atome de carbone auquel ils sont liés, un groupe cycloalkyle en C₅-C₆ qui est substitué par un groupe alkylènediyle éventuellement substitué par méthyle ou éthyle, contenant éventuellement un ou deux atomes d'oxygène et/ou atomes de soufre non directement voisins, ou par un groupe alkylènedioxy ou par un groupe alkylènedithiol, qui forme, avec l'atome de carbone auquel il est lié, un autre cycle à cinq ou six chaînons, ou
A et B représentent ensemble, avec l'atome de carbone auquel ils sont liés, un groupe cycloalkyle en C₃-C₆ ou cycloalcényle en C₅-C₆, dans lequel deux substituants représentent ensemble, avec les atomes de carbone auxquels ils sont liés, un groupe alcane(C₂-C₄)diyle, alcène(C₂-C₄)diyle ou butadiènediyle chacun éventuellement substitué par alkyle en C₁-C₂ ou alcoxy en C₁-C₂,
G représente l'un des groupes 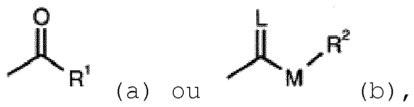 où
L représente un atome d'oxygène ou de soufre,
M représente un atome d'oxygène ou de soufre,
R¹ représente un groupe alkyle en C₁-C₁₆, alcényle en C₂-C₁₆, alcoxy (C₁-C₆)-alkyle (C₁-C₄), alkyl (C₁-C₆)-thio-alkyle(C₁-C₄) ou poly-alcoxy(C₁-C₆)-alkyle(C₁-C₄), chacun éventuellement une à trois fois substitué par fluoro ou chloro, ou représente un groupe cycloalkyle en C₃-C₇ éventuellement une ou deux fois substitué par fluoro, chloro, alkyle en C₁-C₅ ou alcoxy en C₁-C₅, dans lequel éventuellement un ou deux groupes méthylène non directement voisins sont remplacés par un atome d'oxygène et/ou un atome de soufre,
représente un groupe phényle éventuellement une à trois fois substitué par fluoro, chloro, bromo, cyano, nitro, alkyle en C₁-C₄, alcoxy en C₁-C₄, halogénoalkyle(C₁-C₃), halogénoalcoxy(C₁-C₃), alkyl (C₁-C₄)thio ou alkyl (C₁-C₄) sulfonyle,
représente un groupe phényl-alkyle(C₁-C₄) éventuellement une ou deux fois substitué par fluoro, chloro, bromo, alkyle en C₁-C₄, alcoxy en C₁-C₄, halogénoalkyle(C₁-C₃) ou halogéno-alcoxy(C₁-C₃),
représente un groupe pyrazolyle, thiazolyle, pyridyle, pyrimidyle, furanyle ou thiényle, chacun éventuellement une ou deux fois substitué par fluoro, chloro, bromo ou alkyle en C₁-C₄,
représente un groupe phénoxy-alkyle(C₁-C₅) éventuellement une ou deux fois substitué par fluoro, chloro, bromo ou alkyle en C₁-C₄ ou
représente un groupe pyridyloxy-alkyle(C₁-C₅), pyrimidyloxy-alkyle(C₁-C₅) ou thiazolyloxy-alkyle(C₁-C₅), chacun éventuellement une ou deux fois substitué par fluoro, chloro, bromo, amino ou alkyle en C₁-C₄,
R² représente un groupe alkyle en C₁-C₁₆, alcényle en C₂-C₁₆, alcoxy (C₁-C₆)-alkyle (C₂C₆) ou poly-alcoxy(C₁-C₆)-alkyle(C₂-C₆) chacun éventuellement une à trois fois substitué par fluoro ou chloro,
représente un groupe cycloalkyle en C₃-C₇ éventuellement une à trois fois substitué par fluoro, chloro, alkyle en C₁-C₄ ou alcoxy en C₁-C₄ ou
représente un groupe phényle ou benzyle chacun éventuellement une à trois fois substitué par fluoro, chloro, bromo, cyano, nitro, alkyle en C₁-C₄, alcoxy en C₁-C₃, halogénoalkyle(C₁-C₃) ou halogénoalcoxy (C₁-C₃) .

4. Composés de formule (I) selon la revendication 1, dans lesquels
W représente un atome d'hydrogène, de chlore, brome, le groupe méthyle, éthyle, propyle, vinyle, éthynyle, propynyle, cyclopropyle, méthoxy, éthoxy ou trifluorométhyle,
X représente un atome de chlore, de brome, le groupe méthyle, éthyle, propyle, isopropyle, vinyle, éthynyle, propynyle, cyclopropyle, méthoxy, éthoxy, trifluorométhyle, difluorométhoxy, trifluorométhoxy ou cyano,
Y et Z représentent indépendamment l'un de l'autre un atome d'hydrogène, de fluor, chlore, brome, d'iode, le groupe méthyle, éthyle, vinyle, éthynyle, propynyle, cyclopropyle, méthoxy, trifluorométhyle, trifluorométhoxy, cyano ou un radical phényle, 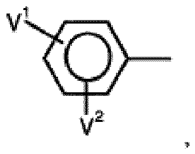 dans le cas d'un radical phényle, un seul des radicaux
Y ou Z pouvant représenter le radical phényle,
V¹ représente un atome d'hydrogène, de fluor ou de chlore,
V² représente un atome d'hydrogène, de fluor, chlore, le groupe méthyle, éthyle, n-propyle, isopropyle, méthoxy, éthoxy ou trifluorométhyle,
A représente un atome d'hydrogène, un groupe alkyle en C₁-C₄ ou alcoxy(C₁-C₂)alkyle(C₁-C₂), chacun éventuellement une à trois fois substitué par fluoro, le groupe cyclopropyle, cyclopentyle ou cyclohexyle,
B représente un atome d'hydrogène, le groupe méthyle ou éthyle,
étant entendu que A et B ne peuvent représenter chacun que le groupe méthyle lorsque, dans le cas où W, X et Y représentent chacun le groupe méthyle ou éthyle et Z représente un atome d'hydrogène, W et X représentent chacun le groupe méthyle ou W et X représentent chacun le groupe éthyle,
ou
A et B représentent ensemble, avec l'atome de carbone auquel ils sont liés, un groupe cycloalkyle en C₅-C₆ saturé, dans lequel éventuellement un chaînon du cycle est remplacé par un atome d'azote, d'oxygène ou de soufre et qui est éventuellement une ou deux fois substitué par méthyle, éthyle, méthoxyméthyle, éthoxyméthyle, méthoxyéthyle, éthoxyéthyle, trifluorométhyle, méthoxy, éthoxy, propoxy, butoxy, méthoxyéthoxy, éthoxyéthoxy, allyloxy, trifluoroéthoxy ou cyclopropylméthoxy, les radicaux nommés précédemment (hormis trifluorométhyle) étant également pris en considération en tant que substituants sur N, ou
A et B représentent ensemble, avec l'atome de carbone auquel ils sont liés, un groupe cycloalkyle en C₆ qui est éventuellement substitué par un groupe alkylidènediyle éventuellement interrompu par un atome d'oxygène ou par un groupe alkylènedioxy contenant deux atomes d'oxygène non directement voisins, avec formation d'un autre cycle pentagonal ou hexagonal (qui peut éventuellement être une ou deux fois substitué par méthyle), ou
A et B représentent ensemble, avec l'atome de carbone auquel ils sont liés, un groupe cycloalkyle en C₅-C₆ ou cycloalcényle en C₅-C₆, dans lequel deux substituants représentent ensemble, avec les atomes de carbone auxquels ils sont liés, un groupe alcane (C₂-C₄)diyle ou alcène (C₂-C₄)diyle ou butadiènediyle,
G représente l'un des groupes 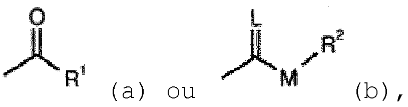 où
L représente un atome d'oxygène ou de soufre,
M représente un atome d'oxygène ou de soufre,
R¹ représente un groupe alkyle en C₁-C₁₀, alcényle en C₂-C₁₀, alcoxy(C₁-C₄)-alkyle (C₁-C₂), alkyl(C₁-C₄)-thio-alkyle(C₁-C₂), chacun éventuellement une à trois fois substitué par fluoro ou chloro, ou représente un groupe cycloalkyle en C₃-C₆ éventuellement une fois substitué par fluoro, chloro, méthyle, éthyle ou méthoxy,
représente un groupe phényle éventuellement une ou deux fois substitué par fluoro, chloro, bromo, cyano, nitro, méthyle, éthyle, n-propyle, isopropyle, méthoxy, éthoxy, trifluorométhyle ou trifluorométhoxy,
représente un groupe furanyle, thiényle ou pyridyle, chacun éventuellement une fois substitué par chloro, bromo ou méthyle,
R² représente un groupe alkyle en C₁-C₁₀, alcényle en C₂-C₁₀ ou alcoxy(C₁-C₄)-alkyle(C₂-C₄), chacun éventuellement une à trois fois substitué par fluoro ou chloro,
représente le groupe cyclopentyle ou cyclohexyle
ou représente un groupe phényle ou benzyle chacun éventuellement une ou deux fois substitué par fluoro, chloro, cyano, nitro, méthyle, éthyle, méthoxy, trifluorométhyle ou trifluorométhoxy.

5. Composés de formule (I) selon la revendication 1, dans lesquels
W représente le groupe méthyle, éthyle ou propyle,
X représente le groupe méthyle, éthyle, un atome de chlore, de brome ou le groupe méthoxy,
Y représente le groupe méthyle, un atome de chlore, d'iode ou de brome,
Z représente un atome d'hydrogène,
A représente le groupe méthyle,
B représente le groupe méthyle,
étant entendu que A et B ne peuvent représenter chacun que le groupe méthyle lorsque, dans le cas où W, X et Y représentent chacun le groupe méthyle ou éthyle et Z représente un atome d'hydrogène, W et X représentent chacun le groupe méthyle ou W et X représentent chacun le groupe éthyle, ou
A, B représentent ensemble, avec l'atome de carbone auquel ils sont liés, un groupe cycloalkyle en C₆ qui est éventuellement substitué par méthyle, méthoxy ou méthoxyméthyle, le substituant pouvant se trouver en la position 4 du cycle,
G représente l'un des groupes 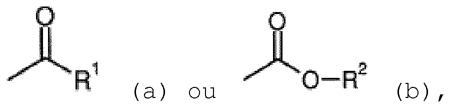 où
R¹ représente le groupe méthyle, éthyle, propyle, isopropyle, butyle, isobutyle, sec-butyle ou tert-butyle,
R² représente le groupe méthyle, éthyle, propyle, isopropyle, butyle, isobutyle, sec-butyle ou tert-butyle.

6. Composés de formule (I) selon la revendication 1, dans lesquels
W représente un atome d'hydrogène,
X représente le groupe méthyle,
Y représente un atome d'hydrogène,
Z représente le groupe méthyle,
A, B représentent ensemble, avec l'atome de carbone auquel ils sont liés, un groupe cycloalkyle en C₆ qui est éventuellement substitué par méthoxy ou méthoxyméthyle, le substituant pouvant se trouver aussi bien en la position 3 qu'en la position 4 du cycle,
G représente l'un des groupes 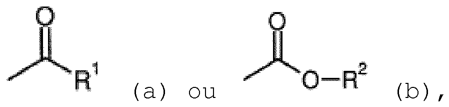 où
R¹ représente le groupe méthyle, éthyle, propyle, isopropyle, butyle, isobutyle, sec-butyle ou tert-butyle,
Ruz représente le groupe méthyle, éthyle, propyle, isopropyle, butyle, isobutyle, sec-butyle ou tert-butyle.

7. Procédé pour la préparation de composés de formule (I) selon la revendication 1, **caractérisé en ce que** pour l'obtention
(A) de composés de formule (I-a) indiquée plus haut, dans laquelle R¹, A, B, W, X, Y et Z ont les significations indiquées plus haut, on fait réagir des composés de formule (II) dans laquelle A, B, W, X, Y et Z ont les significations indiquées plus haut, 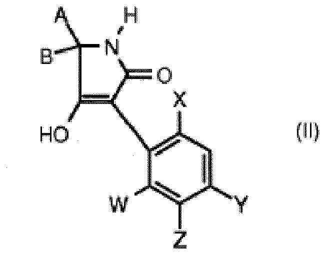 dans laquelle
A, B, W, X, Y et Z ont les significations indiquées plus haut,
α) avec des composés de formule (III) 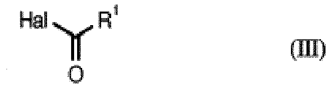 dans laquelle
R¹ a la signification indiquée plus haut et
Hal représente un atome d'halogène
ou
β) avec des anhydrides d'acides carboxyliques de formule (IV)
**R¹-CO-O-CO-R¹** (IV)
dans laquelle
R¹ a la signification indiquée plus haut,
éventuellement en présence d'un diluant et éventuellement en présence d'un capteur d'acide ;
(B) de composés de formule (I-b) indiquée plus haut, dans laquelle R², A, B, M, W, X, Y et Z ont les significations indiquées plus haut et L représente un atome d'oxygène, on fait réagir des composés de formule (II) présentée plus haut, dans laquelle A, B, W, X, Y et Z ont les significations indiquées plus haut,
avec des esters d'acide chloroformique ou des thioesters d'acide chloroformique de formule (V)
R²-M-CO-Cl (V)
dans laquelle
R² et M ont les significations indiquées plus haut,
éventuellement en présence d'un diluant et éventuellement en présence d'un capteur d'acide.

8. Pesticides et/ou herbicides, **caractérisés par** une teneur en au moins un composé de formule (I) selon la revendication 1.

9. Procédé pour la lutte contre des ravageurs animaux et/ou une végétation indésirable, **caractérisé en ce qu'**on fait agir sur les ravageurs et/ou leur habitat des composés de formule (I) selon la revendication 1, à l'exclusion des procédés destinés au traitement chirurgical et thérapeutique de l'organisme humain/animal.

10. Utilisation de composés de formule (I) selon la revendication 1, pour la lutte contre des ravageurs animaux et/ou une végétation indésirable, à l'exclusion de l'utilisation dans des procédés destinés au traitement chirurgical et thérapeutique de l'organisme humain/animal.

11. Procédé pour la fabrication de pesticides et/ou d'herbicides, **caractérisé en ce qu'**on mélange des composés de formule (I) selon la revendication 1 avec des excipients et/ou des substances tensioactives.

12. Utilisation de composés de formule (I) selon la revendication 1, pour la fabrication de pesticides et/ou d'herbicides.

13. Composition contenant une quantité efficace d'une association de substances actives comprenant les composants
a') au moins un composé de formule (I) selon la revendication 1, dans laquelle A, B, W, X, Y et Z ont la signification indiquée plus haut et
b') au moins un composé améliorant la tolérance par les plantes cultivées, choisi dans le groupe suivant de composés : S1, S2, S3, S4, S5, S6, S7, S8, S9, S10, 511, S12, S13, S14, S15, S16 :
S1) composés de formule (S1), 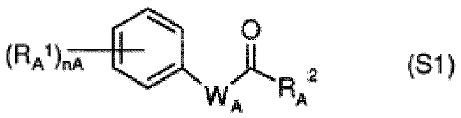 dans laquelle les symboles et indices ont les significations suivantes :
n_{A} est un nombre naturel valant de 0 à 5 ;
R_{A}¹ est un atome d'halogène, un groupe alkyle en C₁-C₄, alcoxy en C₁-C₄, nitro ou halogénoalkyle(C₁-C₄) ;
W_{A} est un radical hétérocyclique divalent substitué ou non substitué choisi dans le groupe des hétérocycles à cycle pentagonal partiellement insaturés ou aromatiques comportant 1 à 3 hétéroatomes dans le cycle, choisis dans le groupe constitué par N et O, au moins un atome d'azote et au maximum un atome d'oxygène étant contenus dans le cycle,
R_{A}² est OR_{A}³, SR_{A}³ ou NR_{A}³R_{A}⁴ ou un hétérocycle saturé ou insaturé à 3 à 7 chaînons, comportant au moins un atome d'azote et jusqu'à 3 hétéroatomes, de préférence choisis dans le groupe constitué par O et S, qui est lié par l'atome d'azote au groupe carbonyle dans (S1) et est non substitué ou substitué par des radicaux choisis dans le groupe constitué par alkyle en C₁-C₄, alcoxy en C₁-C₄ ou phényle éventuellement substitué, de préférence un radical de formule OR_{A}³, NHR_{A}³ ou N(CH₃)₂, en particulier de formue OR_{A}³ ;
R_{A}³ est un atome d'hydrogène ou un radical hydrocarboné aliphatique substitué ou non substitué, de préférence ayant au total 1 à 18 atomes de carbone ;
R_{A}⁴ est un atome d'hydrogène, un groupe alkyle en C₁-C₆, alcoxy en C₁-C₆ ou phényle substitué ou non substitué ;
S2) dérivés de quinoléine de formule (S2), 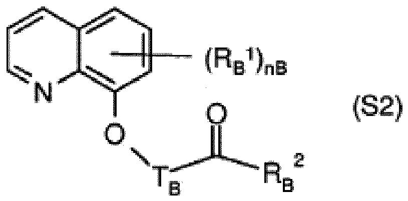 dans laquelle les symboles et indices ont les significations suivantes :
R_{B}¹ est un atome d'halogène, un groupe alkyle en C₁-C₄, alcoxy en C₁-C₄, nitro ou halogénoalkyle(C₁-C₄) ;
n_{B} est un nombre naturel valant de 0 à 5 ;
R_{B}² est OR_{B}³, SR_{B}³ ou NR_{B}³R_{B}⁴ ou un hétérocycle saturé ou insaturé à 3 à 7 chaînons, comportant au moins un atome d'azote et jusqu'à 3 hétéroatomes, qui est lié par l'atome d'azote au groupe carbonyle dans (S2) et est non substitué ou substitué par des radicaux choisis dans le groupe constitué par alkyle en C₁-C₄, alcoxy en C₁-C₄ ou phényle éventuellement substitué ;
R_{B}³ est un atome d'hydrogène ou un radical hydrocarboné aliphatique substitué ou non substitué ;
R_{B}⁴ est un atome d'hydrogène, un groupe alkyle en C₁-C₆, alcoxy en C₁-C₆ ou un groupe phényle substitué ou non substitué ;
T_{B} est une chaîne alcane(C₁ ou C₂)diyle, qui est non substituée ou substituée par un ou deux radicaux alkyle en C₁-C₄ ou par [alcoxy(C₁-C₃)]-carbonyle.
S3) Composés de formule (S3) 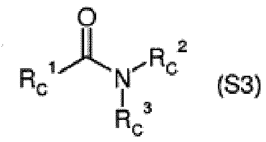 dans laquelle les symboles et indices ont les significations suivantes :
R_{c}¹ est un groupe alkyle en C₁-C₄, halogéno-alkyle(C₁-C₄), alcényle en C₂-C₄, halogéno-alcényle(C₂-C₄), cycloalkyle en C₃-C₇ ;
R_{c}², R_{c}³ sont identiques ou différents et représentent un atome d'hydrogène, un groupe alkyle en C₁-C₄, alcényle en C₂-C₄, alcynyle en C₂-C₄, halogéno-alkyle(C₁-C₄), halogénoalcényle(C₂-C₄), alkyl(C₁-C₄)carbamoyl-alkyle(C₁-C₄), alcényl(C₂C₄)-carbamoyl-alkyle(C₁-C₄), alcoxy(C₁-C₄)-alkyle(C₁-C₄), dioxolanyl-alkyle(C₁-C₄), thiazolyle, furyle, furylalkyle, thiényle, pipéridyle, phényle substitué ou non substitué, ou R_{c}² et R_{c}³ forment ensemble un cycle hétérocyclique substitué ou non substitué.
S4) N-acylsulfonamides de formule (S4) et leurs sels, 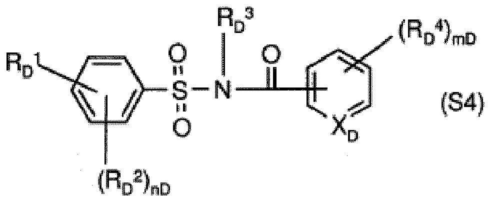 dans laquelle les symboles et indices ont les significations suivantes :
X_{D} est CH ou N ;
R_{D}¹ est CO-NR_{D}⁵R_{D}⁶ ou NHCO-R_{d}⁷ ;
R_{D}² est un atome d'halogène, un groupe halogéno-alkyle(C₁-C₄), halogénoalcoxy(C₁-C₄), nitro, alkyle en C₁-C₄, alcoxy en C₁-C₄, alkyl (C₁-C₄) sulfonyle, alcoxy(C₁-C₄)carbonyle ou alkyl(C₁-C₄)carbonyle ;
R_{D}³ est un atome d'hydrogène, un groupe alkyle en C₁-C₄, alcényle en C₂-C₄ ou alcynyle en C₂-C₄ ;
R_{d}⁴ est un atome d'hydrogène, un groupe nitro, alkyle en C₁-C₄, halogénoalkyle(C₁-C₄), halogéno-alcoxy(C₁-C₄), cycloalkyle en C₃-C₆, phényle, alcoxy en C₁-C₄, cyano, alkyl(C₁-C₄)thio, alkyl(C₁-C₄)sulfinyle, alkyl(C₁-C₄)sulfonyle, alcoxy(C₁-C₄)carbonyle ou alkyl(C₁-C₄)carbonyle ;
R_{d}⁵ est un atome d'hydrogène, un groupe alkyle en C₁-C₆, cycloalkyle en C₃-C₆, alcényle en C₂-C₆, alcynyle en C₂-C₆, cycloalcényle en C₅-C₆, phényle ou un groupe hétérocyclyle à 3 à 6 chaînons contenant V_{D} hétéroatomes choisis dans le groupe constitué par les atomes d'azote, d'oxygène et de soufre, les sept radicaux nommés en dernier étant substitués par V_{D} substituants choisis dans le groupe constitué par halogéno, alcoxy en C₁-C₆, halogénoalcoxy(C₁-C₆), alkyl(C₁-C₂)sulfinyle, alkyl(C₁-C₂)sulfonyle, cycloalkyle en C₃-C₆, alcoxy(C₁-C₄)carbonyle, alkyl(C₁-C₄)carbonyle et phényle et dans le cas de radicaux cycliques également alkyle en C₁-C₄ et halogéno-alkyle(C₁-C₄) ;
R_{D}⁶ est un atome d'hydrogène, un groupe alkyle en C₁-C₆, alcényle en C₂-C₆ ou alcynyle en C₂-C₆, les trois radicaux nommés en dernier étant substitués par V_{D} radicaux choisis dans le groupe constitué par halogéno, hydroxy, alkyle en C₁-C₄, alcoxy en C₁-C₄ et alkyl(C₁-C₄)thio, ou
R_{D}⁵ et R_{D}⁶ forment ensemble avec l'atome d'azote qui les porte un radical pyrrolidinyle ou pipéridinyle ;
R_{D}⁷ est un atome d'hydrogène, un groupe alkyl(C₁-C₄)amino, dialkyl(C₁-C₄)amino, alkyle en C₁-C₆, cycloalkyle en C₃-C₆, les 2 radicaux nommés en dernier étant substitués par V_{D} substituants choisis dans le groupe constitué par halogéno, alcoxy en C₁-C₄, halogénoalcoxy(C₁-C₆) et alkyl(C₁-C₄)thio et dans le cas de radicaux cycliques également alkyle en C₁-C₄ et halogénoalkyle(C₁-C₄) ;
n_{D} est 0, 1 ou 2 ;
m_{D} est 1 ou 2 ;
V_{D} est 0, 1, 2 ou 3.
S5) Substances actives choisies dans la classe des composés hydroxyaromatiques et des dérivés d'acides carboxyliques aromatiques-aliphatiques (S5), par exemple
3,4,5-triacétoxybenzoate d'éthyle, acide 3,5-diméthoxy-4-hydroxybenzoïque, acide 3,5-dihydroxybenzoïque, acide 4-hydroxysalicylique, acide 4-fluorosalicylique, acide 2-hydroxy-cinnamique, 1,2-dihydro-2-oxo-6-trifluorométhyl-pyridine-3-carboxamide, acide 2,4-dichloro-cinnamique, tels qu'ils sont décrits dans les documents WO-A-2004/084631, WO-A-2005/015994, WO-A-2005/016001.
S6) Substances actives choisies dans la classe des 1,2-dihydroquinoxalin-2-ones (S6), par exemple 1-méthyl-3-(2-thiényl)-1,2-dihydroquinoxalin-2-one, 1-méthyl-3-(2-thiényl)-1,2-dihydro-quinoxaline-2-thione, 1-(2-aminoéthyl)-3-(2-thiényl)-1,2-dihydro-quinoxalin-2-one-chlorhydrate, 1-[2-(diéthylamino)éthyl]-6,7-diméthyl-3-thiophén-2-ylquinoxalin-2(1H)-one, 1-(2-méthylsulfonylaminoéthyl)-3-(2-thiényl)-1,2-dihydro-quinoxalin-2-one, tels qu'elles sont décrites dans le document WO-A-2005/112630.
S7) Composés de formule (S7), tels qu'ils sont décrits dans le document WO-A-1998/38856 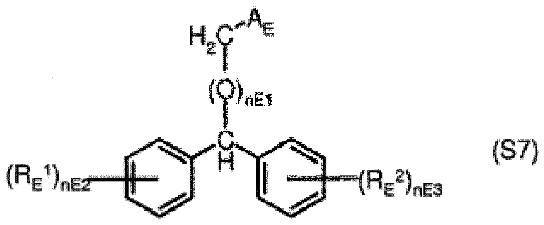 dans laquelle les symboles et indices ont les significations suivantes :
R_{E}¹, R_{E}² représentent indépendamment l'un de l'autre un atome d'halogène, un groupe alkyle en C₁-C₄, alcoxy en C₁-C₄, halogénoalkyle(C₁-C₄), alkyl(C₁-C₄)amino, dialkyl(C₁-C₄)amino, nitro ;
A_{E} est COOR_{E}³ ou COSR_{E}⁴
R_{E}³, R_{E}⁴ représentent indépendamment l'un de l'autre un atome d'hydrogène, un groupe alkyle en C₁-C₄, alcényle en C₂-C₆, alcynyle en C₂-C₄, cyanoalkyle, halogénoalkyle(C₁-C₄), phényle, nitrophényle, benzyle, halogénobenzyle, pyridinylalkyle et alkylammonium,
n_{E}¹ est 0 ou 1
n_{E}², n_{E}³ valent indépendamment l'un de l'autre 0, 1 ou 2.
S8) Composés de formule (S8), tels qu'ils sont décrits dans le document WO-A-98/27049 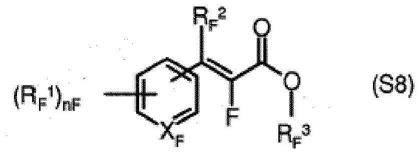 dans laquelle
X_{F} représente CH ou N,
n_{F} représente, dans le cas où X_{F} = N, un nombre entier valant de 0 à 4 et
dans le cas où X_{F} = CH, un nombre entier valant de 0 à 5,
R_{F}¹ représente un atome d'halogène, un groupe alkyle en C₁-C₄, halogénoalkyle(C₁-C₄), alcoxy en C₁-C₄, halogénoalcoxy(C₁-C₄), nitro, alkyl(C₁-C₄)thio, alkyl(C₁-C₄)sulfonyle, alcoxy (C₁-C₄)carbonyle, phényle éventuellement substitué, phénoxy éventuellement substitué,
R_{F}² représente un atome d'hydrogène ou un groupe alkyle en C₁-C₄
R_{F}³ représente un atome d'hydrogène, un groupe alkyle en C₁-C₈, alcényle en C₂-C₄, alcynyle en C₂-C₄, ou aryle, chacun des radicaux carboné précités étant non substitué ou substitué par un ou plusieurs radicaux identiques ou différents, choisis dans le groupe constitué par halogéno et alcoxy, ou leurs sels.
S9) Substances actives choisies dans la classe des 3-(5-tétrazolylcarbonyl)-2-quinolones (S9), par exemple 1,2-dihydro-4-hydroxy-1-éthyl-3-(5-tétrazolylcarbonyl)-2-quinolone (n° d'enregistrement CAS 219479-18-2), 1,2-dihydro-4-hydroxy-1-méthyl-3-(5-tétrazolyl-carbonyl)-2-quinolone (n° d'enregistrement CAS 95855-00-8), tels qu'elles sont décrites dans le document WO-A-1999/000020.
S10) Composés de formules (S10^{a}) ou (S10^{b}), tels qu'ils sont décrits dans les documents WO-A-2007/023719 et WO-A-2007/023764 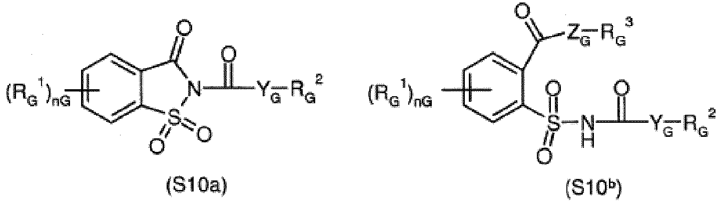 dans lesquelles
R_{G}¹ représente un atome d'halogène, un groupe alkyle en C₁-C₄, méthoxy, nitro, cyano, CF₃, OCF₃
Y_{G}, Z_{G} représentent indépendamment l'un de l'autre O ou S,
n_{G} représente un nombre entier valant de 0 à 4,
R_{G}² représente un groupe alkyle en C₁-C₁₆, alcényle en C₂-C₆, cycloalkyle en C₃-C₆, aryle ; benzyle, halogénobenzyle,
R_{G}³ représente un atome d'hydrogène ou un groupe alkyle en C₁-C₆.
S11) Substances actives du type des composés oxyimino (S11), qui sont connues comme agents de traitement de semences, comme par exemple « oxabétrinile » ((Z)-1,3-dioxolan-ylméthoxyimino(phényl)acéto-nitrile) (S11-1), qui est connu en tant qu'agent de traitement de semences-phytoprotecteur pour le millet contre les dommages du métolachlore,
« fluxofénim » (1-(4-chlorophényl)-2,2,2-trifluoro1-éthanone-O-(1,3-dioxolan-2-ylméthyl)-oxime) (S11-2), qui est connu en tant qu'agent de traitement de semences-phytoprotecteur pour le millet contre les dommages du métolachlore, et
« cyométrinil » ou «CGA-43089» ((Z)-cyano-méthoxyimino(phényl)acétonitrile) (S11-3), qui est connu en tant qu'agent de traitement de semences-phytoprotecteur pour le millet contre les dommages du métolachlore.
S12) Substances actives de la classe des isothiochromanones (S12), comme par exemple [(3-oxo-1H-2-benzothiopyran-4(3H)-ylidène)méthoxy]-acétate de méthyle (n° d'enregistrement CAS 205121-04-6) (S12-1) et composés apparentés de WO-A-1998/13361.
S13) Un ou plusieurs composés du groupe (S13) :
« anhydride naphtalique » (anhydride d'acide 1,8-naphtalènedicarboxylique) (S13-1), qui est connu en tant qu'agent de traitement de semences-phytoprotecteur pour le maïs contre les dommages d'herbicides thiocarbamate,
« fenclorim » (4,6-dichloro-2-phénylpyrimidine) (S13-2), qui est connu comme phytoprotecteur pour le prétilachlore dans le riz en semis,
« flurazole » (2-chloro-4-trifluorométhyl-1,3-thiazole-5-carboxylate de benzyle) (S13-3), qui est connu comme agent de traitement de semences-phytoprotecteur pour le millet contre les dommages de l'alachlore et du métolachlore,
« CL 304415 » (n° d'enregistrement CAS 31541-57-8) (acide 4-carboxy-3,4-dihydro-2H-1-benzopyran-4-acétique) (S13-4) de la société American Cyanamid, qui est connus comme phytoprotecteur contre les dommages d'imidazolinones,
« MG 191 » (n° d'enregistrement CAS 96420-72-3) (2-dichlorométhyl-2-méthyl-1,3-dioxolane) (S13-5) de la société Nitrokemia, qui est connu comme phytoprotecteur pour le maïs,
« MG-838 » (n° d'enregistrement CAS 133993-74-5) (2-propényl 1-oxa-4-azaspiro[4.5]décane-4-carbodithioate) (S13-6) de la société Nitrokemia,
« disulfoton » (0,0-diéthyl S-2-éthylthioéthyl phosphorodithioate) (S 13-7),
« diétholate » (0,0-diéthyl-0-phénylphosphoro-thioate) (S13-8),
« méphénate » (méthylcarbamate de 4-chlorophényle) (S13-9) .
S14) Substances actives qui, outre un effet herbicide contre des plantes nuisibles, présentent également un effet phytoprotecteur sur des plantes cultivées telles que le riz, comme par exemple « dimépipérate » ou « MY-93 » (*S*-1-méthyl-1-phényléthyl-pipéridin-1-carbothioate), qui est connu comme phytoprotecteur pour le riz contre les dommages de l'herbicide molinate,
« daimuron » ou « SK 23 » (1-(1-méthyl-1-phényléthyl)-3-p-tolyl-urée), qui est connu comme phytoprotecteur pour le riz contre les dommages de l'herbicide imazosulfuron,
« cumyluron » = « JC-940 » (3-(2-chlorophényl-méthyl)-1-(1-méthyl-1-phényléthyl)urée, voir JP-A-60087254), qui est connu comme phytoprotecteur pour le riz contre les dommages de certains herbicides,
« méthoxyphénone » ou « NK 049 » (3,3'-diméthyl-4-méthoxy-benzophénone), qui est connue comme phytoprotecteur pour le riz contre les dommages de certains herbicides,
« CSB » (1-bromo-4-(chlorométhylsulfonyl)benzène) de Kumiai, (n° d'enregistrement CAS 54091-06-4), qui est connu comme phytoprotecteur chez le riz contre les dommages de certains herbicides,
S15) Composés de formule (S15) ou leurs tautomères 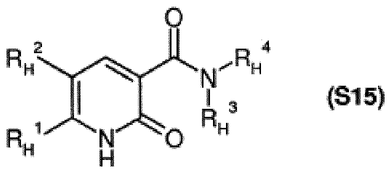 tels qu'ils sont définis dans les documents WO-A-2008/131861 et WO-A-2008/131860
formule dans laquelle
R_{H}¹ représente un radical halogénoalkyle(C₁-C₆) et
R_{H}² représente un atome d'hydrogène ou d'halogène et
R_{H}³, R_{H}⁴ représentent indépendamment l'un de l'autre un atome d'hydrogène, un groupe alkyle en C₁-C₁₆, alcényle en C₂-C₁₆ ou alcynyle en C₂-C₁₆ ,
chacun des 3 radicaux nommés en dernier étant non substitué ou substitué par un ou plusieurs radicaux choisis dans le groupe constitué par halogéno, hydroxy, cyano, alcoxy en C₁-C₄, halogénoalcoxy(C₁-C₄), alkyl(C₁-C₄)thio, alkyl(C₁-C₄)amino, di[alkyl(C₁-C₄)]-amino, [alcoxy(C₁-C₄)]-carbonyle, [halogéno-alcoxy(C₁-C₄)]-carbonyle, cycloalkyle en C₃-C₆ qui est substitué ou non substitué, phényle qui est substitué ou non substitué, et hétérocyclyle qui est substitué ou non substitué,
ou cycloalkyle en C₃-C₆, cycloalcényle en C₄-C₆, cycloalkyle en C₃-C₆ qui est condensé sur un côté du cycle avec un cycle carbocyclique saturé ou insaturé à 4 à 6 chaînons, ou cycloalcényle en C₄-C₆ qui est condensé sur un côté du cycle avec un cycle carbocyclique saturé ou insaturé à 4 à 6 chaînons,
chacun des 4 radicaux nommés en dernier étant non substitué ou substitué par un ou plusieurs radicaux choisis dans le groupe constitué par halogéno, hydroxy, cyano, alkyle en C₁-C₄, halogénoalkyle(C₁-C₄), alcoxy en C₁-C₄, halogénoalcoxy (C₁-C₄), alkyl (C₁-C₄) thio, alkyl(C₁-C₄)amino, di[alkyl(C₁-C₄)]amino, [alcoxy(C₁-C₄)]-carbonyle, [halogénoalcoxy(C₁-C₄)]-carbonyle, cycloalkyle en C₃-C₆ qui est substitué ou non substitué, phényle qui est substitué ou non substitué, et hétérocyclyle qui est substitué ou non substitué,
ou
R_{H}³ représente un groupe alcoxy en C₁-C₄, alcényloxy(C₂-C₄), alcynyloxy(C₂-C₆) ou halogénoalcoxy(C₂-C₄) et
R_{H}⁴ représente un atome d'hydrogène ou un groupe alkyle en C₁-C₄ ou
R_{H}³ et R_{H}⁴ forment ensemble avec l'atome d'azote directement lié un cycle hétérocyclique à quatre à huit chaînons, qui outre l'atome d'azote peut également contenir d'autres hétéroatomes dans le cycle et qui est non substitué ou substitué par un ou plusieurs radicaux choisis dans le groupe constitué par halogéno, cyano, nitro, alkyle en C₁-C₄, halogénoalkyle(C₁-C₄), alcoxy en C₁-C₄, halogénoalcoxy(C₁-C₄) et alkyl(C₁-C₄)thio.
S16) Substances actives qui sont en premier lieu utilisées comme herbicides, mais peuvent également présenter un effet phytoprotecteur sur des plantes cultivées, par exemple
acide (2,4-dichlorophénoxy)acétique (2,4-D),
acide (4-chlorophénoxy)acétique,
acide (R,S)-2-(4-chloro-o-tolyloxy)propionique (mécoprop),
acide 4-(2,4-dichlorophénoxy)butyrique (2,4-DB), acide (4-chloro-o-tolyloxy)acétique (MCPA),
acide 4-(4-chloro-o-tolyloxy)butyrique,
acide 4-(4-chlorophénoxy)butyrique,
acide 3,6-dichloro-2-méthoxybenzoïque (dicamba), 3,6-dichloro-2-méthoxybenzoate de 1-(éthoxy-carbonyl)éthyle (lactidichlore-éthyle).

14. Procédé pour la lutte contre une végétation indésirable, **caractérisé en ce qu'**on fait agir sur les plantes ou leur environnement une composition selon la revendication 13.

15. Utilisation d'une composition selon la revendication 13 pour la lutte contre une végétation indésirable.

16. Procédé pour la lutte contre une végétation indésirable, **caractérisé en ce qu'**on fait agir sur les plantes ou leur environnement, séparément en un court intervalle de temps, un composé de formule (I) selon la revendication 1 et le composé selon la revendication 13, améliorant la tolérance par les plantes.
